# EUROPEAN PATENT SPECIFICATION

(11) **EP 1 200 395 B1**
(45) Date of publication and mention of the grant of the patent: **29.03.2006**
(21) Application number: 00950266.7
(22) Date of filing: 28.07.2000
(51) Int. Cl.: C07C 275/28, C07C 275/30, A61K 31/17

(54) **UREA DERIVATIVES AS INHIBITORS OF CCR-3 RECEPTOR**
Harnstoffderivate als CCR-3 Rezeptor-Inhibitoren
DERIVES D'UREE UTILISES COMME INHIBITEURS DU RECEPTEUR CCR-3

(30) Priority: 28.07.1999 US 146219 P; 22.03.2000 US 191094 P
(43) Date of publication of application: 02.05.2002
(73) Proprietor: KIRIN BEER KABUSHIKI KAISHA, Chuo-ku Tokyo 104-8283 (JP)
(72) Inventor: PADIA, Janak, Tucson, AZ 85718 (US); HOCKER, Michael, D., Tucson, AZ 85740 (US); OHASHI, Hiroshi, Takasaki-shi Gunma-ken 370-1295 (JP); NISHITOBA, Tsuyoshi, Takasaki-shi Gunma-ken 370-1295 (JP); SAWA, Eiji, Takasaki-shi Gunma-ken 370-1295 (JP)
(74) Representative: HOFFMANN EITLE
(86) International application number: PCT/US2000/017868
(87) International publication number: WO 2001/009088

(56) References cited:
- EP-A- 0 432 442
- EP-A- 0 903 349
- DE-C- 888 699
- US-A- 4 880 802
- PATENT ABSTRACTS OF JAPAN vol. 1998, no. 02, 30 January 1998 (1998-01-30) -& JP 09 278737 A (TANABE SEIYAKU CO LTD), 28 October 1997 (1997-10-28)
- KAZUYA NAKAO ET AL.: "Quantitative Structure-Activity Analyses of Novel Hydroxyphenylurea Derivatives as Antioxidants" BIOOGANIC & MEDICINAL CHEMISTRY, vol. 6, no. 6, 1998, pages 849-868, XP000961127
- PATRICK M. O'BRIEN ET AL.: "Inhibitors of Acyl-CoA: Cholesterol O-Acyl Transferase (ACAT) as Hypocholesterolemic Agents 8. Incorporation of Amide or Amine Functionalities into a Series of Disubstituted Ureas and Carbamates. Effects on ACAT Inhibition in Vitro and Efficacy in Vivo" JOURNAL OF MEDICINAL CHEMISTRY., vol. 37, 1994, pages 1810-1822, XP002153795 AMERICAN CHEMICAL SOCIETY. WASHINGTON., US ISSN: 0022-2623
- WILLIAM J. ROST ET AL.: "N-Aralkyl-N-methylaminoethyl Carbanilates as Hypocholesteremic Agents" JOURNAL OF PHARMACEUTICAL SCIENCES., vol. 56, no. 12, December 1967 (1967-12), pages 1598-1603, XP002153796 WASHINGTON US

## Description

### BACKGROUND OF THE INVENTION

The present invention relates to certain urea derivatives that are inhibitors of CCR-3 receptor activity, methods for preparing these compounds, pharmaceutical compositions containing such compounds and methods for their use.

Chemokines are chemotactic cytokines that are produced by a variety of cells to attract leukocytes to sites of inflammation or lymphoid tissue. CCR-3 is a chemokine receptor that is expressed in a variety of cells, including, but not limited to, eosinophils, basophils, T cells and dendritic cells. See Ponath, P.D. *et al., J. Exp. Med.* (1996) 183, 2437-2448; Yamada, H. *et al., Biochem. Biophys. Res. Comm.* (1997) 231, 365-368; Sallusto, F. *et al., Science* (1997) 277, 2005-2007; Sato, K. *et al., Blood* (1999) 93, 34-42. CCR-3 is also known as a co-receptor to HIV virus infection. See He, J. *et al., Nature* (1997) 385, 645-649. Several chemokines including eotaxin, eotaxin-2, RANTES, MCP-2, MCP-3, MCP-4 bind to CCR-3 and activate cell functions such as intracellular Ca²⁺ mobilization, chemotactic response, superoxide anion generation and cell aggregation. See Forssmann, U*. et al., J. Exp. Med.* (1997) 185, 2171-2176; Heath, H*. et al., J. Clin. Invest.* (1997) 99, 178-184; Uguccioni, M. *et al., J. Exp. Med.* (1996) 183, 2379-2384; Tenscher, K. *et al., Blood* (1996) 88, 3195-3199; Sato, K*. et al., Blood* (1999) 93 , 34-42. In particular, eotaxin exhibits a potent and specific chemotactic activity for eosinophils via binding to CCR-3, *in vitro* and *in vivo.* See Ponath, P. D. *et al., J. Clin. Invest.* (1996) 97, 604-612.

Tissue eosinophilia is observed in a number of pathological conditions such as asthma, rhinitis, eczema, inflammatory bowel diseases and parasitic infections. See Bousquest J. *et al., N. Eng. J. Med.* 323, 1033-1039; Middleton, Jr., E*. et al.,* Chapter 42, Allergy Principles and Practice 4^{th} ed. vol.2 Mosby-Year Book, Inc. 1993 U.S.A. In asthma, the airways of patients are infiltrated by a large numbers of eosinophils, and eotaxin production in bronchial mucosa and bronchoalveolar lavage (BALF) is increased. Several studies have suggested a strong correlation between the number of eosinophils in. BALF, the eotaxin level in BALF and the clinical parameters of disease severity. See Walker, C*. et al., J. Allergy Clin. Immunol.* (1991) 88, 935-942; Ying, S. *et al., Eur. J. Immunol.* (1997) 27, 3507-3516. Furthermore, pretreatment with a CCR-3-antibody has been shown to block chemotaxis and Ca²⁻ influx induced by eotaxin, RANTES, MCP-3 or MCP-4, suggesting that most of the eosinophilic response to these chemokines in allergic and eosinophilic patients is mediated through CCR-3. See Heath, H. *et al., J. Clin. Invest.* (1997) 99, 178-184. Similarly, it has recently been disclosed that certain cyclic amine derivatives are antagonistic to CCR-3 and may be useful for treating eosinophil-mediated allergic diseases. See EP 0903349A2. Also, CCR-3 expression on human Th2 type T-cells and human cultured dendritic cells mediates cell functions such as chemotactic response. See Sallusto, F*. et al., Science* (1997) 277, 2005-2007; Sato, K. *et al., Blood* (1999) 93, 34-42. In addition, anti-CCR-3 antibody has been shown to inhibit aggregation of T-cells and dendritic cells, suggesting CCR-3 may regulate the interaction of these cells during the process of antigen presentation. See Sato, K. *et al., Blood* (1999) 93 , 34-42. Therefore, CCR-3 inhibitors may also be useful for regulating immune responses.

These examples suggest that CCR-3 mediated diseases may be treated using compounds that inhibit CCR-3 activity. Because CCR-3 is present on many cell types, however, and is responsible for a variety of disease states, an arsenal of compounds which inhibit CCR-3 activity is required to treat CCR-3 mediated diseases effectively.

### SUMMARY OF THE INVENTION

It is therefore one object of the present invention to provide compounds which inhibit CCR-3 receptor activity.

It is another object of the present invention to provide a method of treating CCR-3 mediated diseases.

In accomplishing these and other objects of the invention, there is provided, in accordance with one aspect of the present invention, a compound having the following Formula (I) : 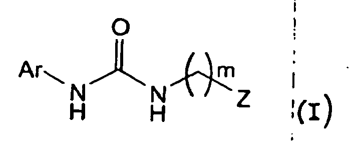 or a salt, hydrate, or complex thereof, wherein:
m is 3,4, 5 or 6;
Ar is aryl
   optionally substituted with one or more groups independently selected from hydroxy, halogen, trihalomethyl, trihalomethoxy, C₁₋₅alkyl, C₁₋₅ alkoxy, cyano, nitro, amino, carboxy, alkyloxycarbonyl, arylmethyloxycarbonyl, carbamoyl, alkylcarbamoyl, arylcarbamoyl, acyl, acyloxy, sulfonyl, alkylsulfonyl, arylsulfonyl, sulfamoyl, alkylsulfamoyl, arylsulfamoyl, alkylthio, alkylsulfonamide, arylsulfonamide, hydrazino, acylamino, alkylamino, hydroxyamino, amidino, guanidino, cyanoguanidino, aryl
   optionally substituted with one or more groups independently selected from hydroxy, halogen, trihalomethyl, C₁₋₅alkyl, C₁₋₅ alkoxy, cyano, nitro, amino and carboxy,
and aryloxy
   optionally substituted with one or more groups independently selected from hydroxy, halogen, trihalomethyl, C₁₋₅ alkyl, C₁₋₅ alkoxy, cyano, nitro, amino and carboxy,
Z is: 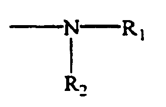 or 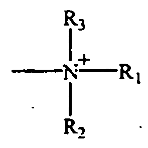
wherein R₁ is: 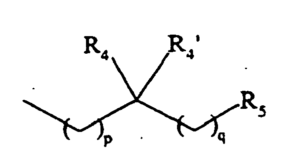 or 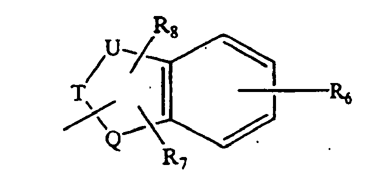 or 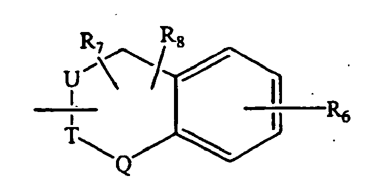 or 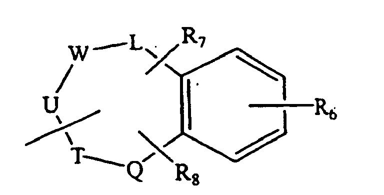 p is 0,1 or 2;
   q is 0, 1 or 2;
R₄ and R4' are independently selected from hydrogen, halogen, C₁₋₅ alkyl, aryl, heteroaryl
   wherein the aryl or heteroaryl is optionally substituted with one or more groups independently selected from hydrogen, hydroxy, halogen, trihalomethyl, C₁₋₅ alkyl, C₁₋₅alkoxy, cyano, nitro, amino, carboxy, carbamoyl, alkylcarbamoyl, arylcarbamoyl, acyl, acyloxy, sulfonyl, alkylsulfonyl, arylsulfonyl, sulfamoyl, alkylsulfamoyl, arylsulfamoyl, alkylthio, alkylsulfonamide, arylsulfonamide, hydrazino, acylamino, alkylamino, hydroxyamino, amidino, guanidino, and cyanoguanidino;
and COR₉; wherein R₉ is hydroxy, C₁₋₅alkyl, C₁₋₅alkoxy, amino, alkylamino or arylamino; R₅ is aryl or heteroaryl
   optionally substituted with one or more groups independently selected from hydroxy, halogen, trihalomethyl, C₁₋₅alkyl, C₁₋₅ alkoxy, cyano, nitro, amino, carboxy, carbamoyl, alkylcarbamoyl, arylcarbamoyl, acyl, acyloxy, sulfonyl, alkylsulfonyl, arylsulfonyl, sulfamoyl, alkylsulfamoyl, arylsulfamoyl, alkylthio, alkylsulfonamide, arylsulfonamide, hydrazino, acylamino, alkylamino, hydroxyamino, amidino, guanidino, cyanoguanidino, aryl
   optionally substituted with one or more groups independently selected from hydroxy, halogen, trihalomethyl, C₁₋₅ alkyl, C₁₋₅ alkoxy, cyano, nitro, amino and carboxy,
   and aryloxy
   optionally substituted with one or more groups independently selected from hydroxy, halogen, trihalomethyl, C₁₋₅ alkyl, C₁₋₅ alkoxy, cyano, nitro, amino and carboxy, R₆ is selected from hydrogen, hydroxy, halogen, trihalomethyl, C₁₋₅ alkyl, C₁₋₅ alkoxy, cyano, nitro, amino and carboxy,
R₇ and R₈ are independently selected hydrogen, hydroxy, halogen, trihalomethyl, C₁₋₅ alkyl, C₁₋₅ alkoxy, cyano, nitro, amino and carboxy Q, T, U, W and L independently selected from the group of atoms consisting of C, N, O and S; wherein adjacent atoms U-T, T-Q, U-W, W-L may from one or more double bonds;
R₂ is selected from C₁₋₈ alkyl, C₁₋₈ alkenyl and C₁₋₈ alkynyl
   substituted with one or more groups independently selected from carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, acyloxy, acylamino, aryl
   substituted with one or more groups independently selected from C₁₋₅ alkyl or C₁₋₅ alkoxy, wherein the alkyl or alkoxy is substituted with carboxy or alkyloxycarbonyl, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, aryloxy, arylmethyloxy, acylamino hydroxy, and halogen,
   heteroaryl
   substituted with one or more groups independently selected from C₁₋₅ alkyl or C₁₋₅ alkoxy which are substituted with carboxy or alkyloxycarbonyl, acylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, hydroxy, and halogen,
   C₁₋₅ alkoxy
   optionally substituted with one or more groups independently selected from C₁₋₅ alkyl or C₁₋₅ alkoxy which may be optionally substituted with carboxy or alkyloxycarbonyl, cyano, nitro, amino, acylamino, alkylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, alhylthio, acyl, acyloxy, hydrazino, hydroxyamino, amidino, guanidino, cyanoguanidino, hydroxy, and halogen,
   arylmethyloxy
   substituted with one or more groups independently selected from C₁₋₅ alkyl or C₁₋₅ alkoxy which are substituted with carboxy or alkyloxycarbonyl, cyano, nitro, amino, acylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, hydroxy, and halogen,
   and C₃₋₇ cycloalkyl
   substituted with one or more groups independently selected from C₁₋₅ alkyl or C₁₋₅ alkoxy which is substituted with carboxy or alkyloxycarbonyl, cyano, nitro, amino, acylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide and aryisulfonamide;

R₃ is selected from C₁₋₈ alkyl, C₁₋₈ alkenyl and C₁₋₈ alkynyl
optionally substituted with one or more groups independently selected from the group consisting of carboxy, carbamoyl, alkylcarbamoyl, arylcarbamoyl, alkylsulfonylcarbmnoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, alkylthio, halogen, acyloxy, hydroxy, nitro, amino, acylamino, alkylamino, cyano, aryl
optionally substituted with one or more groups independently selected from the group consisting of C₁₋₅ alkyl or C₁₋₅ alkoxy; wherein the alkyl or alkoxy may be optionally substituted with carboxy or alkyloxycarbonyl, cyano, nitro, amino, acylamino, alkylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkytsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, alkylthio, acyl, acyloxy, aryloxy, arylmethyloxy, hydrazino, hydroxyamino, amidino, guanidino, cyanoguanidino, hydroxy, and halogen,
heteroaryl optionally substituted with one or more groups independently selected from the group consisting of C₁₋₅ alkyl or C₁₋₅ alkoxy which may be optionally substituted with carboxy or alkyloxycarbonyl, cyano, nitro, amino, acylamino, alkylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, alkylthio, acyl, acyloxy, hydrazino, hydroxyamino, amidino, guanidino, cyanoguanidino, hydroxy, and halogen,
C₁₋₅ alkoxy optionally substituted with one or more groups independently selected from the group consisting of C₁₋₅ alkyl or C₁₋₅ alkoxy which may be optionally substituted with carboxy or alkyloxycarbonyl, cyano, nitro, amino, acylamino, alkylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, alkylthio, acyl, acyloxy, hydrazino, hydroxyamino, amidino, guanidino, cyanoguanidino, hydroxy, and halogen,
arylmethyloxy optionally substituted with one or more groups independently selected from the group consisting of C₁₋₅ alkyl or C₁₋₅ alkoxy which is optionally substituted with carboxy or alkyloxycarbonyl, cyano, nitro, amino, acylamino, alkylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, alkylthio, acyl, acyloxy, hydrazino, hydroxyamino, amidino, guanidino, cyanoguanidino, hydroxy, and halogen,
C₃₋₇ cycloalkyl optionally substituted with one or more groups independently selected from the group consisting of C₁₋₅ alkyl or C₁₋₅ alkoxy which is optionally substituted with carboxy or alkyloxycarbonyl, cyano, nitro, amino, acylamino, alkylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, alkylthio, acyl, acyloxy, hydrazino, hydroxyamino, amidino, guanidino, cyanoguanidino, hydroxy, and halogen, and heterocycle; provided that none of R₁, R₂, and R₃ bond together;
further provided that Ar is not 2-hydroxy-5-(lower)alkoxyphenyl, or when Ar is pyrene, chrysene or phenonthrene m≠3 and Z ≠ NR₁R₂R₃ with R₁=R₂=Me and R₃=B₇Cl; and further provided that when Ar is phenyl,
Z is 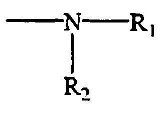 and R₂ is methyl, 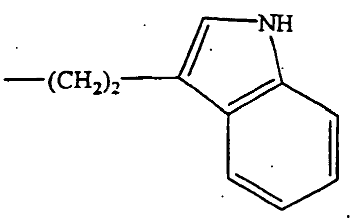 then R₁ is not

In another embodiment of the present invention, there is provided a pharmaceutical composition comprising one or more the disclosed compounds.

In yet another embodiment, there is provided the use of one or more of the inventive compounds of the present invention in the manufacture of a medicament for treating CCR-3 mediated diseases in a patient.

Other objects, features and advantages of the present invention will become apparent from the following detailed description. It should be understood that examples are given by way of illustration only.

### BRIEF DESCRIPTION OF THE DRAWINGS

Scheme 1 provides a schematic representation of the synthesis of N-Phenylcarbamoyl-N'-[2-(4-chlorophenyl)ethyl]-N'-ethyl-1,3-diaminopropane (Compound No. 1).
Scheme 2 provides a schematic representation of the synthesis of N-Phenylcarbamoyl-N'-[2-(4-chlorophenyl)ethyl]-N'-propyl-1,3-diaminopropane (Compound No. 10).
Scheme 3 depicts the synthesis of Methyl 4-[[3-(4-bromophenylureido)propyl](1,2,3,4-tetrahydro-1-naphthyl)amino]burylate (Compound No. 29).
Scheme 4 depicts the synthesis of 4-[[3-(4-Bromophenylureido)propyl](1,2,3,4-tetrahydro-1-naphthyl)amino]butanoic acid (Compound No.60).
Scheme 5 depicts the synthesis of [3-(Phenylureido)propyl][2-(4-chlorophenyl)ethyl]-diethylammonium iodide (Compound No.91).
Scheme 6 depicts the synthesis of Active Compounds by Solid Phase Synthesis.
Scheme 7 depicts the synthesis of 4-[[3-(4-Bromophenylureido)propyl](1,2,3,4-tetrahydro-1-naphthyl)amino]butanamide (Compound No. 193).
Scheme 8 depicts the synthesis of 3-[[3-(4-Bromophenylureido)propyl](1,2,3,4-tetrahydro-1-naphthyl)amino]-1-[(phenylsulfonyl)carbamoyl]propane (Compound No.196).
Scheme 9 depicts the synthesis of 4-[[3-(4-Bromophenylureido)propyl](1,2,3,4-tetrahydro-1-naphthyl)amino]-1-butanol (Compound No.203).
Scheme 10 depicts the synthesis of 3-[[3-(4-Bromophenylureido)propyl](1,2,3,4-tetrahydro-1-naphthyl)amino]-1-(1*H*-tetrazol-5-yl)propane (Compound No.218).
Scheme 11 the synthesis of Methyl 4-[[3-[4-(carboxy)phenylureido]propyl](1,2,3,4-tetrahydro-1-naphthyl)amino]butylate (Compound No:225).
Scheme 12 depicts the synthesis of 4-[[3-[4-(Ethoxycarbonyl)phenylureido]propyl](1,2,3,4-tetrahydro-1-naphthyl)amino]butanoic acid (Compound No.228).
Scheme 13 depicts the synthesis of [3-(Phenylureido)propyl]bis[2-(4-chlorophenyl)ethyl]amine (Compound No.238).
Scheme 14 depicts the synthesis of [3-(Phenylureido)propyl][2-(4-chlorophenyl)ethyl]bis(4-methylbenzyl)ammonium iodide (Compound No.296).
Scheme 5 depicts the synthesis of [3-(4-Bromophenylureido)propyl][(1S)-1-phenylethyl][3-(carboxy)propyl]ethylammonium trifluoroacetate (Compound No.315).
Scheme 16 depicts the synthesis of [3-(Phenylureido)propyl][2-(4-chlorophenyl)ethyl][4-(carboxy)benzyl]ethylammonium iodide (Compound No.322).
Figure 1A demonstrates the inhibitory effects of Compound No. 60 on collagen-induced arthritis.
Figure 1B demonstrates the inhibitory effects of Compound No. 298 on collagen-induced arthritis.
Figure 2A shows the dose-response curves of bronchoconstriction against acetylcholine (murine asthma model) with and without treatment of compound No. 298.
Figure 2B shows the area under each of the dose-response curves of Figure 2A.
Figure 2C shows the suppression of compound No. 298 (CPD No. 298) on eosinophil infiltration to bronchoalveolar lavage fluid (BALF). Two hundred cells were counted in each experiment:

### DETAILED DESCRIPTION OF THE PREFERRED EMBODIMENTS

The present invention provides a new class of compounds which inhibit CCR-3 receptor activity. Because the CCR-3 receptor is understood to mediate a variety of diseases, the disclosed compounds, which are derived from urea, are useful for treating CCR-3-mediated diseases. Examples of such diseases include eosinophil-mediated diseases such as asthma, rhinitis, eczema, inflammatory bowel diseases, parasitic infections, and diseases that are mediated by T-cells, mast cells (Ochi H. et al., *J*. *Exp. Med.* (1999) 190:267-280, Romagnani P. et. al. , *Am. J. Pathol.* (1999) 155:1195-1204) and/or dendritic cells, such as autoimmune and inflammatory diseases and HIV infection.

In one embodiment of the present invention, there is provided a variety of compounds that inhibit cell function mediated by the chemokine receptor CCR-3. These compounds are urea derivatives (Formula I) depicted below (wherein Ar, Z and m are as defined above) :

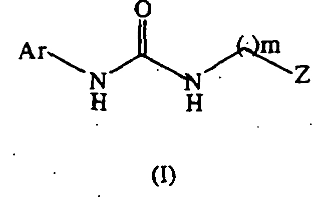

The compounds of Formula (I) as defined above, include variable groups such as an aryl group, a heteroaryl group and a heterocyclic group.

An aryl group is defined as a 6-15 membered aromatic carbocyclic moiety. This includes for instance phenyl, naphthyl, anthryl, indenyl or phenanthrenyl

A heteroaryl group is defined as a 5-15 membered aromatic ring system containing at least one hetero atom selected from the group consisting of N, O, and S. These include but are not limited to 2- or 3-thienyl, 2- or 3-furyl, 2- or 3- pyrrolyl, 2-, 3- or 4- pyridyl, 2-, 4- or 5-oxazolyl, 2-, 4- or 5- thiazolyl, 3-, 4- or 5- pyrazolyl, 2-, 4- or 5- imidazolyl, 3-, 4- or 5- isoxazolyl, 3-, 4- or 5- isothiazolyl, 3- or 5- (1,2,4-oxadiazolyl), 1,3,4-oxadiazolyl, 3- or 5- (1,2,4-thiadiazolyl), 1,3,4-thiadiazoyl, 4- or 5-(1,2,3-thiadiazolyl), 1,2,5-thiadiazolyl, 1,2,3-triazolyl, 1,2,4-triazolyl, 1H- or 2H-tetrazolyl, N-oxido- 2-, 3- or 4- pyridyl, 2-, 4- or 5-pyrimidinyl, N-oxido- 2-, 4- or 5- pyrimidinyl, 3- or 4-pyridazinyl, pyrazinyl, N-oxido-3- or 4-pyridazinyl, benzofuryl, indolyl, benzothiazolyl, benzoxazolyl, triazinyl, oxotriazinyl, tetrazolo[1,5-b]pyridazinyl, triazolo[4,5-b]pyridazinyl, oxoimidazinyl, dioxotriazinyl, pyrrolidinyl, pyranyl, thiopyranyl, 1,4-oxazinyl, 1,4-thiazinyl, 1,3-thiazinyl, benzimidazolyl, quinolyl, isoquinolyl, cinnolinyl, phthalazinyl, quinazolinyl, quinoxalinyl, indolizinyl, quinolizinyl, 1,8-naphthyridinyl, purinyl, pteridinyl, dibenzofuranyl, carbazolyl, acridinyl, phenanthridinyl, phenazinyl, phenothiazinyl and phenoxazinyl.

A heterocyclic group is defined as a 5-15 membered non-aromatic ring system containing at least one hetero atom selected from the group consisting of N, O, and S. These include but are not limited to hydrogenated derivatives of 2- or 3-thienyl, 2- or 3-furyl, 2- or 3- pyrrolyl, 2-, 3- or 4- pyridyl, 2-, 4- or 5-oxazolyl, 2-, 4- or 5- thiazolyl, 3-, 4- or 5- pyrazolyl, 2-, 4- or 5- imidazolyl, 3-, 4- or 5- isoxazolyl, 3-, 4- or 5- isothiazolyl, 3- or 5- (1,2,4-oxadiazolyl), 1,3,4-oxadiazolyl, 3- or 5- (1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl, 1H- or 2H-tetrazolyl, N-oxido- 2-, 3- or 4- pyridyl, 2-, 4- or 5-pyrimidinyl, N-oxido- 2-, 4-or 5- pyrimidinyl, 3- or 4-pyridazinyl, pyrazinyl, N-oxido-3- or 4-pyridazinyl, benzofuryl, indolyl, benzothiazolyl, benzoxazolyl, triazinyl, oxotriazinyl, tetrazolo [1,5-b]pyridazinyl, triazolo[4,5-b]pyridazinyl, oxoimidazinyl, dioxotriazinyl, pyrrolidinyl, pyranyl, thiopyranyl, 1,4-oxazinyl; 1,4-thiazinyl, 1,3-thiazinyl, benzimidazolyl, quinolyl, isoquinolyl, cinnolinyl, phthalazinyl, quinazolinyl, quinoxalinyl, indolizinyl, quinolizinyl, 1,8-naphthyridinyl, purinyl, pteridinyl, dibenzofuranyl, carbazolyl, acridinyl, phenanthridinyl, phenazinyl, phenothiazinyl and phenoxazinyl. The heterocyclic moiety may also include dioxolanyl, morpholinyl, piperidinyl, and piperazinyl.

The compounds of the present invention can be prepared by various methods including for instance liquid phase or a solvent based synthesis and solid phase synthesis involving a polymeric resin.

The liquid phase synthesis generally involves addition of a substituted or unsubstituted alkyl amine containing compound to a protected amine containing starting material bearing a leaving group (e.g., Cl, Br, I, OTs, OMs). The resulting product bearing a protonated amine is reacted with an alkyl halide to yield a substituted amine. Then, the protected amine moiety is deprotected by addition of base or e.g., hydrazine. The resultant free amine is reacted with a compound containing an aromatic isocyanate to yield the aromatic urea derivative.

A second synthesis involves the reaction of aromatic isocyanate with a-haloalkylamine. The resultant product is then further reacted with an optionally substituted amine containing compound, the amine of the optionally substituted amine containing compound is substituted by reaction with an alkyl halide to yield the aromatic urea derivative.

An additional method that can be used to prepare the present compounds involves reaction of a protected amine containing starting compound with an alkylamine. The resultant diamine is reacted with an ester containing a leaving group, after deprotection, the aromatic urea derivative is formed by reaction with a compound containing an aromatic isocyanate.

The aromatic urea derivatives can be further derivatized by conventional organic synthesis techniques, for example, an ester can be converted to an acid by addition of a metal hydroxide. Additionally, salts of the compounds can be formed by conventional synthetic techniques, such as addition to an amine moiety to form an ammonium salt.

Solid phase synthesis involves the use of polymeric resins. Reductive amination of the linker to the resin occurs by reacting a haloalkylamine with the polymeric resin. The protonated amine is then protected by reaction with a substituted or unsubstituted acid chloride. The halogen of the original haloalkylamine is displaced by reaction with an alkyl amine compound and reductive amination follows by reaction with an aldehyde. The protected amine is deprotected by reaction with, for example, tin chloride, an acid or an amine. The deprotected amine is subsequently reacted with an isocyanate to yield the urea moiety, the product is isolated by working up the reaction mixture, for example, in HCl gas.

In one embodiment of the present invention, an effective amount of a pharmaceutical composition comprising one or more of the disclosed compounds is administered to a patient suffering from CCR-3 mediated disease. The active compound of the pharmaceutical composition can be administered in a variety of forms for instance a salt or a hydrate In addition, the pharmaceutical composition can optionally contain suitable carriers or excipients.

A "pharmaceutical composition" refers to a mixture of one or more of the compounds described herein, or pharmaceutically acceptable salts or hydrates thereof, with other chemical components, such as physiologically acceptable carriers and excipients. The purpose of a pharmaceutical composition is to facilitate administration of a compound to an organism.

As used herein, a "physiologically acceptable carrier" refers to a carrier or diluent that does not cause significant irritation to an organism and does not abrogate the biological activity and properties of the administered compound.

An "excipient" refers to an inert substance added to a pharmaceutical composition to further facilitate administration of a compound. Examples of excipients include for instance calcium carbonate, calcium phosphate, sugars, starches, cellulose derivatives, gelatin, vegetable oils and polyethylene glycol.

The form of the administered compound depends, in part, upon the use or the route of entry. Such forms should allow the agent to reach a target cell whether the target cell is present in a multicellular host or in culture. For example, pharmacological agents or compositions injected into the blood stream should be soluble in the concentrations used. Other factors are known in the art, and include considerations such as toxicity and forms which prevent the compound or composition from exerting its effect.

A compound of the present invention also can be formulated as a pharmaceutically acceptable salt, e.g., acid addition salt, and complexes thereof. The preparation of such salts can facilitate the pharmacological use by altering the physical characteristics of the agent without preventing its physiological effect. Examples of useful alterations in physical properties included for instance lowering the melting point to facilitate transmucosal administration and increasing the solubility to facilitate administering higher concentrations of the drug.

A compound of the present invention can be administered to a mammal, including a human patient, using a variety of techniques. For example, for systemic administration. oral administration or injection can be used. For oral administration, a compound of the present invention is formulated into conventional oral administration dosage forms such as capsules, tablets, and tonics. For injection, a compound is formulated in liquid solutions, preferably in physiologically compatible buffers such as Hank's solution or Ringer's solution. In addition, a compound can be formulated in a solid form and redissolved or suspended immediately prior to use. Lyophilized forms can also be produced. Examples of systemic administrations by injection, include intramuscularly, intravenously, intraperitoneally and subcutaneously.

Administration also can be by transmucosal or transdermal means. For transmucosal or transdermal administration, penetrants appropriate to the barrier to be permeated are used in the formulation. Such penetrants are generally known in the art, and include, for example, for transmucosal administration, bile salts and fusidic acid derivatives. In addition, detergents may be used to facilitate permeation. Transmucosal administration also can be achieved, for example, by using nasal sprays or suppositories.

Administration of a compound of the present invention can be achieved by any means which transports the compound to the airways and/or lungs of a mammal, including a human patient. In a preferred embodiment, a compound is administered by generating an aerosol comprised of respirable particles, comprising said compound. Delivery is achieved by animal or patient inhalation of the respirable particles. The respirable particles can be liquid or solid and, optionally, can contain other therapeutic ingredients.

For topical administration, the molecules of the invention are formulated into ointments, salves, gels, or creams, as is generally known in the art.

Generally, a therapeutically effective amount for a human patient is between about 10 nmole and 3 mmole of the compound, preferably 1 µmole to 1 mmole. A therapeutically effective amount for a non-human mammal is between about 0.01 and 50 mg/kg, preferably 0.01 and 20 mg/kg. Optimization of the timing and dosage of a disclosed compound is by convention adapted to, among other things, the particular characteristics of the patient or the non-human mammal and the nature and extent of the disease state, and the EC50 or IC50 of the compound. Such adaptations are routine and do not require abnormal experimentation or skill in the art.

In accordance with yet another aspect of the present invention, there is provided a kit suitable for treating CCR-3 mediated diseases in a patient, comprising a pharmaceutical composition comprising one or more compounds of the present invention, reagents to effect administration of the pharmaceutical composition to the patient and instruments to effect administration of the pharmaceutical composition to the patient. Examples of such instruments include, but are not limited to application devices, such as syringes or inhalers.

In yet another emobobyment, the claimed compounds are useful for treatment and/or prevention of rheumatoid arthritis. The treatment includes for instance administration of the claimed compounds through subcacutaneous, intradermal, intramuscular, intraperitoneal, intravascular, and intracranial injections to human or other mammalian animal bodies.

### EXAMPLES

### SYNTHESIS OF ACTIVE COMPOUNDS

### Example 1. Synthesis of N-Phenylcarbamoyl-N'-[2-(4-chloronhenvilethyl]-N'-ethyl-1.3-diamino.propane (Compound 1)

The following synthesis is depicted in Scheme 1.

Step 1: To a mixture of 2-(4-chlorophenyl)ethylamine (1.56 g. 10 mmol) and potassium carbonate (2.8 g, 20 mmol) in CH₃CN (50 ml) was added N-(3-bromopropyl)phthalimide (3.0 g, 11 mmol). The mixture was refluxed under stirring for 16 h, and then filtered. The filtrate was concentrated under vacuum to dryness, and the residue was chromatographed on silica gel (eluting with 2.5% methanol/chloroform) to afford N-[3-[2-(4-chlorophenyl)ethylamino]propyl]phthalimide (2.28 g, 67%): MS(FD) m/e 343 [M+H]⁺; ¹H NMR (400 MHz, CDCl₃) δ7.84 (m, 2H), 7.71 (m, 2H), 7.24 (d, J = 8.3 Hz, 2H), 7.11 (d, J = 8.3 Hz, 2H), 3.74 (d, J = 6.8 Hz, 2H), 2.82 (t, J = 6.8 Hz, 2H), 2.73 (t, J = 6.8 Hz, 2H), 2.66 (t, J = 6.8 Hz, 2H), 1.84 (m, 2H).

Step 2: To a mixture of N-[3-[2-(4-chlorophenyl)ethylamino]propyl]phthalimide (2.28 g, 6.65 mmol) and potassium carbonate (1.8 g, 13 mmol) in CH₃CN (50 ml) was added ethyl iodide (1.6 ml, 20 mmol). The mixture was stirred at 70 °C for 16 h, and then filtered. The filtrate was concentrated under vacuum to dryness, and the residue was chromatographed on silica gel (eluting with 2% methanol/chloroform) to afford N-[3-[[2-(4-chlorophenyl)ethyl](ethyl)amino]propyl]phthalimide (1.41 g, 57%): MS(ES⁺) m/e 371 [M+H]⁺; ¹H NMR (400 MHz, CDCl₃) δ7.84 (m, 2H), 7.71 (m, 2H), 7.23 (d, J = 8.5 Hz, 2H), 7.11 (d, J = 8.5 Hz, 2H), 3.71 (t, J =7.3 Hz, 2H), 2.66 (m, 4H), 2.57 (m, 4H), 1.83 (m, 2H), 1.00 (t, J .= 7.1 Hz, 3H).

Step 3: To a solution of N-[3-[[2-(4-chlorophenyl)ethyl](ethyl)amino]propyl]-phthalimide (1.41 g, 3.8 mmol) in EtOH (20 ml) was added a solution of hydrazine monohydrate (1.5 g, 30 mmol) in EtOH (5 ml). The solution was stirred at RT for 4h, and then filtered. The filtrate was concentrated under vacuum to dryness. After adding water, the mixture was extracted with chloroform, washed with brine, dried over sodium sulfate, and filtered. Concentrating under vacuum gave N-[2-(4-chlorophenyl)ethyl]-N-ethyl-1,3-diaminopropane (903 mg, 99%) which was used in the next step without further purification.

Step 4: To a solution of N-[2-(4-chlorophenyl)ethyl]-N-ethyl-1,3-diaminopropane (30 mg, 0.125 mmol) in CH₂Cl₂ (1 ml) was added phenyl isocyanate (18 mg. 0.15 mmol). After stirring at RT for 1h, the reaction mixture was chromatographed on silica gel (eluting with 2.5 % methanol/chloroform) to afford N-phenylcarbamoyl-N'-[2-(4-chlorophenyl)ethyl]-N'-ethyl-1,3-diaminopropane (38.7 mg, 86 %): MS(ES⁺) m/e 360 [M+H]⁺; ¹H NMR (400 MHz, CDCl₃) δ 7.34-7.23 (m, 6H), 7.08 (d, J = 8.3 Hz. 2H), 7.03 (m, 1H), 6.93 (br, 2H), 3.34 (m, 2H), 2.76-2.70 (m, 8H), 1.76 (m, 2H), 1.08 (t, J = 7.1 Hz, 3H).

Compound 2, N-(4-Nitrophenylcarbamoyl)-N'-[2-(4-chlorophenyl)ethyl]-N'-ethyl-1,3-diaminopropane, can be obtained in an analogous manner to that described for compound 1 and contains the following characteristics: MS(FD) m/e 405 [M+H]⁺; ¹H NMR (400 MHz, CDCl₃) δ 8.12 (d. J = 9.3 Hz,.2H), 7.65 (br, 1H), 7.51 (d, J = 9.2 Hz, 2H), 7.27 (d, J = 8.3 Hz, 2H), 7.13 (d, J = 8.3 Hz, 2H), 6.38 (br, 1H), 3.33 (m, 2H). 2.83.(m, 4H), 2.74 (m, 4H), 1.76 (m, 2H), 1.11 (t, J = 7.1 Hz, 3H).

Compound 3, N-(4-Bromophenylcarbamoyl)-N'-[2-(4-chlorophenyl)ethyl]-N'-ethyl-1,3-diaminopropane, can be obtained in an analogous manner to that described for compound 1 and contains the following characteristics: MS(ES⁺) m/e 438 [M+H]⁺: ¹H NMR (400 MHz, CDCl₃) δ7.60 (br, 1H), 7.32 (d, J = 8.8, Hz, 2H), 7.26 (m, 4H), 7.08 (d, J = 8.3 Hz, 2H), 6:25 (br, 1H), 3.26 (t, J = 6.1 Hz, 2H), 2.73 (m, 8H), 1.71 (m, 2H), 1.08 (t, J = 7.3 Hz, 3H).

Compounds 4-9, 191, 192, 202, 204, 215, 230-234, 239-245, 274-276, 280, 291, 292 can be obtained in an analogous manner to that of Compound 1.

### Example 2. Synthesis of N-Phenylcarbamoyl-N'-[2-(4-chlorophenyl)ethyl]-N'-propyl-1,3-diaminopropane (Compound 10)

The following synthesis is depicted in Scheme 2.

Step 1: Phenyl isocyanate (1.4 ml, 13 mmol) was added to a solution of 3-bromopropylamine hydrobromide (2.5 g, 11 mmol) and triethylamine (1.7 ml, 12 mmol) in DMF (50 ml) at 0 °C, and the mixture was stirred at 0 °C for 1.5 h. After adding water, the mixture was extracted with ethyl acetate, washed with water and brine, dried over sodium sulfate, and filtered. The filtrate was concentrated under vacuum to dryness, and the residue was chromatographed on silica gel (eluting with 2.5 % ethyl acetate/hexane to 50% ethyl acetate/hexane) to afford N-phenylcarbamoyl-3-bromopropylamine (2.7 g 96%): MS(FD) m/e 256 M⁺; ¹H NMR (400 MHz, CDCl₃)δ 7.35-7.26 (m, 4H), 7.11 (m, 1H), 6.45 (br, 2H), 3.46 (t, J = 6.3 Hz, 2H), 3.41 (t, J = 6.6 Hz, 2H), 2.10 (m, 2H).

Step 2: 2-(4-Chlorophenyl)ethylamine (1.8 g, 12 mmol) was added to a mixture of N-phenylcarbamoyl-3-bromopropylamine (2.5 g, 9.7 mmol) and potassium carbonate (2.6 g, 19 mmol) in CH₃CN (50 ml). The mixture was stirred at 70 °C for 4.5 h, and then filtered. The filtrate was concentrated under vacuum to dryness, and the residue was dissolved with chloroform, washed with water, 1N-HCI and brine. The organic layer was dried over sodium sulfate, filtered, and then concentrated under vacuum to dryness. The residue was chromatographed on silica gel (eluting with 2 % methanol/chloroform) to afford N-phenylcarbamoyl-N'-[2-(4-chlorophenyl)ethyl]-1,3-diaminopropane (1.43 g, 45%): MS(ES⁺) m/e 332 M⁺; ¹H NMR (400 MHz, CD₃OD) δ7.35-7.15 (m, 8H), 6.99 (m, 1H), 3.31 (m, 2H), 3.23 (m, 2H), 3.06 (t, J = 7.1 Hz, 2H), 3.00 (m, 2H), 1.89 (m, 2H).

Step 3: Propyl iodide (51 mg, 0.30 mmol) was added to a mixture of N-phenylcarbamoyl-N'-[2-(4-chlorophenyl)ethyl]-1,3-diaminopropane (33 mg, 0.10 mmol) and potassium carbonate (28 mg, 0.20 mmol) in CH₃CN (2 ml). The mixture was stirred at 75 °C for 5 h, and then filtered. The filtrate was concentrated under vacuum to dryness, and the residue was purified by preparative normal phase HPLC using linear gradients of (A) chloroform and (B) methanol (0-5% B, in 0-10 min; 5-10 % B, in 10-30 min; 10-15% B, in 30-40 min) at a flow rate of 10 ml/min. Fractions containing the major peak were pooled and concentrated to afford N-phenylcarbamoyl-N'-[2-(4-chlorophenyl)ethyl]-N'-propyl-1,3-diaminopropane (27 mg, 59%): MS(ES⁺) m/e 374 [M+H]⁺; ¹H NMR (400 MHz, CDCl₃) δ 7.35 (d, J = 8.3 Hz, 2H), 7.25 (m, 4H), 7.07 (m, 3H), 5.98 (br, 1H), 5.00 (br, 1H), 3.31 (m, 2H), 2.78 (m, 6H), 2.59 (m, 2H), 1.76 (m, 2H), 1.52 (m, 2H), 0.90 (t, J = 7.3 Hz, 3H).

Compounds 11-28, 219-221 can be obtained in an analogous manner to that of Compound 10.

### Example 3. Synthesis of Methyl 4-[[3-(4-bromophenylureido)propyl] (1.2.3.4-tetrahvdro-1-naphthyl)aminolbutylate (Compound 29)

The following synthesis is depicted in Scheme 3.

Step 1: N-(3-Bromopropyl)phthalimide (13.0 g, 48.5 mmol) was added to a mixture of 1,2,3,4-tetrahydro-1-naphthylamine (6.96 ml, 48.5 mmol) and potassium carbonate (13.4 g, 97.0 mmol) in CH₃CN (200 ml). The mixture was refluxed under stirring for 21 h, and then filtered. The filtrate was concentrated under vacuum to dryness, and the residue was chromatographed on silica gel (eluting with 1.5% methanol/chloroform) to afford N-[3-(1,2,3,4-tetrahydro-1-naphthylamino)propyl]phthalimide (23.9 g, 74%): MS(ES⁺) m/e 335 [M+H]⁺; ¹H NMR (400 MHz, CDCl₃) δ 7.84 (m, 2H), 7.71 (m, 2H), 7.39 (m, 1H), 7.13 (m, 2H), 7.06 (m, 1H), 3.81 (m, 3H), 2.81 (m, 2H), 2.71 (m, 2H), 2.00-1.85 (m, 4H), 1.72 (m, 2H).

Step 2: Methyl 4-bromobutylate (16.3 g, 89.8 mmol) was added to a mixture of N-[3-(1,2,3,4-tetrahydro-1-naphthylamino)propyl]phthalimide (10.0 g, 29.9 mmol) and potassium carbonate (8.28 g, 59.9 mmol) in DMF (150 ml). The mixture was stirred at 130 °C for 22 h. After adding water, the mixture was extracted with chloroform, washed with brine, dried over sodium sulfate, and filtered. The filtrate was concentrated under vacuum to dryness, and the residue was chromatographed on silica gel (eluting with 5% methanol/chloroform) to afford methyl 4-[[(3-phthalimido)propyl](1,2,3,4-tetrahydro-1-naphthyl)amino]butylate (4.62 g, 36%): MS(ES⁺) m/e 435 [M+H]⁺; ¹H NMR (400 MHz. CDCl₃) δ 7.75 (m, 2H), 7.63 (m, 2H), 7.60 (d, J = 7.6 Hz, 1H), 7.03 (m, 1H), 6.94 (m. 2H), 3.90 (m, 1H), 3.51 (m, 2H), 2.63 (m, 2H), 2.45-2.20 (m, 6H), 1.91 (m. 2H), 1.74 (m, 4H), 1.52 (m, 2H).

Step 3: Hydrazine monohydrate (1.03 ml, 21.3 mmol) was added to a solution of methyl 4-[[(3-phthalimido)propyl](1,2,3,4-tetrahydro-1-naphthyl)amino]butylate (4.62 g, 10.6 mmol) in EtOH (80 ml) at 0 °C. After stirring at RT for 2h, additional hydrazine monohydrate (1.03 ml, 21.3 mmol) was added. The solution was stirred at RT for 2h, and concentrated under vacuum to dryness. After adding water, the mixture was extracted with chloroform, dried over sodium sulfate, and filtered. The filtrate was dissolved with chloroform, and then extracted with 1N-HCl. The water layer was neutralized with 1N-NaOH at 0 °C, washed with chloroform, and then basified with 1N-NaOH (pH = 14), extracted with chloroform. The organic layer was washed with brine, dried over sodium sulfate, and filtered. Concentrating under vacuum gave methyl 4-[(3-aminopropyl-)(1,2.3,4-tetrahydro-1-naphthyl)amino]butylate (1.05 g, 33%) which was used in the next step without further purification.

Step 4: 4-Bromophenyl isocyanate (83 mg, 0.42 mmol) was added to a solution of methyl 4-[(3-aminopropyl)(1,2,3,4-tetrahydro-1-naphthyl)amino]butylate (106 mg, 0.35 mmol) in CH₂Cl₂ (3 ml). After stirring at RT for 1h, the reaction mixture was concentrated under vacuum to dryness. The residue was adsorbed on a plate of silica gel and the plate was developed with 6% methanol/chloroform to afford methyl 4-[[3-(4-bromophenylureido)propyl](1,2,3,4-tetrahydro-1-naphthyl)amino]butylate (89 mg, 51 %): MS(FD) m/e 502 [M+H]⁺; ¹H NMR (400 MHz, DMSO-d6) δ 8.50 (br, 1H), 7.57 (d, J = 7.6 Hz, 1H), 7.35 (m, 4H), 7.13-7.01 (m, 3H), 6.09 (t, J = 5.5 Hz, 1H), 3.89 (dd, J = 9.0, 5.1 Hz, 1H), 3.53 (s, 3H), 3.12 (m, 1H), 3.03 (m, 1H), 2.67 (m, 2H), 2.43-2.25 (m, 6H), 2.00-1.88 (m, 2H), 1.70-1.50 (m, 6H).

Compound 30, Methyl 4-[[3-(4-bromophenylureido)propyl][(1*R*)-1-phenylethyl]-amino]butylate, can be obtained in an analogous manner to that described for compound 29 and contains the following characteristics: MS(ES⁺) m/e 476 [M+H]⁺; ¹H NMR (400 MHz, CDCl₃) δ 7.73 (m, 1H), 7.38-7.21 (m, 8H), 7.08 (br, 1H), 5.47 (br, 1H), 3.91 (m, 1H), 3.65 (s, 3H), 3.20 (m, 2H), 2.49 (m, 3H), 2.29(m, 3H), 1.77 (m, 2H), 1.61 (m, 2H), 1.31 (d, J = 6.6 Hz, 3H).

Compound 31, Methyl 4-[[3-(4-bromophenylureido)pcopyl][2-(4-chlorophenyl)-ethyl]amino]butylate, can be obtained in an analogous manner to that described for compound 29 and contains the following characteristics: MS(ES⁺) m/e 510 [M+H]⁺; ¹H NMR (400 MHz, CDCl₃) δ 8.61 (br, 1H), 7.34 (d, J = 8.8 Hz, 2H), 7.25 (m, 4H), 7.08 (d, J = 8.3 Hz, 2H), 5.99 (br, 1H), 3.70 (s, 3H), 3.28 (t, J = 5.9 Hz, 2H), 2.68 (br, 4H), 2.59 (t, J = 5.9 Hz, 2H), 2.53 (t, J = 6.8 Hz, 2H), 2.35 (t, J = 7.1 Hz, 2H), 1.79 (m, 2H), 1.68 (m, 2H).

Compound 32, Methyl 4-[[4-(4-bromophenylureido)butyl](1,2,3,4-tetrahydro-1-naphthyl)amino]butylate, can be obtained in an analogous manner to that described for compound 29 and contains the following characteristics: MS(ES⁺) m/e 516 [M+H]⁺; ¹H NMR (400 MHz, CDCl₃) δ 8.09 (br, 1H), 7.62 (m, 1H), 7.31 (d, J = 8.8 Hz, 2H), 7.20 (d, J = 8.8 Hz, 2H), 7.10 (m, 2H), 7.03 (m, 1H), 5.37 (m, 1H), 3.89 (m, 1H), 3.66 (s, 3H), 3.17 (m, 2H), 2.70 (m, 2H), 2.51-2.29 (m, 6H), 1.96 (m, 2H), 1.76 (m, 2H), 1.68-1.40 (m, 6H).

Compound 33. Methyl 4-[[5-(4-bromophenylureido)penryl](1,2.3,4-tetrahvdro-1-naphthyl)amino]burylate, can be obtained in an analogous manner to that described for compound 29 and contains the following characteristics: MS(ES⁺) m/e 530 [M+H]^{+ 1}H NMR (400 MHz, CDCl₃) δ 7.63 (m, 1H), 7.32 (d, J = 8.8 Hz, 2H), 7.29 (br, 1H). 7.21 (d, J = 9.0 Hz, 2H), 7.09 (m, 2H), 7.02 (m, 1H), 5.31 (m, 1H), 3.90 (m, 1H), 3.66 (s, 3H), 3.20 (m, 2H), 2.71 (m, 2H), 2.49-2.27 (m, 6H), 1.97 (m, 2H), 1.76 (m, 2H), 1.60 (m, 2H), 1.43 (m, 4H), 1.27 (m, 2H).

Compound 34, Methyl 4-[[3-(4-methylphenylureido)propyl](1,2,3,4-tetrahydro-1-naphthyl)amino]butylate, can be obtained in an analogous manner to that described for compound 29 and contains the following characteristics: MS(ES⁺) m/e 438 [M+H]⁺; ¹H NMR (400 MHz, DMSO-*d6*) *δ* 8.21 (br, 1H), 7.59 (d, J = 7.6 Hz, 1H), 7.24 (d, J = 8.5 Hz, 2H), 7.13-6.99 (m, 5H), 5.98 (t, J = 5.6 Hz, 1H), 3.90 (dd, J = 9.5, 4.9 Hz, 1H), 3.54 (s, 3H), 3.11 (m, 1H), 3.02 (m, 1H), 2.67 (m, 2H), 2.43-2.23 (m, 6H), 2.20 (s, 3H), 1.94 (m, 2H), 1.70-1.50 (m, 6H).

Compound 35, Methyl 4-[[3-(3,4-dichlorophenylureido)propyl](1,2,3,4-tetrahydro-1-naphthyl)amino]burylate, can be obtained in an analogous manner to that described for compound 29 and contains the following characteristics: MS(ES⁺) m/e 492 [M+H]⁻; ¹H NMR (400 MHz, DMSO-*d6*) *δ*9.20 (br, 1H), 7.89 (d, J = 2.4 Hz, 1H), 7.64 (d, J = 7.3 Hz, 1H), 7.49 (d, J = 8.8 Hz, 1H), 7.28 (dd, J = 8.8, 2.4 Hz, 1H), 7.19-7.07 (m, 3H), 6.27 (m, 1H), 3.95 (m, 1H), 3.60 (s, 3H), 3.18 (m, 1H), 3.10 (m, 1H), 2.73 (m, 2H), 2.57-2.29 (m, 6H), 2.00 (m, 2H), 1.74-1.54 (m, 6H).

Compound 172, Methyl 4-[[3-(4-bromophenylureido)propyl](1-indanyl)amino]burylate, can be obtained in an analogous manner to that described for compound 29 and contains the following characteristics: MS(ES⁺) m/e 490 [M+H]⁺; ¹H NMR (400 MHz, CDCl₃) *δ*8.32 (bs, 1H), 7.43 (m, 1H), 7.30 (m, 2H); 7.26 (m, 2H), 7.20 (m, 3H), 5.77 (br, 1H), 4.50 (m, 1H), 3.65 (s, 3H), 3.27 (m, 2H), 2.82 (m, 2H), 2.51 (m, 1H), 2.40 (m, 4H), 2.31 (m, 1H), 2.04 (m, 1H), 1.95 (m, 1H), 1.81 (m, 2H), 1.66 (m, 2H).

Compound 178, Methyl 4-[[3-(4-bromophenylureido)propyl][(1R)-1-indanyl]amino]butylate, can be obtained in an analogous manner to that described for compound 29 and contains the following characteristics: MS(ES⁺) m/e 490 [M +H]⁺; ¹H NMR (400 MHz, CDCl₃) *δ*8.24 (br, 1H), 7.31 (m, 3H), 7.26 (m, 3H), 7.20 (m, 2H). 5.69 (br, 1H), 4,50 (t, J =6.8 Hz, 1H), 3.66 (s, 3H), 3.28 (m. 2H), 2.90-2.77 (m, 2H), 2.52-2.26 (m, 6H), 2.05 (m, 1H), 1.95 (m, 1H), 1.81 (m, 2H), 1.66 (m, 2H).

Compound 180, Methyl 4-[[3-(4-bromophenylureido)propyl][(1*R*)-1,2,3,4-tetrahydro-1-naphthyl]amino]burylate, can be obtained in an analogous manner to that described for compound 29 and contains the following characteristics: MS(ES⁺) m/e 504 [M+H]⁺; ¹H NMR (400 MHz, CDCl₃) *δ* 8.19 (br, 1H), 7.58 (d, J = 6.8 Hz, 1H), 7.31 (d, J = 8.8 Hz, 2H), 7.20 (d, J = 8.8 Hz, 2H), 7.12 (m, 2H), 7.05 (d, J = 6.9 Hz, 1H), 5.43 (br, 1H), 3.95 (m, 1H). 3.66 (s, 3H), 3.24 (m, 2H), 2.70 (m, 2H), 2.55-2.36 (m, 5H), 2.27 (m, 1H), 1.94 (m, 2H), 1.79 (m, 2H), 1.62 (m, 4H).

Compound 184, Ethyl 4-[[3-(4-bromophenylureido)propyl](1,2,3,4-tetrahydro-1-naphthyl)amino]burylate, can, be obtained in an analogous manner to that described for compound 29 and contains the following characteristics: MS(ES⁺) m/e 516 [M+H]⁺; ¹H NMR (400 MHz, CDCl₃) *δ7.54* (d, J = 7.3 Hz, 1H), 7.27 (d. J = 8.8 Hz, 2H), 7.19 (d. J = 8.8 Hz, 2H), 7.09 (m, 2H), 7.01 (d, J = 6.8 Hz, 1H), 5.33 (br, 2H), 4.06 (q, J = 7.1 Hz, 2H), 3.98 (m, 1H), 3.26 (m, 1H), 3.20 (m, 1H), 2.65 (m, 2H), 2.61-2.31 (m, 5H), 2.22 (m, 1H), 1.91 (m, 2H), 1.74 (m, 2H), 1.60 (m, 4H), 1.18 (t, J = 7.1 Hz, 3H).

Compounds 36-49, 51-59, 174, 176, 182, 185, 187, 189, 194, 198, 200, 206, 208, 212, 213, 224 can be obtained in an analogous manner to that of Compound 29.

### Example 4. Synthesis of 4-[[3-(4-Bromophenylureido)propyl](1,2,3,4-tetrahydro-1-naphthyl)amino]butanoic acid (Compound 60).

The following synthesis is depicted in Scheme 4.

Lithium hydroxide monohydrate (14 mg, 0.33 mmol) was added to a solution of methyl 4-[[3-(4-bromophenylureido)propyl](1,2,3,4-tetrahydro-1-naphthyl)amino]butylate (Compound 29, 83 mg, 0.17 mmol) in 10% water/methanol (2 ml). After stirring at RT for 16 h, additional lithium hydroxide monohydrate (14 mg, 0.33 mmol) was added. The reaction mixture was stirred at RT for 6 h, and then concentrated under vacuum to dryness. The residue was dissolved with ether and water, and partitioned. The water layer was acidified with 1N-HCl (pH = 1), extracted with ethyl acetate, washed with brine, dried over sodium sulfate, and filtered. The filtrate was concentrated under vacuum to dryness. The residue was adsorbed on a plate of silica gel and the plate was developed with 17 % methanol/chloroform to afford 4-[[3-(4-bromophenylureido)propyl](1,2,3,4-tetrahydro-1-naphthyl)amino]butanoic acid (44 mg, 53%): MS(ES⁺) m/e 488 [M+H]⁺: ¹H NMR (400 MHz, CDCl₃) δ 9.40 (br, 1H), 8.76 (br, 1H), 7.72 (m, 1H), 7.38 (m. 4H), 7.23 (m. 3H), 6.35 (br, 1H). 4.92 (br, 1H), 2.97 (m, 2H), 2.85-2.65 (m, 8H), 2.18 (m. 2H). 2.00 (m. 2H), 1.67 (m, 4H).

Compound 61, 4-[[3-(4-Bromophenylureido)propyl][(1*R*)-1-phenylethyl]amino]-butanoic acid, can be obtained in an analogous manner to that described for compound 60 and contains the following characteristics: MS(ES⁺) m/e 462 [M+H]⁺; ¹H NMR (400 MHz, CDCl₃) δ 8.80 (br, 1H), 7.50 (br, 1H), 7.40-7.25 (m, 9H), 6.90 (br, 1H), 4.31 (br, 1H), 3.23 (m, 2H), 2.50-2.21 (m, 6H), 1.74 (m, 4H), 1.27 (m, 3H).

Compound 62, 4-[[4-(4-Bromophenylureido)butyl](1,2,3,4-tetrahydro-1-naphthyl)-amino]butanoic acid, can be obtained in an analogous manner to that described for compound 60 and contains the following characteristics: MS(ES⁺) m/e 502 [M+H]⁺; ¹H NMR (400 MHz, CD₃OD) *δ* 7.52 (d, J = 7.3 Hz, 1H), 7.38-7.24 (m, 7H), 5.01 (t, J = 7.5 Hz,1H), 3.20 (br, 2H), 2.92-2.76 (m, 4H), 2.45-2.29 (m, 4H). 2.04 (m, 4H), 1.87 (m, 2H), 1.75 (m, 2H), 1.53 (br, 2H).

Compound 63, 4-[[5-(4-Bromophenylureido)pentyl](1,2,3,4-tetrahydro-1-naphthyl)-amino]butanoic acid, can be obtained in an analogous manner to that described for compound 60 and contains the following characteristics: MS(ES⁺) m/e 516 [M+H]^{+ 1}H NMR (400 MHz, CD₃OD) *δ* 7.53 (d, J = 7.1 Hz, 1H), 7.36-7.24 (m, 7H), 5.01 (t, J = 7.5 Hz, 1H), 3.17 (br, 2H), 2.91-2.76 (m, 4H), 2.45-2.28 (m, 4H), 2.04 (m, 4H), 1.84 (m, 2H), 1.75 (m, 2H), 1.51 (m, 2H), 1.35 (m, 2H).

Compound 64, 4-[[3-(4-Methylphenylureido)propyl](1,2,3,4-tetrahydro-1-naphthyl)-amino]butanoic acid, can be obtained in an analogous manner to that described for compound 60 and contains the following characteristics: MS(ES⁺) m/e 424 [M+H]⁺; ¹H NMR (400 MHz, DMSO-*d6*) *δ*9.29 (br, 1H), 8.31 (br, 1H); 7.67 (br, 1H), 7.25 (m, 4H), 7.02 (m, 3H), 6.15 (br, 1H), 4.90 (br, 1H), 2.99 (m, 2H), 2.71-2.48 (m, 8H), 2.21 (s,3H), 2.20 (m, 2H), 1.93 (m, 2H), 1.64 (m, 4H).

Compound 65, 4-[[3-(3,4-Dichlorophenylureido)propyl](1,2,3,4-tetrahydro-1-naphthyl)amino]butanoic acid, can be obtained in an analogous manner to that described for compound 60 and contains the following characteristics: MS(ES⁺) m/e 478 [M+H]⁺; ¹H . NMR (400 MHz, DMSO-*d6*) *δ*9.23 (br, 1H), 8.95 (br, 1H), 7.82 (br, 1H), 7.69 (br. 1H). 7.45 (d, J = 8.8 Hz, 1H); 7.29-7.18 (m, 4H), 6.38 (br, 1H), 4.91 (br, 1H), 3.00 (m. 2H). 2.74-2.65 (m, 8H), 2.18 (m, 2H), 1.94 (m, 2H), 1.65 (m, 4H).

Compound 171, 4-[[3-(4-Chlorophenylureido)propyl](1,2,3,4-tetrahydro-1-naphthyl)amino]butanoic acid, can be obtained in an analogous manner to that described for compound 60 and contains the following characteristics: MS(ES⁺) m/e 444 [M+H]⁺; ¹H NMR (400 MHz, DMSO-*d6*) δ 8.99 (s, 1H), 8.31 (s, 1H), 7.66 (m. 1H), 7.46 (m. 4H), 7.24 (m, 3H), 6.21 (br, 1H), 4.87 (m, 1H), 3.00 (m, 2H), 2.72-2.49 (m, 8H), 2.18 (m, 2H), 1.93 (m, 2H), 1.65 (m, 4H).

Compound 173, 4-[[3-(4-Bromopbenylureido)propyl](1-indanyl)amino]butanoic acid, can be obtained in an analogous manner to that described for compound 60 and contains the following characteristics: MS(ES⁺) m/e 476 [M+H]⁺; ¹H NMR (400 MHz, CD₃OD) δ7.63 (m, 1H), 7.39 (m, 2H), 7.34 (m, 4H), 7.27 (m, 1H). 5.25 (dd, J = 8.5, 3.4 Hz, 1H), 3.30 (m, 2H), 3.19 (m, 6H), 3.03 (m, 2H), 2.53 (m, 1H), 2.41 (m, 3H), 2.03 (m, 2H).

Compound 179, 4-[[3-(4-Bromophenylureido)propyl][(1*R*)-1-indanyl]amino]butanoic acid, can be obtained in an analogous manner to that described for compound 60 and contains the following characteristics: MS(ES⁺) m/e 476 [M+H]⁺; ¹H NMR (400 MHz, CD₃OD) δ7.63 (d, J = 7.8 Hz, 1H), 7.38 (m, 3H), 7.33 (m, 3H), 7.27 (m, 1H), 5.24 (dd, J = 8.6, 3.7 Hz, 1H), 3.29 (m, 4H), 3.18 (m, 4H), 3.02 (m, 2H), 2.53 (m, 1H), 2.41 (m, 3H), 2.02 (m, 2H).

Compound 181, 4-[[3-(4-Bromophenylureido)propyl][(1R)-1,2,3,4-tetrahydro-1-naphthyl]amino]butanoic acid, can be obtained in an analogous manner to that described for compound 60 and contains the following characteristics: MS(ES⁺) m/e 490 [M+H]⁺; ¹H NMR (400 MHz, -CD₃OD) *δ*7.64 (m, 1H), 7.39 (d, J = 9.0 Hz, 2H), 7.33 (d, J = 9.0 Hz, 2H), 7.29 (m, 2H), 7.20 (m, 1H), 5.06 (m, 1H), 3.24 (m, 6H), 2.91-2.76 (m, 4H), 2.33 (m, 4H), 2.02 (m, 4H).

Compound 227, 4-[[3-(4-Bromophenylureido)propyl][(1R)-1-(4-methoxyphenyl)ethyl]amino]butanoic acid, can be obtained in an analogous manner to that described for compound 60 and contains the following characteristics: MS(ES⁺) m/e 494 [M+H]⁺;¹H NMR (400 MHz, CDCl₃) *δ*9.57 (s, 1H), 7.88 (br, 1H), 7.44 (d, J = 8.8 Hz, 2H), 7.30 (d, J = 8.8 Hz, 2H), 7.27 (m, 1H), 6.90 (m, 4H), 4.24 (q, J = 6.8 Hz, 1H), 3.75 (s, 3H), 3.26 (m. 2H), 3.13 (m, 1H), 2.98 (m, 1H), 2.91 (in, 2H), 2.43 (m, 2H). 1.90 (m, 3H), 1.81 (m, 1H), 1.63 (d, J = 6.8 Hz; 3H).

Compounds 66- 88, 82, 90, 175, 177, 183, 186, 188, 190, 195, 199, 201. 207, 209, 211, 214, 223, 226 can be obtained in an analogous manner to that of Compound 60.

### Example 5. Synthesis of [3-(Phenylureido)propyl][2-(4-chlorophenyl)ethyl]-diethylammonium iodide (Compound 91).

The following synthesis is depicted in Scheme 5.

A solution of N-phenylcarbamoyl-N'-[2-(4-chlorophenyl)ethyl]-N'-ethyl-1,3-diaminopropane (Compound 1, 13.7 mg, 0.0381 mmol) in ethyl iodide (2 ml) was refluxed for 3. h, and concentrated under vacuum to dryness. The residue was adsorbed on a plate of silica gel and the plate was developed with 17% methanol/chloroform to afford [3-(phenylureido)propyl][2-(4-chlorophenyl)ethyl]diethylammonium iodide (15.4 mg, 78%): MS(ES⁺) m/e 388 [M-I]⁺; ¹H NMR (400 MHz, CDCl₃) δ 8.15 (br, 1H), 7.35 (d, J = 7.6 Hz, 2H), 7.16 (m, 6H), 6.90 (m, 1H), 6.54 (m, 1H), 3.50 (m, 2H); 3.33 (m, 6H), 3.25 (m, 2H), 2.96 (m, 2H), 1.91 (m, 2H), 1.28 (t, J = 7:1 Hz, 6H).

Compound 92, [3-(4-Bromophenylureido)propyl][2-(4-chlorophenyl)ethyl]-diethylammonium iodide, can be obtained in an analogous manner to that described for compound 91 and contains the following characteristics: MS(ES⁺) m/e 466 [M-I]⁺; ¹H NMR (400 MHz, CDCl₃) *δ* 8.25 (br, 1H), 7.30 (d, J = 9.0 Hz, 2H), 7.22 (d, J = 8.8 Hz, 2H), 7.18 (d, J = 8.3 Hz, 2H), 7.12 (d, J = 8.3 Hz, 2H), 6.67 (t. J = 6.0 Hz, 1H), 3.60 (m, 2H), 3.32 (m, 6H), 3.24 (m, 2H), 2.98 (m, 2H), 1.91 (m, 2H), 1.30 (t, J = 7.2 Hz, 6H).

Compound 298, [3-(Phenylureido)propyl][2-(4-chlorophenyl)ethyl](4-chlorobenzyl)ethylammonium iodide, can be obtained in an analogous manner to that described for compound 91 and contains the following characteristics: MS(ES⁺) m/e 484 [M-I]⁺; ¹H NMR (400 MHz, CDCl₃) *δ* 8.00 (s, 1H), 7.40 (m, 4H). 7.34 (d. J = 8.5 Hz, 2H), 7.11 (m, 6H), 6.87 (t, J = 7.3 Hz, 1H), 6.73 (t, J = 6-1 Hz, 1H). 4.65 (d, J = 13.4 Hz, 1H), 4.57 (d, J = 13.4 Hz, 1H), 3.78 (m, 1H), 3.66 (m, 1H), 3.39 (m, 1H), 3.29 (m, 2H), 3.19 (m, 4H), 3.11 (m, 1H), 2.00 (m, 2H), 1.41 (t, J = 7.1 Hz, 3H).

Compound 302, [3-(Phenylureido)propyl][2-(4-chlorophenyl)ethyl](benzyl)ethylammonium iodide, can be obtained in an analogous manner to that described for compound 91 and contains the following characteristics: MS(ES⁻) m/e 450 [M-I]⁺; ¹H NMR (400 MHz. CD₃OD) *δ*7.60 (m, 2H). 7.51 (m, 3H), 7.33 (m. 6H). 7.26 (m, 2H), 7.00 (m, 1H), 4.63 (s, 2H), 3.48-3.30 (m, 8H), 3.18 (m, 2H). 2.17 (m. 2H), 1.51 (t, J = 7.1 Hz, 3H).

Compound 309, [3-(Phenylureido)propyl][2-(3-chlorophenyl)ethyl]diethylammonium iodide, can be obtained in an analogous manner to that described for compound 91 and contains the following characteristics: MS(ES⁺) m/e 388 [M-I]⁺; ¹H NMR (400 MHz, CDCl₃/CD₃OD) *δ* 8.09 (s, 1H), 7.39 (d, J = 8.5 Hz. 2H), 7.13 (m, 5H), 7.08 (d, J = 7.3 Hz, 1H), 6.88 (t, J = 7.3 Hz, 1H), 6.69 (m, 1H). 3.65 (m, 2H), 3.35 (m, 6H), 3.24 (m, 2H), 3.00 (m, 2H), 1.93 (m, 2H), 1.32 (t, J = 7.1 Hz, 6H).

Compound 320, [3-(Phenylureido)propyl][2-(4-chlorophenyl)ethyl][4-(methoxycarbonyl)butyl] ethylammonium iodide, can be obtained in an analogous manner to that described for compound 91 and contains the following characteristics: MS(ES⁺) m/e 474 [M-I]⁺; ¹H NMR (400 MHz, CD₃OD) *δ*7.36 (m, 2H), 7.32 (m, 4H), 7.24 (m, 2H), 6.98 (m, 1H), 3.66 (s, 3H), 3.44 (m, 6H), 3.31 (m, 4H), 3.05 (m, 2H), 2.44 (m, 2H), 1.98 (m, 2H), 1.78 (m, 2H), 1.67 (m, 2H), 1.36 (m, 3H).

Compound 323, [5-(Phenylureido)pentyl][2-(4-chlorophenyl)ethyl]diethylammanium iodide, can be obtained in an analogous manner to that described for compound 91 and contains the following characteristics: MS(ES⁺) m/e 416 [M-I]⁺; ¹H NMR (400 MHz, CDCl₃) *δ*8.37 (bs, 1H), 7.53 (d, J = 7.6 Hz, 2H), 7.24 (m, 4H), 7.16 (m, 2H), 6.89 (t, J = 7.3 Hz, 1H), 6.40 (m, 1H), 3.43-3.28 (m, 10H), 3.01 (m, 2H), 1.78 (m, 2H), 1.58 (m, 4H), 1.29 (t, J = 7.3 Hz, 6H).

Compound 343, [3-(Phenylureido)propyl][2-(4-chlorophenyl)ethyl](2-chlorobenzyl)ethylammonium iodide, can be obtained in an analogous manner to that described for compound 91 and contains the following characteristics: MS(ES⁺) mle 484 [M-I]⁺; ¹H NMR (400 MHz, CDCl₃) *δ* 8.23 (bs, 1H), 7.71 (dd, J = 7.6, 1.5 Hz, 1H), 7.48 (m, 4H), 7.40 (m, 1H), 7.22 (m, 2H), 7.18 (m, 4H), 7.09 (m, 1H), 6.93 (m, 1H), 4.80 (d, J = 2.2 Hz, 2H), 3.98 (m, 2H), 3.57-3.48 (m, 6H), 3.12 (m, 2H), 2.13 (m, 2H), 1.49 (t, J = 7.1 Hz, 3H).

Compound 351, [3-(Phenylureido)propyl][2-(4-chlorophenyl)ethyl](2,5-difluorobenzyl)ethylammonium iodide, can be obtained in an analogous manner to that described for compound 91 and contains the following characteristics: MS(ES⁺) m/e 486 [M-I]⁺; ¹H NMR (400 MHz, CDCl₃) *δ* 8,15 (bs, 1H), 7.43 (m, 3H), 7.18-7.10 (m. 7H). 6.92-6.84 (m, 3H), 4.75 (d, J = 13.9 Hz, 1H), 4.69 (d, J = 13.9 Hz, 1H), 3.85 (m, 2H). 3.47-3.26 (m, 6H), 3.18 (m, 2H), 2.11 (m, 2H), 1.47 (t, J = 7.1 Hz, 3H).

Compound 352, [3-(Phenylureido)propyl][2-(4-chlorophenyl)ethyl](3-fluorobenzyl)ethylammonium iodide, can be obtained in an analogous manner to that described for compound 91 and contains the following characteristics: MS(ES⁺) m/e 470 [M-I]⁺; ¹H NMR (400 MHz, CDCl₃) *δ* 8.14 (bs, 1H), 7.42 (m, 4H), 7.34 (d, J = 7.8 Hz, 1H), 7.23 (m, 1H), 7.17 (m, 6H), 6.91 (m, 1H), 6.77 (m, 1H), 4.73 (d, J = 13.7 Hz, 1H), 4.67 (d, J = 13.7 Hz, 1H), 3.74 (m, 2H), 3.45-3.11 (m, 8H), 2.08 (m, 2H), 1.45 (t, J = 6.8 Hz, 3H).

Compound 394, [3-(4-Cyanophenylureido)propyl][2-(3-chlorophenyl)ethyl][2-(2-methoxyehtoxy)ethyl] ethylammonium iodide, can be obtained in an analogous manner to that described for compound 91 and contains the following characteristics: MS(ES⁺) m/e 487 [M-I]⁺; ¹H NMR (400 MHz, CDCl₃) *δ* 8.62 (bs, 1H). 7.59 (d, J = 8.8 Hz, 2H), 7.40 (d, J = 9.1 Hz, 2H), 7.13 (m, 3H), 7.06 (m, 1H), 6.96 (t, J = 6.1 Hz, 1H), 3.91 (m, 2H), 3.77 (dd, J = 11.2, 5.9 Hz, 2H), 3.66-3.35 (m, 12H), 3.23 (s, 3H), 3.07 (t, J = 8.8 Hz, 2H), 1.92 (m, 2H), 1.37 (t, J = 7.1 Hz, 3H).

Compound 438, [3-(4-Methoxyphenylureido)propyl][2-(3-chlorophenyl)ethyl][2-(2-methoxyehtoxy)ethyl] ethylammonium iodide, can be obtained in an analogous manner to that described for compound 91 and contains the following characteristics: MS(ES⁺) m/e 492. [M-I]⁺; ¹H NMR (400 MHz, CDCl₃) *δ*7.81 (br, 1H), 7.30 (d, J = 9.0 Hz, 2H), 7.14 (m, 3H), 7.09 (m, 1H), 6.68 (m, 3H), 3.89 (m, 2H), 3.76 (m, 2H), 3.66 (s, 3H), 3.59 (m, 4H), 3.46-3.37 (m, 8H), 3.23 (s, 3H), 3.03 (m, 2H), 1.91 (m, 2H), 1.34 (t, J = 7.1 Hz, 3H).

Compounds 294, 295, 297, 299-301, 303-308, 310-314, 317-319, 321, 324-334, 337-342, 344-350, 353-393, 395-437, 439-453 can be obtained in an analogous manner to that of Compound 91.

### Example 6. Synthesis of Active Compounds by Solid Phase Synthesis

The following synthesis is depicted in Scheme 6.

Step 1: ***Reductive amination of the linker to the resin.*** Novabiochem 2-(4-formyl-3-methoxy)phenoxyethyl polystyrene resin (0.5 mmol/g, 100g, 50 mmol) was added to a 500 ml erlenmyer flask. 3-Chloropropylamine hydrochloride (35.03g, 0.3 mole), 100 ml of 1% HOAc in DMF, and DIEA (53 ml, 0.3 mole) were added to the flask: The reaction mixture was stirred for one hour, NaBH(OAc)₃ (0.3 mole) was added and the reaction was stirred for four hours.

The reaction mixture was poured into a 1000 ml sintered glass funnel and the solvent was removed by vacuum. DMF (500 ml) was added and the solution was mixed thoroughly for five minutes. A vacuum again removed the solvent. This wash process was repeated two times. The resin was then washed in this manner three times with MeOH, three times with DCM, and three times with MeOH. The final resin was dried under a vacuum until constant weight.

Step 2: ***Protection of linker.*** The resin prepared above (30g, 15mmole) was placed into a 250 ml roundbottom flask. To this flask, DCM was added until a thick slurry was obtained. DIEA (31.3 ml, 90 mmole) was added followed by the p-nitrobenzylchloroformate, which was added in 5g batches as a solid (19.4g, 45 mmole) while being stirred magnetically. The reaction was stirred for two hours. The reaction mixture was poured into a 1 L sintered glass funnel and a vacuum removed the solvent. The resin was resuspended in DCM and mixed thoroughly for five minutes before the solvent was again removed. This was repeated two more times with DCM, and three times with MeOH. The resin was dried under a vacuum until constant weight.

Step 3: ***Displacement of chlorine by amine**.* The protected resin prepared above was transferred to a 96 well polyfiltronics plate (80mg, 0.04 mmole per well). The plate was placed onto a vacuum block and the resin was washed into their wells with DMSO. The solvent was removed by vacuum. The plate was transferred onto a clamp and the bottom was sealed. To each of the wells with resin, a solution of TBAI (300 µl, 0.16 M) in DMSO and amine (R1-NH2, 0.26 mmole) were added. The plate was sealed from the top and placed into an oven at 80 °C for 48 hours.

The plate was undamped and placed onto the vacuum block where the solvent was removed by vacuum. The plate was placed over a catch tray and each well with resin received roughly 1.5ml of DMSO each with a squirt bottle. The solvent was allowed to drain by gravity, then the remaining solvent was removed by vacuum. This was repeated two more times with DMSO, three times with MeOH, three times with DCM, and three more times with MeOH. The plate was dried under a vacuum.

Step 4: ***Reductive amination of secondary amine**.* The plate was placed onto the vacuum block and the resin was washed down with a solution of 30% EtOH in DMF. The solvent was removed with a vacuum. The plate bottom was sealed with the clamp and the 30% EtOH in DMF (300µl) was added to each well with resin. Aldehydes (R2-CHO, 0.2 mmole) were added to their respective wells. The plate was sealed from the top and shaken for 2 hours. The plate was unclamped from the top and BAP (0.2 mmole) was added to each of the wells with resin. The plate was then reclamped and shaken for 48 hours. The plate was unclamped and a vacuum removed the solvent. Each well was washed three times with DMF, three times MeOH, and three times DCM.

Step 5: ***Deprotection of the p-nitrobenzyl carbamate**.* A solution of SnCl₂. dihydrate in DMF (2.0 M) was prepared. The plate was again clamped and to each of the wells with resin, this solution was added (0.5 ml). The top of the plate was sealed and was allowed to stand overnight. The plate was unclamped, and washed two times with DMF. This deprotection was repeated a second time. The final wash solvents were three times DMF, three times MeOH, three times DMF, two times MeOH, then three times DCM.

Step 6: ***Acylation of linker**.* To the deprotected plate, a solution of DIEA in THF (150 ul, 1.2 M) was added to all wells containing resin. These wells each received the respective isocyanates (R3-NCO, 0.09 mmole) in THF (150 µl). The plate was sealed and allowed to stand.for three hours. The plate was unclamped and washed with the following solvents: three times DCM, three times MeOH, three times DMF, three times MeOH, then three times DCM. The plate was dried under a vacuum.

Step 7: ***Isolation of Final Products**.* The dried plate was placed into the HCl gas cleavage apparatus. The system was flushed with nitrogen for ten minutes followed by a 10 minute flush with HCl gas. The system was sealed and the plate was allowed to sit for one hour in HCl gas. The system was recharged after the hour and the plate was allowed to sit for an additional hour. The system was flushed with nitrogen for ten minutes and the plate was removed. The plate was placed on a tarred 2 ml deepwell plate and the resin treated with a DCM wash (300µl). The solvent was allowed to drain by gravity and was followed by a MeOH wash (300µl). The process was repeated with a DCM and two MeOH washes. The collected filtrate was left out to dry overnight. The final material was placed into a desicator and was dried under a vacuum. The dried plate was weighed and the yield calculated; each well had an average weight of 12 mg.

### Example 7. Synthesis of 4-[[3-(4-Bromophenylureido),propy](1,2,3,4-tetrahydro-1-naphthyl)amino]butanamide (Compound 193)

The following synthesis is depicted in Scheme 7.

To a solution of methyl 4-[[3-(4-bromophenylureido)propyl](1,2,3,4-tetrahydro-1-naphthyl)amino]butylate (40 mg, 0.080 mmol) and formamide (11 mg, 0.24 mmol) in DMF (2 ml) was added sodium methoxide (0.5 M solution in MeOH, 112 µl, 0.056 mmol), and the mixture was stirred at 100 °C for 2.5 h. After adding water, the mixture was extracted with chloroform, washed with brine, dried over sodium sulfate, and filtered. The filtrate was concentrated under vacuum to dryness, and the residue was adsorbed on a plate of silica gel and then developed with 10% methanol/chloroform to afford 4-[[3-(4-bromophenylureido)propyl](1,2,3,4-tetrahydro-1-naphthyl)amino]butanamide (17 mg, 43%): MS(ES⁺) m/e 487 [M+H]⁺;¹H NMR (400 MHz, CDCl₃) *δ* 8.00 (br, 1H), 7.82 (br. 1H), 7.59 (d; J = 7.1 Hz, 1H), 7.31 (s, 4H), 7.12 (m, 2H), 7.05 (m, 1H), 6.08 (br, 1H), 5.64 (br, 1H), 3.99 (m, 1H), 3.41 (m, 1H), 3.22 (m, 1H), 2.70 (m, 1H), 2.61 (m, 1H). 2.55 (m, 1H), 2.39 (m, 3H), 2.12 (m, 2H), 1.96 (m, 3H), 1.87 (m, 1H), 1.64 (m, 4H).

### Example 8. Synthesis of 3-[[3-(4-Bromophenylureido)propyl](1.2,3.4-tetrahdyro-1-naphthyl)amino]-1-[(phenylsulfonyl)carbamoyl]propane (Compound 196)

The following synthesis is depicted in Scheme 8.

To a mixture of 4-[[3-(4-bromophenylureido)propyl](1,2,3,4-tetrahydro-1-naphthyl)amino]butanoic acid (20 mg, 0.041 mmol) and benzenesulfonamide (7.0 mg, 0.045 mmol) in CH₂Cl₂ (1 ml) were added WSC.HCl (8.6 mg, 0.045 mmol) and DMAP (5.5 mg, 0.045 mmol), and the mixture was stirred at RT for 18 h. The reaction mixture was purified by preparative normal phase HPLC using linear gradients of (A) chloroform and (B) methanol (2-4% B, in 0-2 min; 4-5% B, in 2-6 min; 5% B, in 6-12 min) at a flow rate of 12 ml/min. Fractions containing the major peak were pooled and concentrated to afford 3-[[3-(4-bromophenylureido)propyl](1,2,3,4-tetrahydro-1-naphthyl)amino]-1-[(phenylsulfonyl)carbamoyl]propane (5.4 mg, 21%): MS(ES⁺) m/e 627 [M+H]⁺; ¹H NMR (400 MHz, CDCl₃) *δ*8.27 (s, 1H), 8.05 (m, 2H), 7.50 (m, 3H), 7.44 (d, J = 8.8 Hz, 2H), 7.31 (d, J = 9.0 Hz, 2H), 7.23 (m, 3H), 7.15 (d, J = 7.0 Hz, 1H), 7.02 (m, 1H), 6.92 (m, 1H), 4.72 (m, 1H), 3.73 (m, 1H), 3.58 (m, 1H), 3.21 (m, 1H), 2.96 (m, 1H), 2.79-2.65 (m, 5H), 2.57 (m, 1H), 2.17 (m, 1H), 2.03 (m, 1H), 1.94-1.66 (m. 4H).

Compounds 197, 210 can be obtained in an analogous manner to that of Compound 196.

### Example 9. Synthesis of 4-[[3-(4-Bromophenylureido)propyl](1,2,3,4-tetrahydro-1-naphthyl)amino]-1-butanol (Compound 203)

The following synthesis is depicted in Scheme 9.

Lithium hydroxide monohydrate (11 mg, 0.26 mmol) was added to a solution of 4-[[3-(4-bromophenylureido)propyl](1,2,3,4-tetrabydro-1-naphthyl)amino]butyl acetate (67 mg, 0.13 mmol) in 10% water/methanol (1.1 ml). After stirring at RT for 16 h, additional lithium hydroxide monohydrete (22 mg, 0.52 mmol) was added. The reaction mixture was stirred at RT for 17 h, and then concentrated under vacuum to dryness. The residue was adsorbed on a plate of silica gel and then developed with 10% methanol/chloroform to afford 4-[[3-(4-bromophenylureido)propyl](1,2 ,3, 4-tetrahydro-1-naphthyl)amino] - -1 -butanol (48 mg, 77%): MS(ES⁺) m/e 476 [M+H]⁺; ¹H NMR (400 MHz, DMSO-*d6*) *δ*8.52 (br, 1H), 7.63 (d, J = 7.6 Hz, 1H), 7.36 (m, 4H), 7.11-7.03 (m, 3H), 6.10 (br, 1H), 4.33 (br,. 1H), 3.90 (m, 1H). 3.37 (m, 2H), 3.13 (m, 1H), 3.02 (m, 1H), 2.67 (m, 2H), 2.54-2.47 (m, 4H), 1.93 (m, 2H), 1.58 (m, 4H), 1.44 (m, 4H).

Compounds 205, 216 can be obtained in an analogous manner to that of Compound 203.

### Example 10. Synthesis of 3-[[3-(4-Bromophenylureido)propyl](1,2,3,4-tetrahydro-1-naphthyl)amino]-1-(1H-tetrazol-5-yl)propane (Compound 218)

The following synthesis is depicted in Scheme 10.

Step 1: To a mixture of N-[3-(1,2,3,4-tetrahydro-1-naphthylamino)propyl]phthalimide (569 mg, 1.7 mmol), potassium carbonate (709 mg, 5.1 mmol) and potassium iodide (280 mg, 1.7 mmol) in CH₃CN (20 ml) was added 4-bromobutyronitrile (754 mg, 5.1 mmol). The mixture was refluxed under stirring for 18 h, and then filtered. The filtrate was concentrated under vacuum to dryness, and the residue was chromatographed on silica gel (eluting with 20% ethyl acetate/chloroform) to afford 4-[[(3-phthalimido)propyl](1,2,3,4-tetrahydro-1-naphchyl)amino]butyronitrile (336 mg, 50%): MS(ES⁺) m/e 402 [M+H]⁺; ¹H NMR (400 MHz, CDCl₃) *δ*7.82 (dd, J = 5.4, 2.9 Hz. 2H), 7.70 (m, 2H), 7.60 (d, J = 7.6 Hz, 1H), 7.11 (m, 1H), 7.02. (m, 2H), 3.95 (m, 1H), 3.78 (m, 1H), 3.59 (m, 1H), 2.73 (m, 2H), 2.64 (m, 1H), 2.51 (m, 4H), 2.35 (m, 1H), 1.98 (m, 2H), 1.83 (m, 4H), 1.63 (m, 2H)..

Step 2: To a solution of 4-[[(3-phthalimido)propyl](1,2,3,4-tetrahydro-1-naphthyl)amino]buryronitrile (336 mg, 0.84 mmol) in xylene (5 ml) was added trimethyltin azide (378 mg, 1.84 mmol). The mixture was stirrred at 115 °C for 18 h, and then filtered. The filtrate was concentrated under vacuum to dryness, and the residue was dissolved with 17% THF/CH₂Cl₂ (2.4 ml). To the solution was added 10 N NaOH solution (107 µl, 1.07 mmol). After srirring at RT for 30 min, triphenylmethyl chloride (297 mg, 1.07 mmol) was added, and the mixture was stirred at RT for 7 h, After adding water, the mixture was extracted with chloroform, washed with brine, dried over sodium sulfate, and filtered. Concentrating under vacuum gave 3-[[(3-phthalimido)propyl](1,2,3,4-tetrahydro-1-naphthyl)amino]-1-[1-(triphenylmethyl)tetrazol-5-yl]propane (125 mg, 22%) which was used in the next step without further purification.

Step 3: To a solution of 3-[[(3-phthalimido)propyl](1,2,3,4.-tetrahydro-1-naphthyl)amino]-1-[1-(triphenylmethyl)tetrazol-5-yl]propane (50 mg, 0.073 mmol) in EtOH (1 ml) was added hydrazine monohydrate (I7 µl, 0.36 mmol), and the mixture was stirred at RT for 3 h. The reaction mixture was concentrated under vacuum, and then water was added. The mixture was extracted with chloroform, washed with brine, dried over sodium sulfate, and filtered. To the filtrate was added 4-bromophenyl isocyanate (17 mg, 0.088 mmol), and the mixture was stirred at RT for 1 h. The reaction mixture was concentrated under vacuum to dryness, and the residue was adsorbed on a plate of silica gel and then developed with 3% methanol/chloroform to afford 3-[[3-(4-bromophenylureido)propyl](1,2,3,4-tetrahydro-1-naphthyl)amino]-1-[1-(triphenylmethyl)tetrazol-5-yl]propane (Compound 217, 33 mg, 60%): MS(ES⁺) m/e 756 [M+H]⁺;¹H NMR (400 MHz, CDCl₃) *δ*7.62 (m, 1H), 7.53 (br, 1H), 7.34 (m, 10H), 7.09 (m, 12H), 5.79 (br, 1H), 4.05 (m, 1H), 3.48 (m, 1H), 3.31 (m, 1H), 3.17 (m, 1H), 2.74 (m, 4H), 2.55 (m, 1H), 2.44 (m, 2H), 1.96 (m, 3H), 1.86 (m, 1H), 1.72 (m, 2H), 1.61 (m, 2H).

Step 4: To a solution of 3-[[3-(4-bromophenylureido)propyl](1,2,3,4-tetrahydro-1-naphthyl)amino]-1-[1-(triphenylmethyl)tetrazol-5-yl]propane (30 mg, 0.04 mmol) in THF (2 ml) was added 10 wt% HCl solution (1ml), and the mixture was stirred at RT for 4 h. After adding water, the mixture was extracted with chloroform, washed with brine, dried over sodium sulfate, and filtered. The filtrate was adsorbed on a plate of silica gel and then developed with 20% methanol/chloroform to afford 3-[[3-(4-bromophenylureido)propyl](1,2,3,4-tetrahydro-1-naphthyl)amino]-1-(1*H*-tetrazol-5-yl)propane (12 mg, 57%): MS(ES⁺) m/e 514 [M+H]⁺;¹H NMR (400 MHz, CD₃OD) *δ* 7.89 (s, 1H), 7.64 (m, 1H), 7.36 (d, J = 9.0 Hz, 2H), 7.27 (d, J = 9.0 Hz, 2H), 7.21 (m. 2H), 7.11 (m, 1H), 3.31 (m, 1H), 3.21 (t, J = 6.3 Hz, 2H), 3.14 (m, 1H), 3.08 (m, 3H), 2.94-2.72 (m, 4H), 2.24 (m, 1H), 2.17-2.01 (m, 3H), 1.98-1.80 (m, 3H), 1.71 (m, 1H).

Compounds 222 can be obtained in an analogous manner to that of Compound 218.

### Example 11. Synthesis of Methyl 4-[[3-[4-(carboxy)phenylureido]propyl](1,2,3,4-tetrahydro-1-naphthyl)amino]butylate (Compound 225)

The following synthesis is depicted in Scheme 11.

Lithium hydroxide monohydrate (2.5 mg, 0.060 mmol) was added to a solution of methyl 4-[[3-[4-(ethoxycarbonyl)phenylureido]propyl](1,2,3,4-tetrahydro-1 - naphthyl)amino]butylate (28 mg, 0.057 mmol) in 7% water/methanol (4.3 ml). After stirring at RT for 24 h, additional lithium hydroxide monohydrete (5 mg, 0.12 mmol) was added. The reaction mixture was stirred at RT for 24 h, and then concentrated under vacuum to dryness. The residue was adsorbed on a plate of silica gel and then developed with 10% methanol/chloroform to afford methyl 4-[[3-[4-(carboxy)phenylureido]propyl](1,2,3,4-tetrahydro-1-naphthyl)amino]butylate (12 mg, 51 %): MS(ES⁺) m/e 468 [M+H]⁺;¹H NMR (400 MHz, CDCl₃) *δ*9.85 (bs, 1H), 7.91 (m, 3H), 7.61 (m, 3H), 7.21 (m, 1H), 7.13 (m, 3H), 4.72 (m, 1H), 3.86 (s, 3H), 3.33 (m, 3H), 3.25 (m, 1H), 3.02 (m, 1H), 2.88 (m, 2H), 2.75 (m, 2H), 2.56 (m, 1H), 2.22 (m, 2H), 2.00 (m, 3H), 1.85 (m, 1H), 1.71 (m, 2H).

Compounds 235 can be obtained in an analogous manner to that of Compound 225 except for the use of compound 228 as starting material instead of methyl 4-[[3-[4-(ethoxycarbonyl)phenylureido]propyl](1,2,3,4-tetrahydro-1-naphthyl)amino]butylate.

### Example 12 Synthesis of 4-[[3-[4-(Ethoxycarbonyl)phenylureido]propyl(1,2,3,4-tetrahydro-1-naphthyl)amino]butanoic acid (Compound 228)

The following synthesis is depicted in Scheme 12

Step 1: To a solution of N-[3-(1,2,3,4-tetrahydro-1-naphthylamino)propyl]phthalimide (200 mg, 0.60 mmol) in MeOH (10 ml) were added succinic semialdehyde (15 wt. % solution in water, 0.45 ml, 0.72 mmol), HOAc.(41 µl. 0.72 mmol) and NaBH₃CN (45 mg, 0.72 mmol), and the mixture was stirred at RT for 2.5 h. After adding water, the mixture was extracted with chloroform, washed with water and brine, dried over sodium sulfate, and filtered. The filtrate was concentrated under vacuum to dryness, and the residue was chromatographed on silica gel (eluting with 5 % methanol/chloroform) to afford 4-[[(3-phthalimido)propyl](1,2,3,4-tetrahydro-1-naphthyl)amino]butanoic acid (207 mg, 82%): MS(ES⁺) m/e 421. [M+H]⁺; ¹H NMR (400 MHz, CDCl₃) *δ*7.83 (dd, J = 5.6, 2.9 Hz, 2H), 7.72 (m, 2H), 7.65 (d, J = 7.6 Hz, 1H), 7.15 (m, 1H), 7.08 (m, 1H), 7.00 (d, J = 7.3 Hz, 1H), 4.28 (m, 1H), 3.75 (m, 1H), 3.65 (m, 1H), 2.80-2.55 (m, 6H), 2.47 (m, 1H), 2.30 (m, 1H), 2.06-1.86 (m, 5H), 1.74-1.66 (m, 3H).

Step 2: To a solution of 4-[[(3-phthalimido)propyl](1,2,3,4-tetrahydro-1-naphthyl)amino]butanoic acid (137 mg, 0.32 mmol) in EtOH (5 ml) was added hydrazine monohydrate (63 µl, 1.3 mmol), and the mixture was stirred at RT for 4 h. The reaction mixture was concentrated under vacuum, and then water was added. The mixture was extracted with chloroform, washed with water and brine, dried over sodium sulfate, and filtered. To the filtrate was added 4-(ethoxycarbonyl)phenyl isocyanate (62 mg, 0.32 mmol), and the mixture was stirred at RT for 30 min. The reaction mixture was concentrated under vacuum to dryness, and the residue was adsorbed on a plate of silica gel and then developed with 17% methanol/chloroform to afford 4-[[3-[4-(ethoxycarbonyl)phenylureido]propyl](1,2,3,4-tetrahydro-1-naphthyl)amino]butanoic acid (25 mg, 16%): MS(ES⁺) m/e 482 [M+H]⁺;¹H NMR (400 MHz, CDCl₃) *δ*9.86 (s, 1H), 8.80 (br, 1H), 7.91 (m, 3H), 7.61 (m, 3H), 7.21 (m, 1H), 7.12 (m, 2H), 4.70 (t, J = 7.6 Hz, 1H), 4.33 (q, J = 7.1 Hz, 2H), 3.32 (m, 2H), 3.23 (m, 1H), 3.03-2.84 (m, 3H), 2.74 (m, 2H), 2.55 (m, 1H), 2.21 (m, 2H), 2.02-1.78 (m, 5H), 1.70 (m, 2H), 1.37 (t, J = 7.1 Hz, 3H).

Compound 229, 4-[[3-(4-Iodophenylureido)propyl](1,2,3,4-tetrahydro-1-naphthyl)amino]butanoic acid, can be obtained in an analogous manner to that described for compound 228 and contains the following characteristics: MS(ES⁺) m/e 536 [M+H]⁺; ¹H NMR (400 MHz, CDCl₃) *δ*9.50 (bs, 1H), 7.70 (br, 1H), 7.61 (d. J = 7.8 Hz. 1H), 7.50 (d, J = 7.8 Hz, 2H), 7.35 (d, J = 7.8 Hz, 2H), 7.22 (m, 1H), 7.14. (m, 3H), 4.69 (m, 1H), 3.30 (m, 2H), 3.22 (m, 1H), 2.99 (m, 1H), 2.87 (m, 2H), 2.75 (m, 2H), 2.53 (dd, J = 16.6, 7.3 Hz, 1H), 2.20 (m, 2H), 2.00-1.77 (m, 5H), 1.70 (m, 2H).

Compound 237, 4-[[3-[4-(Butoxycarbonyl)phenylureido]propyl](1.2,3,4-tetrahydro-1-naphoyl)amino]butanoic acid, can be obtained in an analogous manner to that described for compound 228 and contains the following characteristics: MS(ES⁺) m/e 510 [M+H]⁺: ¹H NMR (400 MHz, CDCl₃)*δ* 9.70 (br, 1H), 7.91 (d, J = 8.5 Hz; 2H), 7.72 (br, 1H), 7.62 (m, 3H), 7.20 (m, 3H), 7.13 (m, 1H), 4:71 (m, 1H), 4.27 (t, J.= 6.6 Hz. 2H). 3.33 (m, 2H), 3.26 (m, 1H), 3.01 (m, 1H), 2.88 (m, 2H), 2.75 (m, 2H), 2.54 (m, 1H), 2.20 (m. 2H), 2.01 (m, 4H), 1.84 (m, 1H), 1.73 (m, 4H), 1.47 (m, 2H), 0.97 (t, J = 7.3 Hz, 3H).

Compound 258, 4-[[3-(4-Bromophenylureido)propyl] [(1*R*)-1-(4-bromophenyl)ethyl]amino]butanoic acid, can be obtained in an analogous manner to that described for compound 228 and contains the following characteristics: MS(ES⁺) m/e 542 [M+H]⁺;¹H NMR (400 MHz, CDCl₃) *δ*9.29 (bs, 1H), 7.82 (br, 1H), 7.69 (br, 1H), 7.47 (d, J = 8.1 Hz, 2H), 7.40 (d, J = 8.5 Hz, 2H), 7.31 (d, J = 8.5 Hz, 2H), 7.22 (d, J = 8.1 Hz, 2H), 4.24 (m, 1H), 3.20 (m, 2H), 2.99 (m, 1H), 2.84 (m, 3H), 2.40 (m, 2H), 1.82 (m, 4H), 1.56 (d, J = 6.8 Hz, 3H).

Compound 269, 4-[[3-(4-Bromophenylureido)propyl][1-(4-fluorophenyl)ethyl]amino]butanoic acid, can be obtained in an analogous manner to that described for compound 228 and contains the following characteristics: MS(ES⁺) m/e 482 [M+H]⁺;¹H NMR (400 MHz, CDCl₃) *δ*9.47 (s, 1H), 7.68 (br, 1H), 7.43 (d, J = 9.0 Hz, 2H), 7.35 (m, 2H), 7.31 (d, J = 8.8 Hz, 2H), 7.05 (m, 2H), 6.92 (m, 1H), 4.30 (q, J = 6.8 Hz, 1H), 3.22 (m, 2H), 3.04 (m, 1H), 2.89 (m, 3H), 2.42 (m, 2H), 1.84 (m, 4H), 1.60 (d, J = 7.1 Hz, 3H).

Compound 272, 4-[[3-(4-Bromophenylureido)propyl][1-(4-chlorophenyl)ethyl]amino]butanoic acid, can be obtained in an analogous manner to that described for compound 228 and contains the following characteristics: MS(ES⁺) m/e 498 [M+H]⁺;¹H NMR (400 MHz, CDCl₃) *δ*9:38 (bs, 1H), 7.60 (br, 1H), 7.43 (d, J = 8.8 Hz, 2H), 7.31 (m, 6H), 7.20 (m, 1H), 4.26 (q, J = 7.1 Hz, 1H), 3.21 (m, 2H), 3.02 (m, 1H), 2.87 (m, 3H), 2.43 (m, 2H), 1.83 (m, 4H), 1.59 (d, J = 7.1 Hz, 3H).

Compound 293, 4-[[3-(4-Bromophenylureido)propyl][(1S)-1-(4-bromophenyl)ethyl]amino]butanoic acid, can be obtained in an analogous manner to that described for compound 228 and contains the following characteristics: MS(ES⁺) m/e 542 [M+H]⁺;¹H NMR (400 MHz, CDCl₃) *δ*9.37 (bs, 1H), 9.14 (br, 1H), 7.56 (br, 1H), 7.48 (d, J = 8.5 Hz, 2H), 7.43 (d, J = 8.8 Hz, 2H), 7.32 (d, J = 8.8 Hz, 2H), 7.23 (d, J = 8.5 Hz, 2H), 4.24 (m, 1H), 3.21 (m, 2H), 3.01 (m, 1H), 2.87 (m; 3H), 2.44 (m. 2H), 1.84 . (m, 4H), 1.58 (d, J = 6.8 Hz, 3H).

Compounds 236, 259-266,268, 270, 271, 273, 277-278 can be obtained in an analogous manner to that of Compound 228.

### Example 13 Synthesis of [3-(Phenylureido)propyl]bis[2-(4-chlorophenyl)ethyl]amine (Compound 238)

The following synthesis is depicted in Scheme 13.

Step 1: To a mixture of 4-chlorophenylacetic acid (500 mg, 3.0 mmol) and 2-(4-chlorophenyl)ethylamine (456 mg, 3.0 mmol) in DMF (50 ml) were added WSC.HCl (592 mg, 3.1 mmol), HOBt.H₂O (474 mg, 3.1 mmol) and triethylamine (0.43 ml, 3.1 mmol), and the mixture was stirred at RT for 18 h. After adding water, the mixture was extracted with ethyl acetate, washed with brine, dried over magnesium sulfate, and filtered. Concentrating under vacuum gave N-[2-(4-chlorophenyl)ethyl]-(4-chlorophenyl)acetamide (819 mg, 89%) which was used in the next step without further purification.

Step 2: To a solution of N-[2-(4-chlorophenyl)ethyl]-(4-chlorophenyl)acetamide (100 mg, 0.33 mmol) in THF (2 ml) was added borane-methyl sulfide complex (2.0 M solution in THF, 1.6 ml, 3.2 mmol), and the mixture was stirred at 70 °C for 1.5 h. After adding 1 N HCl solution (4 ml), the mixture was stirred at RT for 1 h. After adding 5 wt% NaOH solution (4 ml), the mixture was extracted with chloroform, dried over magnesium sulfate, and filtered. The filtrate was concentrated under vacuum to dryness, and the residue was chromatographed on silica gel (eluting with 10% methanol/chloroform) to afford bis[2-(4-chlorophenyl)ethyl]amine (48 mg, 50%): MS(ES⁺) m/e 294 [M+H]⁺.

Step 3: To a mixture of bis[2-(4-chlorophenyl)ethyl]amine (48 mg, 0.16 mmol), potassium carbonate (44 mg, 0.32 mmol) and potassium iodide (26 mg, 0.16 mmol) in CH₃CN (2 ml) was added N-phenylcarbamoyl-3-bromopropylamine (215 mg, 0.64 mmol) in DMF (2 ml). The mixture was stirred at 80 °C for 18 h, and then concentrated under vacuum to dryness. The residue was adsorbed on a plate of silica gel and then developed with 10% methanol/chloroform to afford [3-(phenylureido)propyl]bis[2-(4-chlorophenyl)ethyl]amine (10 mg, 13%): MS(ES⁺) m/e 470 [M+H]⁺; ¹H NMR (400 MHz, CDCl₃) *δ*7.24 (m, 9H), 7.03 (m, 5H), 6.59 (br, 1H), 3.24 (t, J = 6.3 Hz, 2H), 2.87 (m, 1H), 2.76 (m, 1H), 2.73-2.61 (m. 8H), 1.64 (m, 2H).

### Example 14. Synthesis of [3-(Phenylureido)propyl][2-(4-chlorophenyl)ethyl]bis(4-methylbenzyl)ammonium iodide (Compound 296)

The following synthesis is depicted in Scheme 14.

To a mixture of N-phenylcarbamoyl-N'-[2-(4-chlorophenyl)ethyl]-1,3-diaminopropane (80 mg, 0.24 mmol) and potassium carbonate (100 mg, 0.72 mmol) in CH₃CN (2 ml) was added 4-methylbenzyl bromide (134 mg, 0.72 mmol). The mixture was refluxed under stirring for 1.5 h, and then filtered. The filtrate was concentrated under vacuum to dryness, and the residue was adsorbed on a plate of silica gel and then developed with 33% methanol/chloroform to afford [3-(phenylureido)propyl][2-(4-chlorophenyl)ethyl]bis(4-methylbenzyl)ammonium iodide (101 mg, 68%): MS(ES⁺) m/e 540 [M-Br]⁺; ¹H NMR (400 MHz, CDCl₃) *δ* 8.13 (s, 1H), 7.50 (d, J = 7.6 Hz, 2H). 7.40 (d, J = 8.1 Hz, 4H), 7.19 (m. 8H), 7.07 (d, J = 8.5 Hz, 2H), 6.94 (t, J = 7.3 Hz, 1H). 6.86 (m, 1H), 4.81 (d, J = 13.2 Hz, 2H), 4.57 (d, J = 13.2 Hz, 2H), 3.75 (m, 2H), 3.35 (m, 2H), 3.25 (m, 2H), 3.19 (m, 2H), 2.11 (m, 2H), 1.60 (s, 6H).

### Example 15. Synthesis of [3-(4-Bromophenylureido)propyl][(1S)-1-phenylethlyl][3-(carboxy)propyl]ethylammonium trifluoroacetate (Compound 315)

The following synthesis is depicted in Scheme 15.

Lithium hydroxide monohydrate (10 mg, 0.24 mmol) was added to a solution of [3-(4-bromophenylureido)propyl][(1*S*)-1-phenylethyl][3-(methoxycarbonyl)propyl]ethylammonium iodide (24 mg, 0.05 mmol) in 10% water/methanol (3.3 ml). After stirring at RT for 4.5 h, the reaction mixture was concentrated under vacuum to dryness. The residue was purified by preparative reverse phase HPLC using linear gradients of (A) 0.05% TFA/H₂O and (B) 0.05% TFA/CH₃CN (20-80% B, in 0-15 min; 80% B, in 15-18 min) at a flow rate of 3 ml/min. Fractions containing the major peak were pooled and concentrated to afford [3-(4-bromophenylureido)propyl][(1S)-1-phenylethyl][3-(carboxy)propyl]ethylammonium trifluoroacetate (6 mg, 20%): MS(ES⁺) m/e 492 [M-CF₃COO]⁺;¹H NMR (400 MHz, CD₃OD) δ7.63 (m, 2H), 7.45 (m, 3H), 7.39 (d, J = 9.0 Hz, 2H), 7.31 (d, J = 9:0 Hz, 2H), 3.47 (m, 3H), 3.38 (m, 4H), 3.26 (m, 2H), 2.41 (m, 2H), 1.96 (m, 4H), 1.83 (d, J = 6.8 Hz, 3H), 1.30 (m, 3H).

Compound 316, [3-(4-Bromophenylureido)propyl][(1*R*)-1-phenylethyl][3-(carboxy)propyl]ethylammonium trifluoroacetate, can be obtained in an analogous manner to that described for compound 315 and contains the following characteristics: MS(ES⁺) m/e 492 [M-CF₃COO]⁺;¹H NMR (400 MHz, CD₃OD) *δ*7.63 (m, 2H), 7.45 (m, 3H), 7.39 (d, J.= 9.0 Hz, 2H), 7.31 (d, J = 8.8 Hz, 2H), 3.47 (m, 3H), 3.37 (m. 4H), 3.26 (m. 2H), 2.41 (m, 2H), 1.96 (m, 4H), 1.83 (d, J = 6.8 Hz, 3H), 1.31 (m. 3H).

### Example 16. Synthesis of [3-(Phenylureido)propyl][2-(4-chlorophenyl)ethyl][4-(carboxy)benzyl]ethylammonium iodide (Compound 322)

The following synthesis is depicted in Scheme 16.

Lithium hydroxide monohydrate (4 mg, 0.095 mmol) was added to a solution of [3-(phenylureido)propyl][2-(4-chlorophenyl)ethyl][4-(methoxycarbonyl)benzyl]ethylammonium iodide (28 mg, 0.044 mmol) in 10% water/methanol (1.3 ml). After stirring at RT for 26 h, the reaction mixture was concentrated under vacuum to dryness. The residue was adsorbed on a plate of silica gel and then developed with 33 % methanol/chloroform to afford [3-(phenylureido)propyl][2-(4-chlorophenyl)ethyl][4-(carboxy)benzyl]ethylammonium iodide (19 mg, 70%): MS(ES⁺) m/e 496 [M-I]⁺; ¹H NMR (400 MHz, CD₃OD) *δ*8.01 (d, J = 8.3 Hz, 2H), 7.53 (d, J = 8.3 Hz, 2H), 7.38 (m, 2H), 7.30 (m, 4H), 7.24 (m, 2H), 6.97 (m, 1H), 4.59 (s, 2H), 3.31 (m, 8H), 3.15 (m, 2H), 2.15 (m, 2H), 1.48 (t, J = 7.1 Hz, 3H).

### Abbreviations:

- EtOH: ethanol
- CH₂Cl₂: dichloromethane
- DMSO: dimethylsulfoxide
- MeOH: methanol
- HOAc: acetic acid
- DIEA: diisopropylethylamine
- DCM: dichloromethane
- DMF: N.N-dimethylformamide
- BAP: borane and pyridine
- TBAI: tetrabutylammonium iodide
- SnCh: tin chloride
- Fmoc: 9H-9-fluorenylmethoxycarbonyl
- Asp: aspartic acid residue
- tBu: *tert*-butyl
- WSC: 1-[3-(dimethylamino)propyl]-3-ethylcarbodiimide
- HOBt: 1-hydroxybenzotriazole
- THF: tetrahydrofuran
- TFA: trifluoroacetic acid
- DMAP .: 4-(dimethylamino)pyridine

Table 1a and 1b list a variety of compounds that can be synthesized by using one of the methods described above.

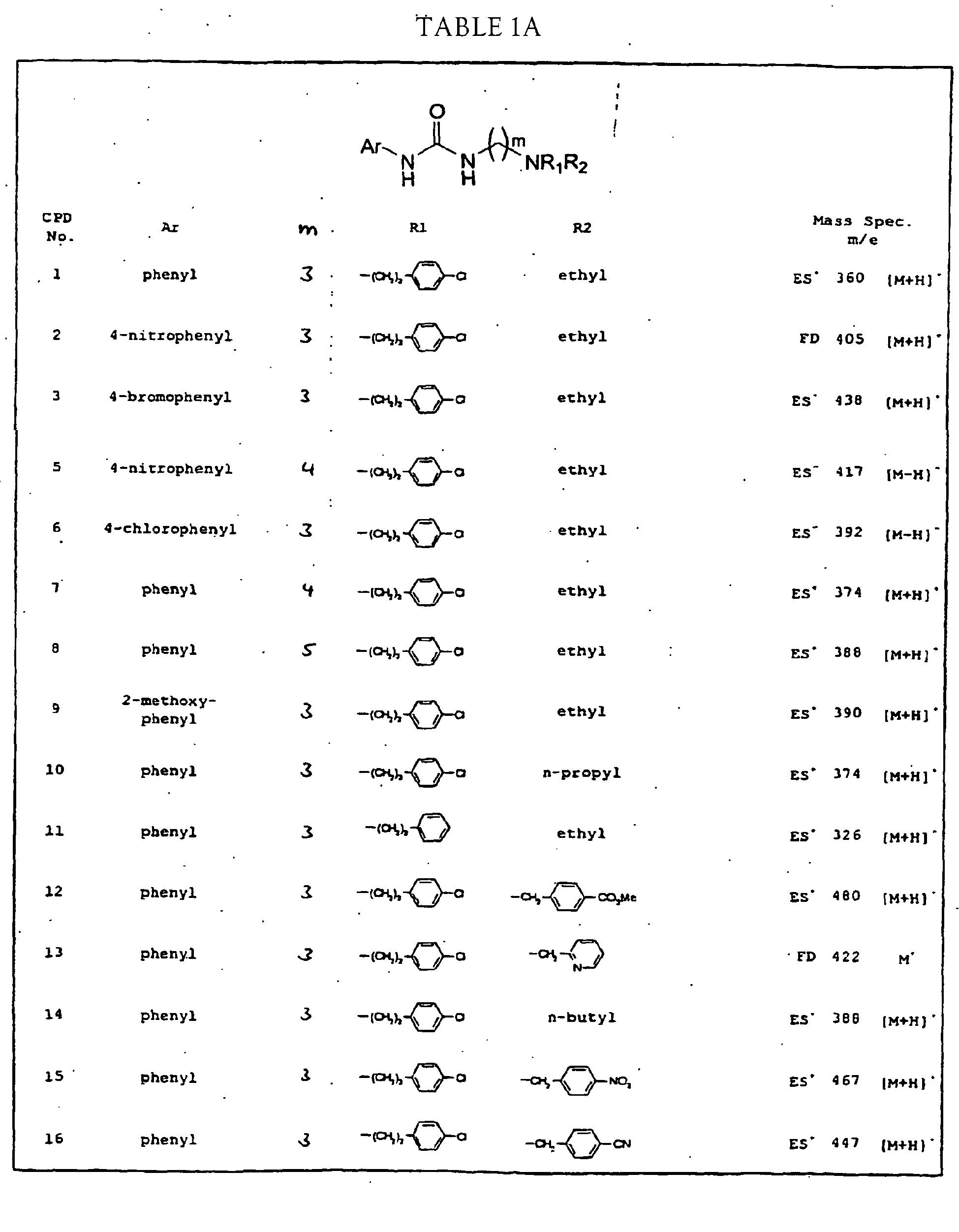

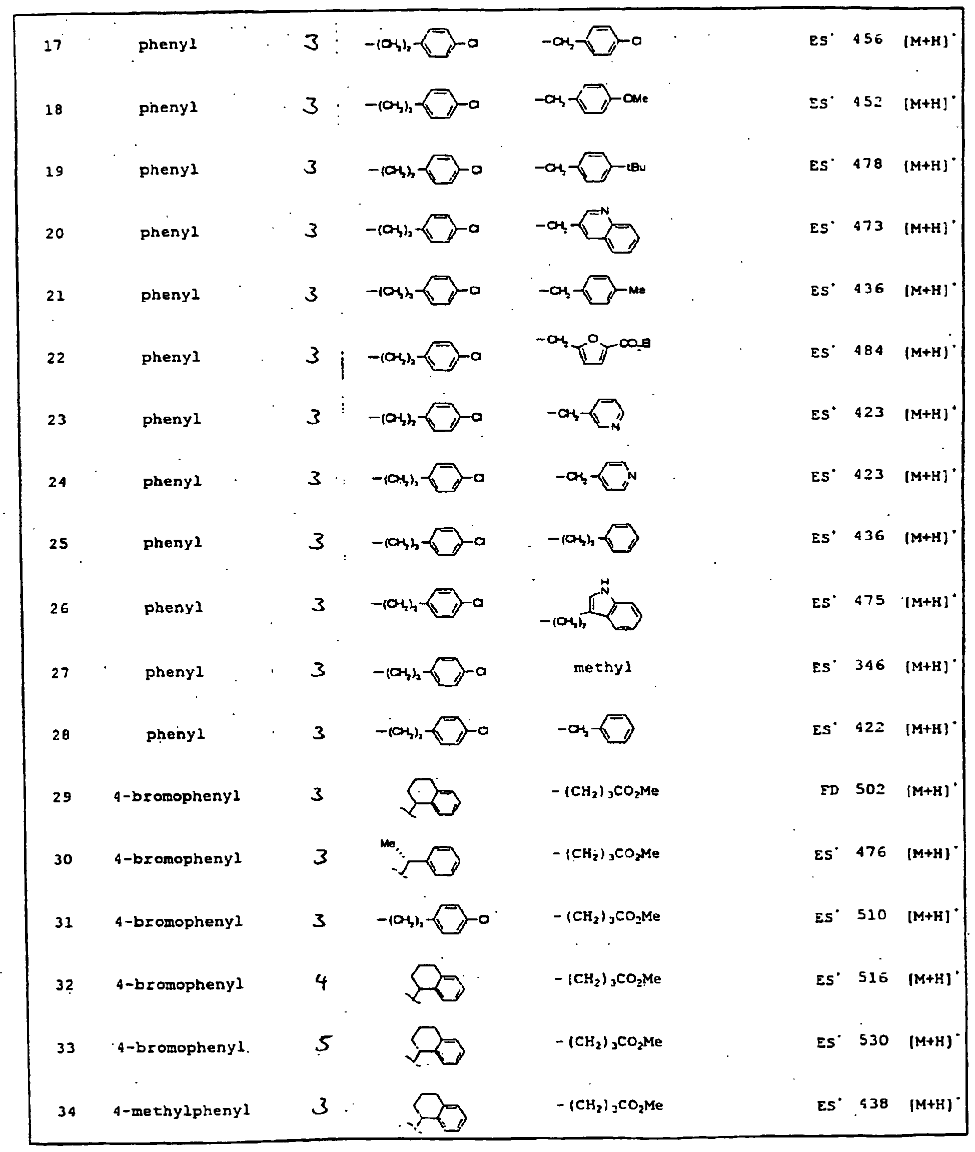

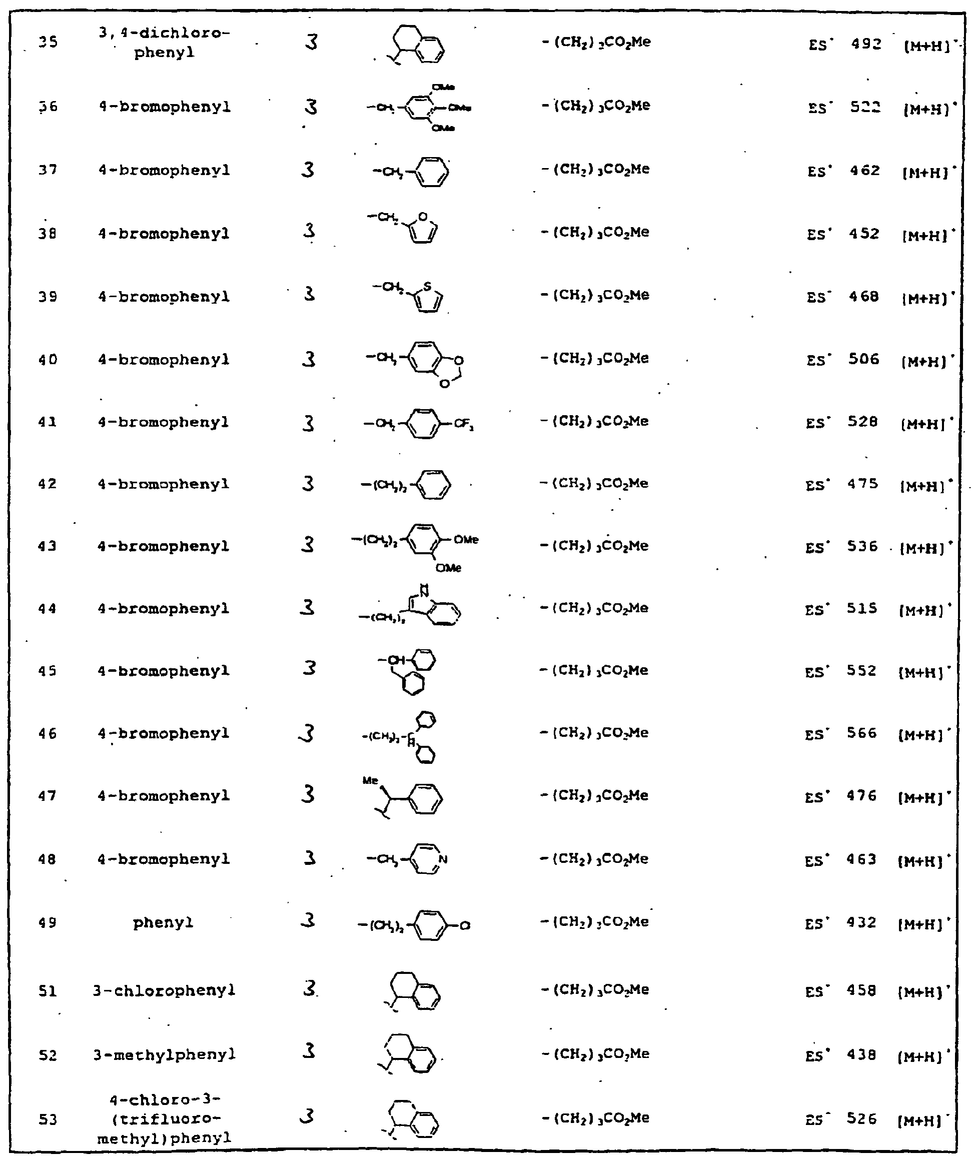

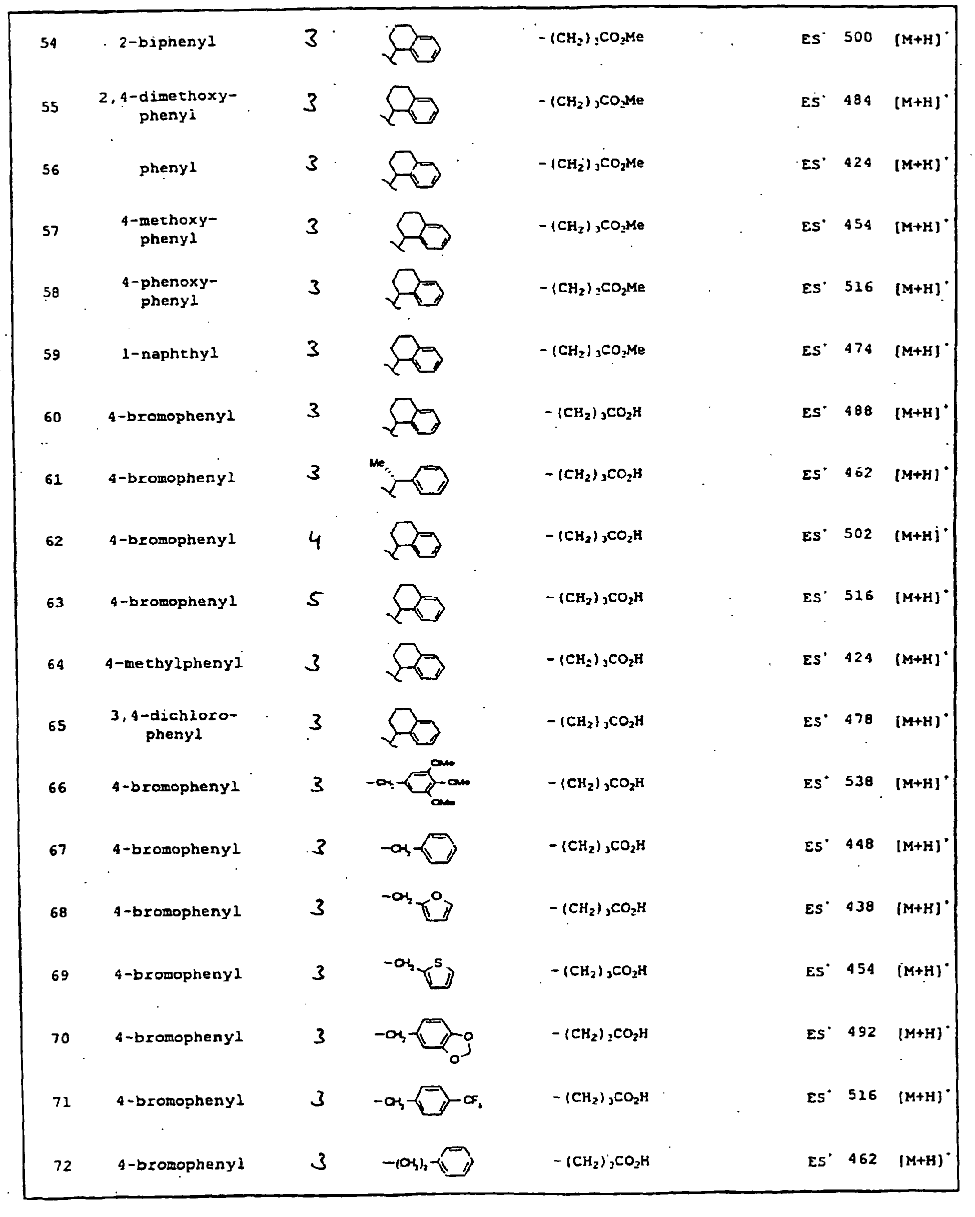

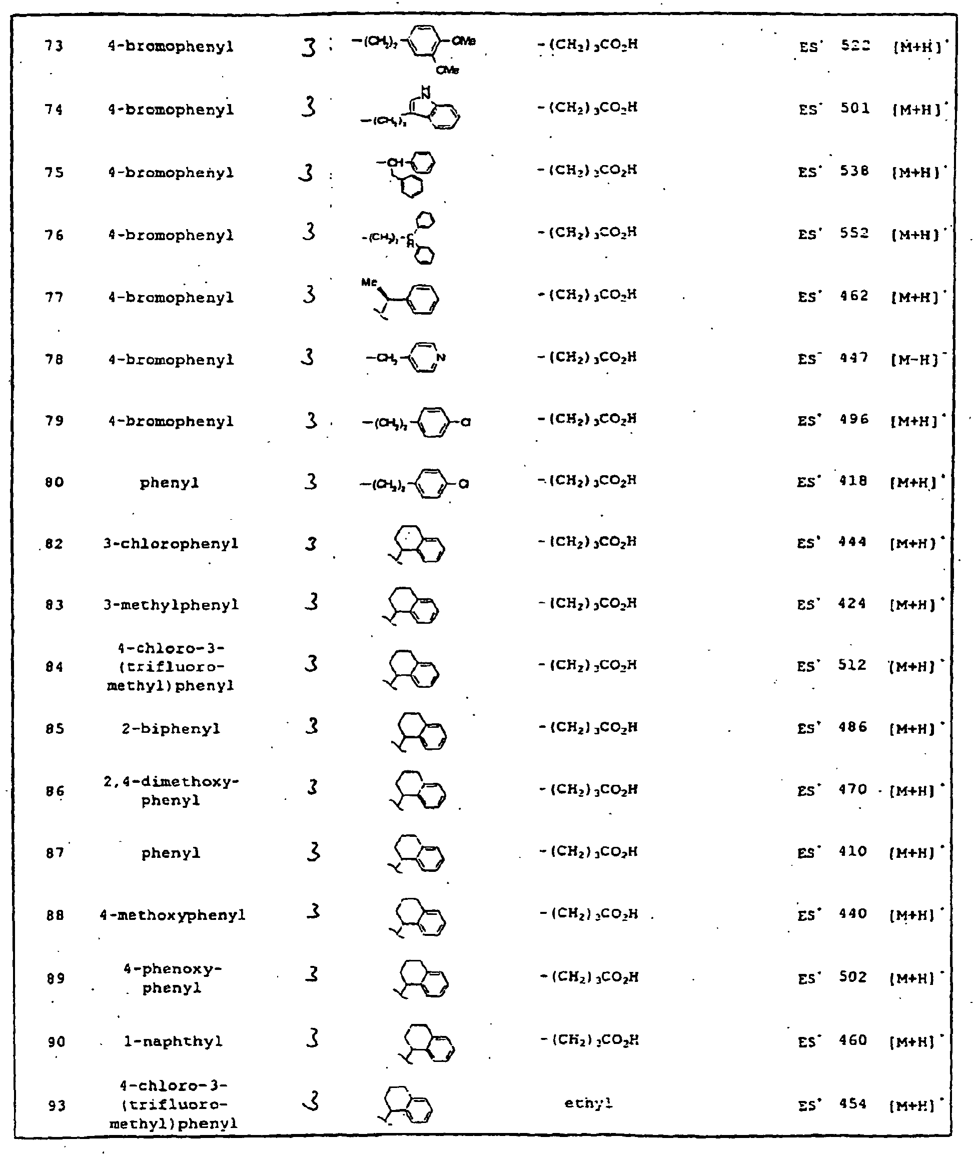

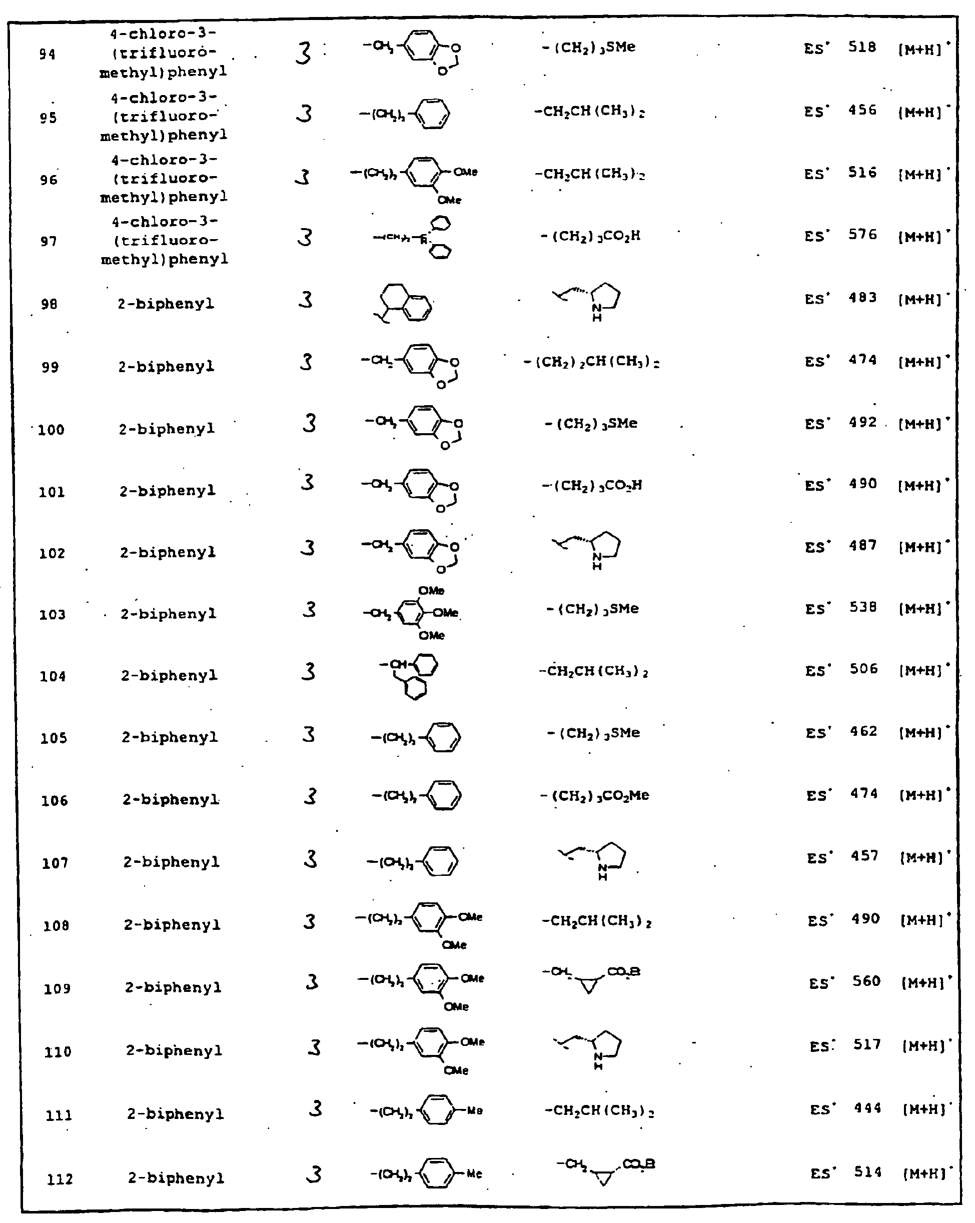

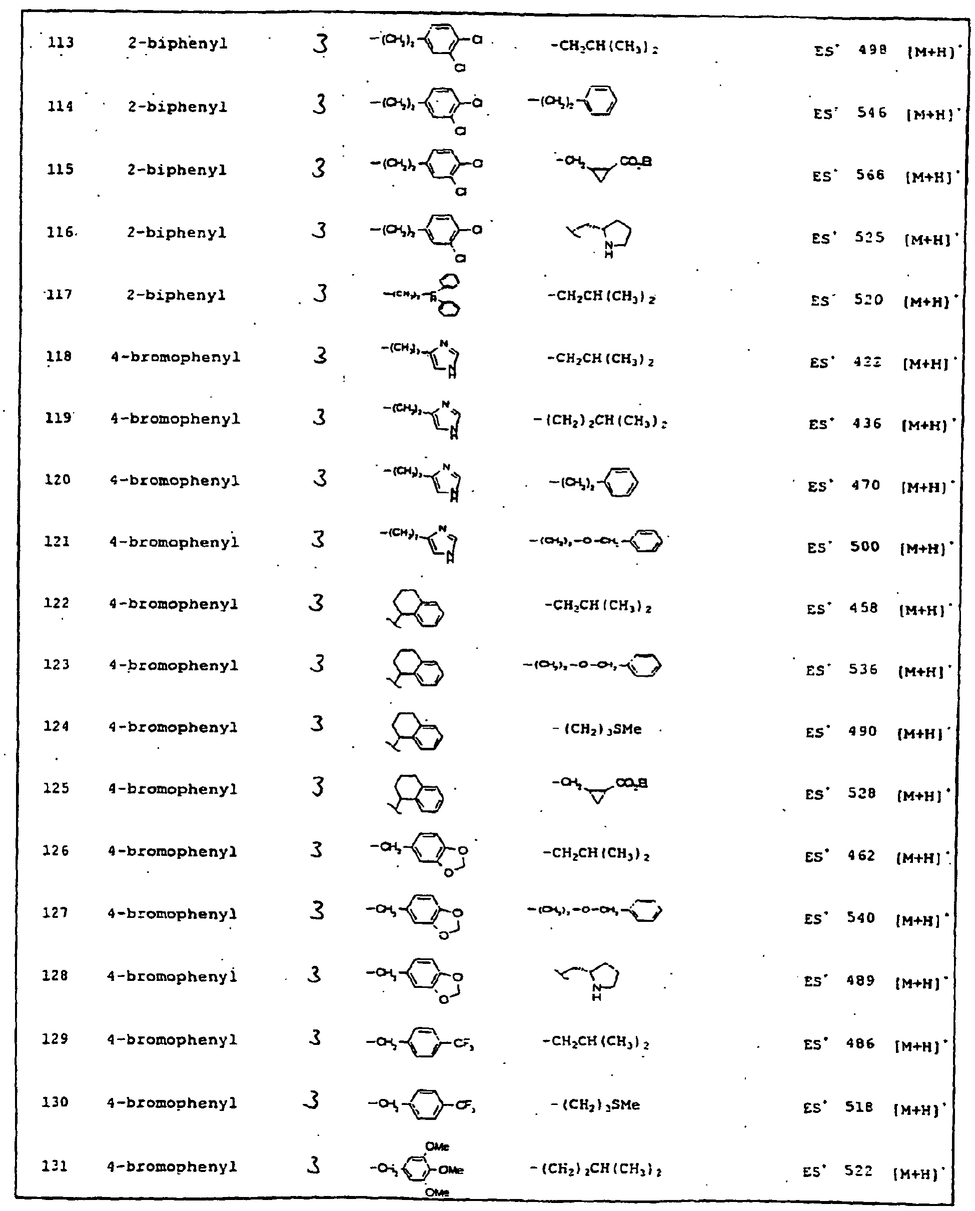

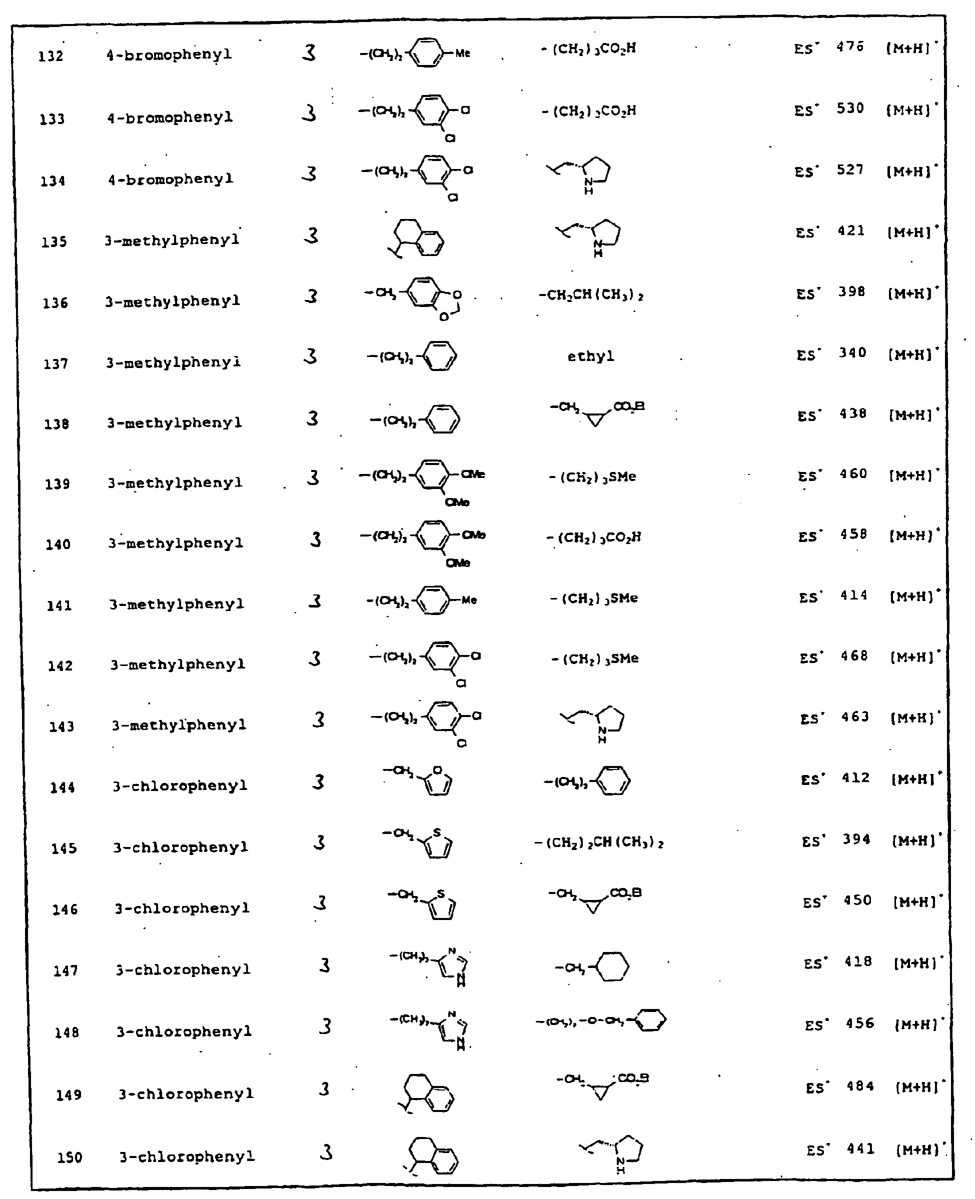

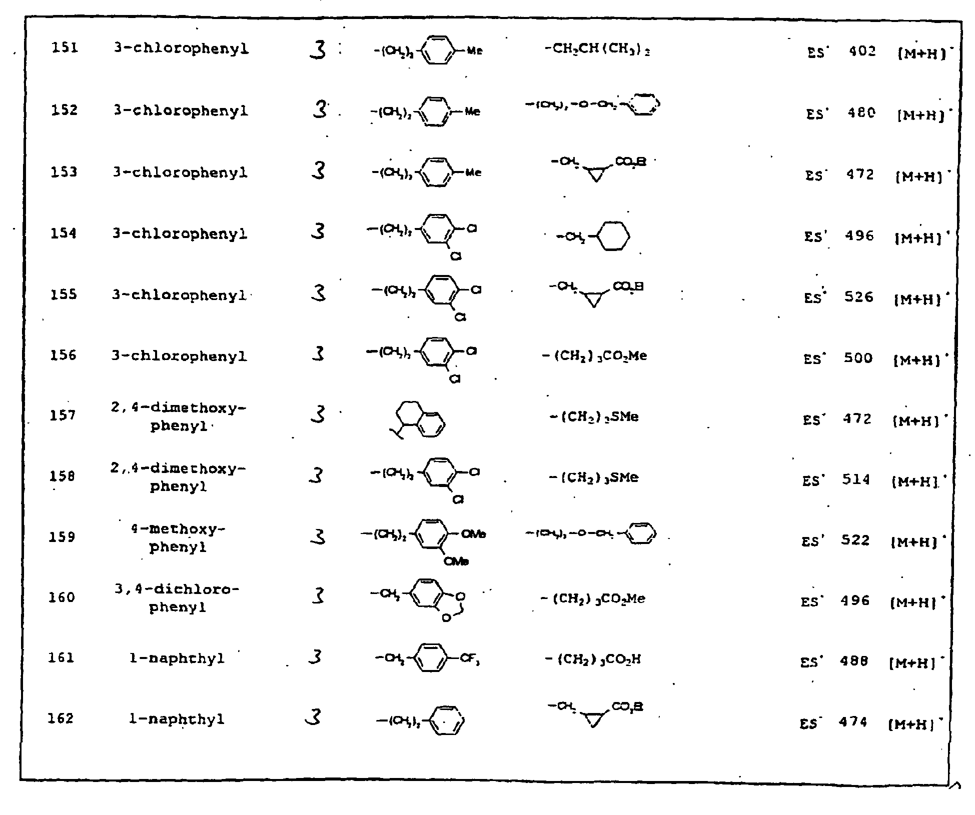

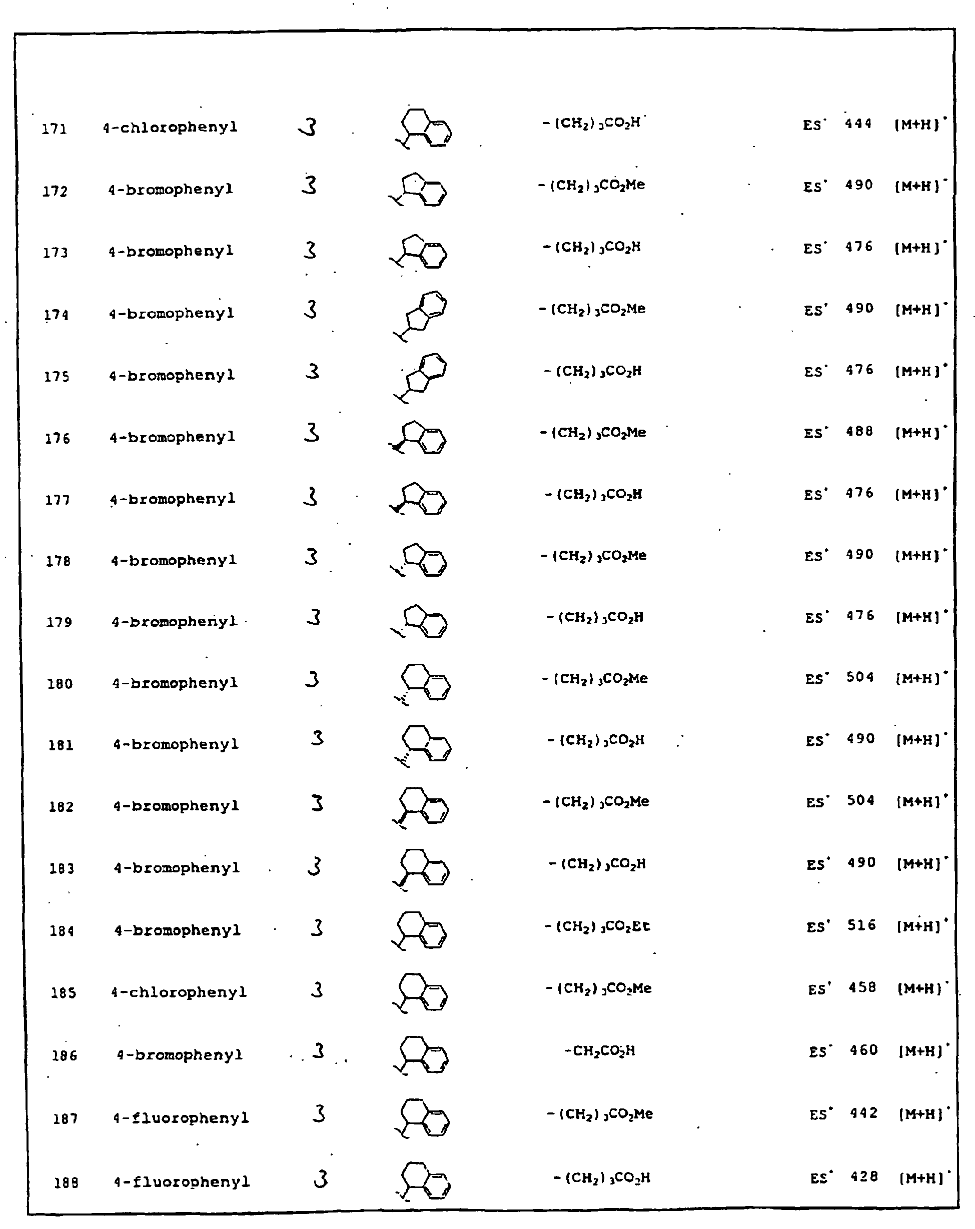

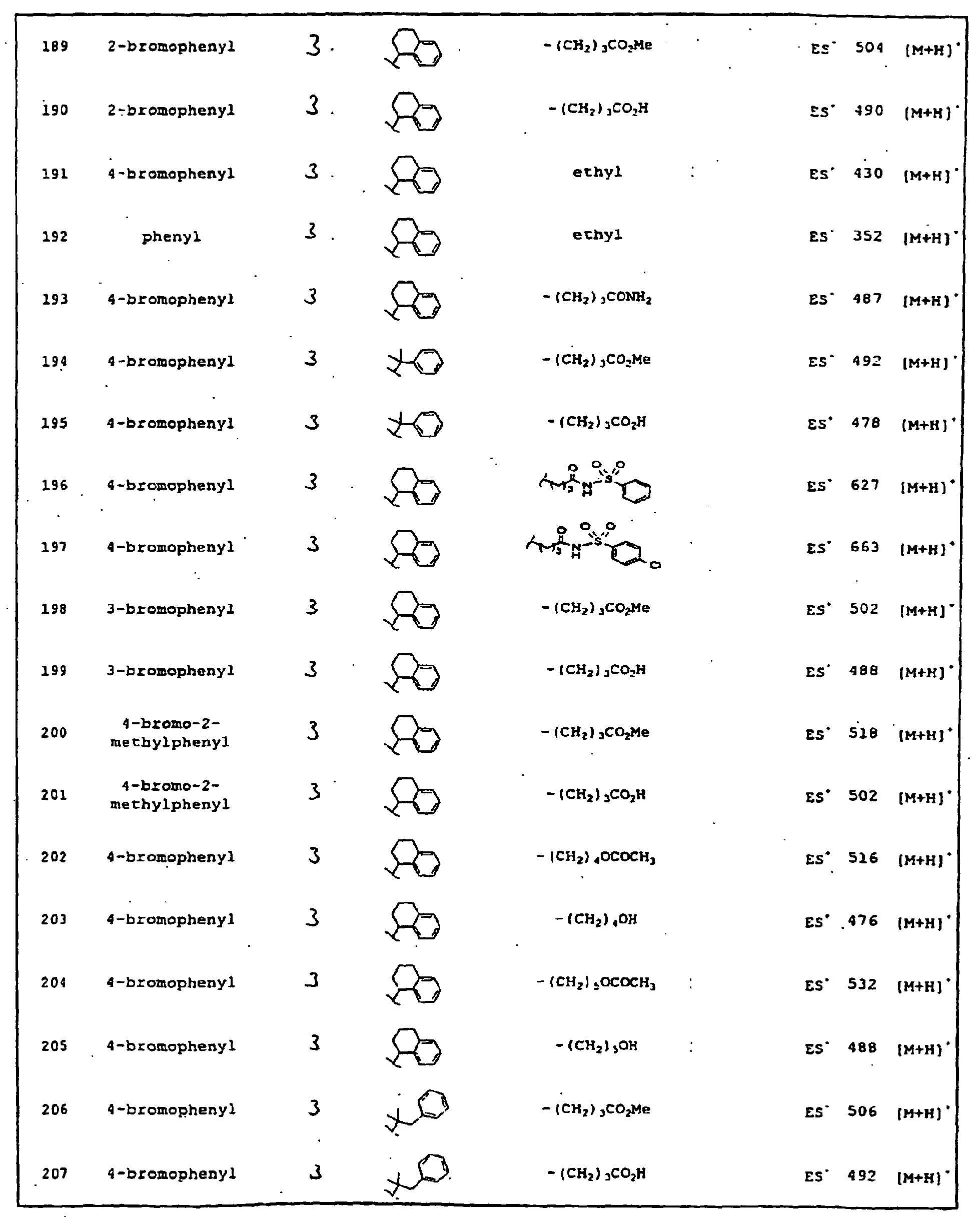

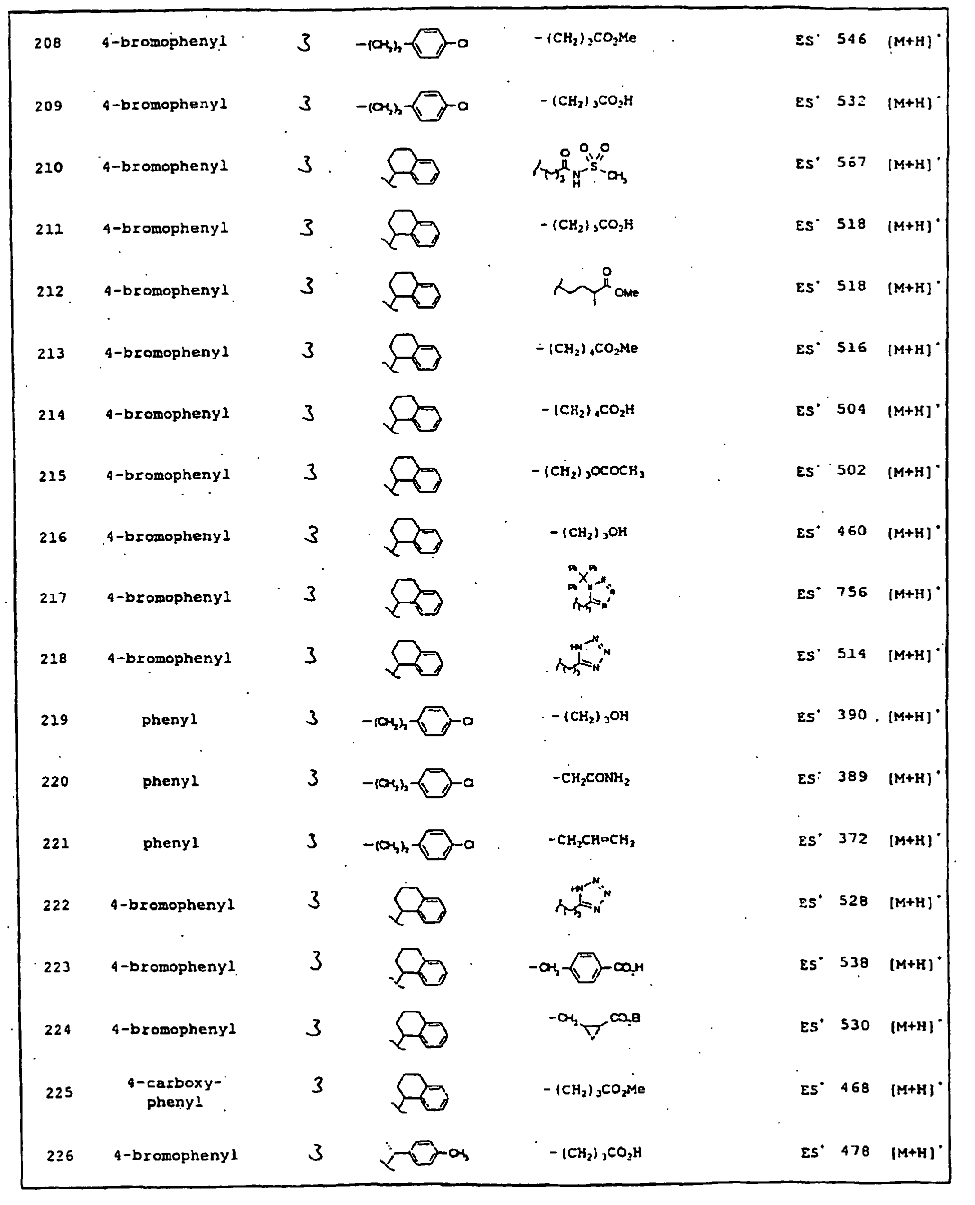

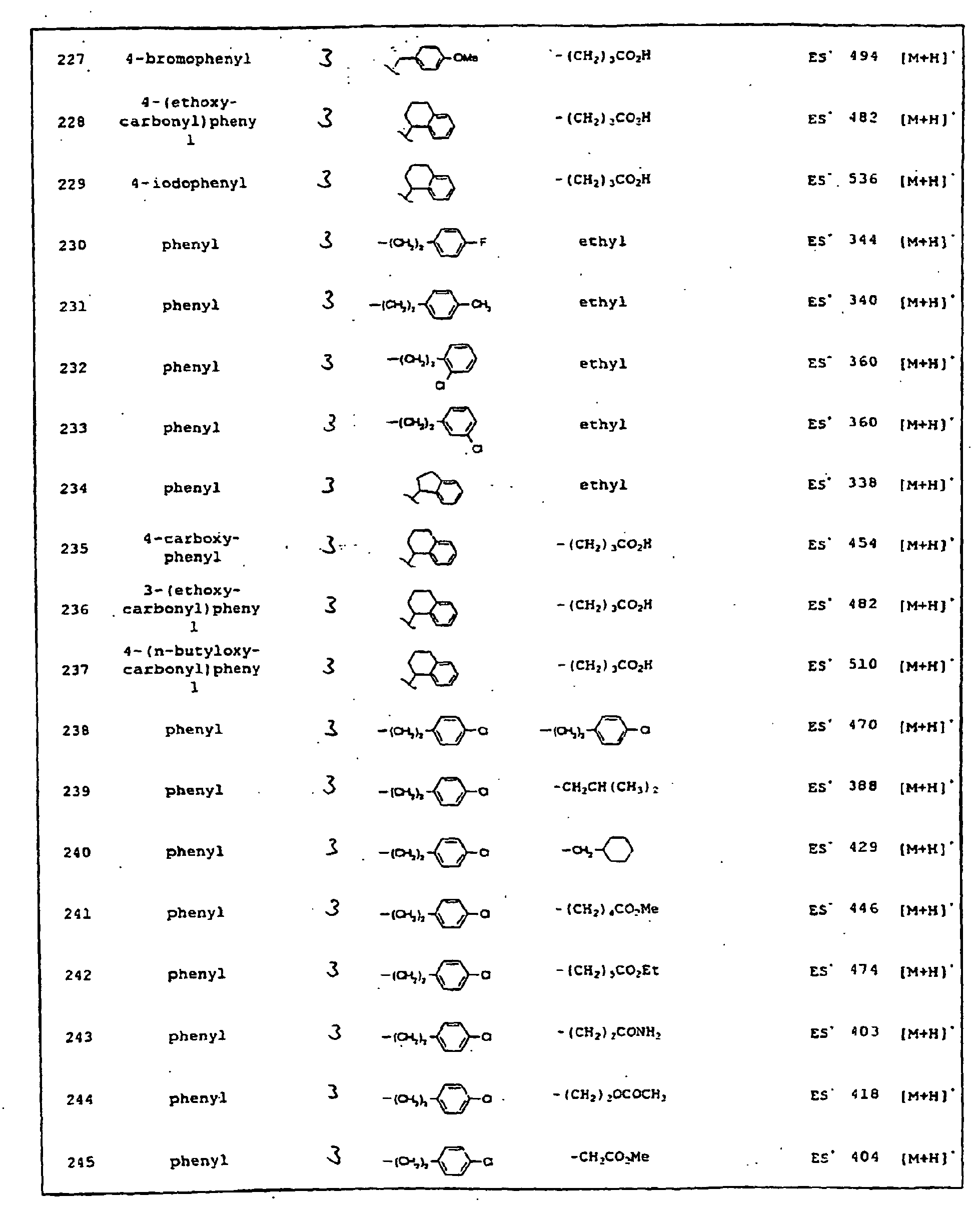

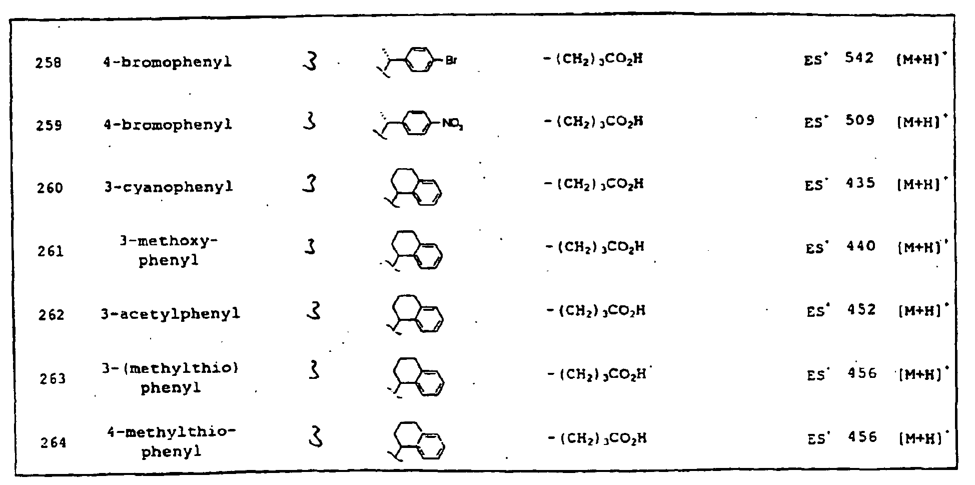

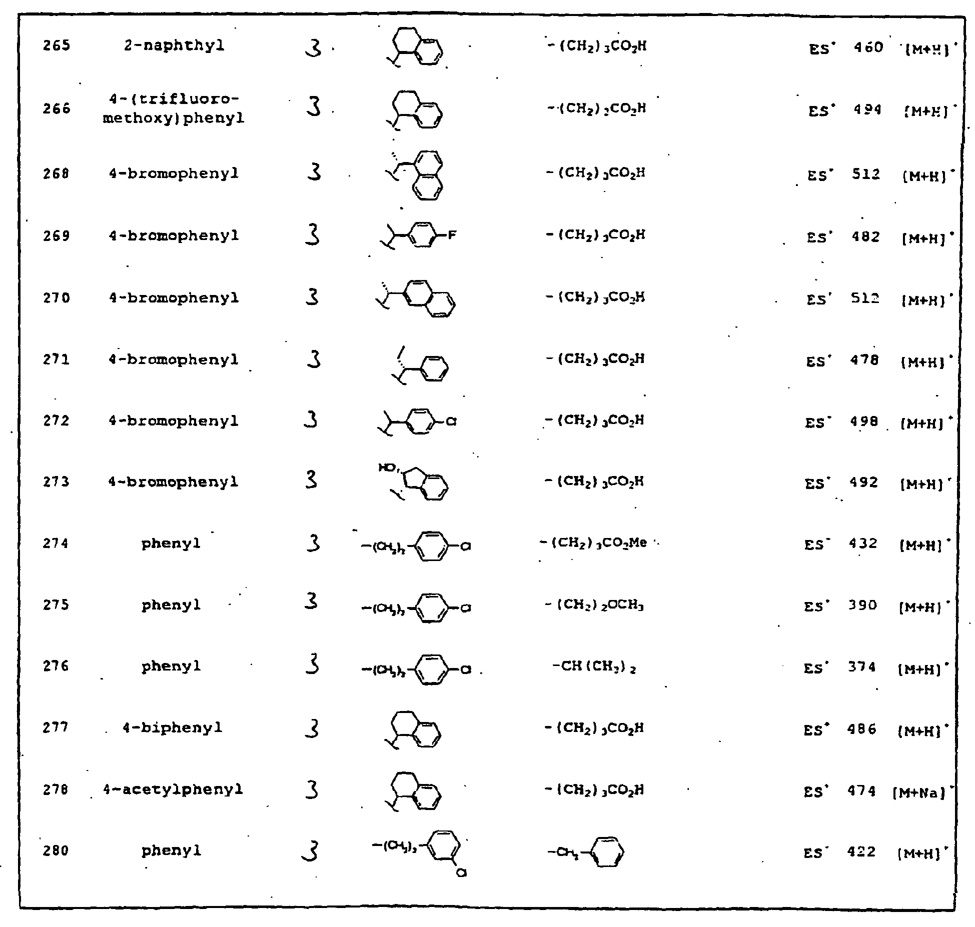

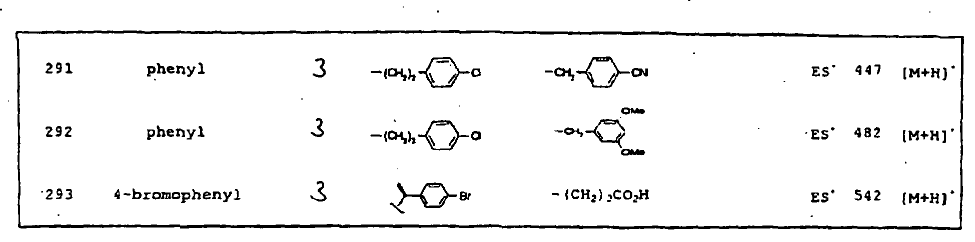

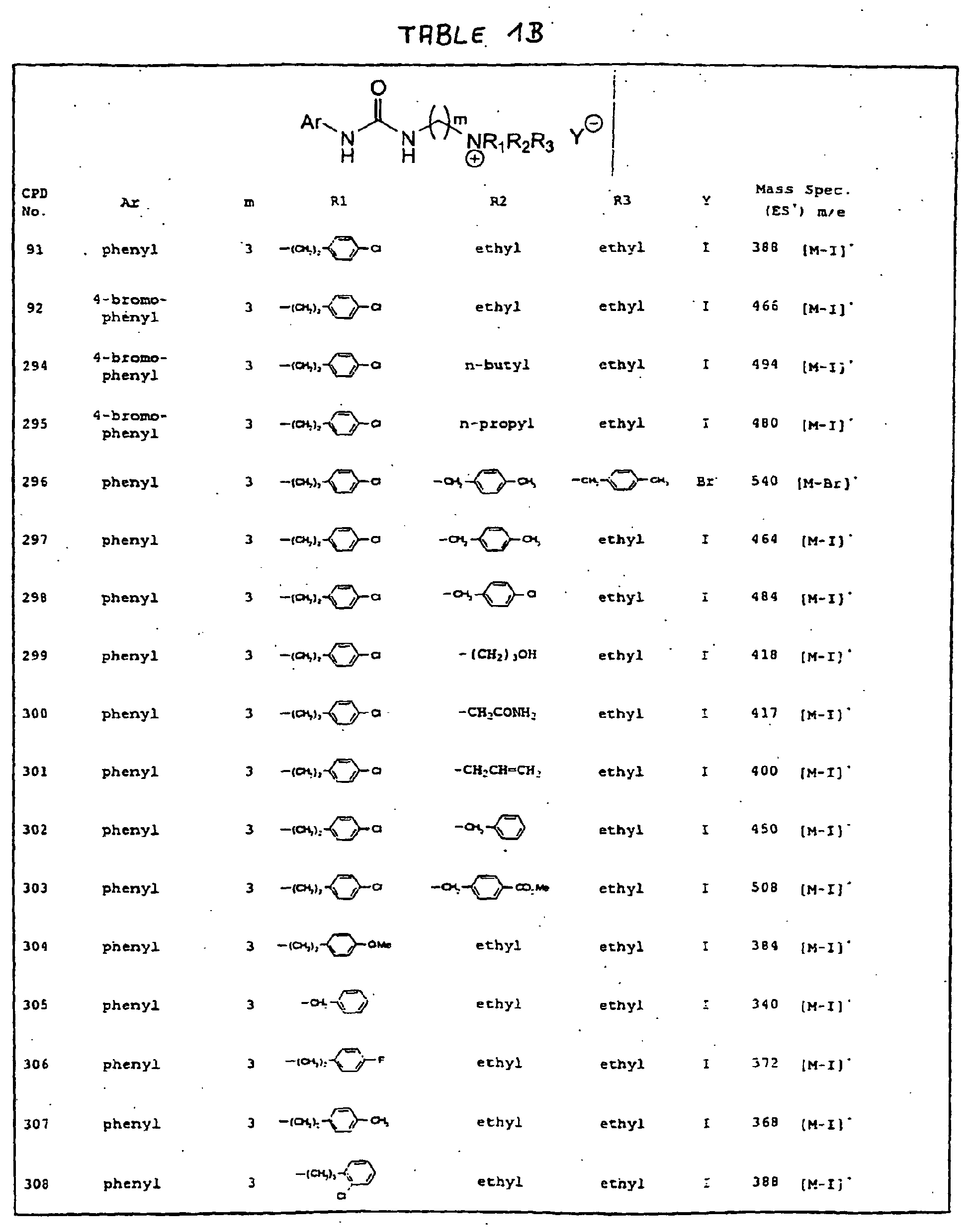

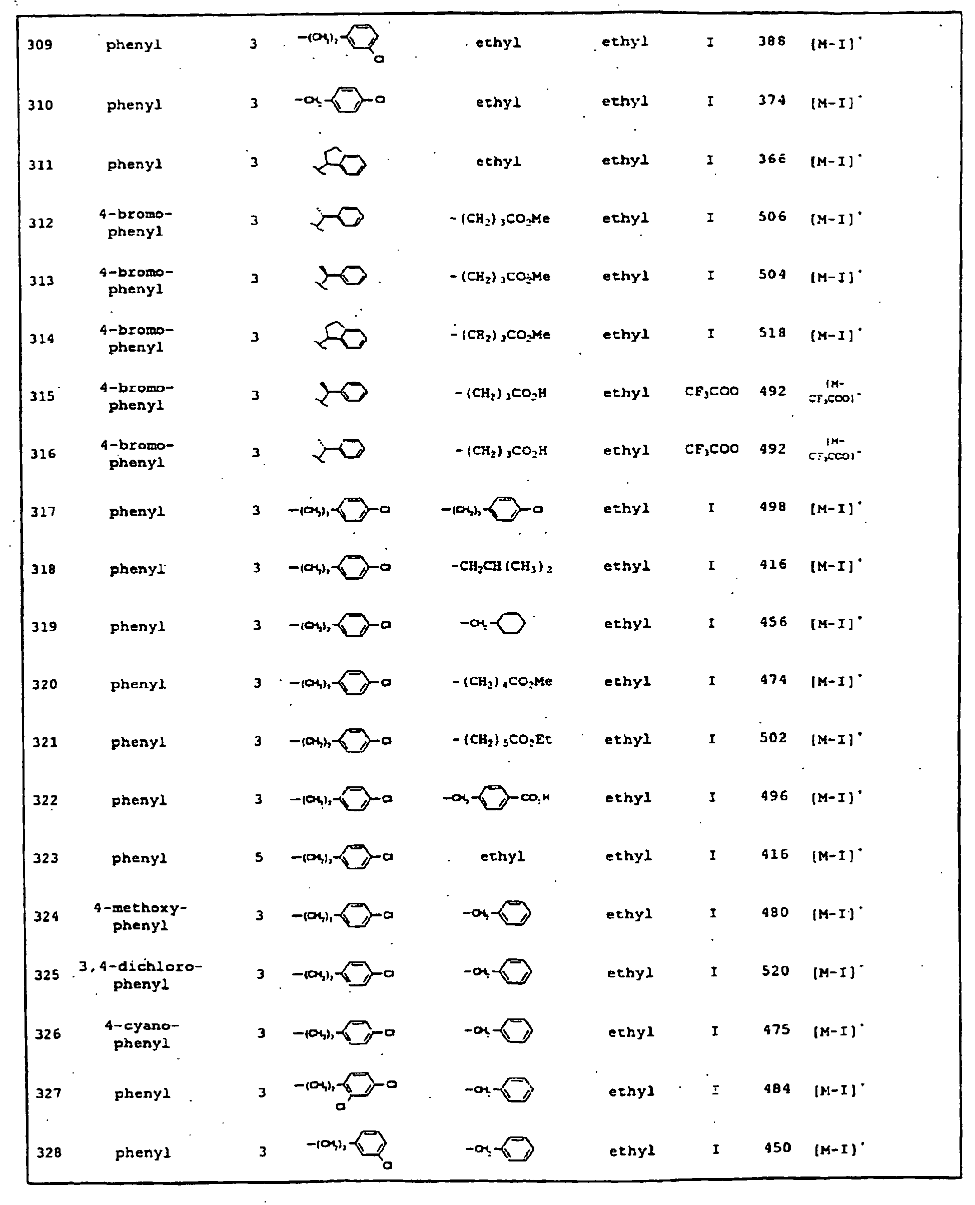

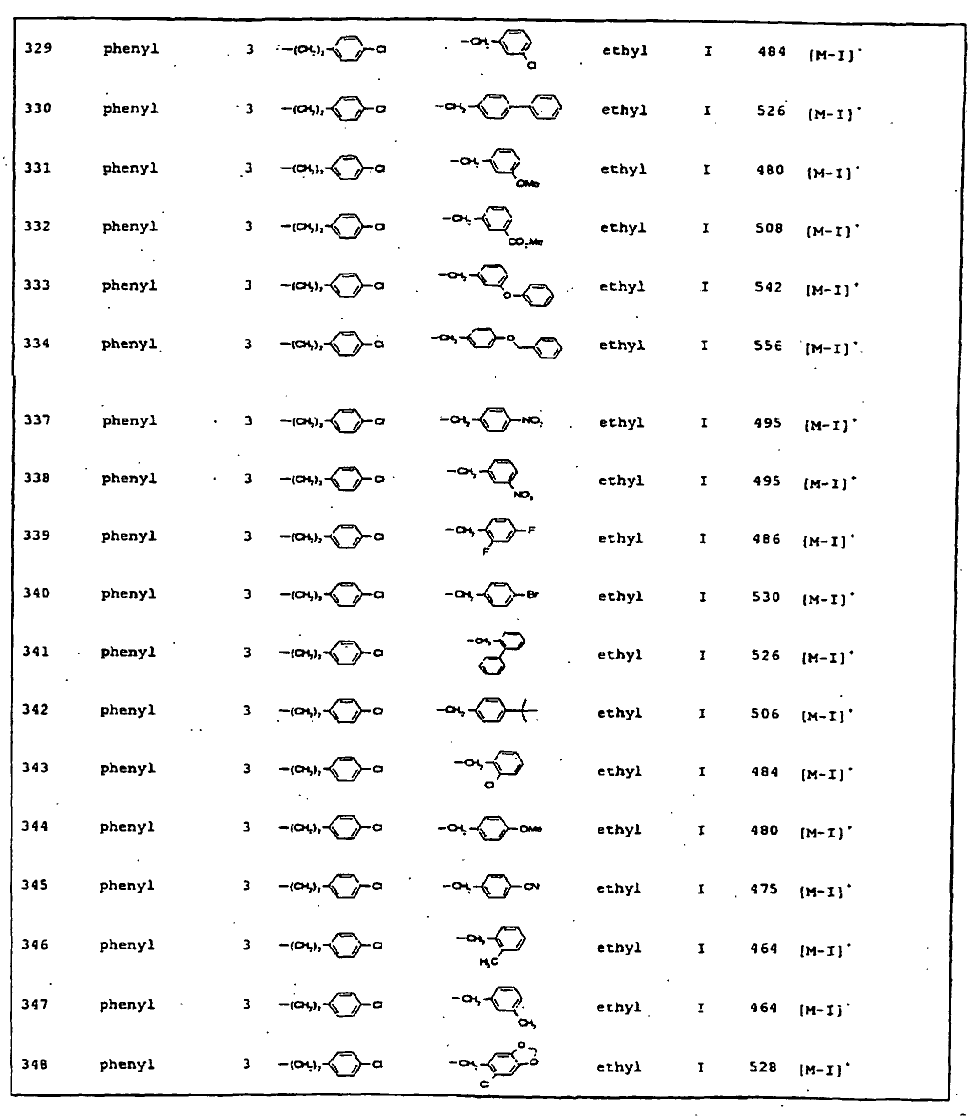

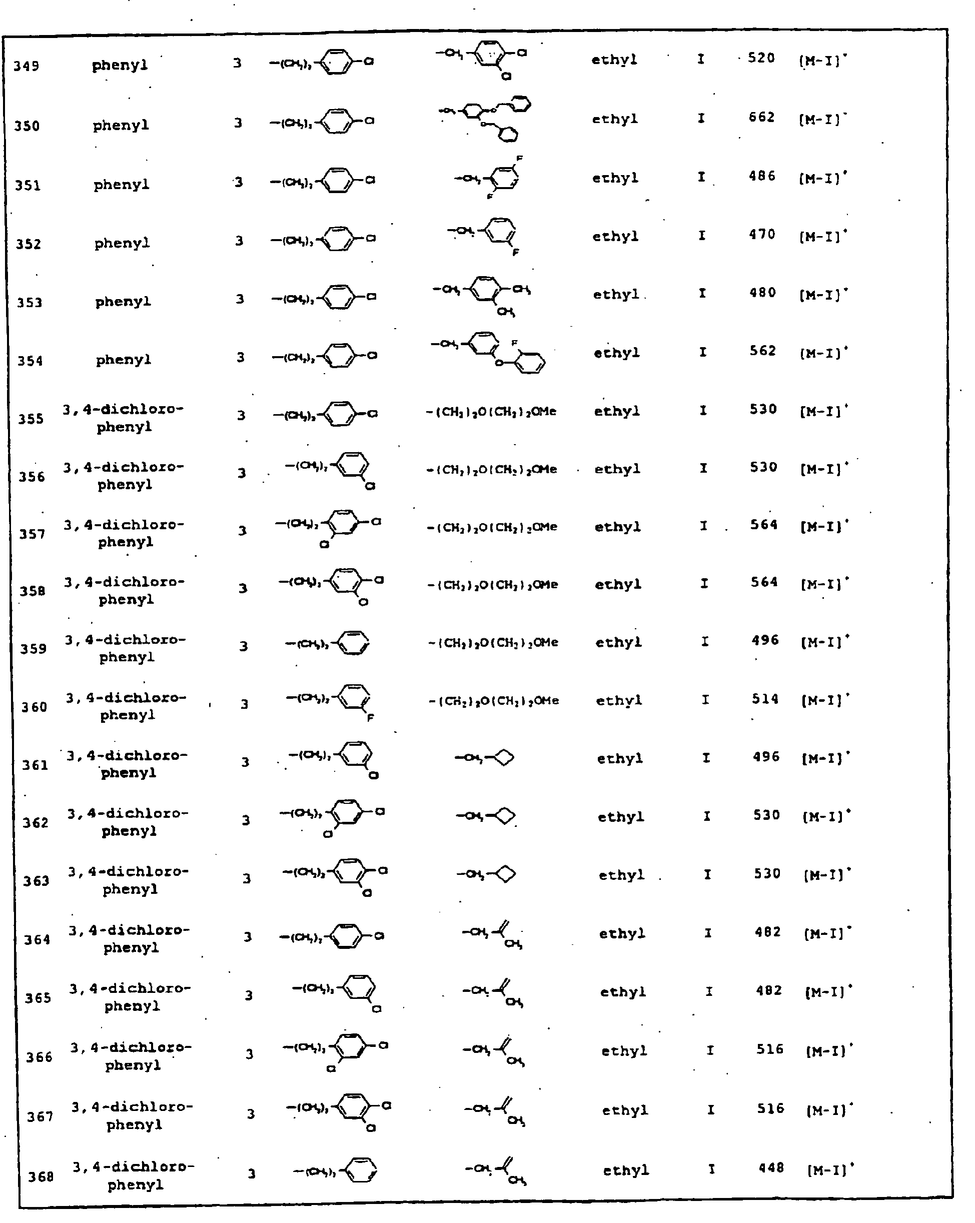

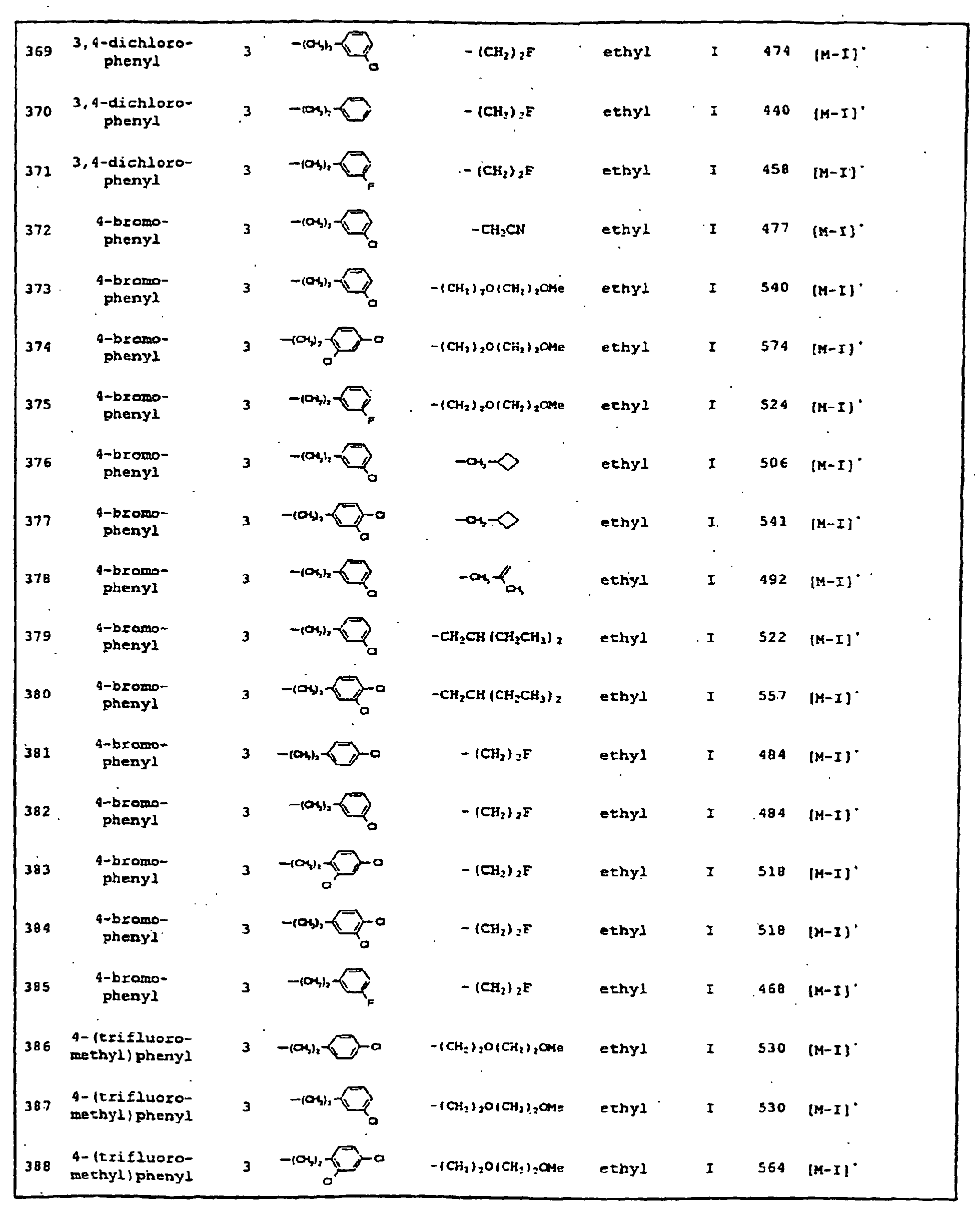

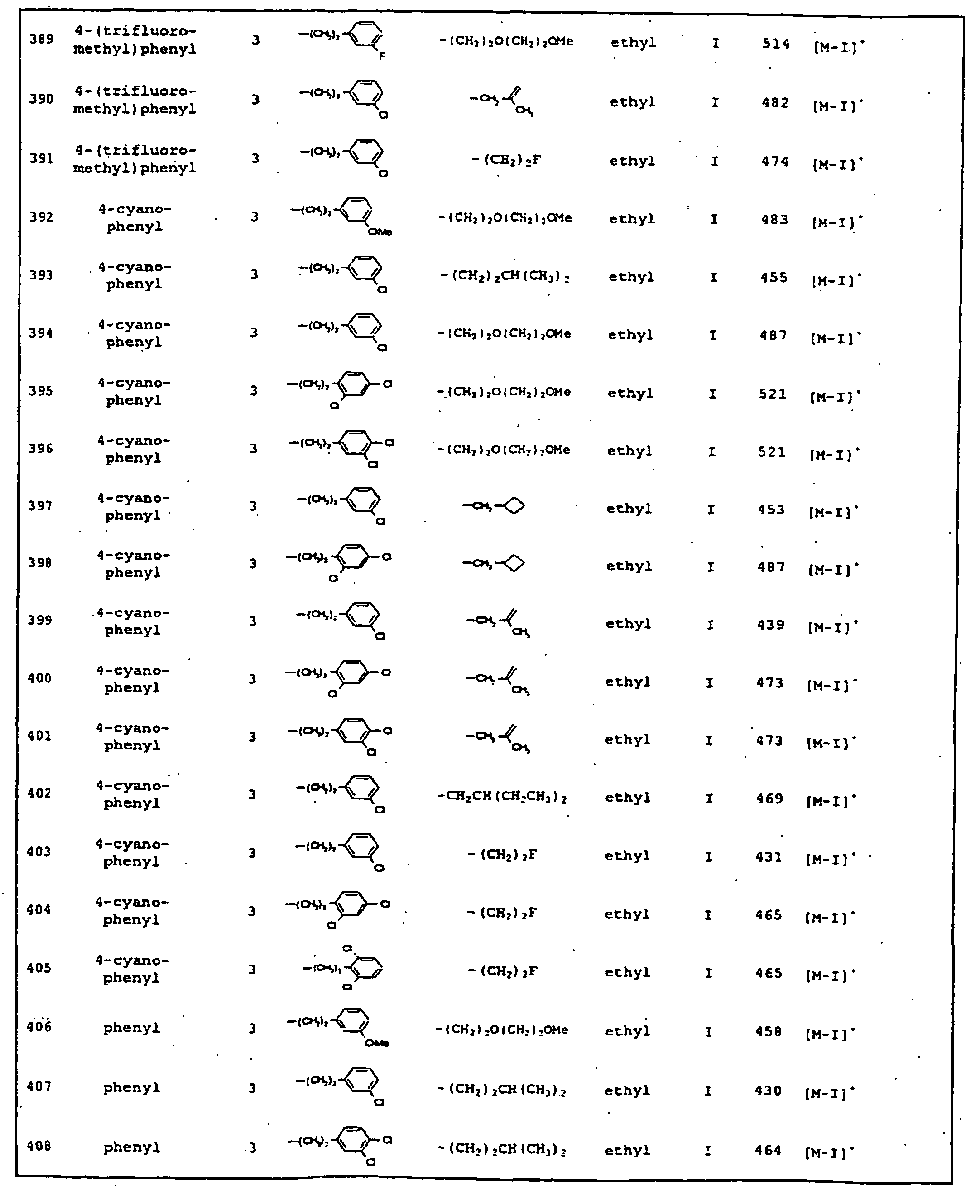

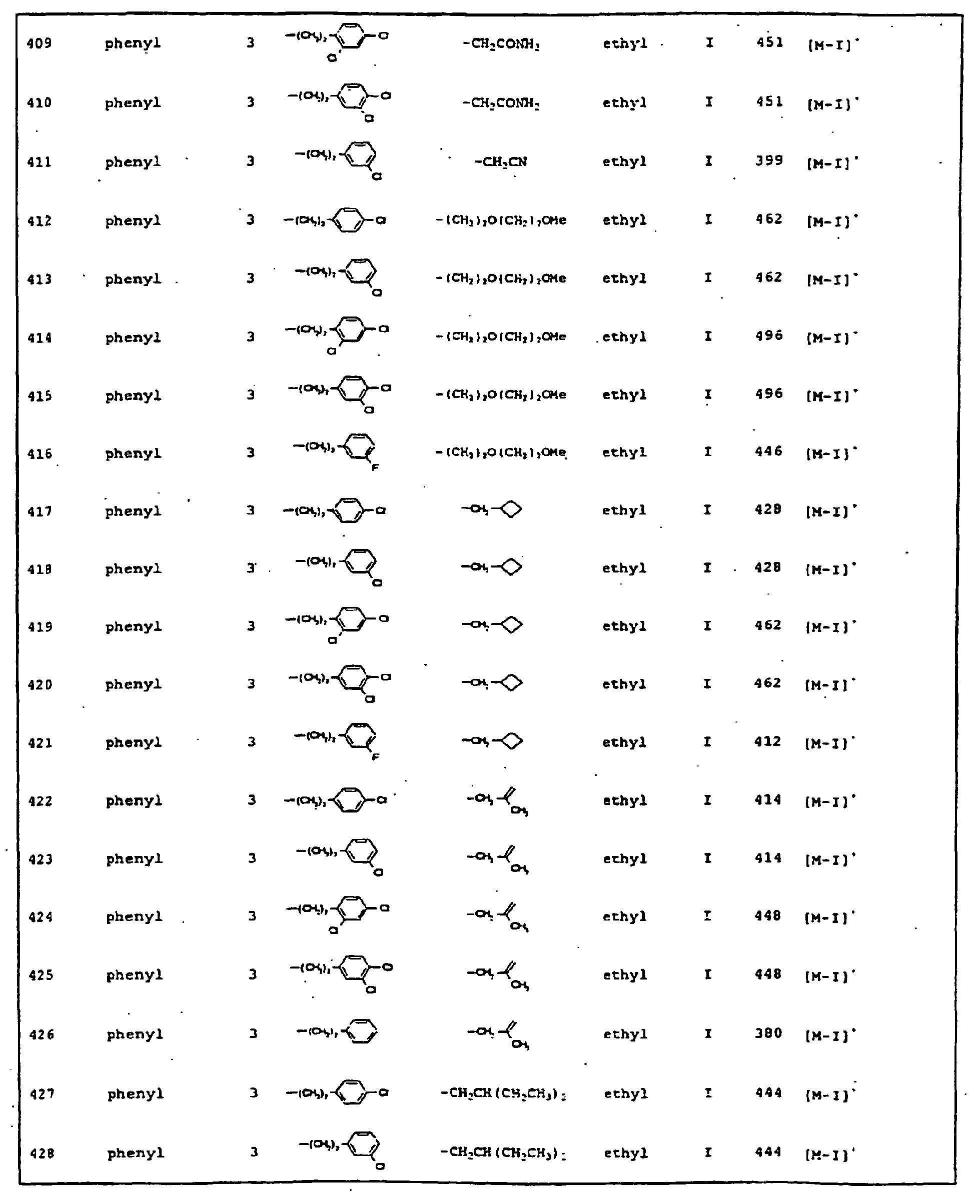

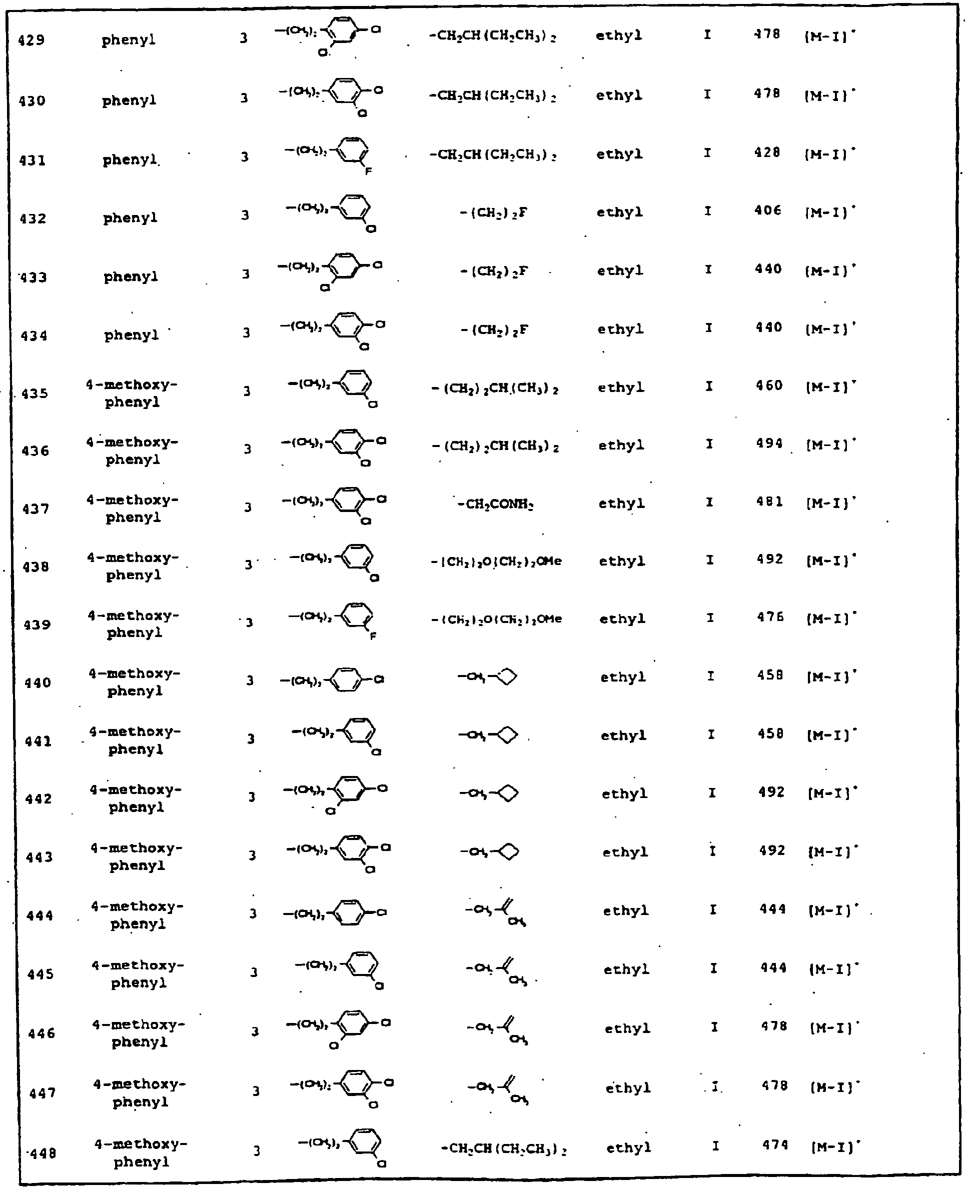

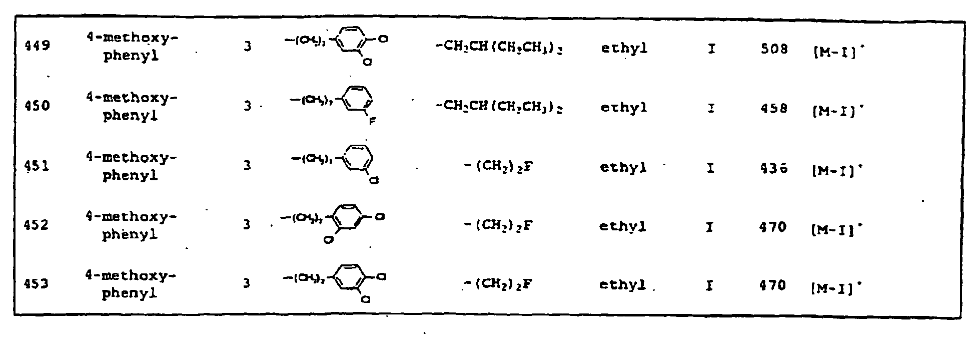

### Examples 17. Evaluation of CCR-3 Inhibition Using a Calcium Mobilization Assay

The CCR-3 inhibitory activity of the disclosed compounds was determined by measuring the inhibition of eotaxin-induced calcium mobilization using the assay described below. Compounds 93-162 used in this assay were synthesized by using the method described in Example 6.

CCR-3 transfectant cells (CCR3/HEK293) were isolated and resuspended with assay buffer (20 mM HEPES, 125 mM NaCl, 5 mM KCl; 0.5 mM glucose, 1 mM CaCl₂, 1 mM MgCl₂, 0.1 % BSA). After washing CCR3/HEK293 cells with assay buffer, cells were loaded with Fura-2/AM in assay buffer for 1 hour at room temperature. Cells were washed and resuspended with assay buffer at 5 x 10⁶ cells/ml in assay buffer, and placed in a tissue culture plate (Falcon, no. 3296). Test compounds dissolved in DMSO were added to the wells, followed by eotaxin (10 nM at final concentration). Cells were excited at 340 nm and 380 nm in a fluorimeter (ARGUS50, FDSS2000, Hamamatsu Photonics) and the relative ratio of the fluorescence emitted at 510 nm was recorded. For a control, DMSO without a test compound was added. Intracellular calcium mobilization was calculated as described in Krogel C. *et al. , FEBS Let.* (1989) 243 , 41-46.

The results shown in Table 2a and Table 2b indicate that the disclosed compounds inhibit calcium mobilization.

**Table 2a Inhibitory effects of compounds on eotaxin-induced intracellular calcium mobilization**

| CPD No. | Ca²⁺ mobilization 6.25 *µ*g/ml (inhibition %) |
|---|---|
| 93 | 50 |
| 94 | 50 |
| 95 | 55 |
| 96 | 53 |
| 97 | 57 |
| 98 | 51 |
| 99 | . 53 |
| 100 | 53 |
| 101 | 63 |
| 102 | 63 |
| 103 | 53 |
| 104 | 51 |
| 105 | 53 |
| 106 | 57 |
| 107 | 53 |
| 108 | 61 |
| 109 | 51 |
| 110 | 57 |
| 111 | 51 |
| 112 | 52 |
| 113 | 51 |
| 114 | 51 |
| 115 | 57 |
| 116 | 68 |
| 117 | 55 |
| 118 | 58 |
| 119 | 62 |
| 120 | 57 |
| 121 | 52 |
| 122 | 66 |
| 123 | 53 |
| 124 | 55 |
| 125 | 65 |
| 126 | 54 |
| 127 | 59 |
| 128 | 55 |
| 129 | 50 |
| 130 | 51 |
| 131 | 52 |
| 132 | 55 |
| 133 | 63 |
| 134 | 59 |
| 135 | 57 |
| 136 | 63 |
| 137 | 50 |
| 138 | 56 |
| 139 | 65 |
| 140 | 54 |
| 141 | 53 |
| 142 | 58 |
| 143 | 64 |
| 144 | 51 |
| 145 | 53 |
| 146 | 52 |
| 147 | 51 |
| 148 | 57 |
| 149 | 56 |
| 150 | 70 |
| 151 | 54 |
| 152 | 54 |
| 153 | 59 |
| 154 | 55 |
| 155 | 69 |
| 156 | 63 |
| 157 | 53 |
| 158 | 50 |
| 159 | 51 |
| 160 | 58 |
| 161 | 51 |
| 162 | 50 |

**Table 2b Inhibitory effects of compounds on eotaxin-induced intracellular calcium mobilization**

| CPD No. | Ca²⁺ mobilization 1.25 *µ*g/ml (inhibition %) |
|---|---|
| 355 | 63 |
| 356 | 57 |
| 357 | 57 |
| 358 | 63 |
| 361 | 70 |
| 362 | 49 |
| 363 | 56 |
| 364 | 66 |
| 365 | 65 |
| 366 | 46 |
| 367 | 50 |
| 393 | 56 |
| 394 | 76 |
| 395 | 69 |
| 396 | 67 |
| 397 | 69 |
| 398 | 70 |
| 399 | 58 |
| 400 | 68 |
| 401 | 57 |
| 435 | 62 |
| 436 | 57 |
| 437 | 64 |
| 438 | 70 |
| 440 | 62 |
| 441 | 71 |
| 442 | 63 |
| 443 | 69 |
| 444 | 72 |
| 445 | 63 |
| 446 | 76 |
| 447 | 65 |

### Example 18. Evaluation of Eotaxin-induced Chemotaxis of CCR-3 Transfectant Cell

The inhibitory activity of the compounds against eotaxin-induced chemotaxis was determined by measuring the inhibition of migration of CCR-3 transfectant cells (CCR3/U937), using a minor modification of the method described by Ohashi, H. *et al., Int Arch Allergy Immunol.* (1999) 118, 44-50. CCR-3 transfectant cells were grown in RPMI1640 medium containing 10% fetal calf serum (FCS) and Geneticin 418 (0.8 mg/ml). For the assay, CCR-3 transfectant-cells were isolated and resuspended at 1 x 10⁷ cells/ml in assay medium (RPMI 1640 medium containing 0.1 % bovine serum albumin (BSA)). The chemotaxis assay was performed in a 24-well culture plate. Human eotaxin suspended in assay medium was added to the wells at 1 x 10⁻⁹ M along with test compounds at various concentrations. For a positive control, eotaxin was added to the wells without a test compound, and for a negative control, neither eotaxin nor a test compound was added to the wells. Chemotaxicell (Kurabo Co., Ltd.) having 5 micrometers pore size were inserted into each well and 100 micro liters of CCR-3 transfectant cells suspension were added to the top chamber. The plates were incubated at 37 °C for 1 hour. After incubation, migrated cells in lower wells were diluted and counted by particle size distribution analyzer (CDP-500, Sysmex Co., Ltd.).

The results shown in Table 3a, 3b. 3c and 3d indicate that the disclosed compounds inhibit eotaxin-induced chemotaxis.

**Table 3a Inhibitory effects of compounds on eotaxin-induced chemotaxis of CCR3 transfectants**

| CPD No. | Chemotaxis Assay 10 µM (inhibition %) |
|---|---|
| 1 | 100 |
| 2 | 100 |
| 3 | 100 |
| 5 | 99 |
| 6 | 100 |
| 7 | 99 |
| 8 | 94 |
| 9 | 100 |
| 10 | 99 |
| 11 | 86 |
| 14 | 97 |
| 16 | 63 |
| 17 | 51 |
| 18 | 58 |
| 19 | 47 |
| 20 | 26 |
| 21 | 40 |
| 22 | 25 |
| 23 | 82 |
| 24 | 100 |
| 25 | 88 |
| 27 | 97 |
| 28 | 76 |
| 29 | 100 |
| 30 | 100 |
| 31 | . 97 |
| 32 | 99 |
| 33 | 95 |
| 34 | 96 |
| 35 | 100 |
| 36 | 34 |
| 37 | 100 |
| 38 | 46 |
| 39 | 78 |
| 40 | 88 |
| 41 | 20 |
| 42 | 96 |
| 43 | 50 |
| 45 | 62 |
| 47 | 58 |
| 48 | 34 |
| 49 | 100 |
| 51 | 100 |
| 52 | 100 |
| 53 | 93 |
| 54 | 19 |
| 55 | 38 |
| 56 | 100 |
| 57 | 100 |
| 58 | 100. |
| 59 | 87 |
| 60 | 98 |
| 61 | 100 |
| 62 | 98 |
| 63 | 100 |
| 64 | 100 |
| 65 | 100 |
| 66 | 32 |
| 67 | 100 |
| 68 | 31 |
| 69 | 81 |
| 70 | 89 |
| 71 | 64 |
| 72 | 68 |
| 73 | 44 |
| 74 | 50 |
| 75 | 68 |
| 76 | 44 |
| 77 | 76 |
| 79 | 78 |
| 80 | 65 |
| 82 | 100 |
| 83 | 100 |
| 84 | 100 |
| 85 | 19 |
| 86 | 37 |
| 87 | 97 |
| 88 | 100 |
| 89 | 89 |
| 90 | 100 |
| 171 | 100 |
| 172 | 100 |
| 173 | 100 |
| 174 | 100 |
| 175 | 100 |
| 176 | 75 |
| 177 | 89 |
| 178 | 100 |
| 179 | 100 |
| 180 | 100 |
| 181 | 100 |
| 182 | 97 |
| 183 | 94 |
| 184 | 100 |
| 185 | 100 |
| 186 | 69 |
| 187 | 100 |
| 188 | 100 |
| 189 | 94 |
| 190 | 90 |
| 191 | 100 |
| 192 | 100 |
| 193 | 100 |
| 194 | 100 |
| 195 | 100 |
| 196 | 100 |
| 197 | 100 |
| 198 | 100 |
| 199 | 100 |
| 200 | 100 |
| 201 | 100 |
| 202 | 100 |
| 203 | 100 |
| 204 | 78 |
| 205 | 97 |
| 206 | 64 |
| 207 | 50 |
| 208 | 63 |
| 209 | 94 |
| 210 | 100 |
| 211 | 67 |
| 212 | 100 |
| 213 | 92 |
| 214 | 99 |
| 215 | 89 |
| 216 | 100 |
| 217 | 87. |
| 218 | 99 |
| 219 | 86 |
| 220 | 77 |
| 221 | 100 |
| 222 | 79 |
| 223 | 86 |
| 224 | 75 |
| 225 | 100 |
| 226 | 100 |
| 227 | 100 |
| 228 | 100 |
| 229 | 100 |
| 230 | 100 |
| 231 | 90 |
| 232 | 100 |
| 233 | 100 |
| 234 | 89 |
| 235 | 91 |
| 236 | 97 |
| 237 | 100 |
| 238 | 69 |
| 239 | 100 |
| 240 | 86 |
| 241 | 100 |
| 242 | 73 |
| 243 | 84 |
| 244 | 81 |
| 245 | 100 |
| 258 | 100 |
| 259 | 100 |
| 260 | 100 |
| 261 | 100 |
| 262 | 62 |
| 263 | 100 |
| 264 | 100 |
| 265 | 95 |
| 266 | 100 |
| 268 | 100 |
| 269 | 100 |
| 270 | 100 |
| 271 | 100 |
| 272 | 89 |
| 273 | 88 |
| 274 | 60 |
| 275 | 100 |
| 276 | 100 |
| 277 | 76 |
| 278 | 100 |
| 280 | 60 |
| 291 | 53 |
| 292 | 56 |
| 293 | 95 |

**Table 3b Inhibitory effects of compounds on eotaxin-induced chemotaxis of CCR3 transfectants**

| CPD No. | CCR-3 Transfectant Chemotaxis Assay 6.25 µg/ml (inhibition %) |
|---|---|
| 97 | 21 |
| 99 | 47 |
| 100 | 54 |
| 102 | 19 |
| 106 | 47 |
| 107 | 55 |
| 108 | 23 |
| 109 | 12 |
| 110 | 32 |
| 111 | 44 |
| 112 | 26 |
| 113 | 66 |
| 114 | 22 |
| 115 | 62 |
| 116 | 82 |
| 118 | 62 |
| 119 | 65 |
| 120 | 34 |
| 121 | 64 |
| 122 | 92 |
| 125 | 90 |
| 126 | 54 |
| 128 | 33 |
| 132 | 11 |
| 133 | 21 |
| 135 | 12 |
| 136 | 32 |
| 137 | 40 |
| 138 | 31 |
| 149 | 31 |
| 155 | 56 |

**Table 3c Inhibitory effects of compounds on eotaxin-induced chemotaxis of CCR3 transfectants .**

| CPD No. | Chemotaxis Assay 10 *µ*M (inhibition %) |
|---|---|
| 91 | 100 |
| 92 | 100 |
| 294 | 100 |
| 295 | 100 |
| 296 | 67 |
| 297 | 100 |
| 298 | 100 |
| 299 | 100 |
| 300 | 100 |
| 301 | 100 |
| 302 | 100 |
| 303 | 100 |
| 304 | 66 |
| 305 | 100 |
| 306 | 100 |
| 307 | 92 |
| 308 | 100 |
| 309 | 100 |
| 310 | 100 |
| 311 | 93 |
| 312 | 97 |
| 313 | 86 |
| 314 | 100 |
| 315 | 63 |
| 316 | 82 |
| 317 | 100 |
| 318 | 100 |
| 319 | 100 |
| 320 | 100 |
| 321 | 100 |
| 322 | 93 |
| 323 | 100 |
| 324 | 100 |
| 325 | 100 |
| 326 | 100 |
| 327 | 100 |
| 328 | 100 |
| 329 | 100 |
| 330 | 100 |
| 331 | 100 |
| 332 | 100 |
| 333 | 100 |
| 334 | 100 |
| 337 | 99 |
| 338 | 100 |
| 339 | 100 |
| 340 | 100 |
| 341 | 100 |
| 342 | 97 |
| 343 | 100 |
| 344 | 100 |
| 345 | 100 |
| 346 | 100 |
| 347 | 100 |
| 348 | 100 |
| 349 | 100 |
| 350 | 59 |
| 351 | 100 |
| 352 | 100 |
| 353 | 100 |
| 354 | 100 |

**Table 3d Inhibitory effects of compounds on eotaxin-induced chemotaxis of CCR3 transfectants**

| CPD No. | Chemotaxis Assay 0.1 µg/ml (inhibition %) |
|---|---|
| 359 | 49 |
| 360 | 70 |
| 368 | 88 |
| 369 | 82 |
| 370 | 64 |
| 371 | 86 |
| 372 | 76 |
| 373 | 100 |
| 374 | 100 |
| 375 | 91 |
| 376 | 87 |
| 377 | 46 |
| 378 | 81 |
| 379 | 80 |
| 380 | 46 |
| 381 | 68 |
| 382 | 98 |
| 383 | 43 |
| 384 | 76 |
| 385 | 68 |
| 386 | 43 |
| 387 | 94 |
| 388 | 56 |
| 389 | 65 |
| 390 | 51 |
| 391 | 47 |
| 392 | 45 |
| 402 | 71 |
| 403 | 77 |
| 404 | 47 |
| 405 | 57 |
| 406 | 43 |
| 407 | 52 |
| 408 | 74 |
| 409 | 53 |
| 410 | . 50 |
| 411 | 42 |
| 412 | 84 |
| 413 | 95 |
| 414 | 98 |
| 415 | 99 |
| 416 | 69 |
| 417 | 59 |
| 418 | 89 |
| 419 | 76 |
| 420 | 99 |
| 421 | 66 |
| 422 | 42 |
| 423 | 92 |
| 424 | 95 |
| 425 | 93 |
| 426 | 44 |
| 427 | 67 |
| 428 | 93 |
| 429 | 64 |
| 430 | 76 |
| 431 | 96 |
| 432 | 96 |
| 433 | 76 |
| 434 | 100 |
| 439 | 51 |
| 448 | 82 |
| 449 | 96 |
| 450 | 35 |
| 451 | 92 |
| 452 | 59 |
| 453 | 87 |

### Example 19. Evaluation of Eotaxin-induced Chemotaxis of Eosinophils.

The inhibitory activity of the compounds against eotaxin-mediated chemotaxis of human-derived eosinophils was determined assay described below.

Eosinophils were prepared from culture of human cord blood mononuclear cells as described by Ohashi, H*. et al., Int Arch Allergy Immunol.* (1999) 118, 44-50. For the assay, eosinophils were resuspended at 1 x 10⁷ cells/ml in assay medium (RPMI 1640 medium containing 0.1 % bovine serum albumin (BSA)). The chemotaxis assay was performed in a 24-well culture plate. Human eotaxin suspended in assay medium was added into wells at 1 x 10⁻⁹ M with test compounds at various concentrations. For a positive control, eotaxin was added without a test compound, and for a negative control, neither eotaxin nor a test compound was added to the wells. Chemotaxicell (Kurabo Co., Ltd.) having 5 micrometers pore size were inserted into each well and 100 microliters of eosinophil suspension were added to the top chamber. The plates were incubated at 37°C for 1 hour. After incubation, migrated cells in lower wells were diluted and counted by particle size distribution analyzer (CDP-500, Sysmex Co., Ltd.).

The results shown in Table 4 indicate that the disclosed compounds inhibit eotaxin-induced chemotaxis in cultured eosinophils.

**Table 4. Inhibitory effects of compounds on Eotaxin-induced chemotaxis of cultured eosinophils.**

| CPD No. | Cultured Eosinophil Chemotaxis assay 10.00 µM (inhibition %) |
|---|---|
| 1 | 100 |
| 3 | 100 |
| 36 | 31 |
| 38 | 11 |
| 60 | 100 |
| 61 | 100 |
| 65 | 100 |
| 66 | 44 |
| 67 | 71 |
| 91 | 100 |
| 92 | 100 |

### Example 20. Use of an Urea Derivative to treat a CCR-3 Mediated Disease

A patient suffering from asthma is administered N-Phenylcarbamovi-N'-[2-(4-chlorophenyl)ethyl]-N'-ethyl-1.3-diaminopropane (Compound I). Approximately 1 mmole of the compound is administered to the patient via inhalation of an aerosol comprising compound 1. The amount of compound administered should be between 0.01 and 20 mg/kg of the patient's weight.

### Example 21. Suppression of Type II Collagen-induced Arthritis in Mice by Compound No. 60 and Compound No. 298

The inhibitory effects of Compound No. 60 (CPD No.60) and Compound No. 298 (CPD No. 298) on collagen-induced arthritis were evaluated in mice.

Male DBA/1 mice were purchased from Japan Charles River Inc. (Kanagawa, Japan) and used at 5 week of age. Mice were immunized intradermally at the base of the tail with mixture of 100 *µ*g of bovine type II collagen (Collagen Gijyutsu-Kenshukai, Japan) and 100 *µ*g of *Mycobacterium tuberculosis* H37Ra (Difco, Detroit, MI) in incomplete Freaund's adjuvant at the base of the tail, and then boosted 21 days later with same emulsion. The compound to be studied (20 mg/kg per day) was administered subcutaneously starting at 2nd immunization. In the control experiment, control vehicle (10% DMSO and 10% Cremophor EL in saline) was administered instead of the compound to be studied. Clinical scoring for each paw was assessed by reference to the following scale: 0=normal, 1 = swelling and/or erythema of one toe, 2 = swelling and/or erythema of two or more toes, 3=swelling and erythema of the entire paw, 4=complete sewlling and erythema of the entire paw and incapacity to bend the ankle. Clinical score for the whole animal was expressed as the cumulative value for all paws, with a maximum of 16. Each group consists of 10 animals.

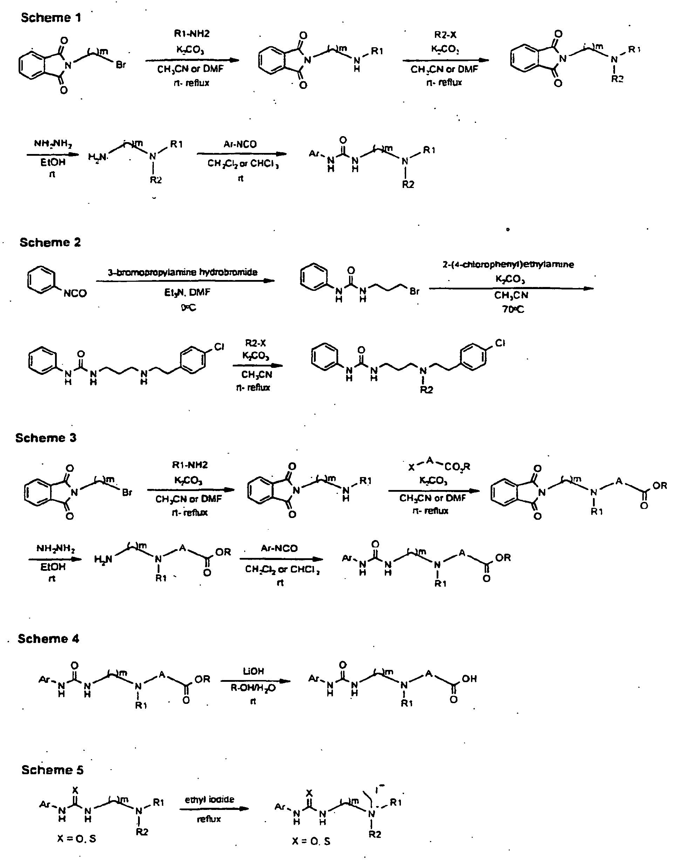

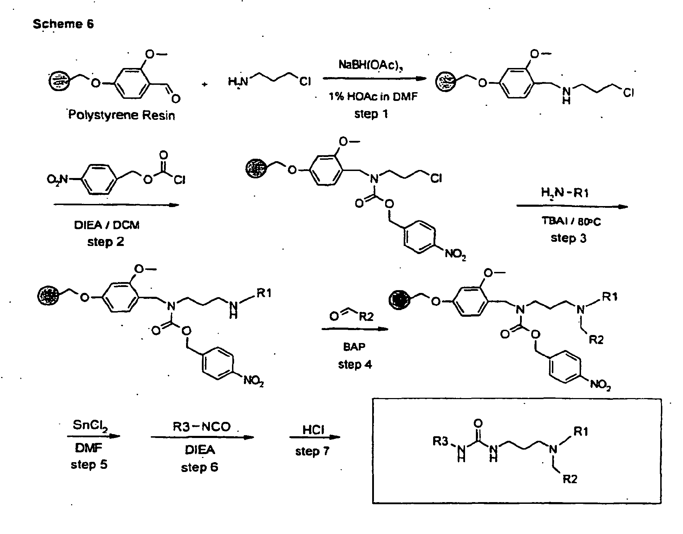

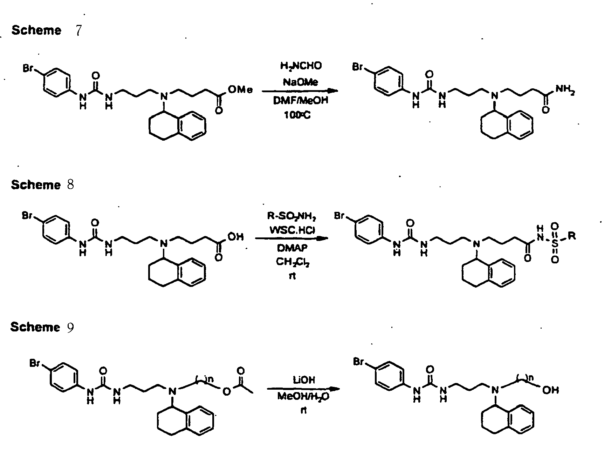

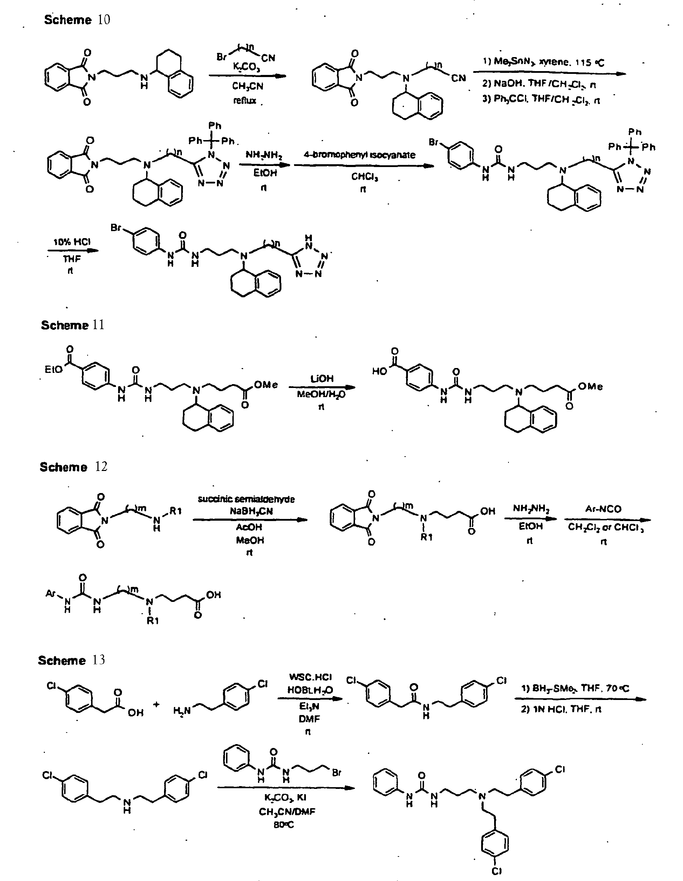

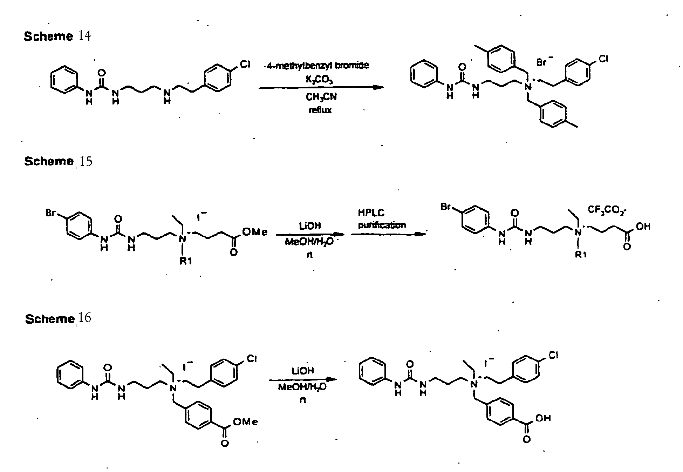

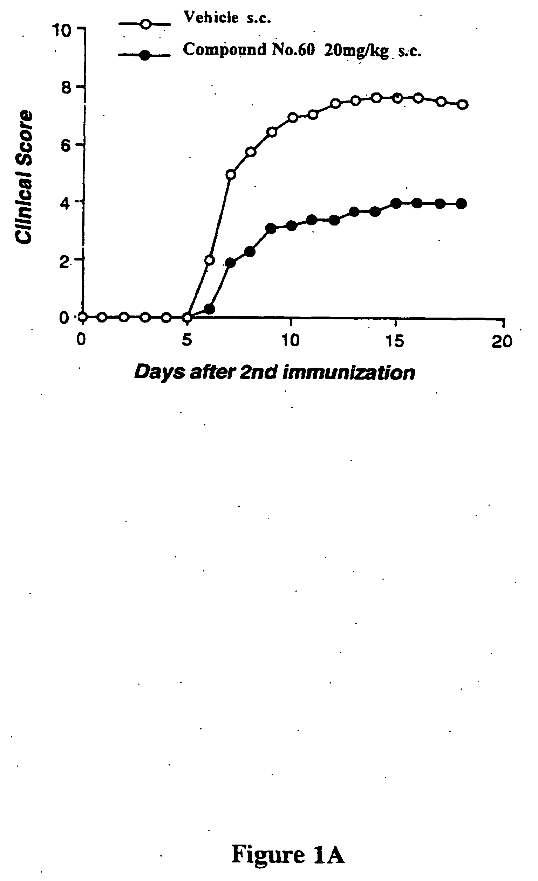

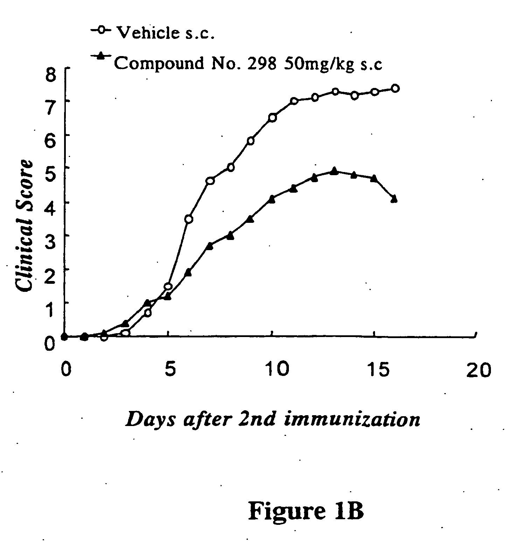

The inhibitory effects of Compound No. 60 and Compound No. 298 on collagen-induced arthritis were shown in Figure 1A and 1B, respectively. A marked prevention in clinical score was observed by administration of Compound No. 60 or Compound No. 298, as opposed to administration of control vehicle..

### Example 22. Suppression of Airway Hyperreactivity and Eosinophil Infiltration in Bronchoalveolar Lavage Fluid (BALF) by Compound No. 298

Male BALB/c mice were immunized by an intraperitoneal injection of 10 *µ*g OVA adsorbed to 1 mg aluminum hydroxide gel (alum). A booster injection of the same dose of alum-adsorbed OVA was given 5 days later. Unitnmunized control mice received saline.

Twelve days after primary immunization, both the immunized and unimmunized mice were exposed to aerosolized antigen. Aerosolization of OVA was performed using a nose-only aerosol chamber adapted for mice. Animals were exposed for 10 minutes to 5 mg/ml OVA aerosolized by an ultrasonic nebulizer (NE-U12, Omron, Tokyo, Japan) driven by a vacuum pump. The antigen bronchoprovocation was repeated on day 16 and day 20 under the same conditions. Compound No. 298 (CPD No. 298) was dissolved in saline containing 2 % DMSO and 2 % Cremophore and administered intraperitoneally for 9 days, starting on the first day of antigen inhalation.

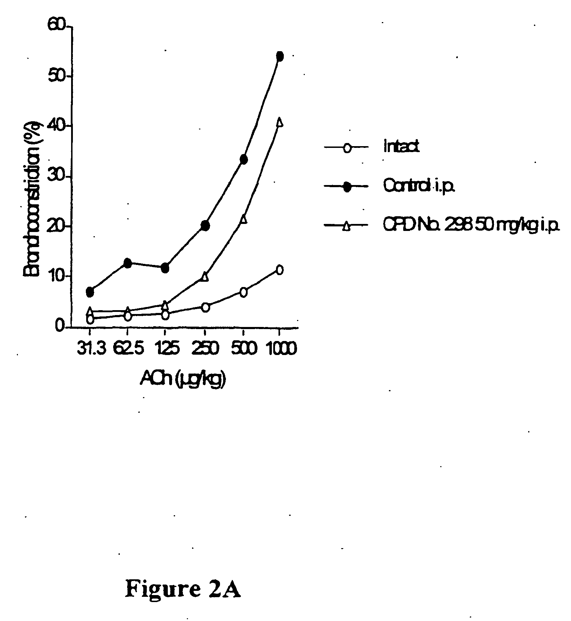

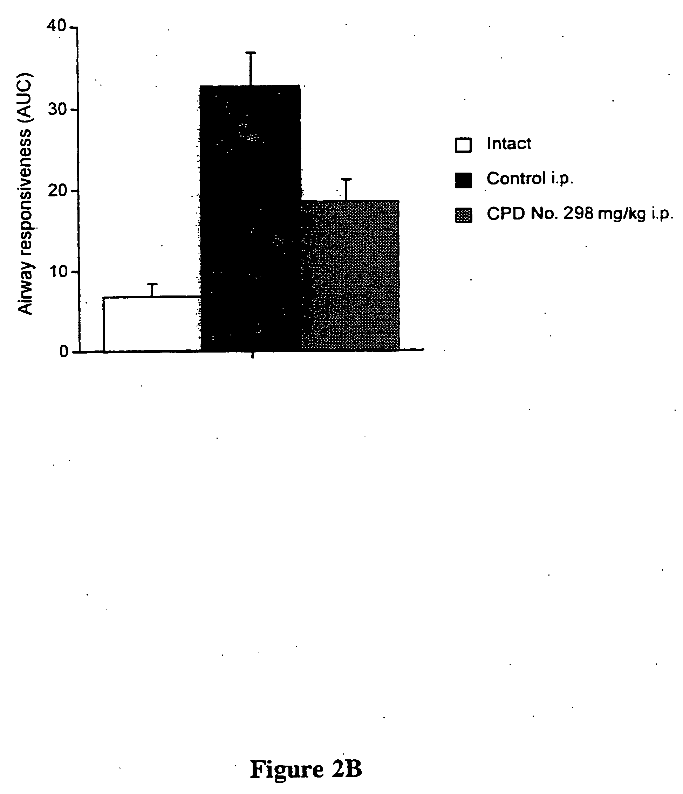

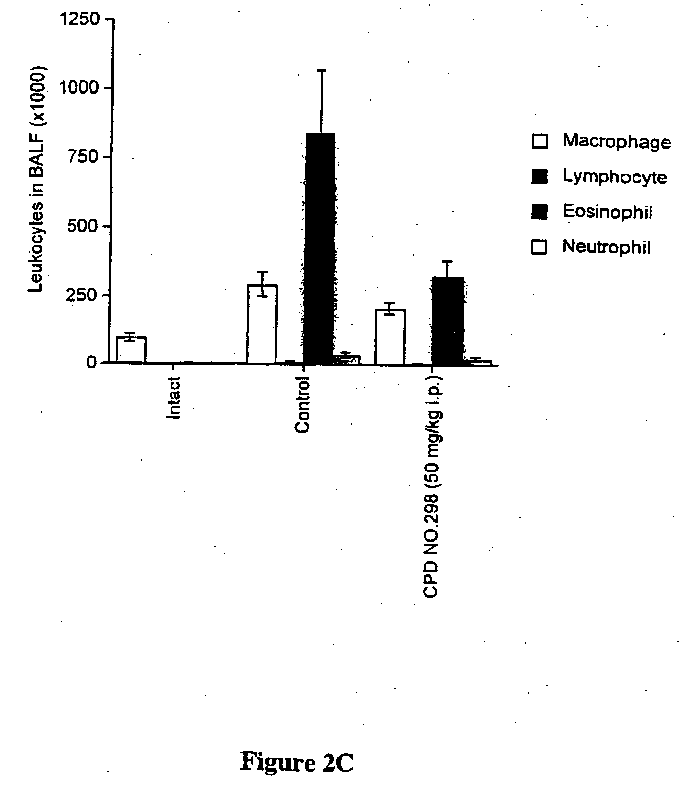

Twenty-four hours after the final aerosol exposure, bronchoconstriction was measured by the overflow method of Konzett and Rössler. Mice were anesthetized by an intraperitoneal injection of sodium pentobarbitone (50 mg/kg), and the tracheas were surgically exposed, cannulated, and connected to a rodent ventilator (Model 683, Harvard Apparatus, South Natick, MA) and a bronchospasm transducer (Model 7020, Ugo Basile, Comerio-Varese, Italy). Animals were mechanically ventilated with air at 60 strokes/min with a stroke volume of 0.6 ml. A paralytic agent, pancuronium bromide, 0.1 mg/kg, was administered to eliminate spontaneous respiration. After a stable baseline airway pressure was established; acetylcholine chloride was injected intravenously in a volume of 1 µl/g of mouse per dose, starting with 31.3 µg/kg, and increasing the concentration two-fold for each subsequent dose. Bronchoconstriction was recorded on a flatbed recorder (Model FBR-252A, TOA Electronics Ltd., Tokyo, Japan). Bronchoconstriction (%) represent the respiratory overflow volume provoked by acetylcholine as a percentage of the maximal overflow volume (100%) obtained by totally occluding the tracheal cannula. See Figure 2A. Inhibition of bronchoconstriction provoked by acetylcholine (Murine Asthma Model) by Compound No. 298 was shown in Figure 2A. In some experiments, airway reactivity was expressed by the area under the dose-response curve (the curves in Figure 2A) of bronchoconstriction against the acetylcholine- concentration. See Figure 2B.

Immediately after the measurement of airway reactivity to acethycholine, BALF was collected by lavaging whole-lung three times with 0.7-ml aliquots of physiological saline containing 0.1 % BSA via the tracheal cannula while gently massaging the thorax. The BALF recovered from one mouse was pooled, centrifuged, and the cells were resuspended in 100 µl saline containing 0.1 % BSA. Cell numbers were determined using a hemocytometer and 2 x 10⁴ cells were cytecetrifuged onto a glass slide. Cells were stained with Diff-Quik (International reagent, Kobe, Japan), and cell types were identified by morphological criteria. Two hundred cells were examined per slide for differential count. *See* Figure 2C. As shown in Figure 2C, Compound No. 298 (CPD No. 298) significantly suppressed eosinophil infiltration to bronchoalveolar lavage fluid (BALF).

## Claims

1. A compound having the following Formula (I) : 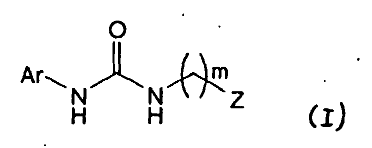 or a salt, hydrate, or complex thereof, wherein:
m is 3,4,5, or 6;
Ar is aryl or heteroaryl
optionally substituted with one or more groups independently selected from the group consisting of hydroxy, halogen, trihalomethyl, tribalomethoxy, C₁₋₅alkyl, C₁. ₅alkoxy, cyano, nitro, amino, carboxy, alkyloxycarbonyl, arylmethyloxycarbonyl, carbamoyl, alkylcarbamoyl, arylcarbamoyl, acyl, acyloxy, sulfonyl, alkylsulfonyl, arylsulfonyl, sulfamoyl, alkylsulfamoyl, arylsulfamoyl, alkylthio, alkylsulfonamide, arylsulfonamide, hydrazino, acylamino, alkylamino, hydroxyamino, amidino, guanidino, cyanoguanidino, aryl
optionally substituted with one or more groups independently selected from hydroxy, halogen, trihalomethyl, C₁₋₅alkyl, C₁₋₅ alkoxy, cyano, nitro, amino and carboxy,
and aryloxy
optionally substituted with one or more groups independently selected from hydroxy, halogen, trihalomethyl, C₁₋₅ alkyl, C₁₋₅ alkoxy, cyano, nitro, amino and carboxy,
Z is: 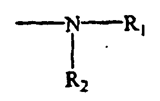 or 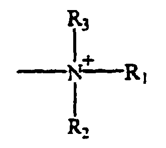
wherein R₁ is: 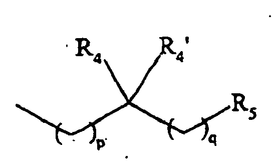 or 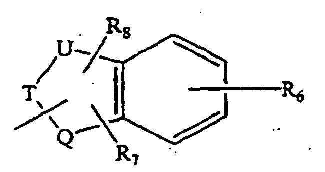 or 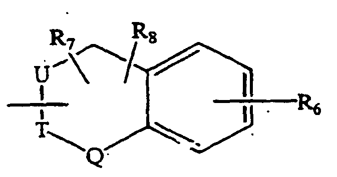 or 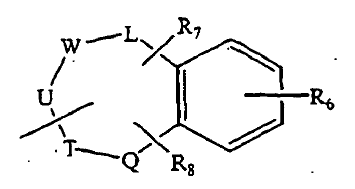 p is 0, 1 or 2;
q is 0, 1 or 2;
R₄ and R4' are independently selected from hydrogen, halogen. C₁₋₅ alkyl, aryl, heteroaryl
wherein the aryl or heteroaryl is optionally substituted with one or more groups independently selected from hydrogen, hydroxy, halogen, trihalomethyl, C₁₋₅ alkyl, C₁₋₅alkoxy, cyano, nitro, amino, carboxy, carbamoyl, alkylcarbamoyl, arylcarbamoyl, acyl, acyloxy, sulfonyl, alkylsulfonyl, arylsulfonyl, sulfamoyl, alkylsulfamoyl, arylsulfamoyl, alkylthio, alkylsulfonamide, arylsulfonamide, hydrazino, acylamino, alkylamino, hydroxyamino, amidino, guanidino, and cyanoguanidino;
and COR₉; wherein R₉ is hydroxy, C₁₋₅alkyl, C₁₋₅alkoxy, amino, alkylamino or arylamino; R₅ is aryl or heteroaryl
optionally substituted with one or more groups independently selected from hydroxy, halogen, trihalomethyl, C₁₋₅alkyl, C₁₋₅ alkoxy, cyano, nitro, amino, carboxy, carbamoyl, alkylcarbamoyl, arylcarbamoyl, acyl, acyloxy, sulfonyl, alkylsulfonyl, arylsulfonyl, sulfamoyl, alkylsulfamoyl, arylsulfamoyl, alkylthio, alkylsulfonamide, arylsulfonamide, hydrazino, acylamino, alkylamino, hydroxyamino, amidino, guanidino, cyanoguanidino, aryl
optionally substituted with one or more groups independently selected from hydroxy, halogen, trihalomethyl, C₁₋₅ alkyl, C₁₋₅ alkoxy, cyano, nitro, amino and carboxy,
and aryloxy
optionally substituted with one or more groups independently selected from hydroxy, halogen, trihalomethyl, C₁₋₅ alkyl, C₁₋₅ alkoxy, cyano, nitro, amino and carboxy;
R₆ is selected from hydrogen, hydroxy, halogen, trihalomethyl. C₁₋₅ alkyl, C₁₋₅ alkoxy, cyano, nitro, amino and carboxy,
R₇ and R₈ are independently selected from hydrogen, hydroxy, halogen, trihalomethyl, C₁₋₅ alkyl, C₁₋₅ alkoxy, cyano, nitro, amino and carboxy,
Q, T, U, W and L are independently selected from the group of atoms consisting of C, N, O and S; wherein adjacent atoms U-T, T-Q, U-W, W-L may form one or more double bonds;
R₂ is selected from C₁₋₈ alkyl, C₁₋₈ alkenyl and C₁₋₈ alkynyl
substituted with one or more groups independently selected from carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, acyloxy, acylamino, aryl
substituted with one or more groups independently selected from C₁₋₅ alkyl or C₁₋₅ alkoxy, wherein the alkyl or alkoxy is substituted with carboxy or alkyloxycarbonyl, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, aryloxy, arylmethyloxy, acylamino, hydroxy, and halogen,
heteroaryl
substituted with one or more groups independently selected from C₁₋₅ alkyl or C₁₋₅ alkoxy which are substituted with carboxy or alkyloxycarbonyl, acylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, hydroxy, and halogen,
C₁₋₅ alkoxy
optionally substituted with one or more groups independently selected from C₁₋₅ alkyl or C₁₋₅ alkoxy which may be optionally substituted with carboxy or alkyloxycarbonyl, cyano, nitro, amino, acylamino, alkylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido. arylsulfonamido, sulfonyl, alkylsulfonyl, arylsulfonyl, sulfamoyl, alkylsulfamoyl, arylsulfamoyl, alkylsulfonamide, arylsulfonamide, alkylthio, acyl, acyloxy, hydrazino, hydroxyamino, amidino, guanidino, cyanoguanidino, hydroxy, and halogen,
arylmethyloxy
substituted with one or more groups independently selected from C₁₋₅ alkyl or C₁₋₅ alkoxy which are substituted with carboxy or alkyloxycarbonyl, acylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, hydroxy; and halogen,
and C₃₋₇ cycloalkyl
substituted with one or more groups independently selected from C₁₋₅ alkyl or C₁₋₅ alkoxy which is substituted with carboxy or alkyloxycarbonyl, acylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide and arylsulfonamide;
R₃ is selected from C₁₋₈ alkyl, C₁₋₈ alkenyl and C₁₋₈ alkynyl
optionally substituted with one or more groups independently selected from the group consisting of carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, alkylthio, halogen, acyloxy, hydroxy, nitro, amino, acylamino, alkylamino, cyano, aryl
optionally substituted with one or more groups independently selected from the group consisting of C₁₋₅, alkyl or C₁₋₅, alkoxy, wherein the alkyl or alkoxy may be optionally substituted with carboxy or alkyloxycarbonyl, cyano, nitro, amino, acylamino, alkylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, alkylthio, acyl, acyloxy, aryloxy, arylmethyloxy, hydrazino, hydroxyamino, amidino, guanidino, cyanoguanidino, hydroxy, and halogen,
heteroaryl
optionally substituted with one or more groups independently selected from the group consisting of C₁₋₅ alkyl or C₁₋₅ alkoxy which may be optionally substituted with carboxy or alkyloxycarbonyl, cyano, nitro, amino, acylamino, alkylamino, carboxy, carbarmoyl, alkylcarbamoyl, arylcarbamoyl, alkylsulfonylcarbamoyl, arylsulfonylcarbamoyl, alkyloxycarbonyl, tetrazolyl, isoxazolyl; isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, alkylthio, acyl, acyloxy, hydrazino, hydroxyamino, amidino, guanidino, cyanoguanidino, hydroxy, and halogen,
C₁₋₅ alkoxy
optionally substituted with one or more groups independently selected from the group consisting of C₁₋₅, alkyl or C₁₋₅ alkoxy which may be optionally substituted with carboxy or alkyloxycarbonyl, cyano, nitro, amino, acylamino, alkylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, alkylthio, acyl, acyloxy, hydrazino, hydroxyamino, amidino, guanidino, cyanoguanidino, hydroxy, and halogen,
arylmethyloxy
optionally substituted with one or more groups independently selected from the group consisting of C₁₋₅ alkyl or C₁₋₅ alkoxy which is optionally substituted with carboxy or alkyloxycarbonyl, cyano, nitro, amino, acylamino, alkylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, alkylthio, acyl, acyloxy, hydrazino, hydroxyamino, amidino, guanidino, cyanoguanidino, hydroxy, and halogen,
C₃₋₇ cycloalkyl optionally substituted with one or more groups independently selected from the group consisting of C₁₋₅ alkyl or C₁₋₅ alkoxy which is optionally substituted with carboxy or alkyloxycarbonyl, cyano, nitro, amino, acylamino, alkylamino, carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, alkylthio, acyl, acyloxy, hydrazino, hydroxyamino, amidino, guanidino, cyanoguanidino, hydroxy, and halogen, and heterocycle; provided that none of R₁, R₂, and R₃ bond together;
further provided that Ar is not 2-hydroxy-5-methoxyphenyl, 2-hydroxy-5-(lower) alkoxyphenyl, and when Ar is pyrene, chrysene or phenanthrene then m≠3 and Z≠N⁺R₁R₂R₃ with R₁=R₂=Me and R₃=Bz and further provided that when
Ar is phenyl,
Z is 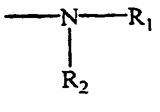 and R₂ is methyl, then R₁ is not

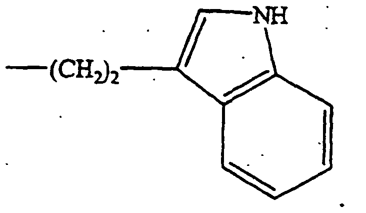

2. The compound according to claim 1, wherein Z is

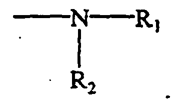

3. The compound according to claim 2, wherein m is 3, 4 or 5.

4. The compound according to claim 3, wherein is 3 or 4.

5. The compound of claim 1, wherein R₂ is independently selected from C₁₋₈ alkyl, C₁₋₈ alkenyl and C₁₋₈ alkynyl, substituted with one or more groups independently selected from carboxy, carbamoyl, alkylcarbamoyl, arylcarbamoyl, alkylsulfonylcarbamoyl, arylsulfonylcarbamoyl, alkyloxycarbonyl, tetrazolyl, isoxazolyl, isothiazolyl, alkylsulfonamido, arylsulfonamido, sulfonyl, alkylsulfonyl, arylsulfonyl, sulfamoyl, alkylsulfamoyl, arylsulfamoyl, alkylsulfonamide, arylsulfonamide, and acylamino.

6. The compound of claim 5, wherein R₂ is independently selected from C₁₋₈ alkyl, C₁₋₈ alkenyl and C₁₋₈ alkynyl, substituted with one or more groups independently selected from carboxy and alkyloxycarbonyl.

7. The compound according to claim 1, wherein Z is

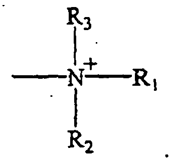

8. The compound according to claim 7, wherein m is 3, 4 or 5,

9. The compound according to claim 8, wherein m is 3 or 4.

10. The compound according to claim 9, wherein R₃ is C₁₋₈ alkyl optionally substituted with one or more groups independently selected from the group consisting of hydroxy, halogen, trihalomethyl, C₁₋₅ alkyl, C₁₋₅ alkoxy, cyano, nitro, amino, and carboxy.

11. The compound according to claim 1, wherein
Ar is as defined in claim 1 except for aryl substituted with trihalomethoxy;
R₂ is selected from C₁₋₈-alkyl, C₁₋₈-alkenyl and C₁₋₈₋alkynyl, substituted with one or more groups as defined in claim 1, except for
(i) substituted acyloxy,
(ii) carboxy, carbamoyl, alkylcarbamoyl, arylcarbamoyl, alkylsulfonylcarbamoyl, arylsulfonylcarbamoyl, alkoxycarbonyl, tetrazolyl, isoxazolyl, isothiazolyl, alkylsulfonylamido, arylsulfonylamido, sulfonyl, alkylsulfonyl, arylsulfonyl, acylamino, aryl, each substituted with acylamino, aryloxy, arylmethyloxy and
(iii) optionally substituted C₁₋₅-alkoxy;
R₃ is selected from C₁₋₈-alkyl, C₁₋₈-alkenyl and C₁₋₈₋alkynyl, optionally substituted with one or more groups as defined in claim 1, except for
(i) optionally substituted acyloxy, and
(ii) carboxy, carbamoyl, alkylcarbamoyl, arylcarbamoyl, alkylsulfonylcarbamoyl, arylsulfonylcarbamoyl, alkoxycarbonyl, tetrazolyl, isoxazolyl, isothiazolyl, alkylsulfonylamido, arylsulfonylamido, sulfonyl, alkylsulfonyl, arylsulfonyl, acylamino, aryl, each substituted with aryloxy, arylmethyloxy and
(iii) optionally substituted C₁₋₅-alkoxy; and
R₄' is hydrogen.

12. The compound according to claim 11, wherein Ar is aryl
optionally substituted with one or more groups independently selected from hydroxy, halogen, trihalomethyl, C₁₋₅alkyl, C₁₋₅alkoxy, cyano, nitro, amino, carboxy, carbamoyl, alkylcarbamoyl, arylcarbamoyl, acyl, acyloxy, sulfonyl, alkylsulfonyl; arylsulfonyl, sulfamoyl, alkylsulfamoyl, arylsulfamoyl, alkylthio, alkylsulfonamide, arylsulfonamide, hydrazino, acylamino, alkylamino, hydroxyamino, amidino, guanidino, cyanoguanidino, aryl
optionally substituted with one or more groups independently selected from the group consisting of hydroxy, halogen, trihalomethyl, C₁₋₅alkyl, C₁₋₅ alkoxy, cyano, nitro, amino and carboxy,
and aryloxy
optionally substituted with one or more groups independently selected from hydroxy, halogen, trihalomethyl, C₁₋₅alkyl, C₁₋₅ alkoxy, cyano, nitro, amino and carboxy.

13. The compound according to claim 1 selected from :
N-Phenylcarbamoyl-N' -[2-(4-chlorophenyl)ethyl]-N' -ethyl-1,3-diaminopropane;
N-(4-Nitrophenylcarbamoyl-N'-[2-(4-chlorophenyl)ethyl]-N'-ethyl-1,3-diaminopropane;
N-(4-Bromophenylcarbamoyi-N'-[2-(4-chloropbenyl)ethyl]-N'-ethyl-1,3-diaminopropane;
N-Phenylcarbamoyl-N'-[2-(4-*chlorophenyl*)ethyl]-N'-propyl-1,3-diaminopropane;
Methyl 4-[[3-(4-bromophenylureido)propyl] (1,2,3,4-tetrahydro-1-naphthyl)amino] butylate;
Methyl 4-([3-(4-bromophenylureido)propyl] [(1*R*)-1-phenylethyl]amino]butylate;
Methyl 4-[[3-(4-bromophenylureido)propyl][2-(4-chlorophenyl)ethyl]amino] butylate;
Methyl 4-[[4-(4-bromophenylureido)butyl](1,2,3,4-tetrahydro-1-naphthyl)amino] butylate;
Methyl 4-[[5-(4-bromophenylureido)pentyl](1,2,3,4-tetrahydro-1-naphthyl)amino] burylate;
Methyl 4-[[3-(4-methylphenylureido)propyl](1,2,3,4-tetrahydro-1-naphthyl)amino] butylate;
Methyl 4-([3-(3,4-dichlorophenylureido)propyl](1,2,3,4-tetrahydro-1-naphthyl)amino] butylate;
4-[[3-(4-Bromophenylureido)propyl](1,2,3,4-tetrahydro-1-naphthyl)amino] butanoic acid;
4-[[3-(4-Bromophenylureido)propyl][(1*R*)-1-phenylethyl]amino] butanoic acid;
4-[[4-(4-Bromophenylureido)butyl](1,2,3,4-tetrahydro-1-naphthyl)amino]butanoic acid;
4-[[5-(4-Bromophenylureido)pentyl](1,2,3,4-tetrahydro-1-naphthyl)amino]butanoic acid;
4-[[3-(4-Methylphenylureido)propyl](1,2,3,4-tetrahydro-1-naphthyl)amino]butanoic acid;
4-[[3-(3,4-Dichlorophenylureido)propyl](1,2,3,4--tetrahydro-1-naphthyl)anzino]butanoic acid;
[3-(Phenylureido)propyl][2-(4-chlorophenyl)ethyl]diethylammonium iodide;
[3-(4-Bromophenylureido)propyl][2-(4-chlorophenyl)ethyl]diethylammonium iodide;
N-Phenylcarbamoyl-N'-[2-(4-chlorophenyl)ethyl]-N'-ethyl-2-hydroxy-1,3-diaminopropane;
4-[[(3S)-3-(4-Bromophenylureido)-3-(tert-butoxycarboayl)propyl](1,2,3,4-tetrahydro-1-naphthyl)amino]butanoic acid;
4-[[3-(4-Bromophenylureido)-2-hydroxypropyl](1,2,3,4-tetrahydro-1-naphthyl)amino]butanoic acid;
4-[[3-(4-Chlorophenylureido)propyl](1,2,3,4-tetrahydro-1-naphthyl)amino]butanoic:
Methyl 4-[[3-(4-bromophenylureido)propyl](1-indanyl)amino]butylate;
4-[[3-(4-Bromophenylureido)propyl](1-indanyl)amino]butanoic acid;
Methyl 4-[[3-(4-bromophenylureido)propyl][(1*R*)-1-indanyl]amino]burylate;
4-[[3-(4-Bromophenylureido)propyl][(1*R*)-1-indanyl]amino]butanoic acid;
Methyl 4-[[3-(4-bromophenylureido)propyl][(1*R*)-1,2,3,4-tetrahydro-1-naphthyl]amino]butylate;
4-[[3-(4-Bromophenylureido)propyl][(1*R*)-1,2,3,4-tetrahydro-1-naphthyl]amino]butanoic acid;
Ethyl 4-[[3-(4-bromophenylureido)propyl](1,2,3.4-tetrahydro-1-naphthyl)amino)butylate;
4-[[3-(4-Bromophenylureido)propyl](1,2,3,4-tetrahydro-1-naphthyl)amino]butanamide;
3-[[3-(4-Bromophenylureido)propyl](1,2,3,4-tetrahydro-1-naphthyl)amino]-1-[(phenylsulfonyl)carbamoyl]propane;
4-[[3-(4-Bromopbenylureido)propyl](1,2,3,4-tetrahydro-1-naphthyl)amino]-1-butanol;
3-[[3-(4-Bromophenylureido)propyl](1,2,3,4-tetrahydro-1-naphthyl)amino]-1-[1-(triphenylmethyl)tetrazol-5-yl]propane;
3-[[3-(4-Bromophenylureido)propyl](1,2,3,4-tetrahydro-1-naphthyl)amino]-1-(1*H-*tetrazol-5-yl)propane;
Methyl 4-[[3-[4-(carboxy)phenylureido]propyl](1,2,3,4-tetrahydro-1-naphthyl)amino]butylate;
4-[[3-(4-Bromophenylureido)propyl][(1*R*)-1-(4-methoxyphenyl)ethyl]amino]butanoic acid;
4-[[3-[4-(Ethoxycarbonyl)phenylureido]propyl](1,2,3,4-tetrahydro-1-naphthyl)amino]butanoic acid;
4-[[3-(4-Iodophenylureido)propyl](1,2, 3,4-tetrahydro-1-naphthyl)amino]butanoic acid;
4-[[3-[4-(Butoxycarbonyl)phenylureido]propyl](1,2,3,4-tetrahydro-1-naphthyl)amino]butanoic acid;
[3-(Phenylureido)propyl]bis[2-(4-chlorophenyl)ethyl]amine;
4-[[3-(4-Bromophenylureido)propyl][(1*R*)-1-(4-bromohpenyl)ethyl]amino]butanoic acid;
4-[[3-(4-Bromophenylureido)propyl][1-(4-fluorophenyl)ethyl]amino]butanoic acid;
4-[[3-(4-Bromophenylureido)propyl][1-(4-chlorophenyl)ethyl]amino]butanoic acid;
Methyl 4-[[(3S)-3-(4-bromophenylureido)-3-(tert-butoxycarbonyl)propyl](1,2,3,4-tetrahydro-1-naphthyl)amino]butylate;
Methyl 4-[[(3S)-3-(4-bromophenylureido)-3-(isopropylcarbamoyl)propyl](1,2,3,4-tetrahydro-1-naphthyl)amino]butylate;
Methyl 4-[[(3S)-3-(4-bromophenylureido)-3-(benzylcarbamoyl)propyl](1,2,3,4-tetrahydro-1-naphthyl)amino]butylate;
4-[[(3S)-3-(4-Bromophenylureido)-3-(isopropylcarbamoyl)propyl](1,2,3,4-tetrahydro-1-naphthyl)amino]butanoic acid;
4-[[(3S)-3-(4-Bromophenylureido)-3-(benzylcarbamoyl)propyl](1,2,3,4-tetrahydro-1-naphthyl)amino]butanoic acid;
4-[[3-(4-Bromophenylureido)propyl][(1S)-1-(4-bromophenyl)ethyl]amino]butanoic acid;
[3-(Phenylureido)propyl] [2-(4-chlorophenyl)ethyl]bis(4-methylbenzyl)ammonium iodide;
[3-(Phenylureido)propyl][2-(4-chlorophenyl)ethyl](4-chlorobenzyl)ethylammonium iodide;
[3-(Phenylureido)propyl][2-(4-chlorophenyl)ethyl](benzyl)ethylammonium iodide;
[3-(Phenylureido)propyl][2-(3-chlorophenyl)ethyl]diethylammonium iodide;
[3-(4-Bromophenylureido)propyl][(1S)-1-phenylethyl][3-(carboxy)propyl]ethylammonium trifluoroacetate;
[3-(4-Bromophenylureido)propyl][(1*R*)-1-phenylethyl][3-(carboxy)propyl]ethylammnonium trifluoroacetate;
[3-(Phenylureido)propyl][2-(4-chlorophenyl)ethyl][4-(methoxycarbonyl)butyl] ethylammonium iodide;
[3-(Phenylureido)propyl][2-(4-chlorophenyl)ethyl][4-(carboxy)benzyl]ethylammonium iodide;
[5-(Phenylureido)pentyl][2-(4-chlorophenyl)ethyl]diethylammonium iodide;
[3-(Phenylureido)propyl][2-(4-chlorophenyl)ethyl](2-chlorobenzyl)ethylammonium iodide;
[3-(Phenylureido)propyl][2-(4-chlorophenyl)ethyl](2,5-difluorobenzyl)ethylammonium iodide;
[3-(Phenylureido)propyl][2-(4-chlorophenyl)ethyl](3-fluorobenzyl)ethylammonium iodide;
[3-(4-Cyanophenylureido)propyl] [2-(3-chlorophenyl)ethyl][2-(2-methoxyehtoxy)ethyl] ethylammonium iodide; and
[3-(4-Methoxyphenylureido)propyl][2-(3-chlorophenyl)ethyl][2-(2-methoxyehtoxy)ethyl] ethylammonium iodide.

14. The compound according to claim 1, wherein the compound is selected from the following formulae 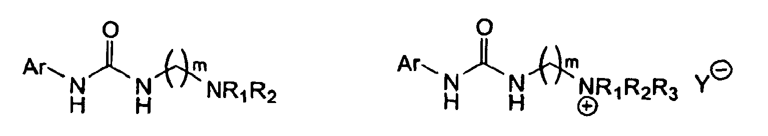 wherein Ar, R₁, R₂, R₃, Y and m are defined as shown in the following Tables:

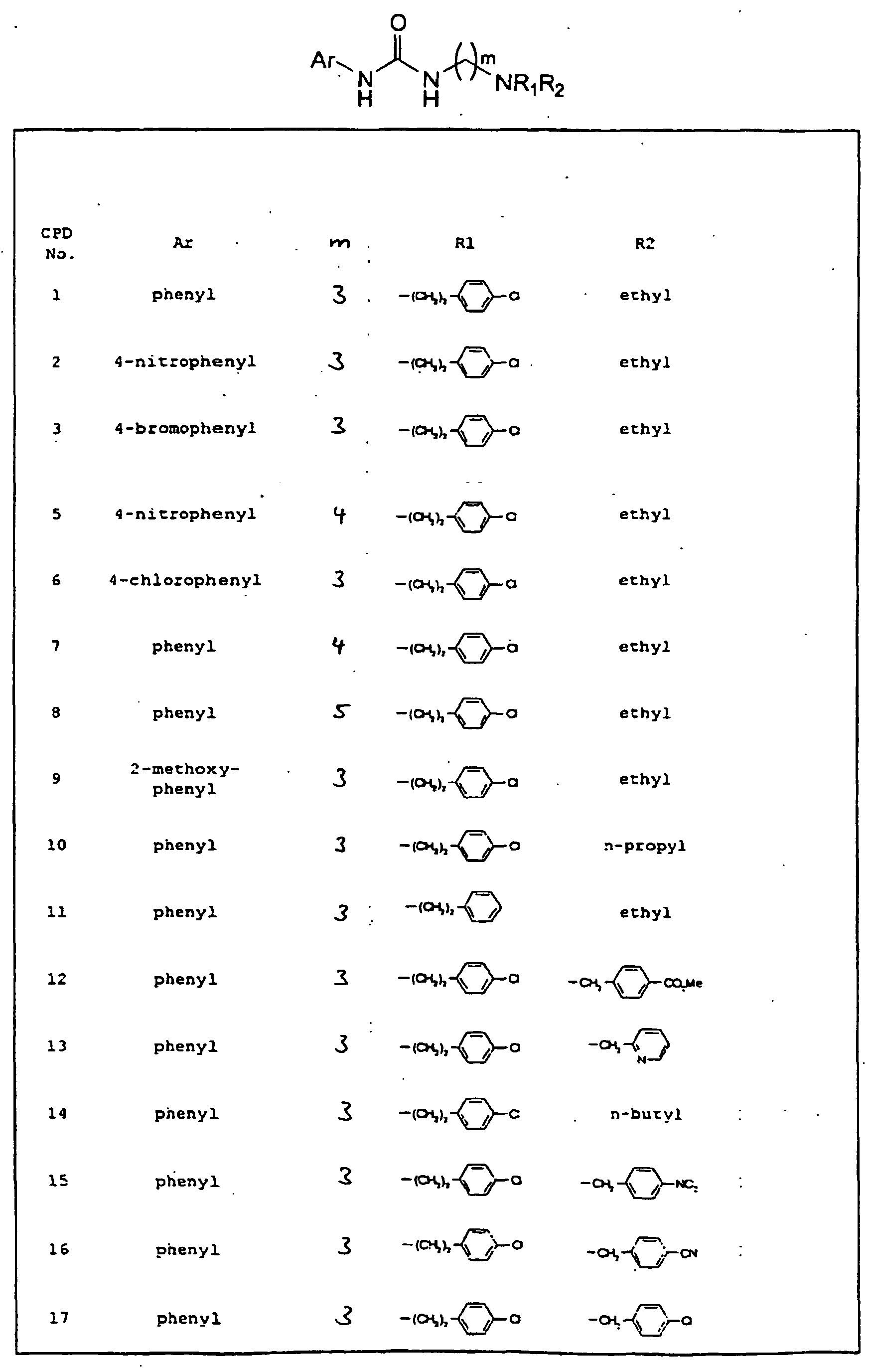

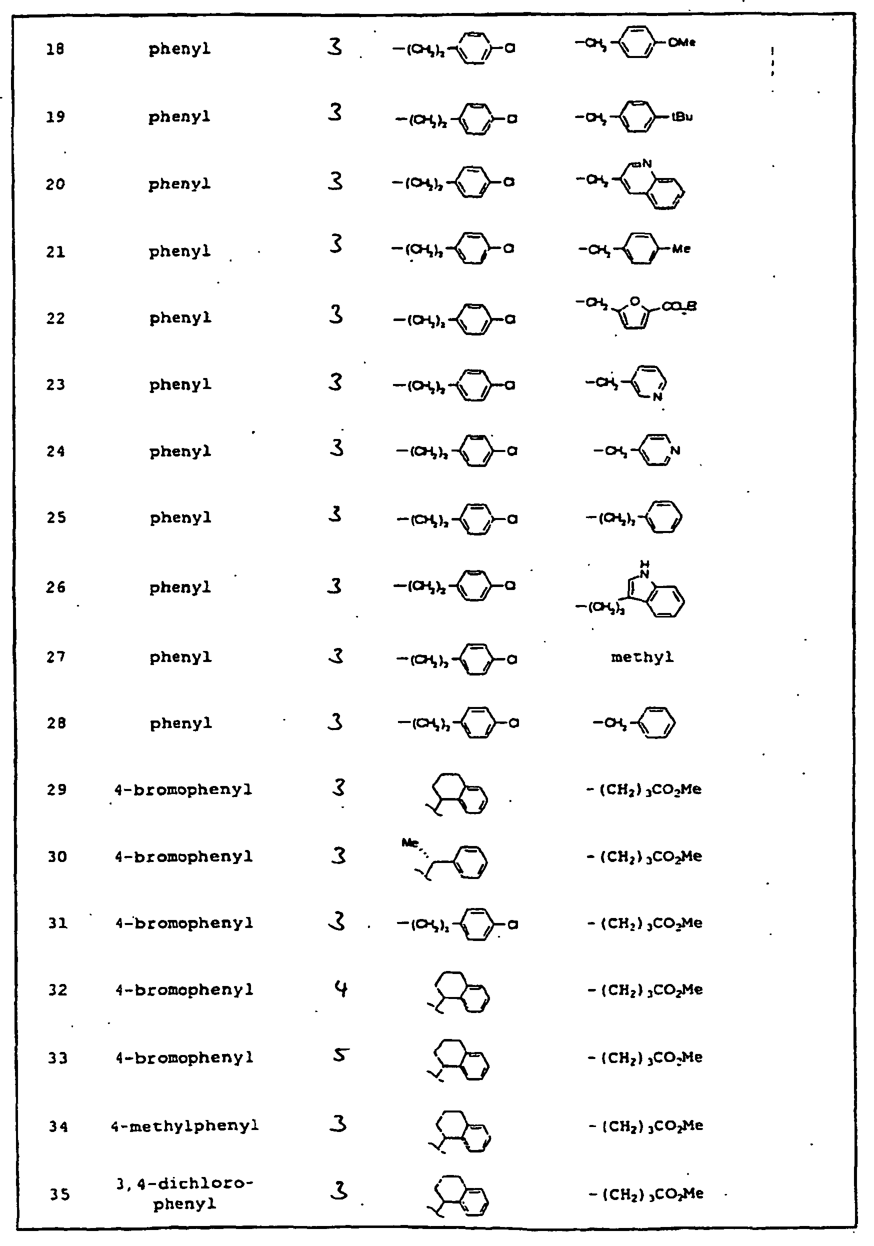

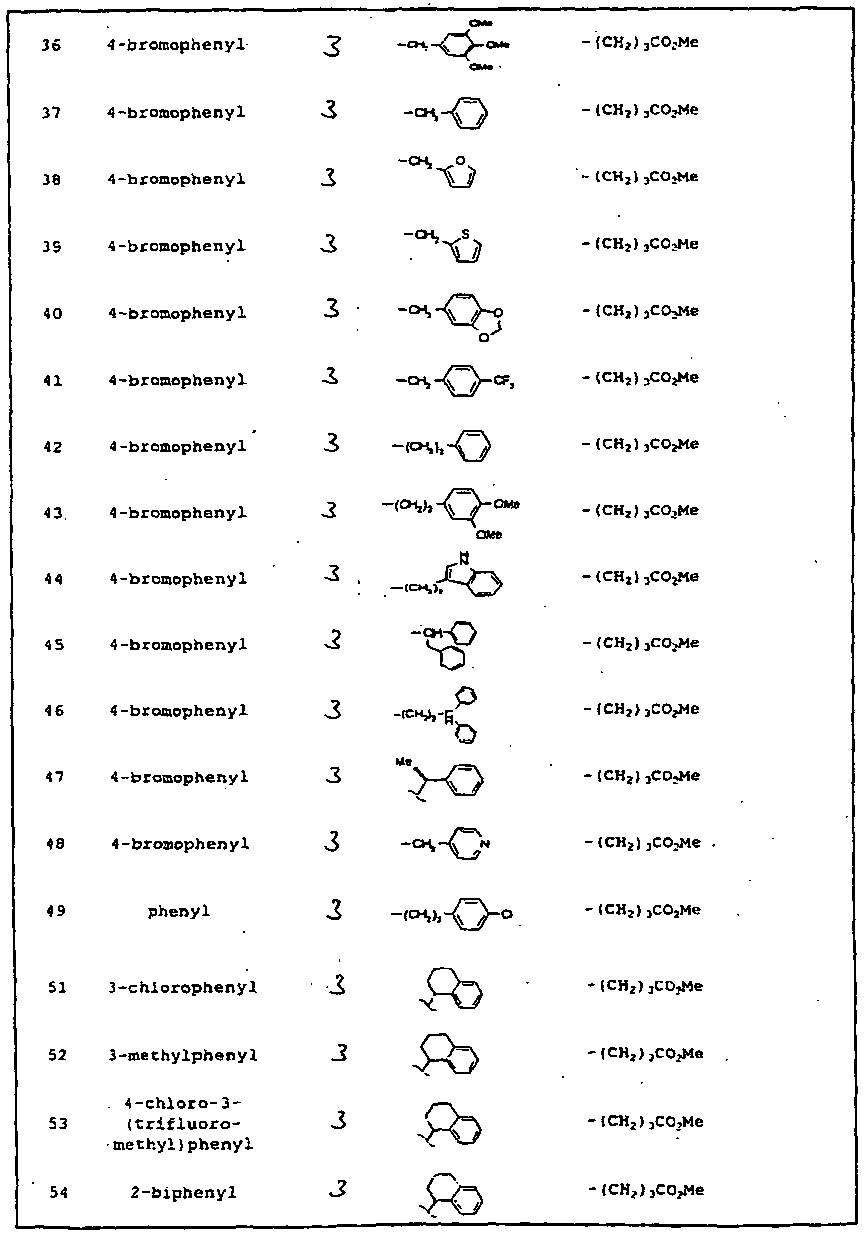

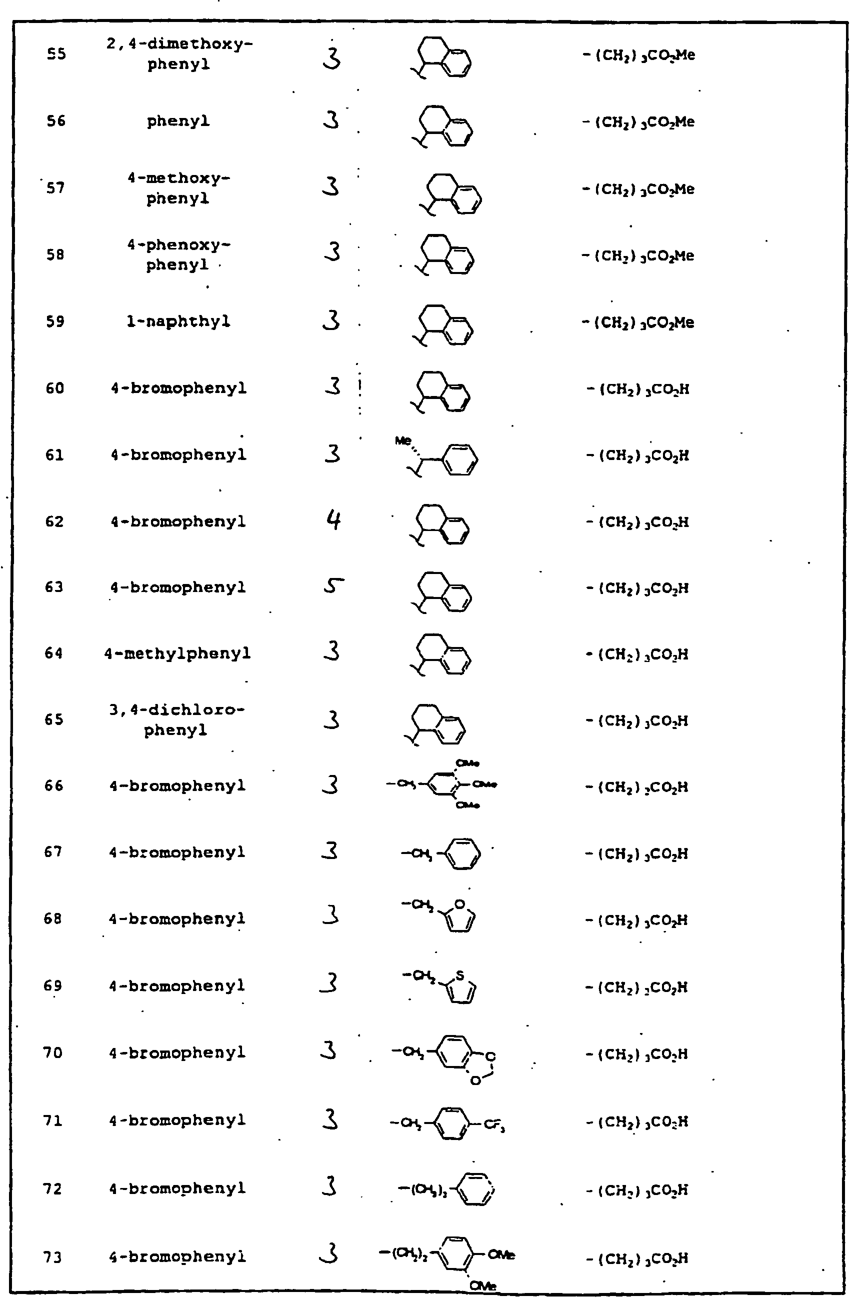

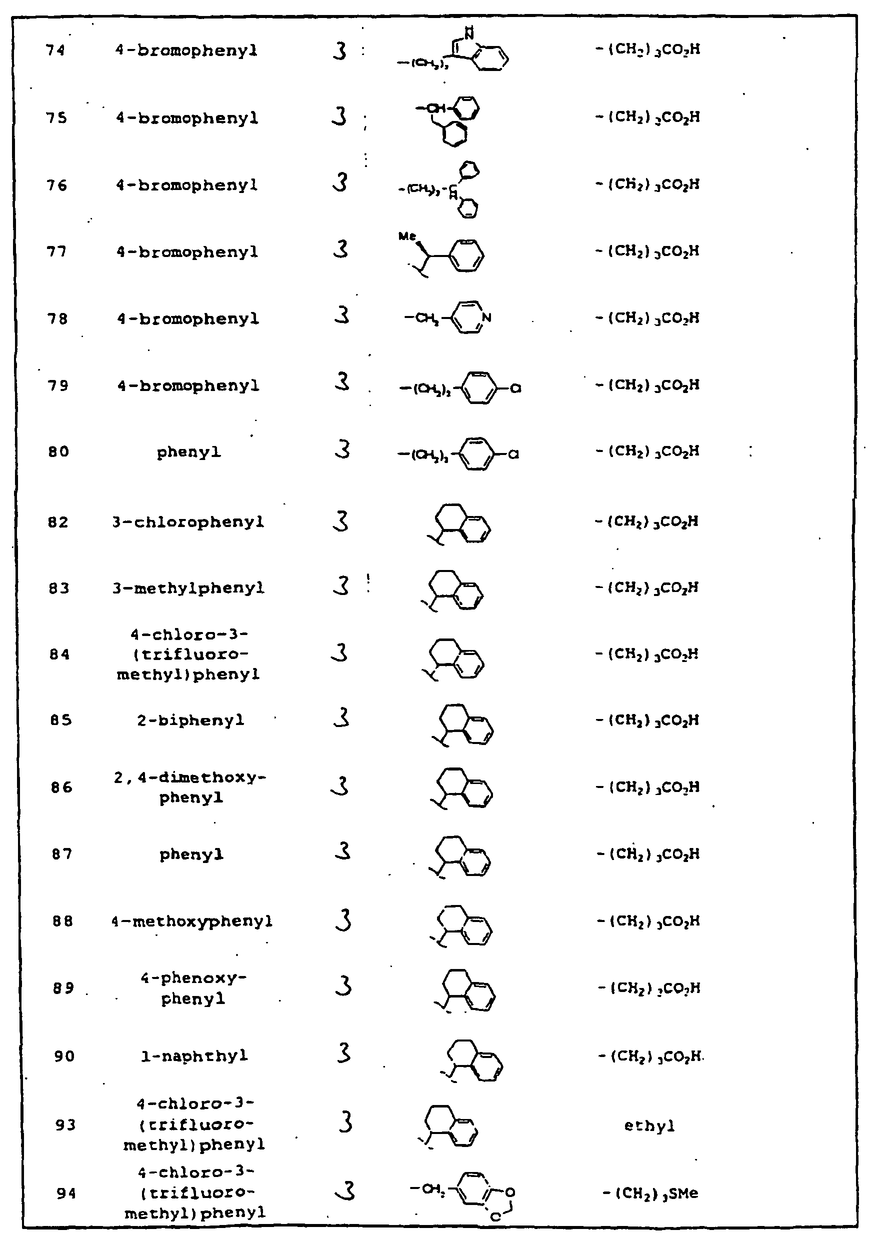

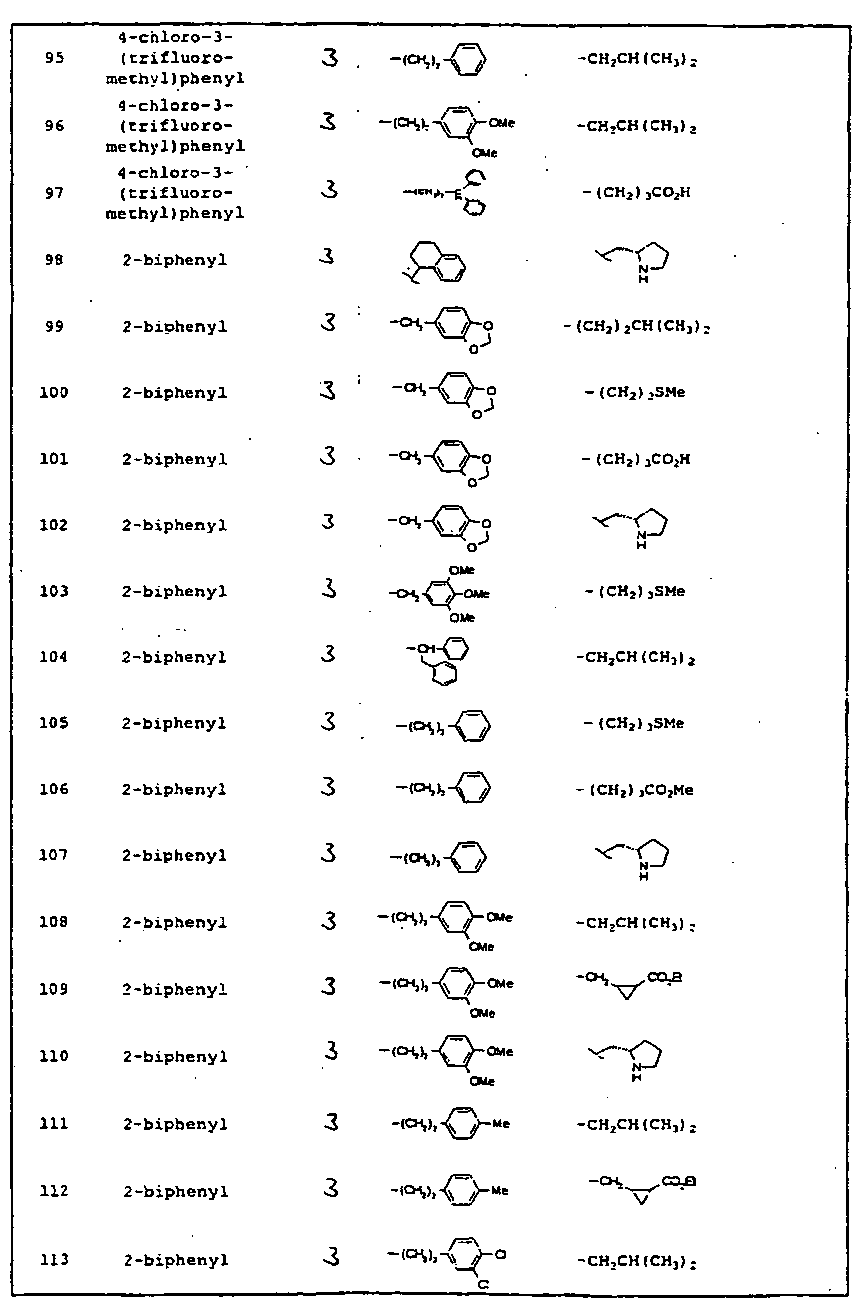

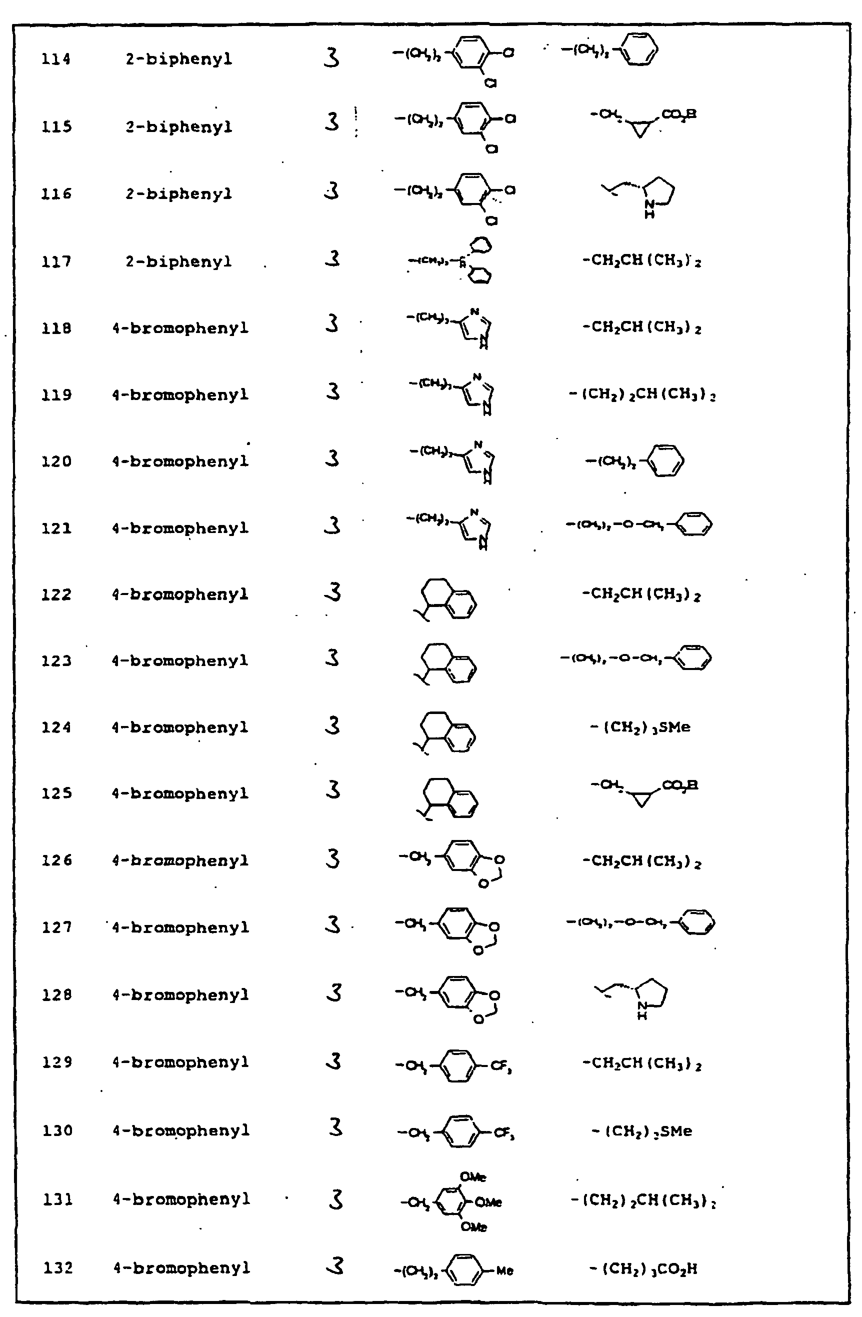

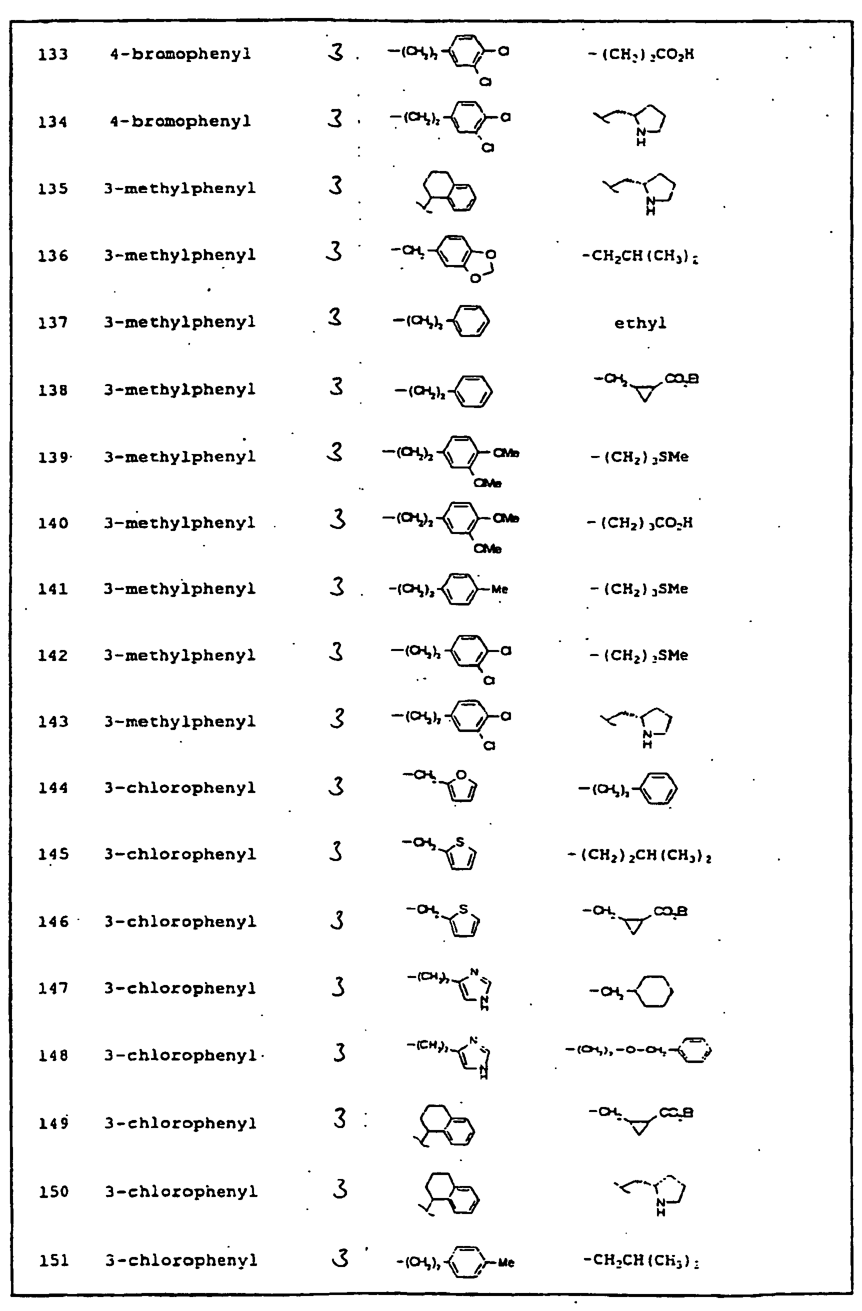

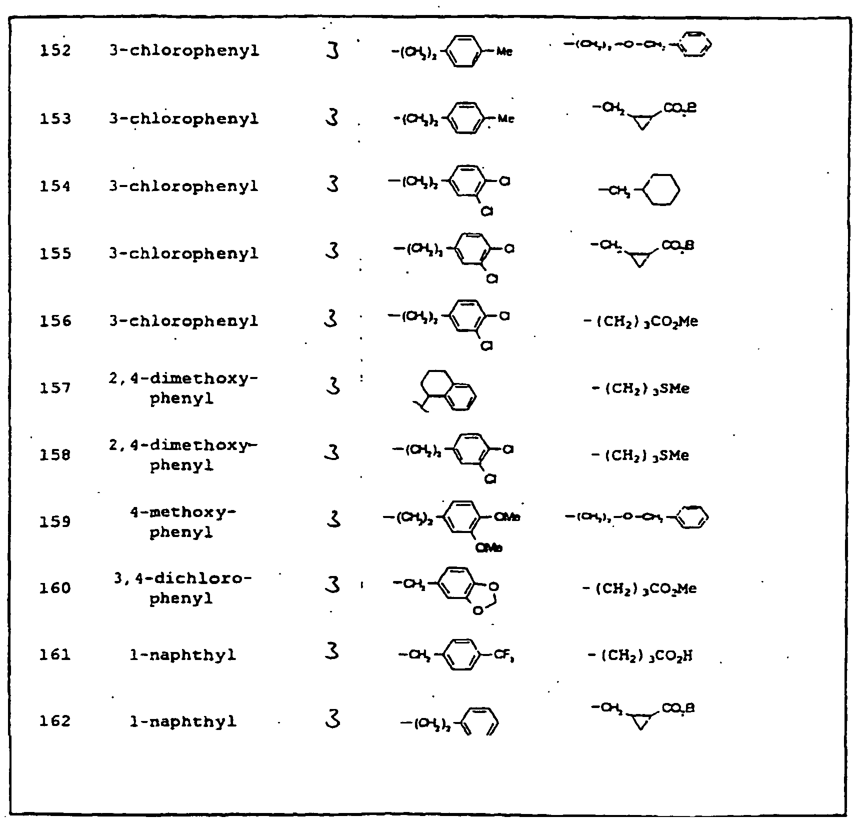

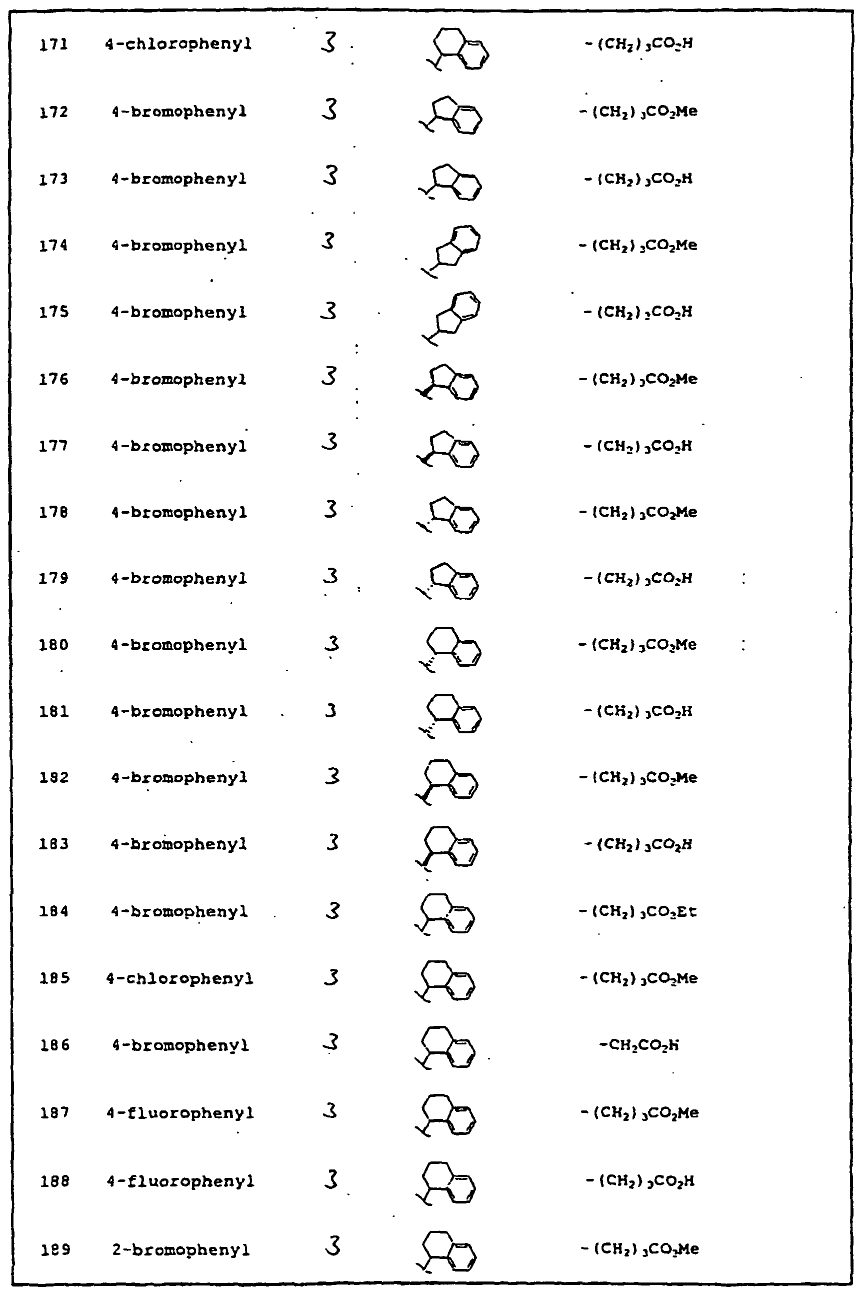

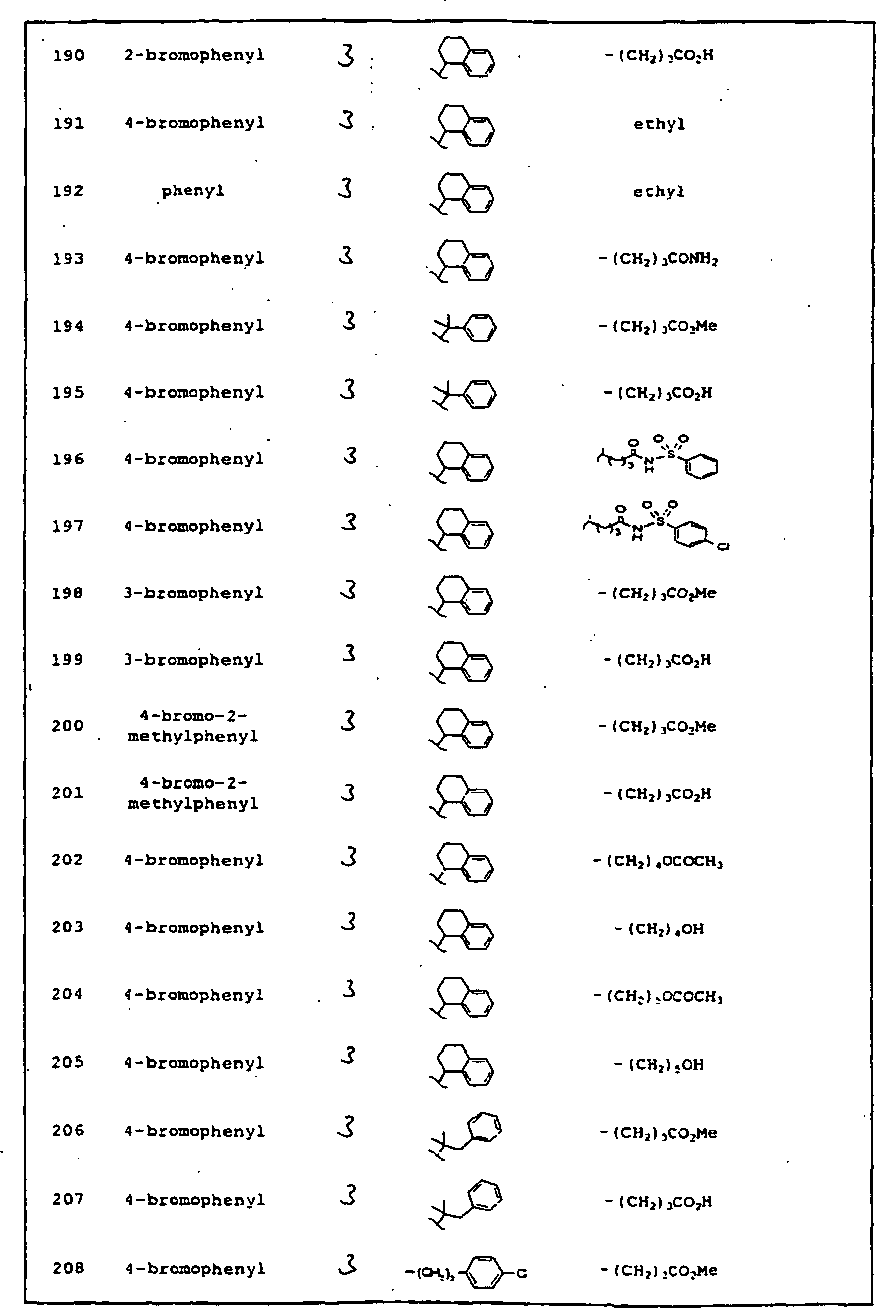

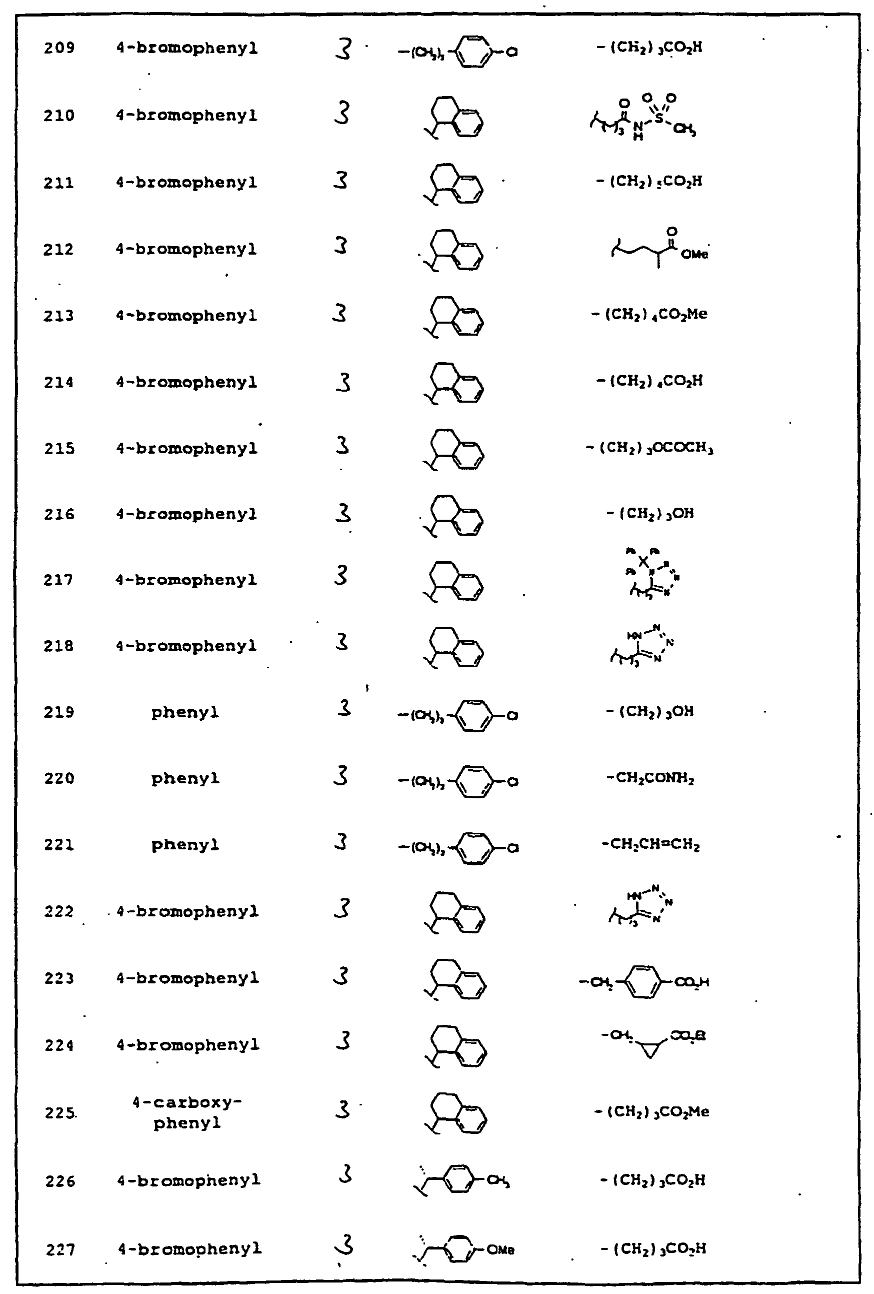

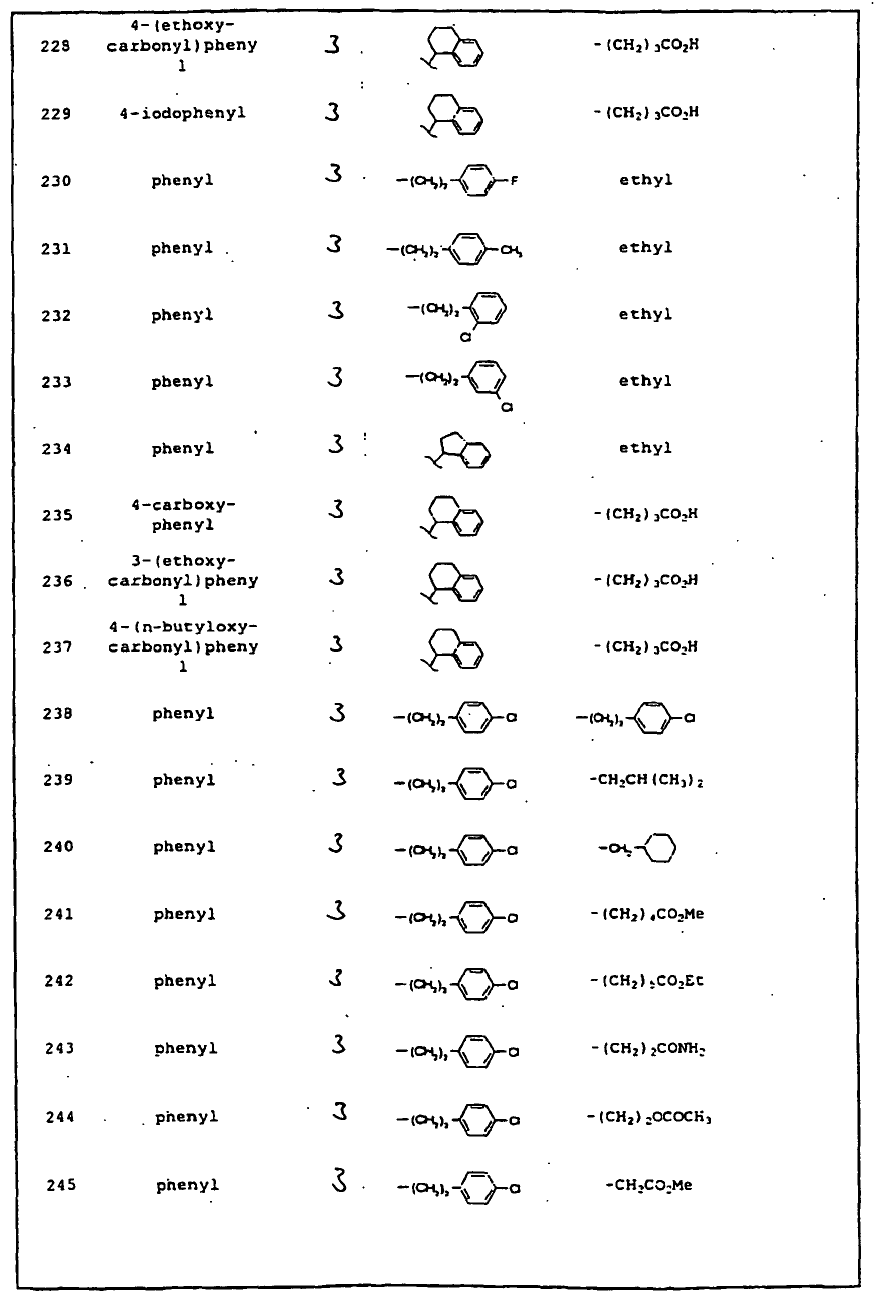

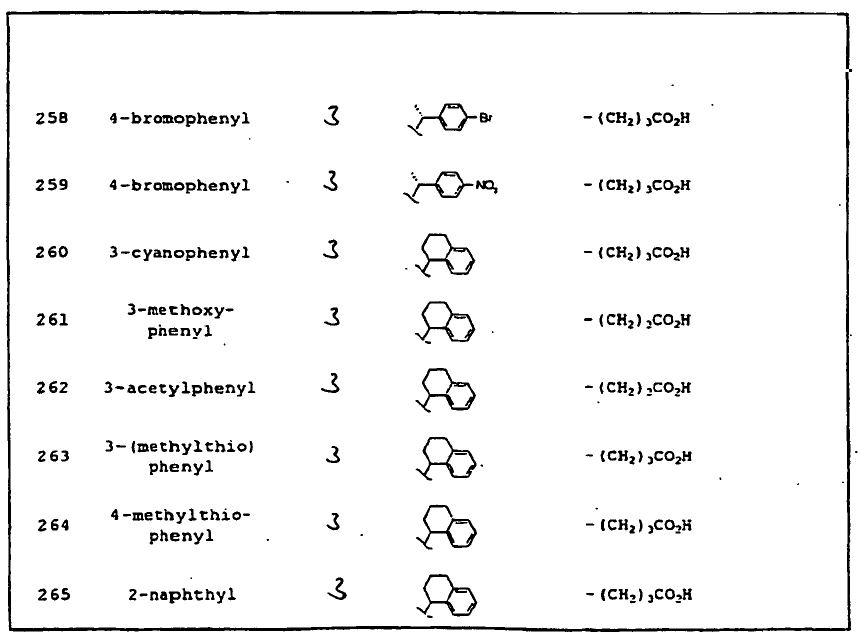

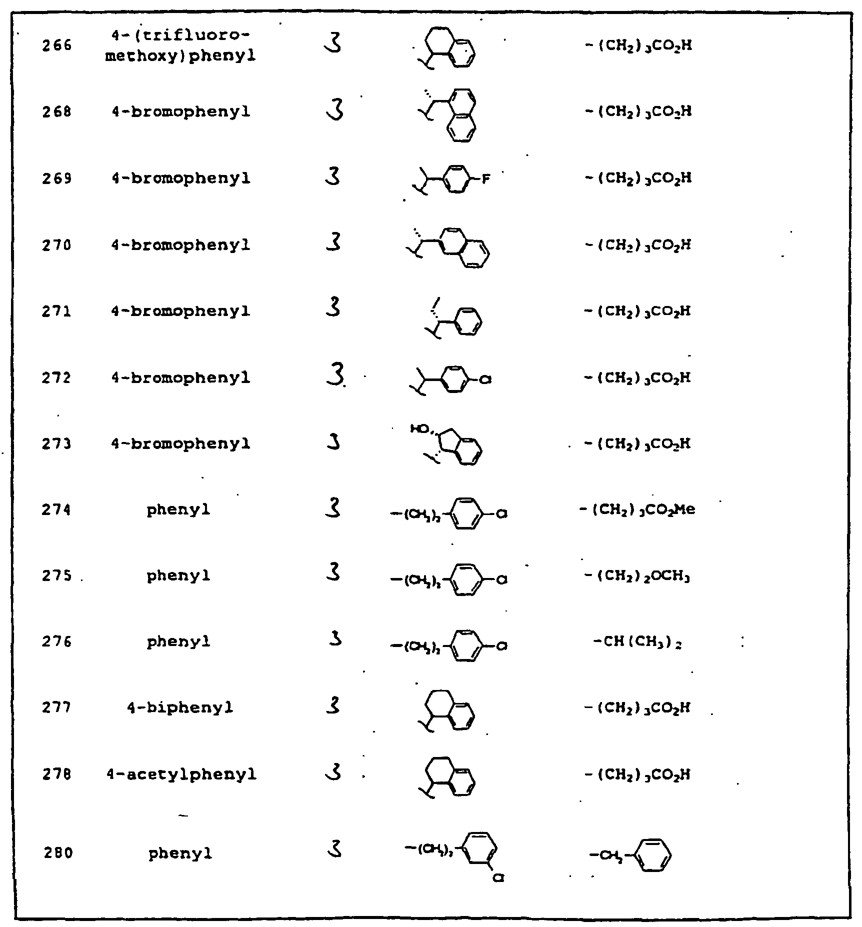

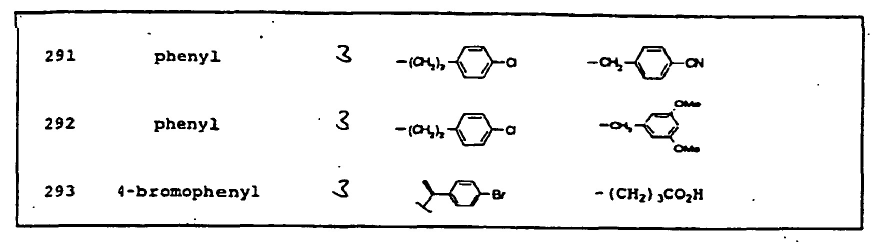

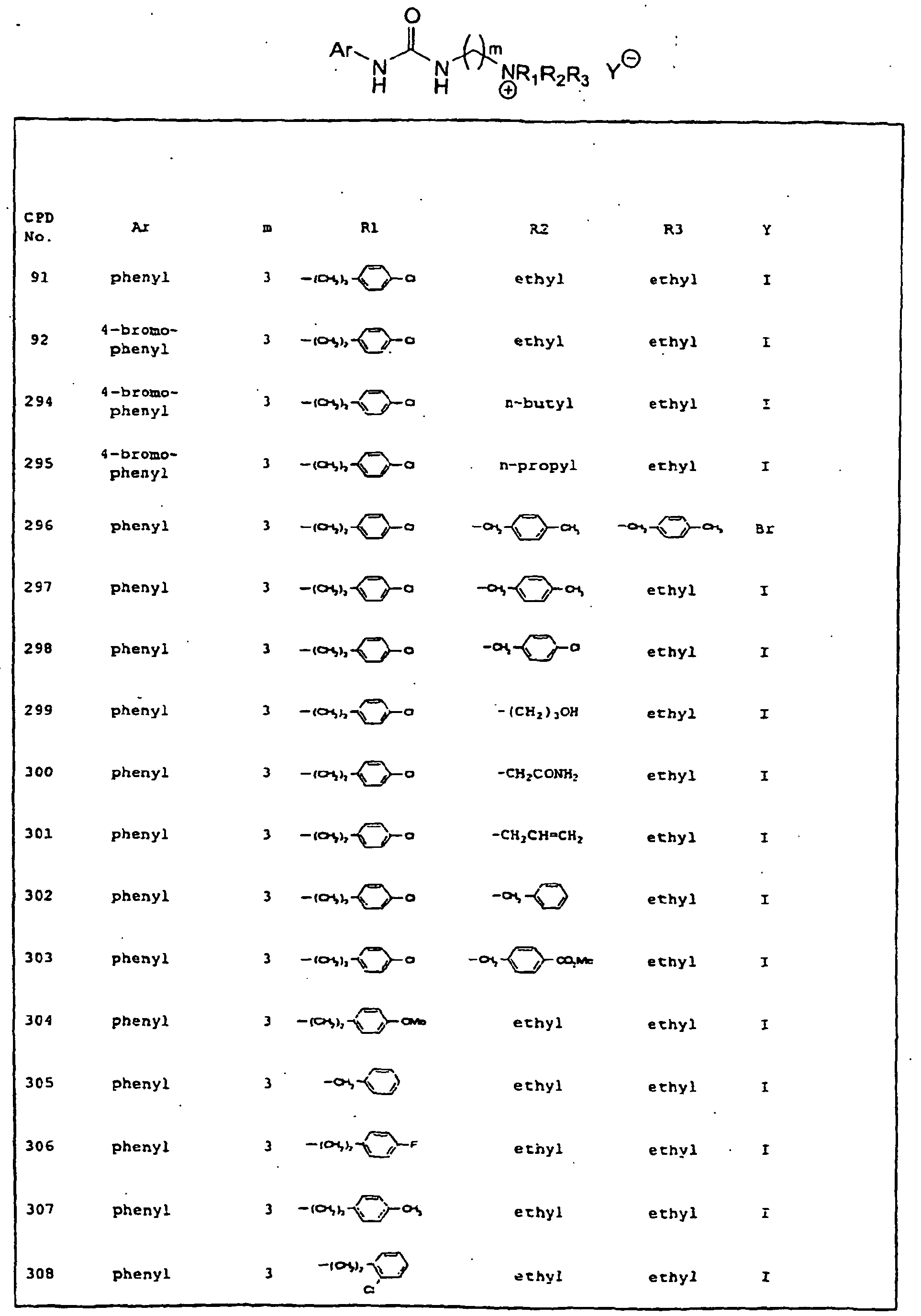

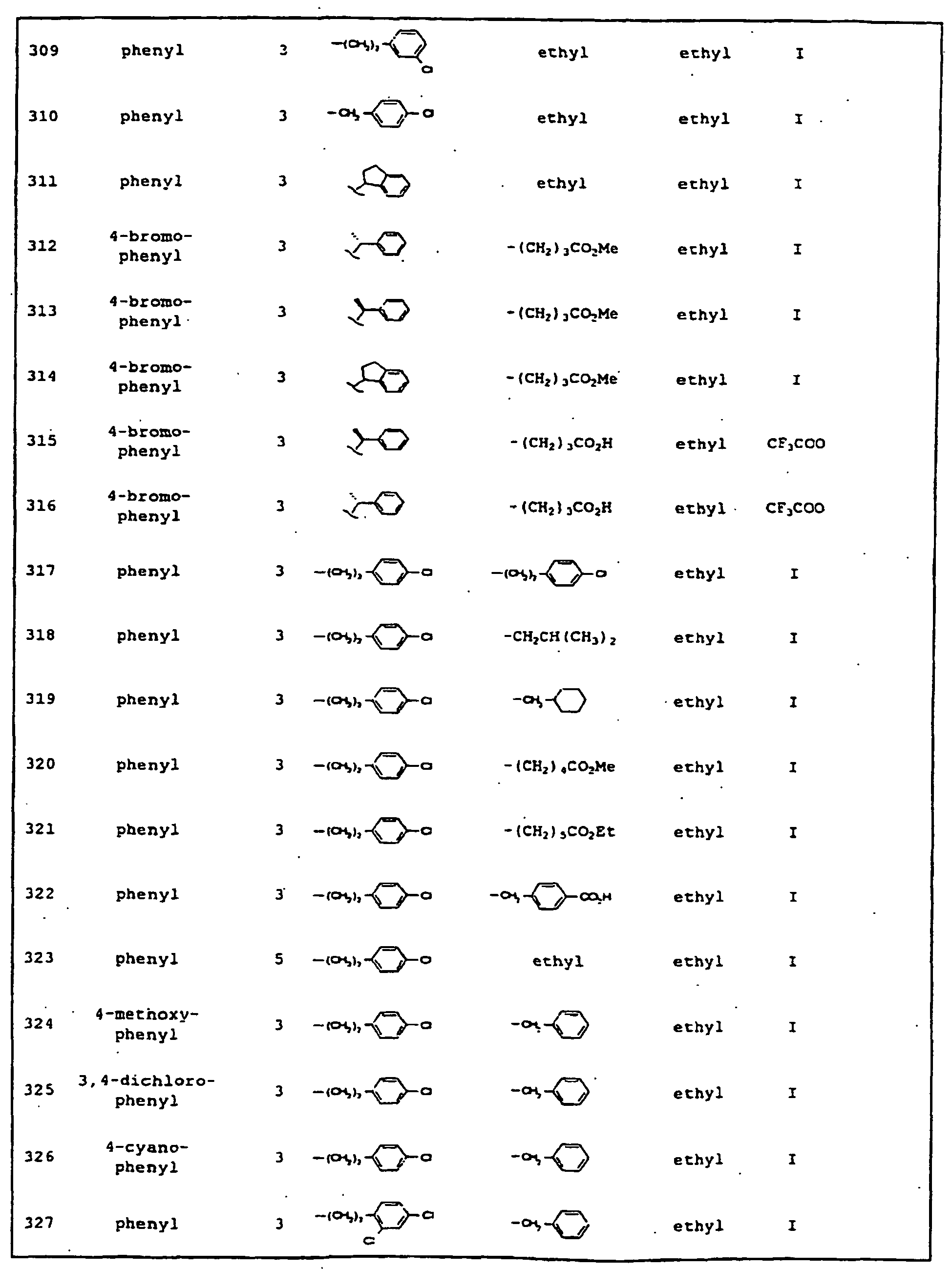

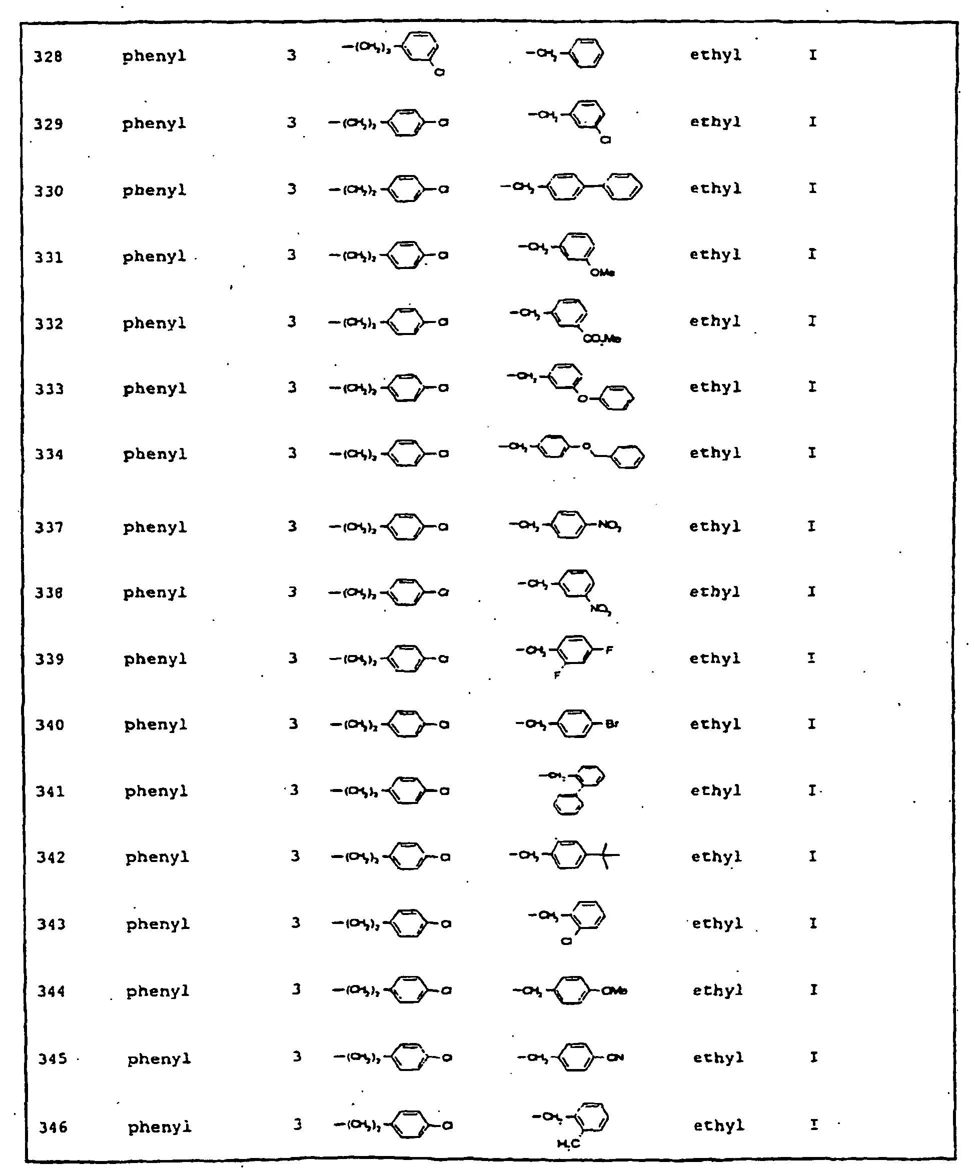

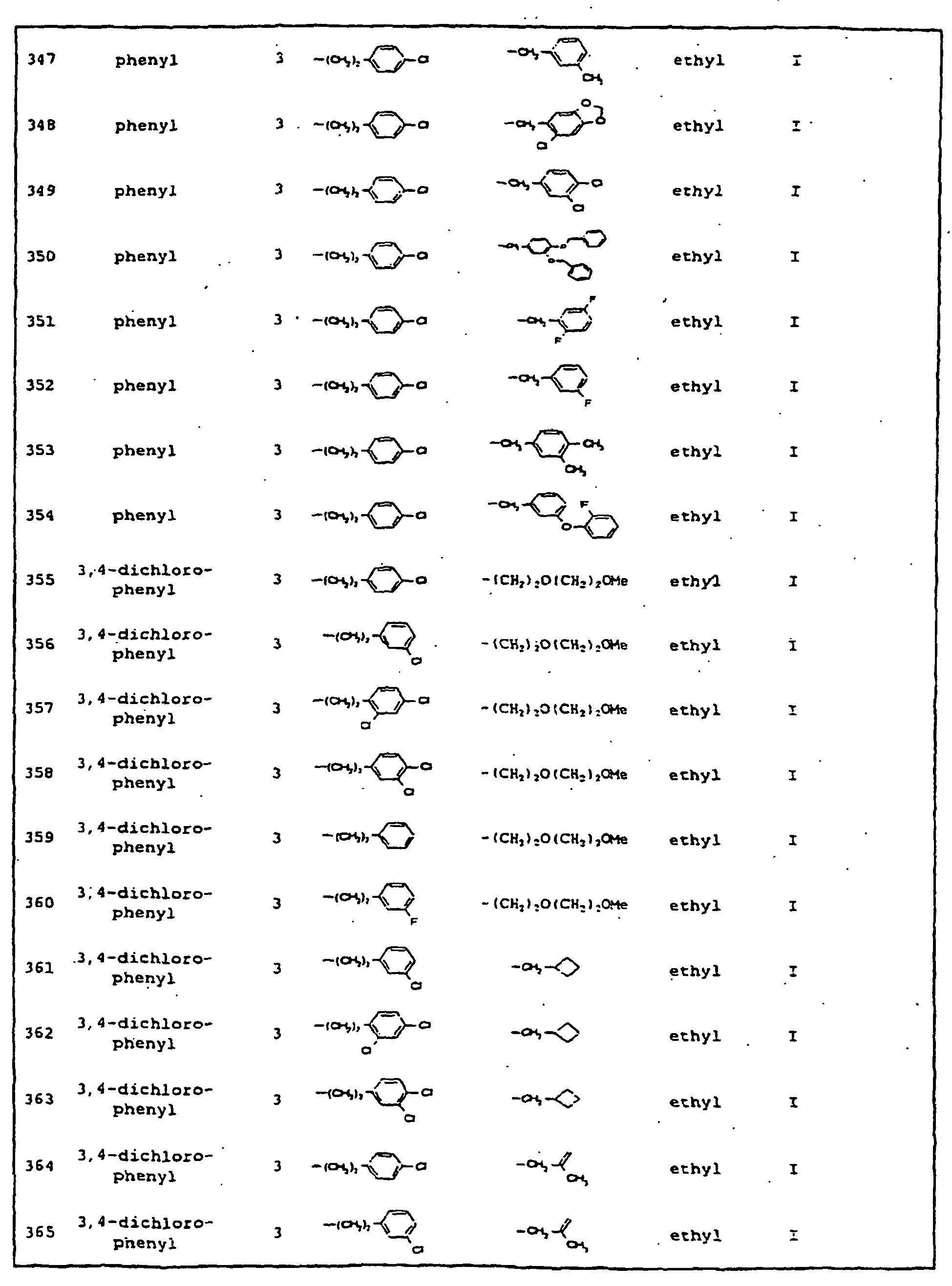

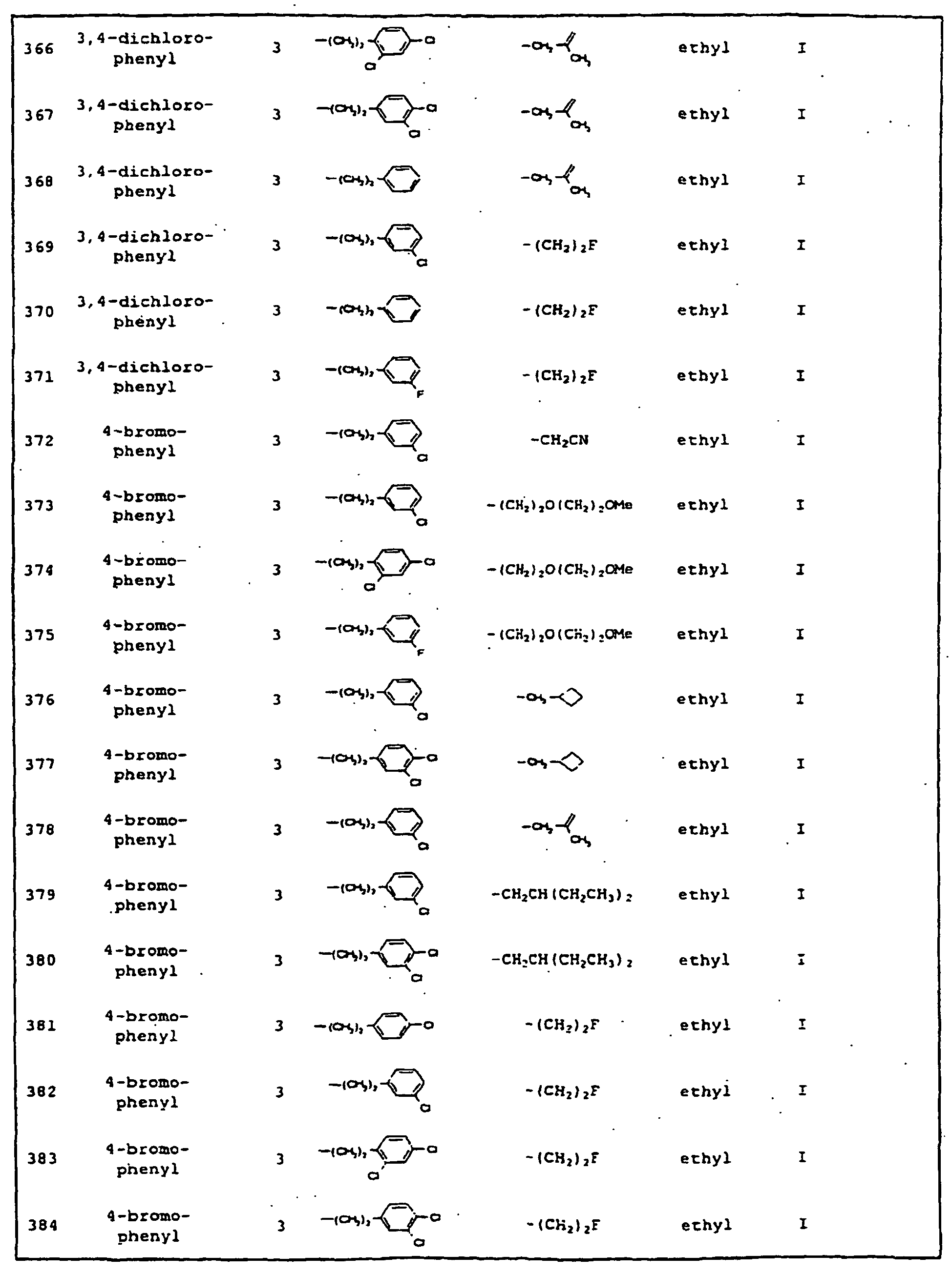

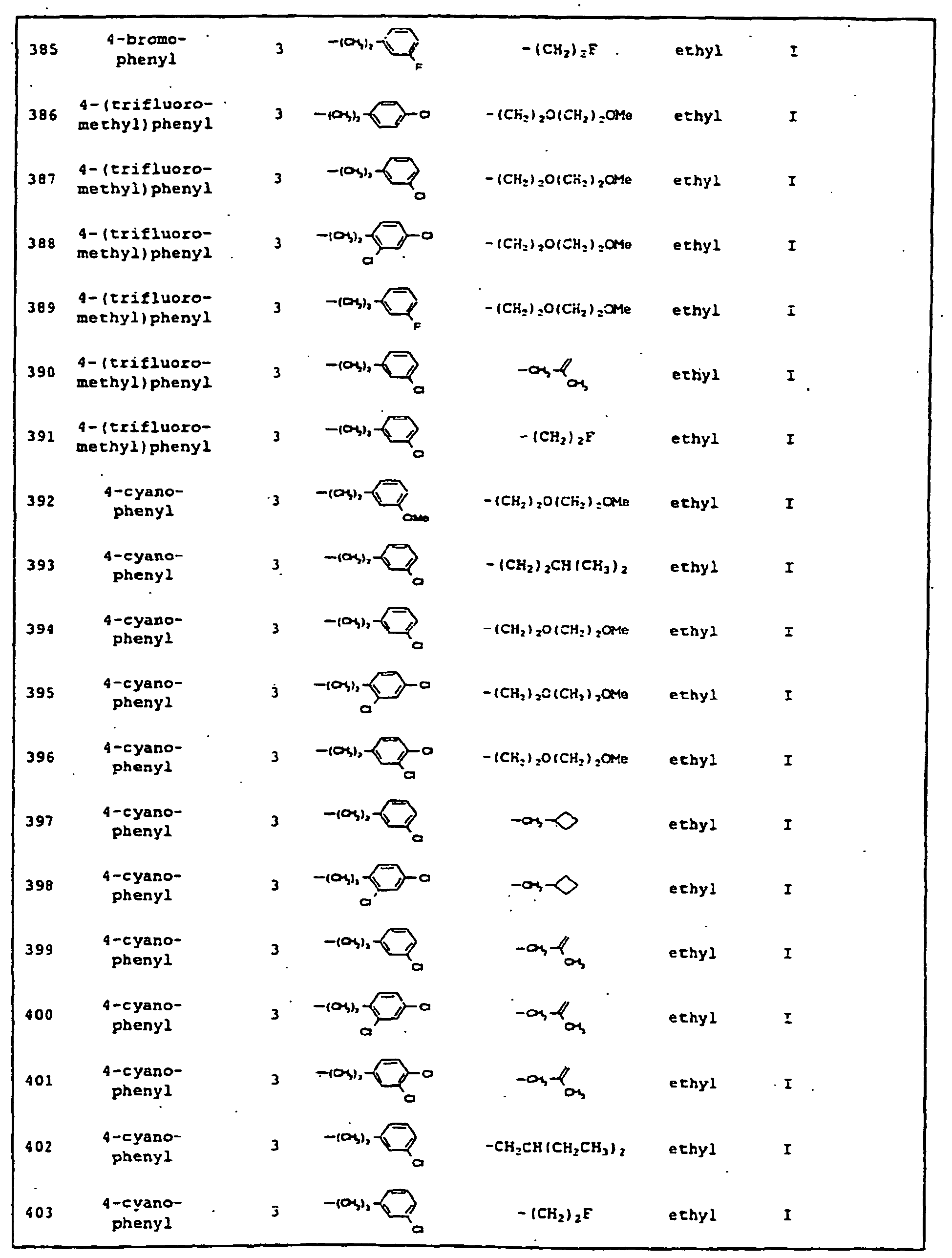

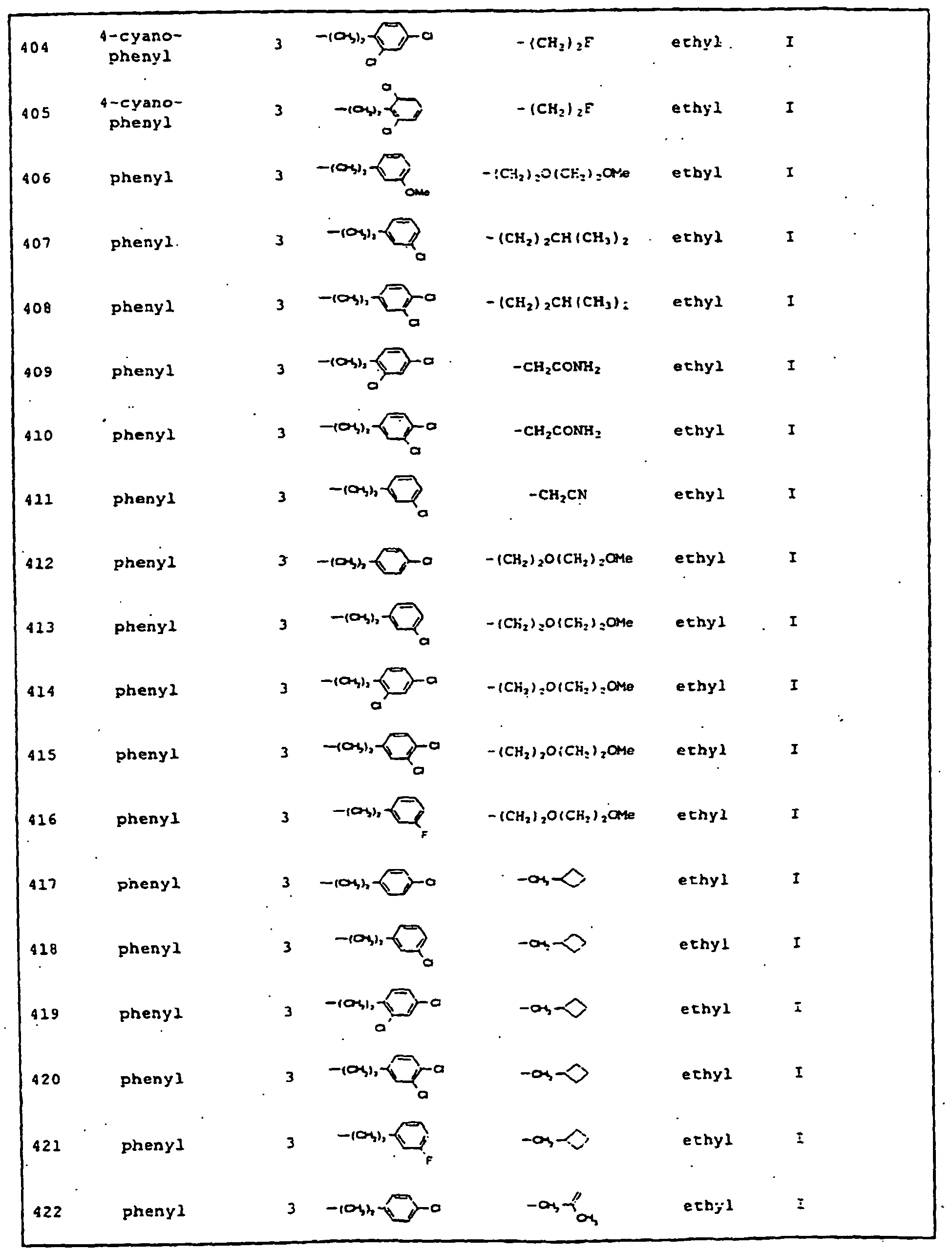

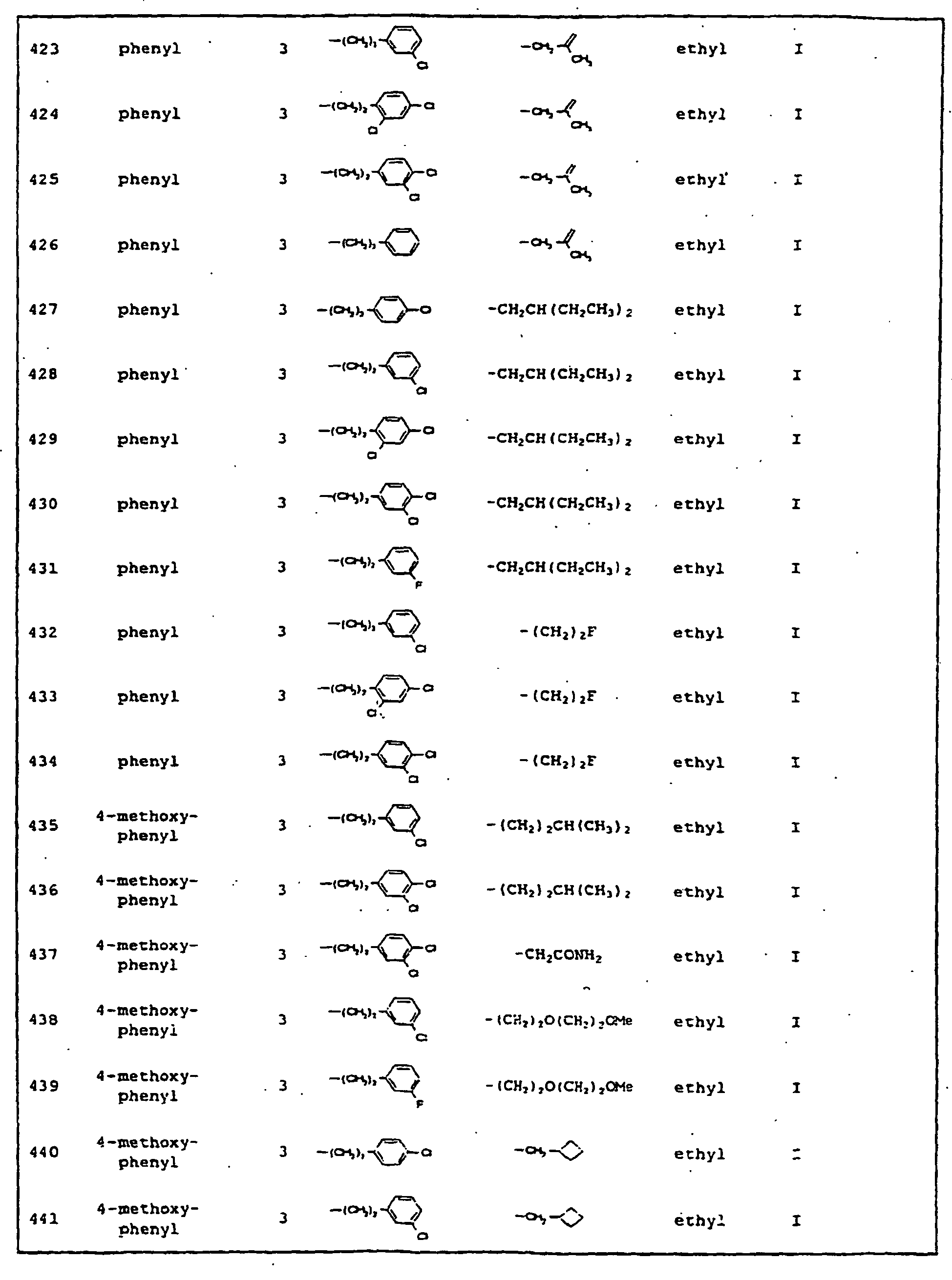

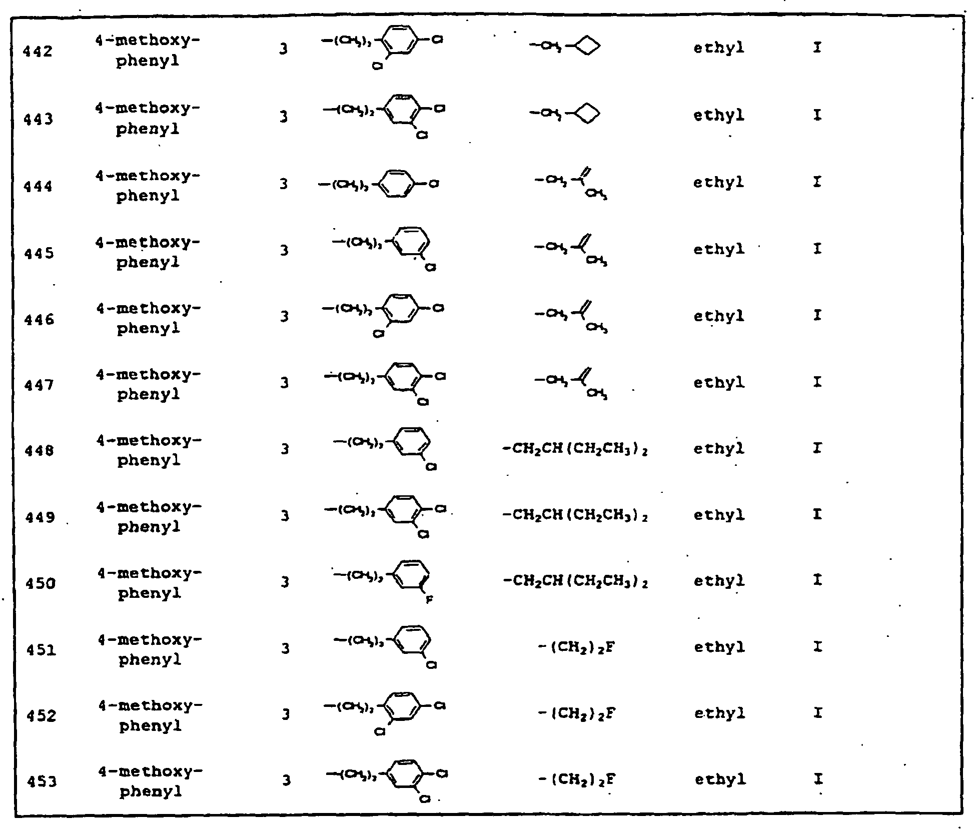

15. Pharmaceutical composition comprising a compound according to any of claims 1-14.

16. Use of a compound according to any of claims 1-14 in the manufacture of a medicament for treating a CCR-3 mediated disease.

17. Use of a compound according to claim 16, wherein the medicament is for treating a CCR-3 mediated disease in a mammal.

18. Use of claim 16 or 17, wherein the CCR-3 mediated disease is an eosinophil-mediated allergic disease.

19. Use of claim 18, wherein the disease is selected from asthma, rhinitis, eczema, inflammatory bowel diseases and parasitic infections.

20. Use of claim 16 or 17, wherein the CCR-3 mediated disease is a T-cell or a dendritic cell mediated disease.

21. The use of claim 20, wherein the disease is selected from autoimmune diseases and HIV.

## Patentansprüche

1. Verbindung der folgenden Formel (I) : 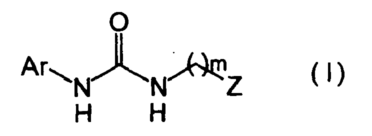 oder ein Salz, Hydrat oder Komplex davon, worin
m 3, 4, 5 oder 6 ist;
Ar ist Aryl oder Heteroaryl
gegebenenfalls substituiert mit einer oder mehreren Gruppe(n), unabhängig voneinander ausgewählt aus Hydroxy, Halogen, Trihalogenmethyl, Trihalogenmethoxy, C₁₋₅-Alkyl, C₁₋₅-Alkoxy, Cyano, Nitro, Amino, Carboxy, Alkyloxycarbonyl, Arylmethyloxycarbonyl, Carbamoyl, Alkylcarbamoyl, Arylcarbamoyl, Acyl, Acyloxy, Sulfonyl, Alkylsulfonyl, Arylsulfonyl, Sulfamoyl, Alkylsulfamoyl, Arylsulfamoyl, Alkylthio, Alkylsulfonamid, Arylsulfonamid, Hydrazino, Acylamino, Alkylamino, Hydroxyamino, Amidino, Guanidino, Cyanoguanidino, Aryl
gegebenenfalls substituiert mit einer oder mehreren Gruppe(n), unabhängig voneinander ausgewählt aus Hydroxy, Halogen, Trihalogenmethyl, C₁₋₅-Alkyl, C₁₋₅-Alkoxy, Cyano, Nitro, Amino und Carboxy
und Aryloxy
gegebenenfalls substituiert mit einer oder mehreren Gruppe(n), unabhängig voneinander ausgewählt aus Hydroxy, Halogen, Trihalogenmethyl, C₁₋₅-Alkyl, C₁₋₅-Alkoxy, Cyano, Nitro, Amino und Carboxy,
Z ist: 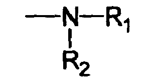 oder 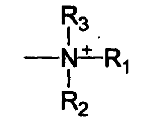 worin R₁ folgendes ist: 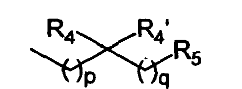 oder 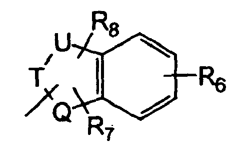 oder 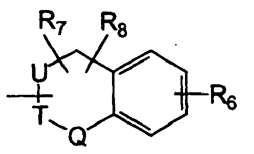 oder 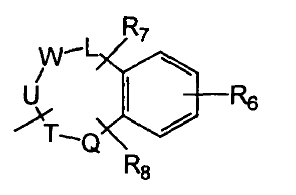 p ist 0, 1 oder 2;
q ist 0, 1 oder 2;
R₄ und R₄' sind unabhängig voneinander ausgewählt aus Wasserstoff, Halogen, C₁₋₅-Alkyl, Aryl, Heteroaryl,
worin das Aryl oder Heteroaryl gegebenenfalls substituiert ist mit einer oder mehreren Gruppe(n), unabhängig voneinander ausgewählt aus Wasserstoff, Hydroxy, Halogen, Trihalogenmethyl, C₁₋₅-Alkyl, C₁₋₅-Alkoxy, Cyano, Nitro, Amino, Carboxy, Carbamoyl, Alkylcarbamoyl, Arylcarbamoyl, Acyl, Acyloxy, Sulfonyl, Alkylsulfonyl, Arylsulfonyl, Sulfamoyl, Alkylsulfamoyl, Arylsulfamoyl, Alkylthio, Alkylsulfonamid, Arylsulfonamid, Hydrazino, Acylamino, Alkylamino, Hydroxyamino, Amidino, Guanidino und Cyanoguanidino;
und COR₉, worin R₉ Hydroxy, C₁₋₅-Alkyl, C₁₋₅-Alkoxy, Amino, Alkylamino oder Arylamino ist;
R₅ ist Aryl oder Heteroaryl,
gegebenenfalls substituiert mit einer oder mehreren Gruppe(n), unabhängig voneinander ausgewählt aus Hydroxy, Halogen, Trihalogenmethyl, C₁₋₅-Alkyl, C₁₋₅₋Alkoxy, Cyano, Nitro, Amino, Carboxy, Carbamoyl, Alkylcarbamoyl, Arylcarbamoyl, Acyl, Acyloxy, Sulfonyl, Alkylsulfonyl, Arylsulfonyl, Sulfamoyl, Alkylsulfamoyl, Arylsulfamoyl Alkylthio, Alkylsulfonamid, Arylsulfonamid, Hydrazino, Acylamino, Alkylamino, Hydroxyamino, Amidino, Guanidino, Cyanoguanidino, Aryl
gegebenenfalls substituiert mit einer oder mehreren Gruppe(n), unabhängig voneinander ausgewählt aus Hydroxy, Halogen, Trihalogenmethyl, C₁₋₅-Alkyl, C₁₋₅-Alkoxy, Cyano, Nitro, Amino und Carboxy,
und Aryloxy
gegebenenfalls substituiert mit einer oder mehreren Gruppe(n), unabhängig voneinander ausgewählt aus Hydroxy, Halogen, Trihalogenmethyl, C₁₋₅-Alkyl, C₁₋₅-Alkoxy, Cyano, Nitro, Amino und Carboxy;
R₆ ist ausgewählt aus Wasserstoff, Hydroxy, Halogen, Trihalogenmethyl, C₁₋₅-Alkyl, C₁₋₅-Alkoxy, Cyano, Nitro, Amino und Carboxy;
R₇ und R₈ sind unabhängig voneinander ausgewählt aus Wasserstoff, Hydroxy, Halogen, Trihalogenmethyl, C₁₋₅-Alkyl, C₁₋₅-Alkoxy, Cyano, Nitro, Amino und Carboxy;
Q, T, U, W und L sind unabhängig voneinander ausgewählt aus den Atomen C, N, O und S, worin benachbarte Atome U-T, T-Q, U-W, W-L eine oder mehrere Doppelbindungen bilden können;
R₂ ist ausgewählt aus C₁₋₈-Alkyl, C₁₋₈-Alkenyl und C₁₋₈-Alkinyl,
substituiert mit einer oder mehreren Gruppe(n), unabhängig voneinander ausgewählt aus Carboxy, Carbamoyl, Alkylcarbamoyl, Arylcarbamoyl, Alkylsulfonylcarbamoyl, Arylsulfonylcarbamoyl, Alkyloxycarbonyl, Tetrazolyl, Isoxazolyl, Isothiazolyl, Alkylsulfonamido, Arylsulfonamido, Sulfonyl, Alkylsulfonyl, Arylsulfonyl, Sulfamoyl, Alkylsulfamoyl, Arylsulfamoyl, Alkylsulfonamid, Arylsulfonamid, Acyloxy, Acylamino, Aryl,
substituiert mit einer oder mehreren Gruppe(n), unabhängig voneinander ausgewählt aus C₁₋₅-Alkyl oder C₁₋₅-Alkoxy, worin die Alkyl- oder Alkoxygruppe mit Carboxy oder Alkyloxycarbonyl substituiert ist, Carboxy, Carbamoyl, Alkylcarbamoyl, Arylcarbamoyl, Alkylsulfonylcarbamoyl, Arylsulfonylcarbamoyl, Alkyloxycarbonyl, Tetrazolyl, Isoxazolyl, Isothiazolyl, Alkylsulfonamido, Arylsulfonamido, Sulfonyl, Alkylsulfonyl, Arylsulfonyl, Sulfamoyl, Alkylsulfamoyl, Arylsulfamoyl, Alkylsulfonamid, Arylsulfonamid, Aryloxy, Arylmethyloxy, Acylamino, Hydroxy und Halogen,
Heteroaryl
substituiert mit einer oder mehreren Gruppe(n), unabhängig voneinander ausgewählt aus C₁₋₅-Alkyl oder C₁₋₅-Alkoxy, die mit Carboxy oder Alkyloxycarbonyl substituiert sind, Acylamino, Carboxy, Carbamoyl, Alkylcarbamoyl, Arylcarbamoyl, Alkylsulfonylcarbamoyl, Arylsulfonylcarbamoyl, Alkyloxycarbonyl, Tetrazolyl, Isoxazolyl, Isothiazolyl, Alkylsulfonamido, Arylsulfonamido, Sulfonyl, Alkylsulfonyl, Arylsulfonyl, Sulfamoyl, Alkylsulfamoyl, Arylsulfamoyl, Alkylsulfonamid, Arylsulfonamid, Hydroxy und Halogen,
C₁₋₅-Alkoxy
gegebenenfalls substituiert mit einer oder mehreren Gruppe(n), unabhängig voneinander ausgewählt aus C₁₋₅-Alkyl oder C₁₋₅-Alkoxy, die gegebenenfalls mit Carboxy oder Alkyloxycarbonyl substituiert sein können, Cyano, Nitro, Amino, Acylamino, Alkylamino, Carboxy, Carbamoyl, Alkylcarbamoyl, Arylcarbamoyl, Alkylsulfonylcarbamoyl, Arylsulfonylcarbamoyl, Alkyloxycarbonyl, Tetrazolyl, Isoxazolyl, Isothiazolyl, Alkylsulfonamido, Arylsulfonamido, Sulfonyl, Alkylsulfonyl, Arylsulfonyl, Sulfamoyl, Alkylsulfamoyl, Arylsulfamoyl, Alkylsulfonamid, Arylsulfonamid, Alkylthio, Acyl, Acyloxy, Hydrazino, Hydroxyamino, Amidino, Guanidino, Cyanoguanidino, Hydroxy und Halogen,
Arylmethyloxy
substituiert mit einer oder mehreren Gruppe(n), unabhängig voneinander ausgewählt aus C₁₋₅-Alkyl oder C₁₋₅-Alkoxy, die mit Carboxy oder Alkyloxycarbonyl substituiert sind, Acylamino, Carboxy, Carbamoyl, Alkylcarbamoyl, Arylcarbamoyl, Alkylsulfonylcarbamoyl, Arylsulfonylcarbamoyl, Alkyloxycarbonyl, Tetrazolyl, Isoxazolyl, Isothiazolyl, Alkylsulfonamido, Arylsulfonamido,, Sulfonyl, Alkylsulfonyl, Arylsulfonyl, Sulfamoyl, Alkylsulfamoyl, Arylsulfamoyl, Alkylsulfonamid, Arylsulfonamid, Hydroxy und Halogen,
und C₃₋₇-Cycloalkyl
substituiert mit einer mehr mehreren Gruppe(n), unabhängig voneinander ausgewählt aus C₁₋₅-Alkyl oder C₁₋₅-Alkoxy, das mit Carboxy oder Alkyloxycarbonyl substituiert ist, Acylamino, Carboxy, Carbamoyl, Alkylcarbamoyl, Arylcarbamoyl, Alkylsulfonylcarbamoyl, Arylsulfonylcarbamoyl, Alkyloxycarbonyl, Tetrazolyl, Isoxazolyl, Isothiazolyl, Alkylsulfonamido, Arylsulfonamido, Sulfonyl, Alkylsulfonyl, Arylsulfonyl, Sulfamoyl, Alkylsulfamoyl, Arylsulfamoyl, Alkylsulfonamid und Arylsulfonamid;
R₃ ist ausgewählt aus C₁₋₈-Alkyl, C₁₋₈-Alkenyl und C₁₋₈-Alkinyl
gegebenenfalls substituiert mit einer oder mehreren Gruppe(n), unabhängig voneinander ausgewählt aus Carboxy, Carbamoyl, Alkylcarbamoyl, Acylcarbamoyl, Alkylsulfonylcarbamoyl, Arylsulfonylcarbamoyl, Alkyloxycarbonyl, Tetrazolyl, Isoxazolyl, Isothiazolyl, Alkylsulfonamido, Arylsulfonamido, Sulfonyl, Alkylsulfonyl, Arylsulfonyl, Sulfamoyl, Alkylsulfamoyl, Arylsulfamoyl, Alkylsulfonamid, Arylsulfonamid, Alkylthio, Halogen, Acyloxy, Hydroxy, Nitro, Amino, Acylamino, Alkylamino, Cyano, Aryl
gegebenenfalls substituiert mit einer oder mehreren Gruppe(n), unabhängig voneinander ausgewählt aus C₁₋₅-Alkyl oder C₁₋₅-Alkoxy, worin die Alkyl- oder Alkoxygruppe gegebenenfalls mit Carboxy oder Alkyloxycarbonyl substituiert sein kann, Cyano, Nitro, Amino, Acylamino, Alkylamino, Carboxy, Carbamoyl, Alkylcarbamoyl, Arylcarbamoyl, Alkylsulfonylcarbamoyl, Arylsulfonylcarbamoyl, Alkyloxycarbonyl, Tetrazolyl, Isoxazolyl, Isothiazolyl, Alkylsulfonamido, Arylsulfonamido, Sulfonyl, Alkylsulfonyl, Arylsulfonyl, Sulfamoyl, Alkylsulfamoyl, Arylsulfamoyl, Alkylsulfonamid, Arylsulfonamid, Alkylthio, Acyl, Acyloxy, Aryloxy, Arylmethyloxy, Hydrazino, Hydroxyamino, Amidino, Guanidino, Cyanoguanidino, Hydroxy und Halogen,
Heteroaryl
gegebenenfalls substituiert mit einer oder mehreren Gruppe(n), unabhängig voneinander ausgewählt aus C₁₋₅-Alkyl oder C₁₋₅-Alkoxy, die gegebenenfalls mit Carboxy oder Alkyloxycarbonyl substituiert sein können, Cyano, Nitro, Amino, Acylamino, Alkylamino, Carboxy, Carbamoyl, Alkylcarbamoyl, Arylcarbamoyl, Alkylsulfonylcarbamoyl, Arylsulfonylcarbamoyl, Alkyloxycarbonyl, Tetrazolyl, Isoxazolyl, Isothiazolyl, Alkylsulfonamido, Arylsulfonamido, Sulfonyl, Alkylsulfonyl, Arylsulfonyl, Sulfamoyl, Alkylsulfamoyl, Arylsulfamoyl, Alkylsulfonamid, Arylsulfonamid, Alkylthio, Acyl, Acyloxy, Hydrazino, Hydroxyamino, Amidino, Guanidino, Cyanoguanidino, Hydroxy oder Halogen,
C₁₋₅-Alkoxy
gegebenenfalls substituiert mit einer oder mehreren Gruppe(n), unabhängig voneinander ausgewählt aus C₁₋₅-Alkyl oder C₁₋₅-Alkoxy, das gegebenenfalls mit Carboxy oder Alkyloxycarbonyl substituiert sein kann, Cyano, Nitro, Amino, Acylamino, Alkylamino, Carboxy, Carbamoyl, Alkylcarbamoyl, Arylcarbamoyl, Alkylsulfonylcarbamoyl, Arylsulfonylcarbamoyl, Alkyloxycarbonyl, Tetrazolyl, Isoxazolyl, Isothiazolyl, Alkylsulfonamido, Arylsulfonamido, Sulfonyl, Alkylsulfonyl, Arylsulfonyl, Sulfamoyl, Alkylsulfamoyl, Arylsulfamoyl, Alkylsulfonamid, Arylsulfonamid, Alkylthio, Acyl, Acyloxy, Hydrazino, Hydroxyamino, Amidino, Guanidino, Cyanoguanidino, Hydroxy und Halogen,
Arylmethyloxy
gegebenenfalls substituiert mit einer oder mehreren Gruppe(n), unabhängig voneinander ausgewählt aus C₁₋₅-Alkyl oder C₁₋₅-Alkoxy, das gegebenenfalls mit Carboxy oder Alkyloxycarbonyl substituiert ist, Cyano, Nitro, Amino, Acylamino, Alkylamino, Carboxy, Carbamoyl, Alkylcarbamoyl, Arylcarbamoyl, Alkylsulfonylcarbamoyl, Arylsulfonylcarbamoyl, Alkyloxycarbonyl, Tetrazolyl, Isoxazolyl, Isothiazolyl, Alkylsulfonamido, Arylsulfonamido, Sulfonyl, Alkylsulfonyl, Arylsulfonyl, Sulfamoyl, Alkylsulfamoyl, Arylsulfamoyl, Alkylsulfonamid, Arylsulfonamid, Alkylthio, Acyl, Acyloxy, Hydrazino, Hydroxyamino, Amidino, Guanidino, Cyanoguanidino, Hydroxy und Halogen,
C₃₋₇-Cycloalkyl
gegebenenfalls substituiert mit einer oder mehreren Gruppe(n), unabhängig voneinander ausgewählt aus C₁₋₅-Alkyl oder C₁₋₅-Alkoxy, das gegebenenfalls mit Carboxy oder Alkyloxycarbonyl substituiert ist, Cyano, Nitro, Amino, Acylamino, Alkylamino, Carboxy, Carbamoyl, Alkylcarbamoyl, Arylcarbamoyl, . Alkylsulfonylcarbamoyl, Arylsulfonylcarbamoyl, Alkyloxycarbonyl, Tetrazolyl, Isoxazolyl, Isothiazolyl, Alkylsulfonamido, Arylsulfonamido, Sulfonyl, Alkylsulfonyl, Arylsulfonyl, Sulfamoyl, Alkylsulfamoyl, Arylsulfamoyl, Alkylsulfonamid, Arylsulfonamid, Alkylthio, Acyl, Acyloxy, Hydrazino, Hydroxyamino, Amidino, Guanidino, Cyanoguanidino, Hydroxy und Halogen,
und Heterocyclus;
mit der Massgabe, dass keine der Gruppen R₁, R₂ und R₃ miteinander verbunden sind;
ferner mit der Massgabe, dass Ar nicht 2-Hydroxy-5-methoxyphenyl, 2-Hydroxy-5-(nieder)alkoxyphenyl ist, und wenn Ar Pyren, Chrysen oder Phenanthren ist, dann ist m # 3 und Z # N⁺R₁R₂R₃, worin R₁ = R₂ = Me und R₃ = Bz ist, und mit der weiteren Massgabe, dass, wenn Ar Phenyl ist, Z 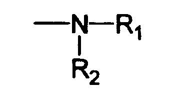 und R₂ Methyl ist, R₁ nicht 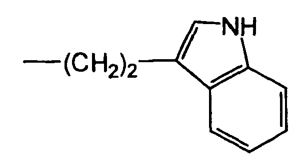 ist.

2. Verbindung gemäss Anspruch 1, worin Z 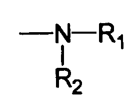 ist.

3. Verbindung gemäss Anspruch 2, worin m 3, 4 oder 5 ist.

4. Verbindung gemäss Anspruch 3, worin m 3 oder 4 ist.

5. Verbindung gemäss Anspruch 1, worin R₂ unabhängig voneinander ausgewählt ist aus C₁₋₈-Alkyl, C₁₋₈₋Alkenyl und C₁₋₈-Alkinyl,
substituiert mit einer oder mehreren Gruppe(n), unabhängig voneinander ausgewählt aus Carboxy, Carbamoyl, Alkylcarbamoyl, Arylcarbamoyl, Alkylsulfonylcarbamoyl, Arylsulfonylcarbamoyl, Alkyloxycarbonyl, Tetrazolyl, Isoxazolyl, Isothiazolyl, Alkylsulfonamido, Arylsulfonamido, Sulfonyl, Alkylsulfonyl, Arylsulfonyl, Sulfamoyl, Alkylsulfamoyl, Arylsulfamoyl, Alkylsulfonamid, Arylsulfonamid und Acylamino.

6. Verbindung gemäss Anspruch 5, worin R₂ unabhängig voneinander ausgewählt ist aus C₁₋₈-Alkyl, C₁₋₈₋Alkenyl und C₁₋₈-Alkinyl, substituiert mit einer oder mehreren Gruppe(n), unabhängig voneinander ausgewählt aus Carboxy und Alkyloxycarbonyl.

7. Verbindung gemäss Anspruch 1, worin Z 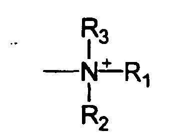 ist.

8. Verbindung gemäss Anspruch 7, worin m 3, 4 oder 5 ist.

9. Verbindung gemäss Anspruch 8, worin m 3 oder 4 ist.

10. Verbindung gemäss Anspruch 9, worin R₃ C₁₋₈-Alkyl ist, gegebenenfalls substituiert mit einer oder mehreren Gruppe(n), unabhängig voneinander ausgewählt aus Hydroxy, Halogen, Trihalogenmethyl, C₁₋₅-Alkyl, C₁₋₅-Alkoxy, Cyano, Nitro, Amino und Carboxy.

11. Verbindung gemäss Anspruch 1, worin
Ar wie in Anspruch 1 definiert ist, mit Ausnahme von mit Trihalogenmethoxy substituiertem Aryl;
R₂ ist ausgewählt aus C₁₋₈-Alkyl, C₁₋₈- Alkenyl und C₁₋₈-Alkinyl, substituiert mit einer oder mehreren Gruppe(n), wie in Anspruch 1 definiert, mit Ausnahme von
(i) substituiertem Acyloxy,
(ii) Carboxy, Carbamoyl, Alkylcarbamoyl, Arylcarbamoyl, Alkylsulfonylcarbamoyl, Arylsulfonylcarbamoyl, Alkoxycarbonyl, Tetrazolyl, Isoxazolyl, Isothiazolyl, Alkylsulfonylamido, Arylsulfonylamido, Sulfonyl, Alkylsulfonyl, Arylsulfonyl, Acylamino, Aryl, jeweils substituiert mit Acylamino, Aryloxy, Arylmethyloxy und
(iii) gegebenenfalls substituiertem C₁₋₅-Alkoxy;
R₃ ist ausgewählt aus C₁₋₈-Alkyl, C₁₋₈-Alkenyl und C₁₋₈-Alkinyl, gegebenenfalls substituiert mit einer oder mehreren Gruppe(n), wie in Anspruch 1 definiert, mit Ausnahme von
(i) gegebenenfalls substituiertem Acyloxy,
(ii) Carboxy, Carbamoyl, Alkylcarbamoyl, Arylcarbamoyl, Alkylsulfonylcarbamoyl, Arylsulfonylcarbamoyl, Alkoxycarbonyl, Tetrazolyl, Isoxazolyl, Isothiazolyl, Alkylsulfonylamido, Arylsulfonylamido, Sulfonyl., Alkylsulfonyl, Arylsulfonyl, Acylamino, Aryl, jeweils substituiert mit Aryloxy, Arylmethoxy und
(iii) gegebenenfalls substituiertem C₁₋₅-Alkoxy; und
R₄' ist Wasserstoff, Carboxy, Carbamoyl, Alkylcarbamoyl, Arylcarbamoyl, Alkylsulfonylcarbamoyl, Arylsulfonylcarbamoyl, Alkoxycarbonyl, Tetrazolyl, Isoxazolyl, Isothiazolyl, Alkylsulfonylamido, Arylsulfonylamido, Sulfonyl, Alkylsulfonyl, Arylsulfonyl, Acylamino, Aryl, jeweils substituiert mit Aryloxy, Arylmethoxy.

12. Verbindung gemäss Anspruch 11, worin Ar Aryl ist,
gegebenenfalls substituiert mit einer oder mehreren Gruppe(n), unabhängig voneinander ausgewählt aus Hydroxy, Halogen, Trihalogenmethyl, C₁₋₅-Alkyl, C₁₋₅₋Alkoxy, Cyano, Nitro, Amino, Carboxy, Carbamoyl, Alkylcarbamoyl, Arylcarbamoyl, Acyl, Acyloxy, Sulfonyl, Alkylsulfonyl, Arylsulfonyl, Sulfamoyl, Alkylsulfamoyl, Arylsulfamoyl, Alkylthio, Alkylsulfonamid, Arylsulfonamid, Hydrazino, Acylamino, Alkylamino, Hydroxyamino, Amidino, Guanidino, Cyanoguanidino, Aryl
gegebenenfalls substituiert mit einer oder mehreren Gruppe(n), unabhängig voneinander ausgewählt aus Hydroxy, Halogen, Trihalogenmethyl, C₁₋₅-Alkyl, C₁₋₅-Alkoxy,
Cyano, Nitro, Amino und Carboxy,
und Aryloxy
gegebenenfalls substituiert mit einer oder mehreren Gruppe(n), unabhängig voneinander ausgewählt aus Hydroxy, Halogen, Trihalogenmethyl, C₁₋₅-Alkyl, C₁₋₅-Alkoxy, Cyano, Nitro, Amino und Carboxy.

13. Verbindung gemäss Anspruch 1, ausgewählt aus
N-Phenylcarbamoyl-N'-[2-(4-chlorphenyl)ethyl]-N'-ethyl-1,3-diaminopropan;
N-(4-Nitrophenylcarbamoyl-N'-[2-(4-chlorphenyl)ethyl]-N'-ethyl-1,3-diaminopropan;
N-(4-Bromphenylcarbamoyl-N'-[2-(4-chlorphenyl)ethyl]-N'-ethyl-1,3-diaminopropan;
N-Phenylcarbamoyl-N'-[2-(4-chlorphenyl)ethyl]-N'-propyl-1,3-diaminopropan;
Methyl-4-[[3-(4-bromphenylureido)propyl](1,2,3,4-tetrahydro-1-naphthyl)amino]butylat;
Methyl-4-[[3-(4-bromphenylureido)propyl][(1R)-1-phenylethyl]amino]butylat;
Methyl-4-[[3-(4-bromphenylureido)propyl][2-(4-chlorphenyl)ethyl]amino]butylat;
Methyl-4-[[4-(4-bromphenylureido)butyl](1,2,3,4-tetrahydro-1-naphthyl)amino]butylat;
Methyl-4-[[5-(4-bromphenylureido)pentyl](1,2,3,4-tetrahydro-1-naphthyl) amino] butylat;
Methyl-4-[[3-(4-methylphenylureido)propyl](1,2,3,4-tetrahydro-1-naphthyl)amino]butylat;
Methyl-4-[[3-(3,4-dichlorphenylureido)propyl]-(1,2,3,4-tetrahydro-1-naphthyl)amino]butylat;
4-[[3-(4-Bromphenylureido)propyl](1,2,3,4-tetrahydro-1-naphthyl)amino]butansäure;
4- [[3- (4-Bromphenylureido)propyl] [(1R) -1-phenylethyl]amino]butansäure;
4-[[4-(4-Bromphenylureido)butyl](1,2,3,4-tetrahydro-1-naphthyl)amino]butansäure;
4-[[5-(4-Bromphenylureido)pentyl](1,2,3,4-tetrahydro-1-naphthyl)amino]butansäure;
4-[[3-(4-Methylphenylureido)propyl](1,2,3,4-tetrahydro-1-naphthyl)amino]butansäure;
4-[[3-(3,4-Dichlorphenylureido)propyl](1,2,3,4-tetrahydro-1-naphthyl)amino]butansäure;
[3-(Phenylureido)propyl][2-(4-chlorphenyl)ethyl]diethylammoniumiodid;
[3-(4-Bromphenylureido)propyl][2-(4-chlorphenyl)ethyl]diethylammoniumiodid;.
N-Phenylcarbamoyl-N'-[2-(4-chlorphenyl)ethyl]-N'-ethyl-2-hydroxy-1,3-diaminopropan;
4-[[(3S)-3-(4-Bromphenylureido)-3-(tert-butoxycarbonyl)propyl](1,2,3,4-tetrahydro-1-naphthyl)amino]butansäure;
4-[[3-(4-Bromphenylureido)-2-hydroxypropyl](1,2,3,4-tetrahydro-1-naphthyl)amino]butansäure;
4-[[3-(4-Chlorphenylureido)propyl] (1,2,3,4-tetrahydro-1-naphthyl)amino]butansäure;
Methyl-4-[[3-(4-bromphenylureido)propyl](1-indanyl)amino]butylat;
4-[[3-(4-Bromphenylureido)propyl](1-indanyl)amino]butansäure;
Methyl-4-[[3-(4-bromphenylureido)propyl][(1R)-1-indanyl]amino]butylat ;
4-[[3-(4-Bromphenylureido)propyl][(1R)-1-indanyl]amino]butansäure;
Methyl-4-[[3-(4-bromphenylureido)propyl][(1R)-1,2,3,4-tetrahydro-1-naphthyl]amino]butylat;
4-[[3-(4-Bromphenylureido)propyl][(1R)-1,2,3,4-tetrahydro-1-naphthyl]amino]butansäure;
Ethyl-4-[[3-(4-bromphenylureido)propyl)(1,2,3,4-tetrahydro-1-naphthyl)amino]butylat;
4-[[3-(4-Bromphenylureido)propyl](1,2,3,4-tetrahydro-1-naphthyl)amino]butanamid;
3-[[3-(4-Bromphenylureido)propyl](1,2,3,4-tetrahydro-1-naphthyl)amino]-1-[(phenylsulfonyl)carbamoyl]-propan;
4-[[3-(4-Bromphenylureido)propyl] (1,2,3,4-tetrahydro-1-naphthyl)amino]-1-butanol;
3-[[3-(4-Bromphenylureido)propyl](1,2,3,4-tetrahydro-1-naphthyl)amino]-1-[1-(triphenylmethyl)tetrazol-5-yl]propan;
3-[[3-(4-Bromphenylureido)propyl] (1,2,3,4-tetrahydro-1-naphthyl) amino]-1-(1H-tetrazol-5-yl) propan;
Methyl-4-[[3-(4-carboxy)phenylureido]propyl](1,2,3,4-tetrahydro-1-naphthyl) amino] butylat;
4- [[3-(4-Bromphenylureido)propyl] [(1R)-1-(4-methoxyphenyl)ethyl]amino]butansäure;
4-[[3-[4-(Ethoxycarbonyl) phenylureido]propyl] (1,2,3,4-tetrahydro-1-naphthyl)amino]butansäure;
4-[[3-(4-Iodphenylureido)propyl](1,2,3,4-tetrahydro-1-naphthyl)amino]butansäure;
4-[[3-[4-(Butoxycarbonyl)phenylureido]propyl](1,2,3,4-tetrahydro-1-naphthyl)amino]butansäure;
[3-(Phenylureido)propyl]bis[2-(4-chlorphenyl)-ethyl]amin;
4-[[3-(4-Bromphenylureido)propyl] [(1R)-1-(4-bromphenyl)ethyl]amino]butansäure;
4- [[3- (4-Bromphenylureido)propyl] [1- (4-fluorphenyl) - ethyl]amino]butansäure;
4- [[3- (4-Bromphenylureido) propyl] [1- (4-chlorphenyl)ethyl]amino]butansäure;
Methyl-4-[[(3S)-3-(4-bromphenylureido)-3-(tert-butoxycarbonyl)propyl](1,2,3,4-tetrahydro-1-naphthyl)amino]butylat;
Methyl-4-[[(3S)-3-(4-bromphenylureido)-3-(isopropylcarbamoyl)propyl](1,2,3,4-tetrahydro-l-naphthyl)amino]butylat;
Methyl-4-[[(3S)-3-(4-bromphenylureido)-3-benzylcarbamoyl)propyl](1,2,3,4-tetrahydro-1-naphthyl)amino]butylat;
4-[[(3S)-3-(4-Bromphenylureido)-3-(isopropylcarbamoyl)propyl](1,2,3,4-tetrahydro-1-naphthyl)amino]butansäure;
4-[[(3S)-3-(4-Bromphenylureido)-3-(benzylcarbamoyl)propyl](1,2,3,4-tetrahydro-1-naphthyl)amino]butansäure;
4-[[3-(4-Bromphenylureido)propyl][(1S)-1-(4-bromphenyl)ethyl]amino]butansäure;
[3-(Phenylureido)propyl]-2-(4-chlorphenyl)ethyl]bis-(4-methylbenzyl)ammoniumiodid;
[3-(Phenylureido)propyl] [2-(4-chlorphenyl)ethyl] (4-chlorbenzyl)ethylammoniumiodid;
[3-(Phenylureido)propyl][2-(4-chlorphenyl)ethyl]-(benzyl)ethylammoniumiodid;
[3-(Phenylureido)propyl][2-(3-chlorphenyl)ethyl]-diethylammoniumiodid;
[3-(4-Bromphenylureido)propyl][(1S)-1-phenylethyl][3-(carboxy)propyl]ethylammoniumtrifluoracetat;
[3-(4-Bromphenylureido)propyl][(1R)-1-phenylethyl][3-(carboxy)propyl]ethylammoniumtrifluoracetat;
[3-(Phenylureido)propyl][2-(4-chlorphenyl)ethyl][4-(methoxycarbonyl)butyl]ethylammoniumiodid;
[3-(Phenylureido)propyl][2-(4-chlorphenyl)ethyl][4-(carboxy)phenyl]ethylammoniumiodid;
[5-(Phenylureido)pentyl][2-(4-chlorphenyl)ethyl]diethylammoniumiodid;
[3-(Phenylureido)propyl][2-(4-chlorphenyl)ethyl](2-chlorbenzyl)ammoniumiodid;
[3-(Phenylureido)propyl][2-(4-chlorphenyl)ethyl](2,5-difluorbenzyl)ethylammoniumiodid;
[3-(Phenylureido)propyl][2-(4-chlorphenyl)ethyl](3-fluorbenzyl)ethylammoniumiodid;
[3-(4-Cyanophenylureido)propyl] [2- (3-chlorphenyl) - ethyl][2-(2-methoxyethoxy)ethyl]ethylammoniumiodid; und
[3-(4-Methoxyphenylureido)propyl][2-(3-chlorphenyl) ethyl] [2- (2-methoxyethoxy)ethyl]ethylammoniumiodid.

14. Verbindung gemäss Anspruch 1, worin die Verbindung ausgewählt ist aus den folgenden Formeln: 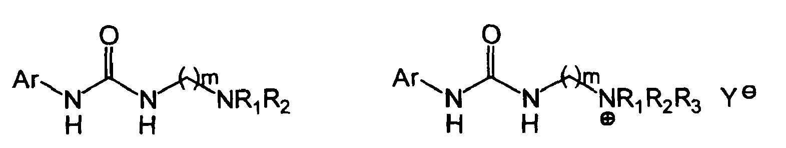 worin Ar, R₁, R₂, R₃, Y und m wie in den folgenden Tabellen gezeigt definiert sind:

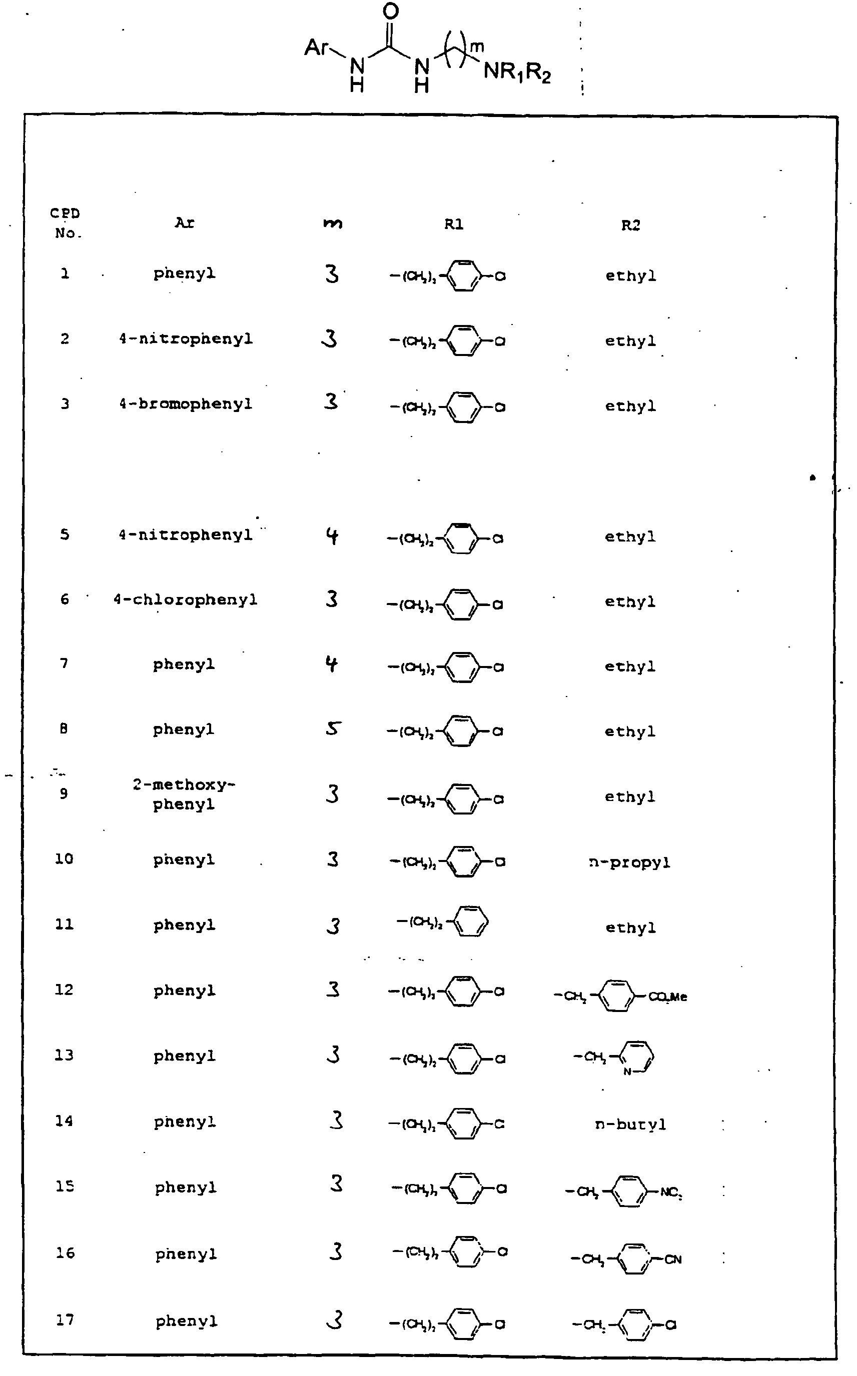

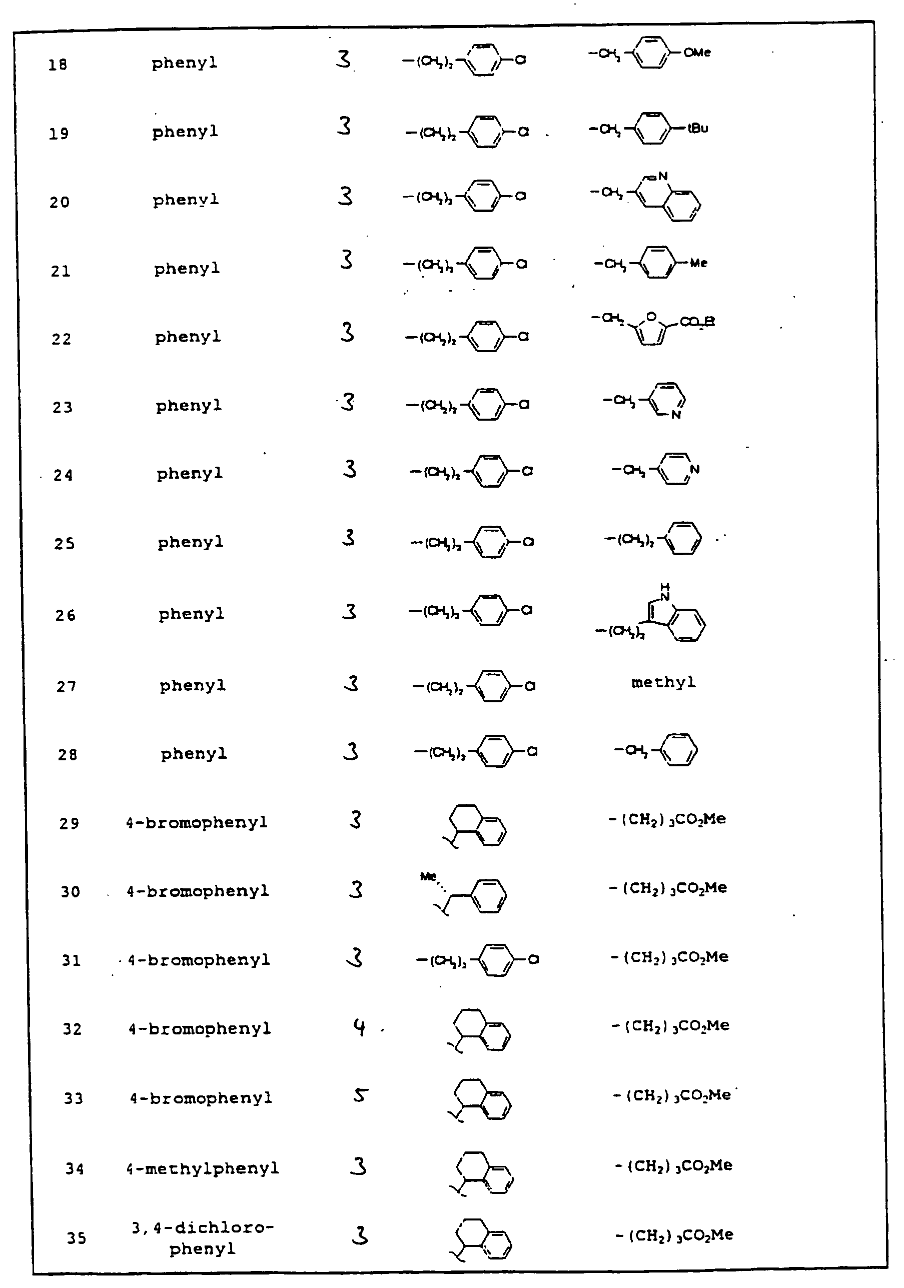

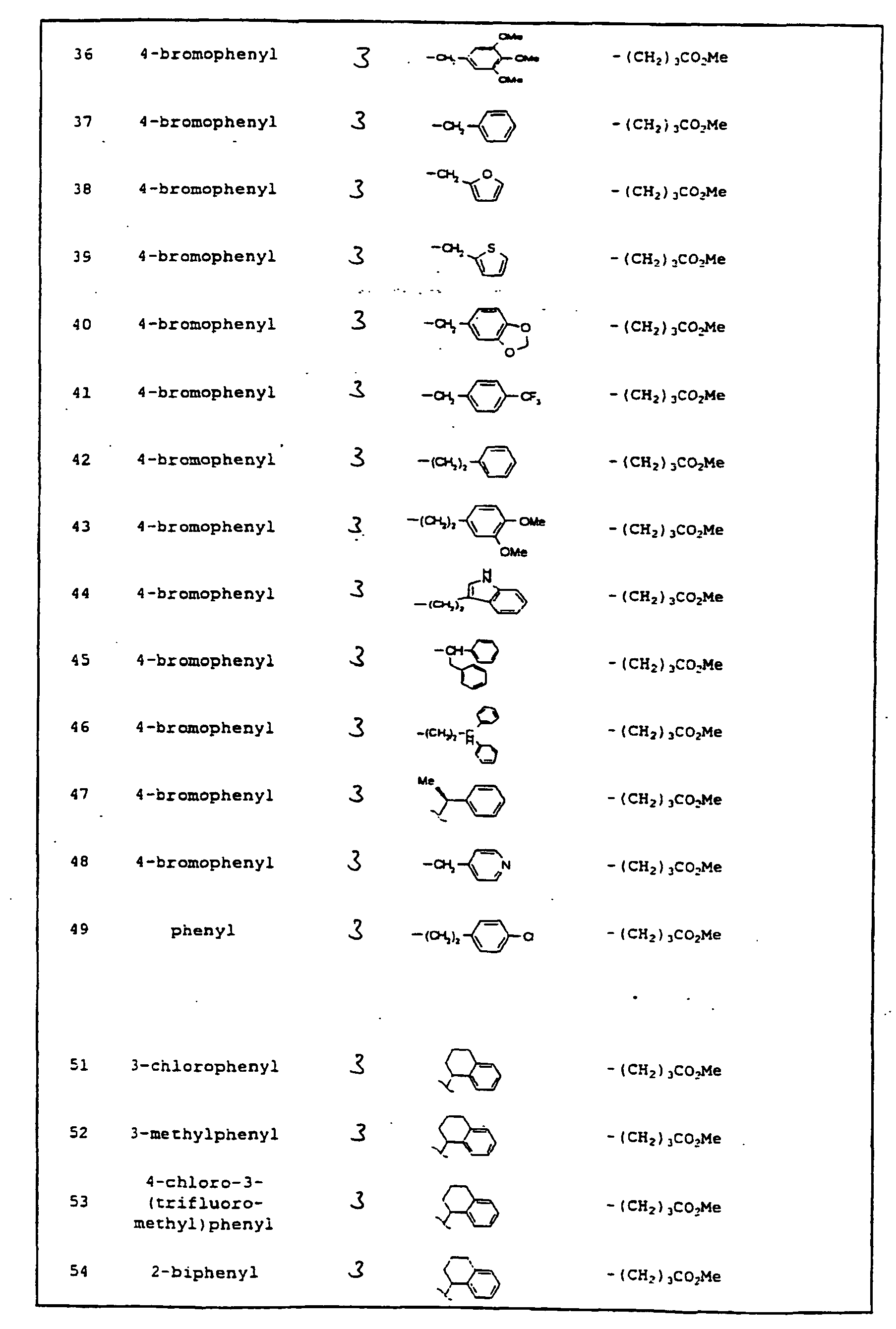

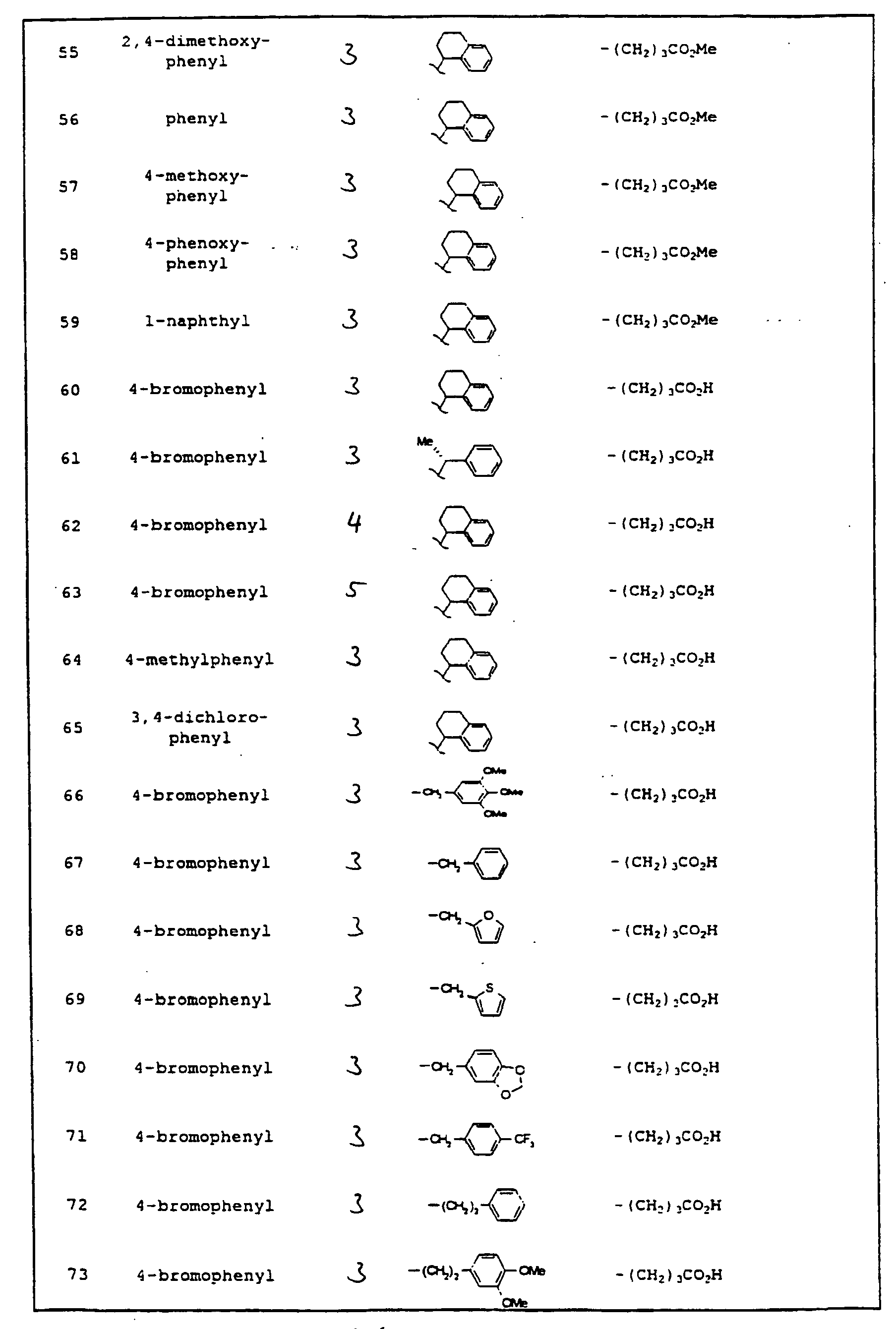

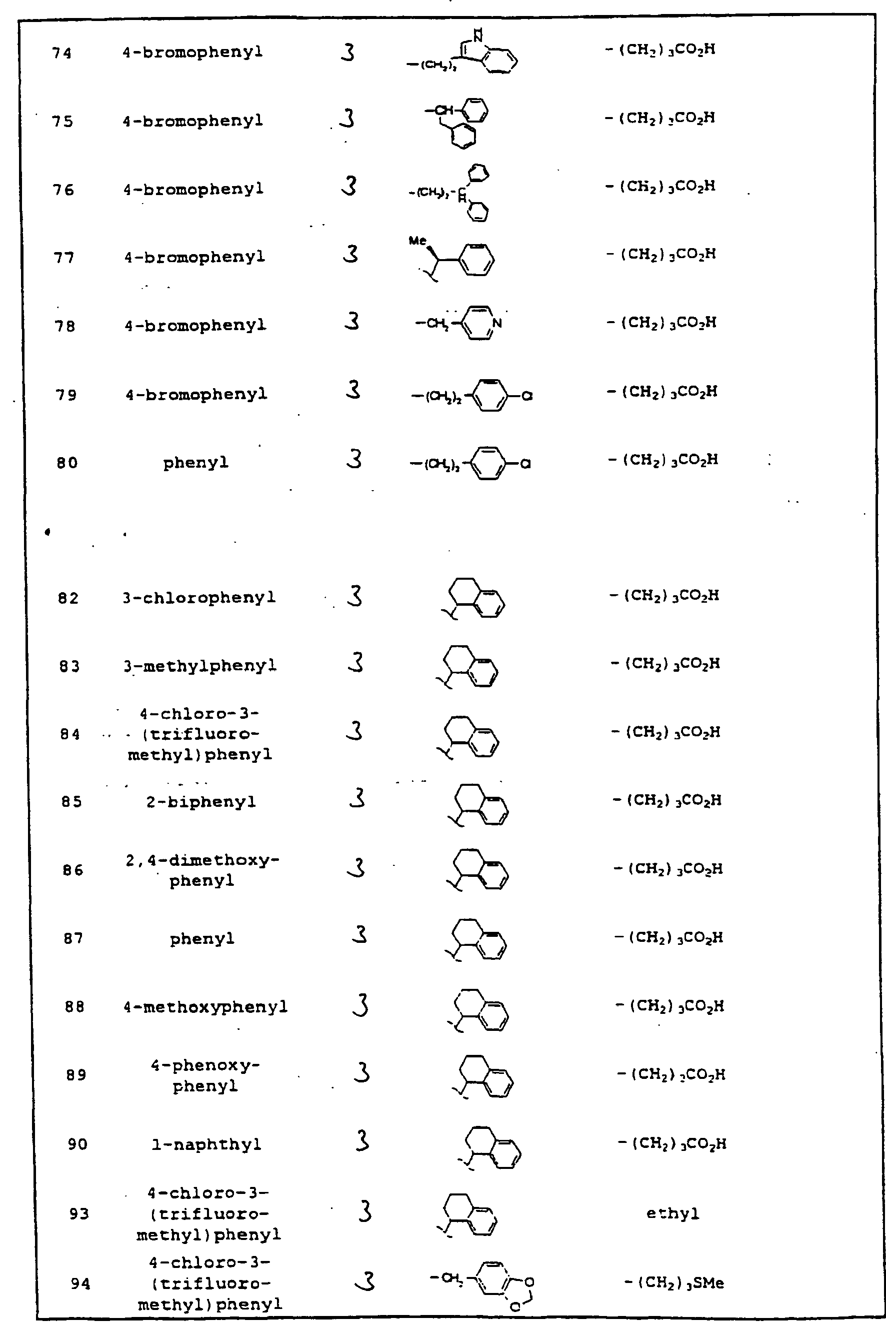

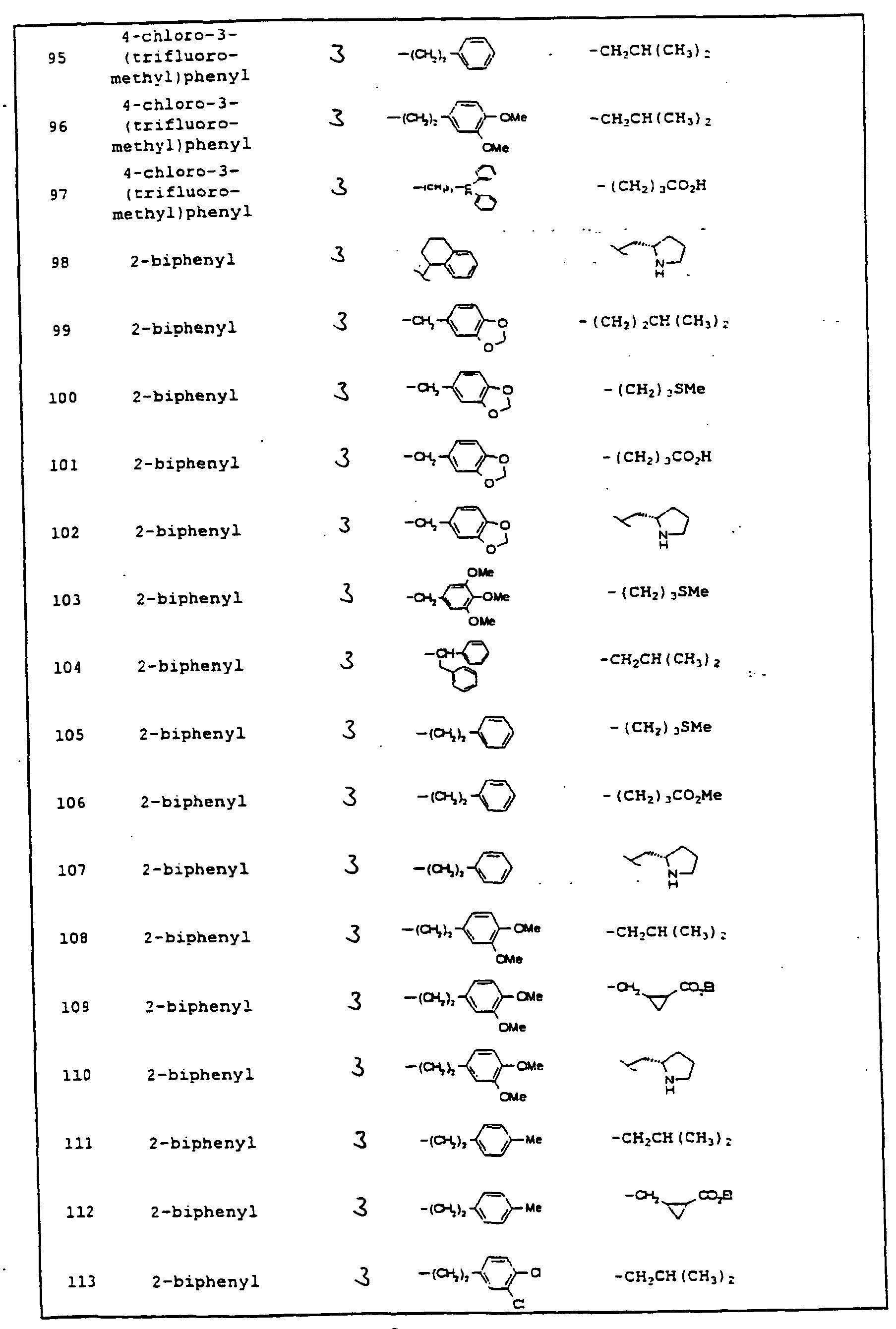

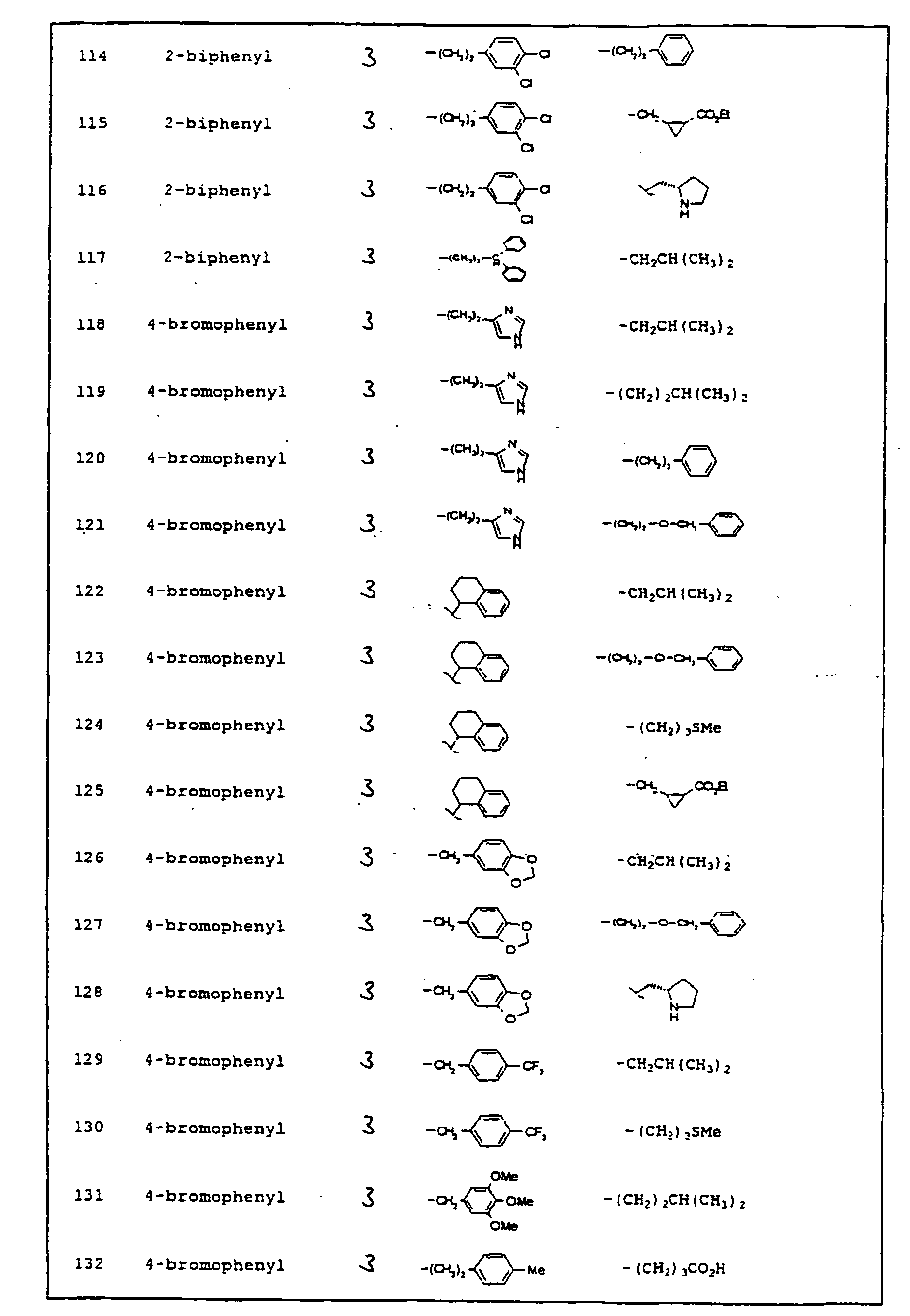

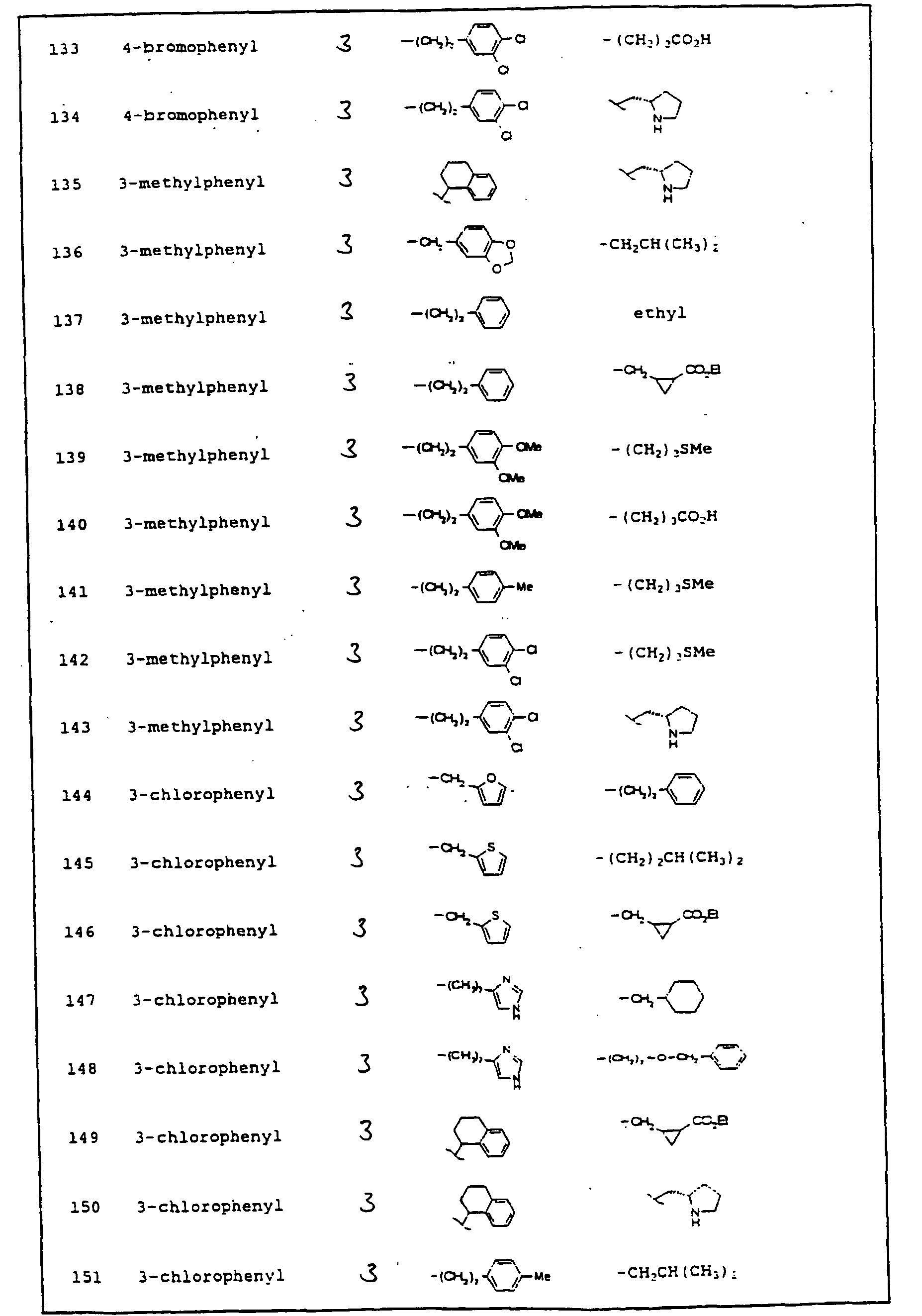

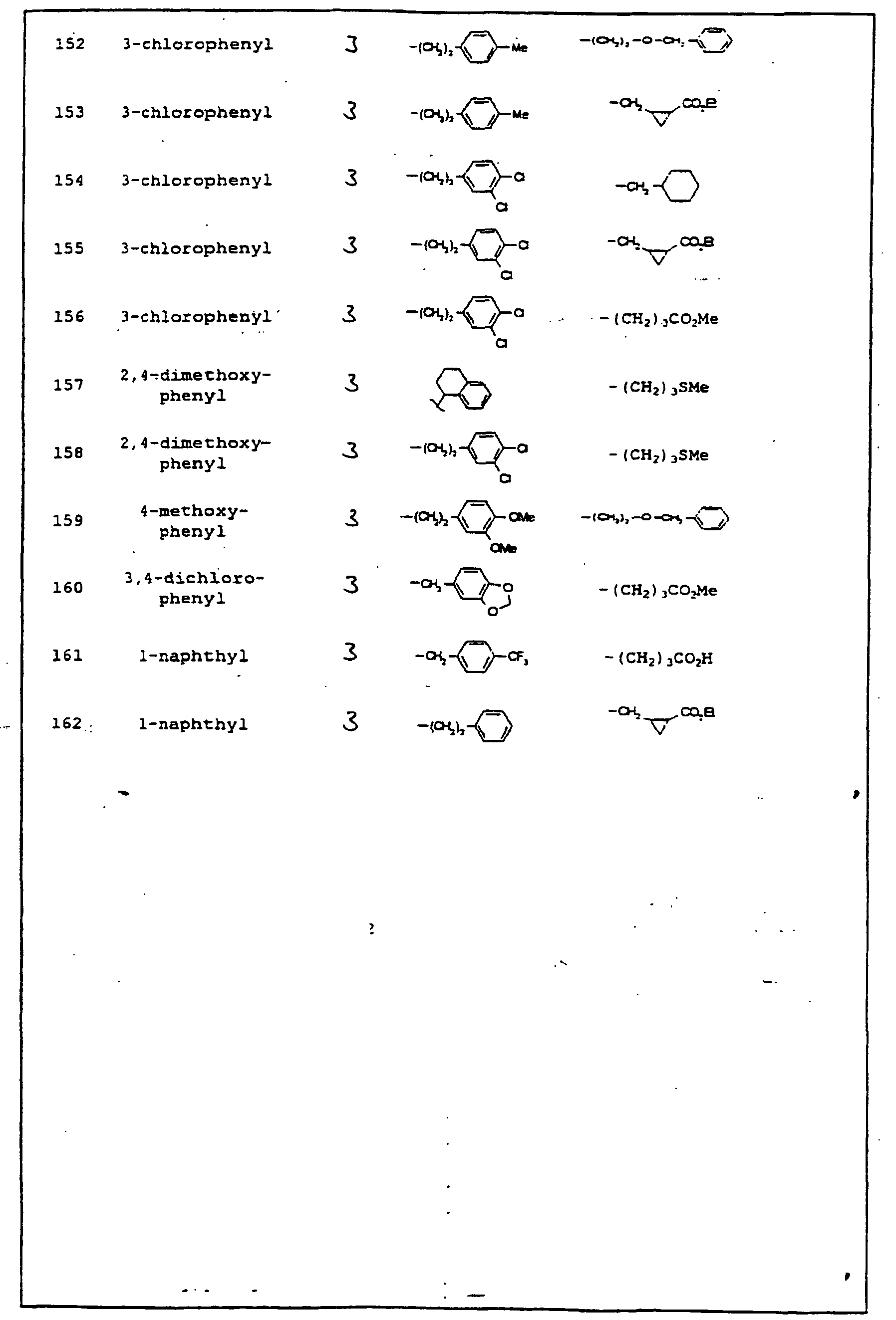

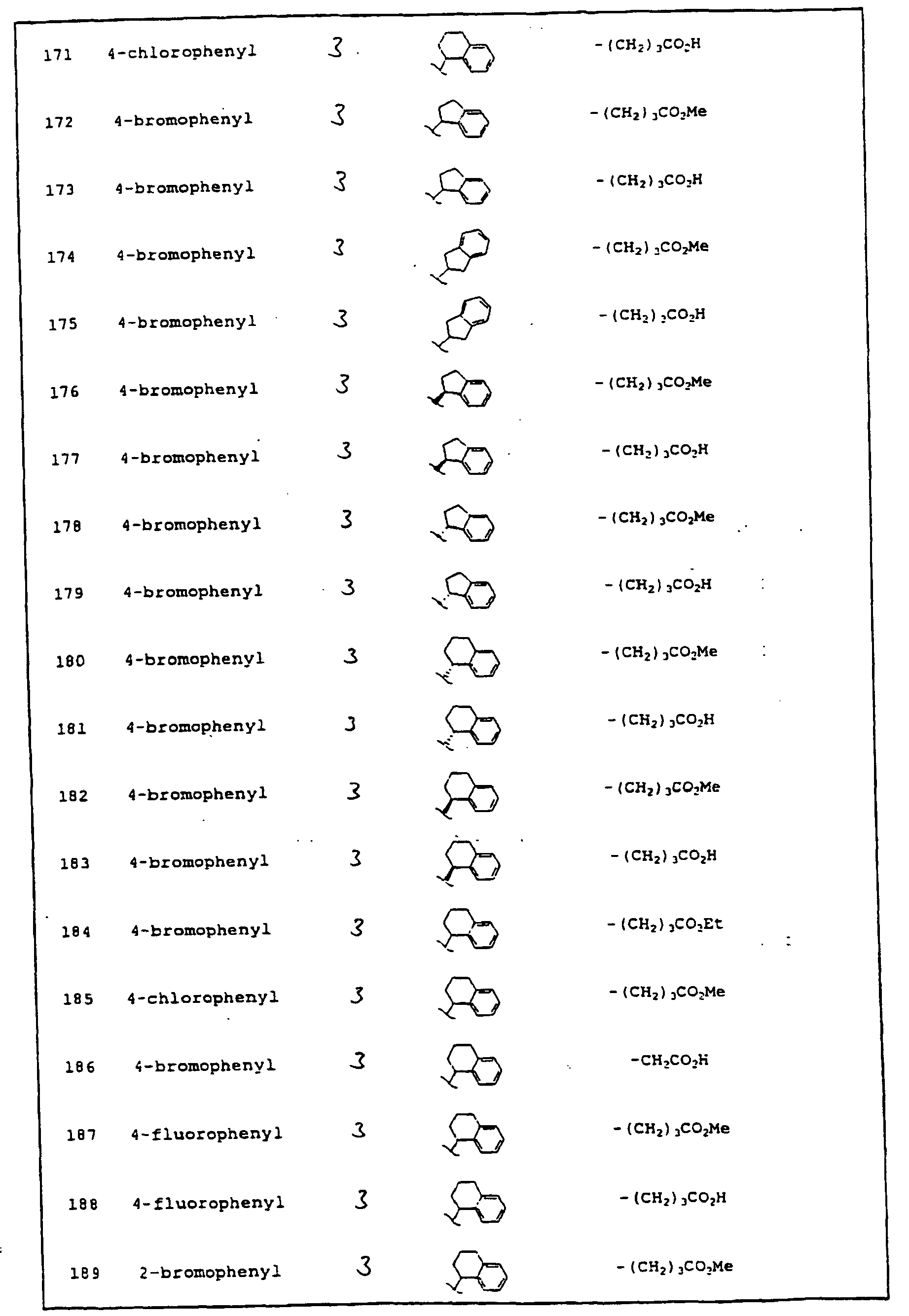

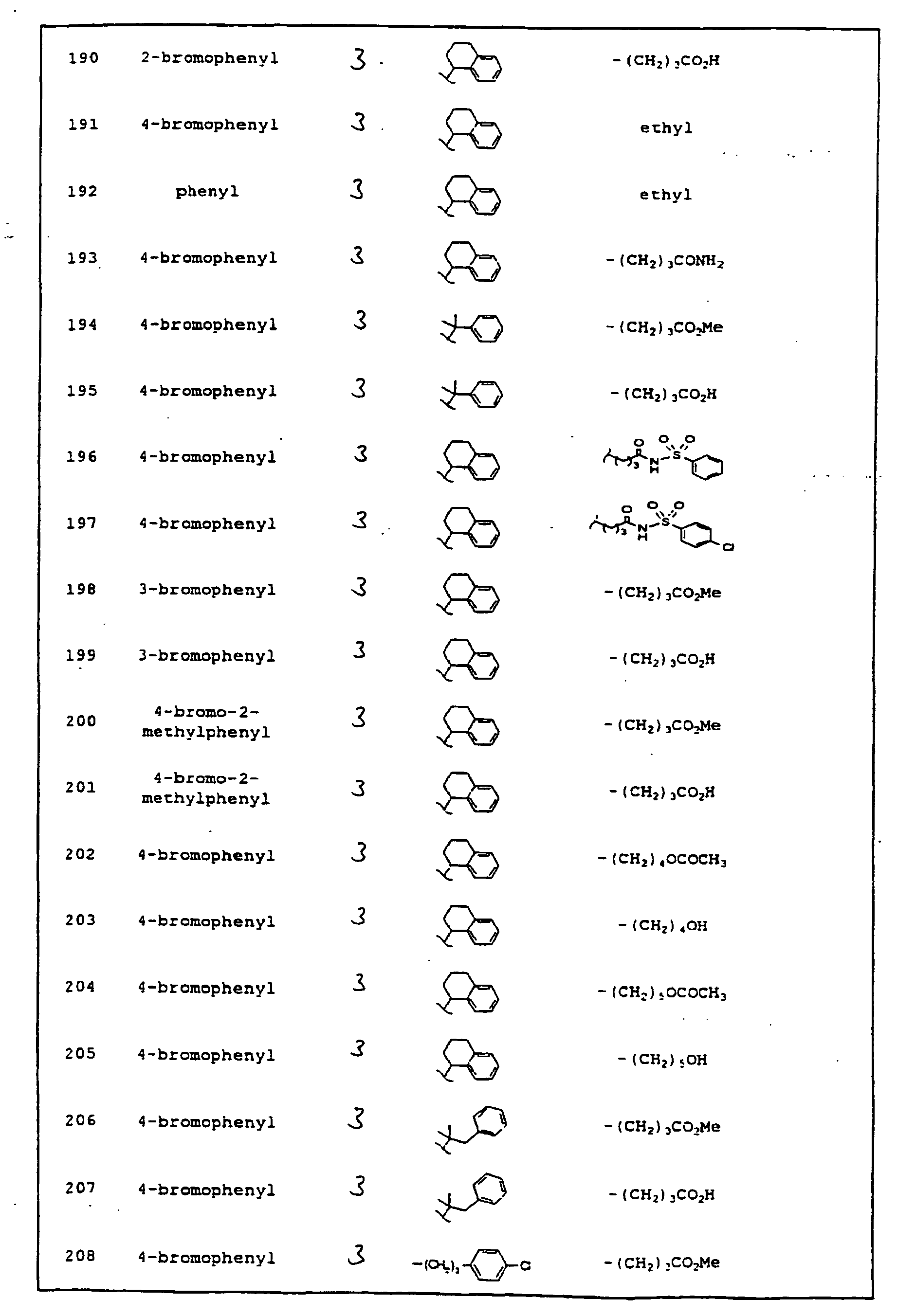

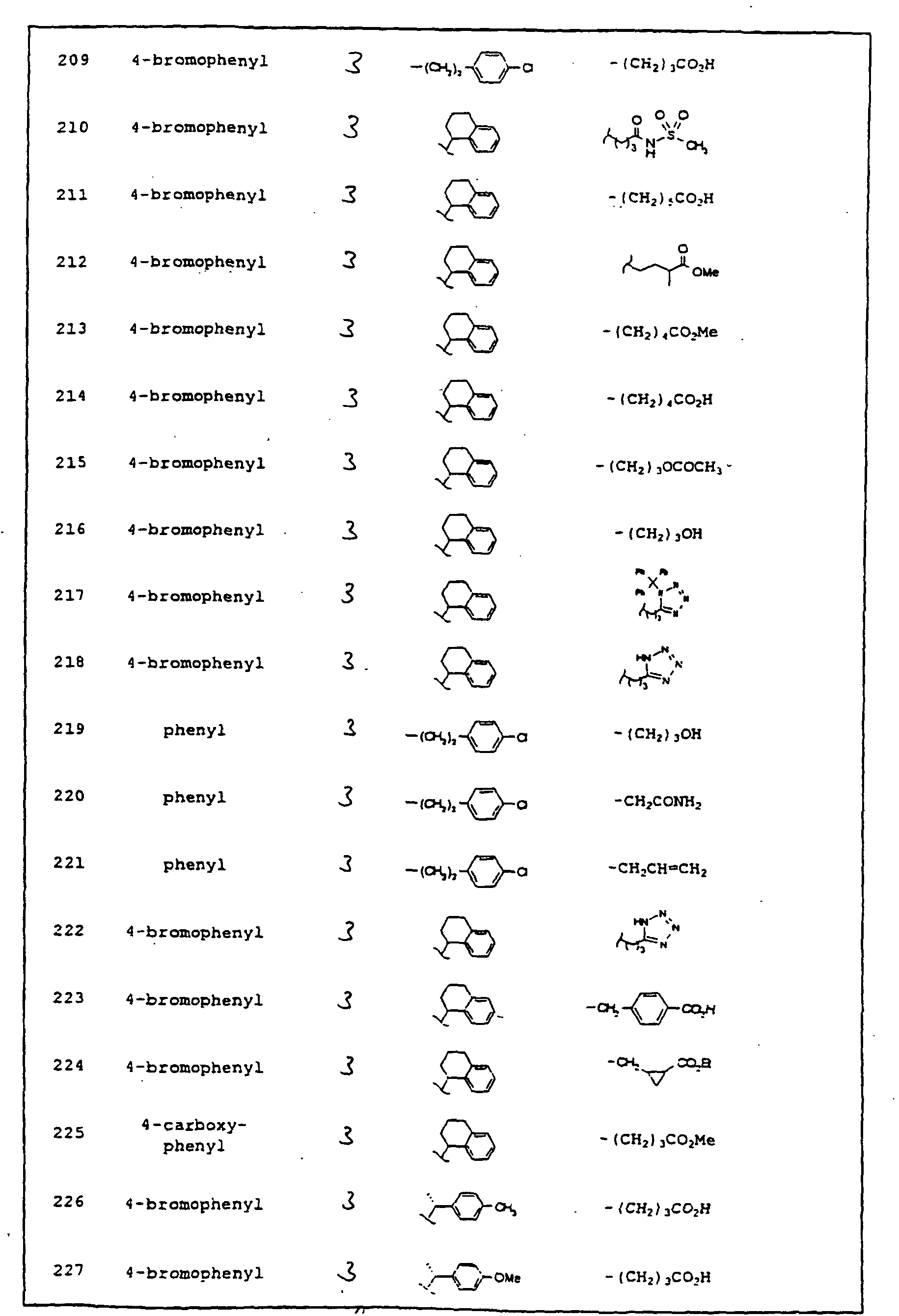

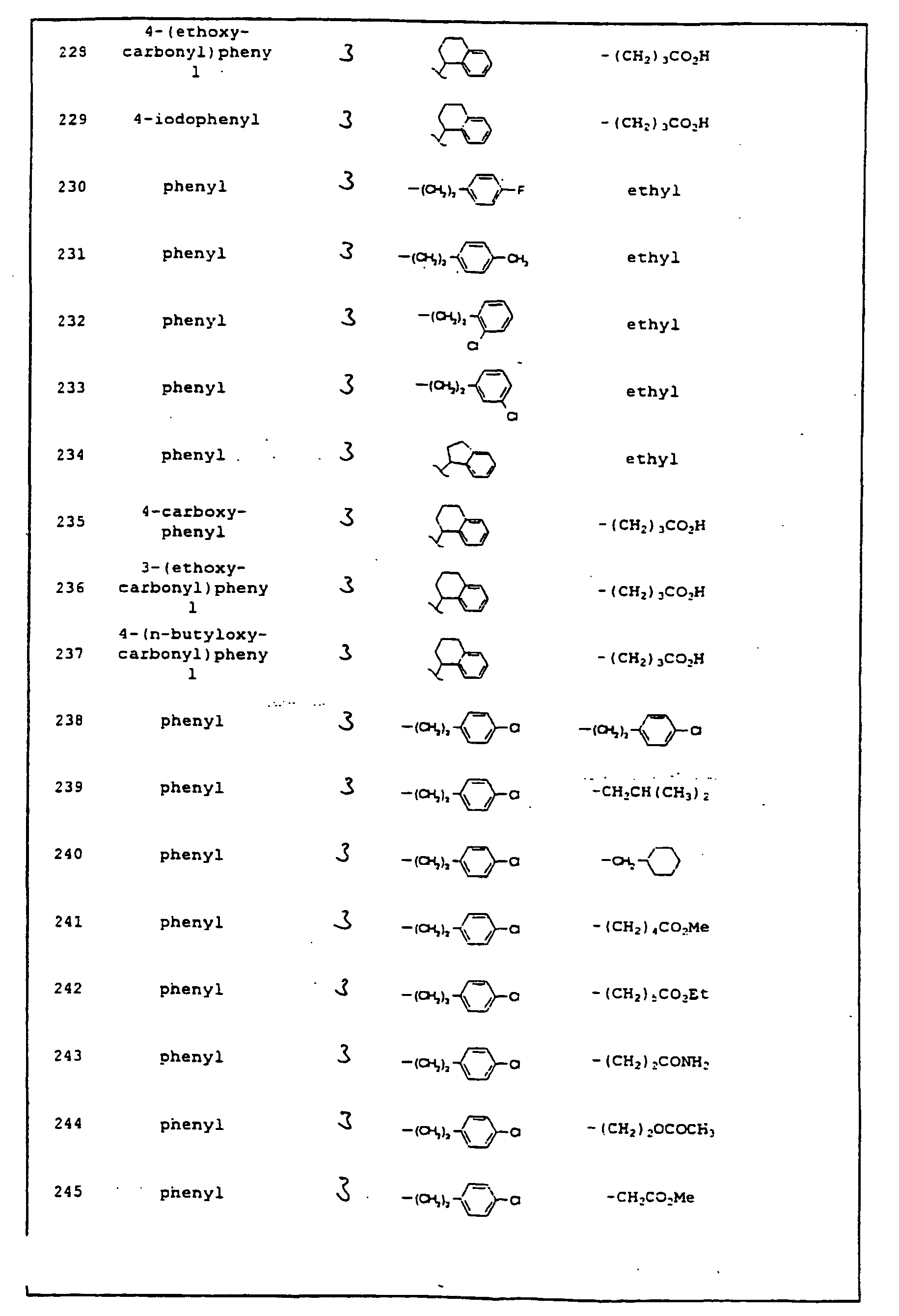

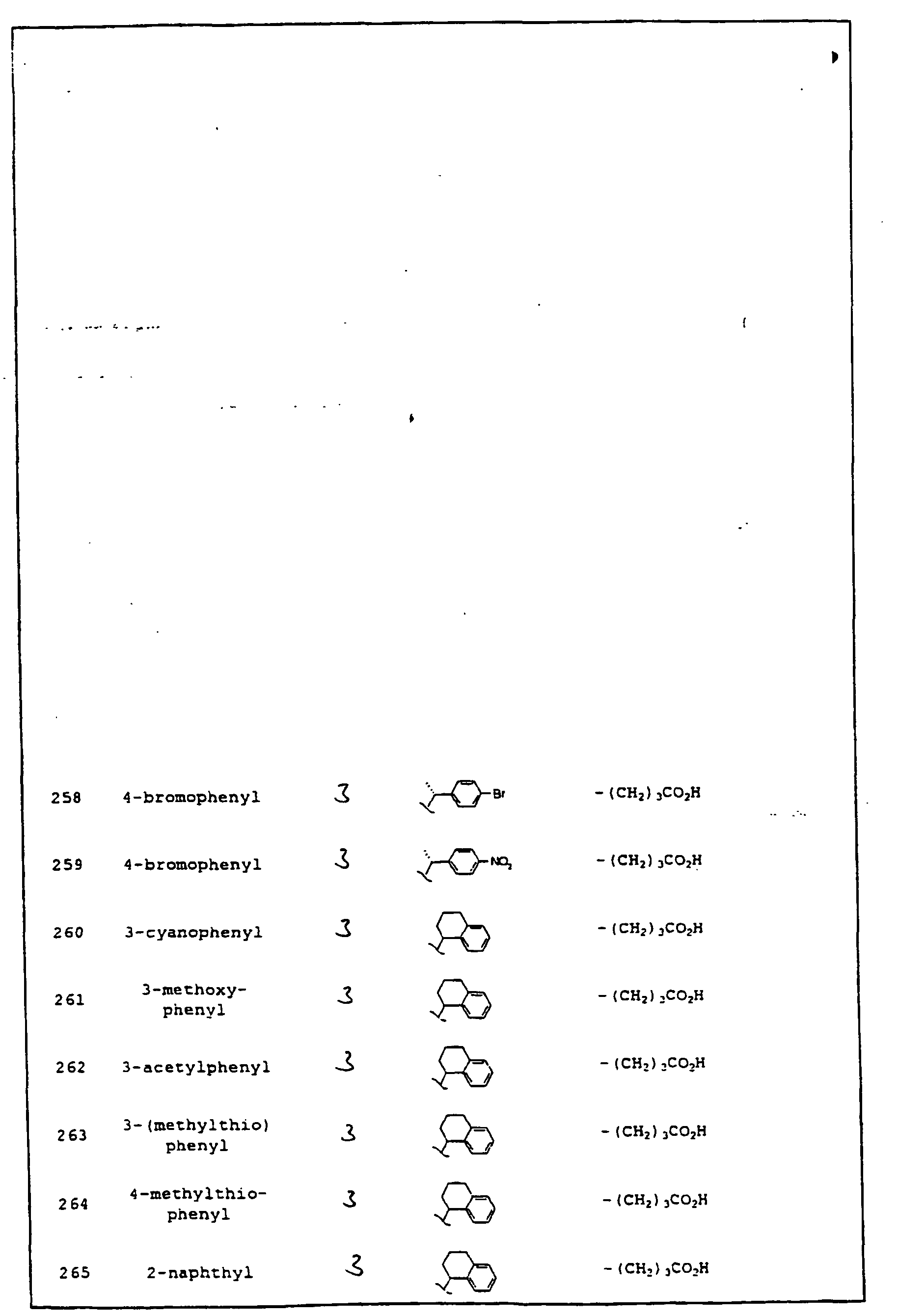

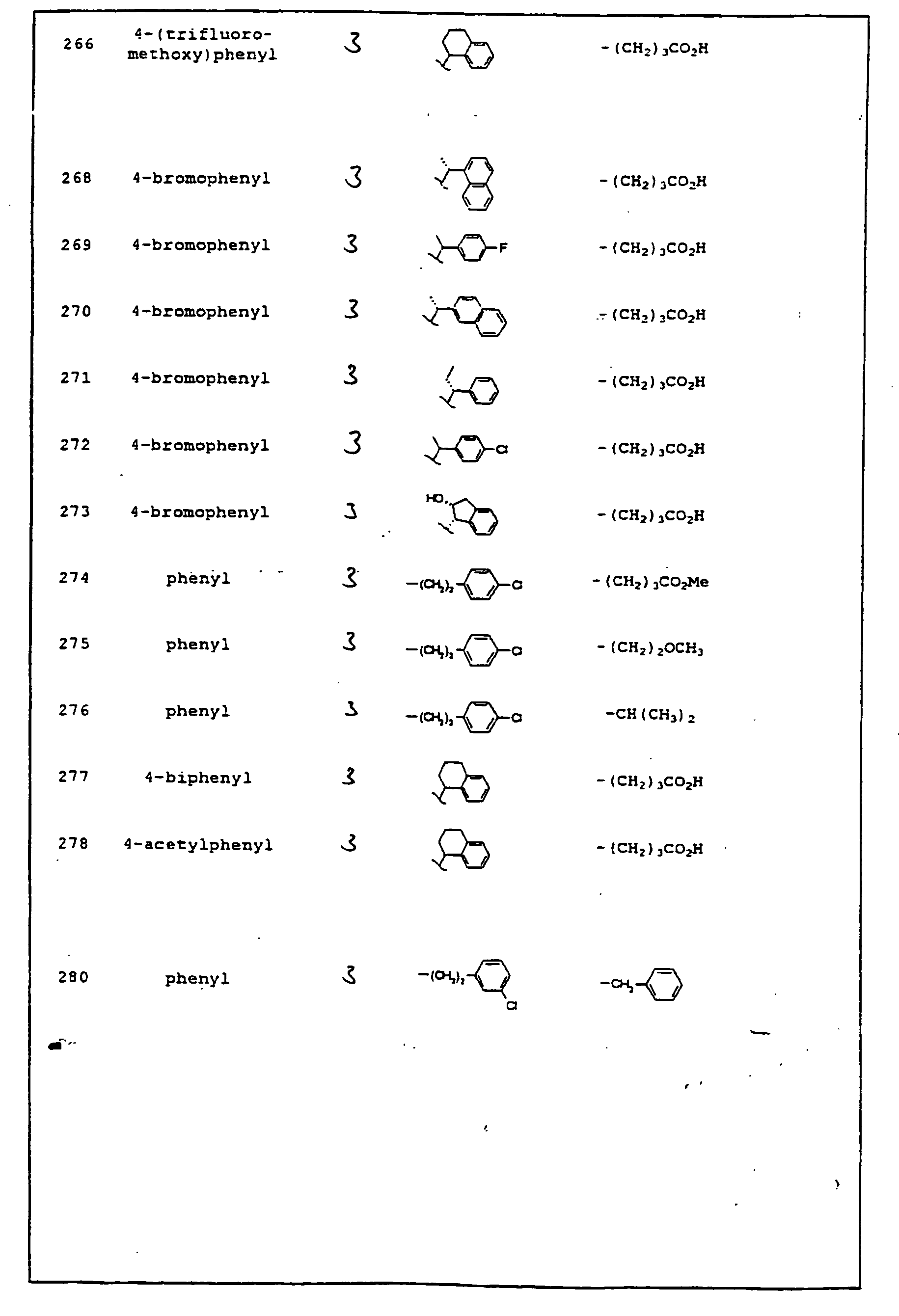

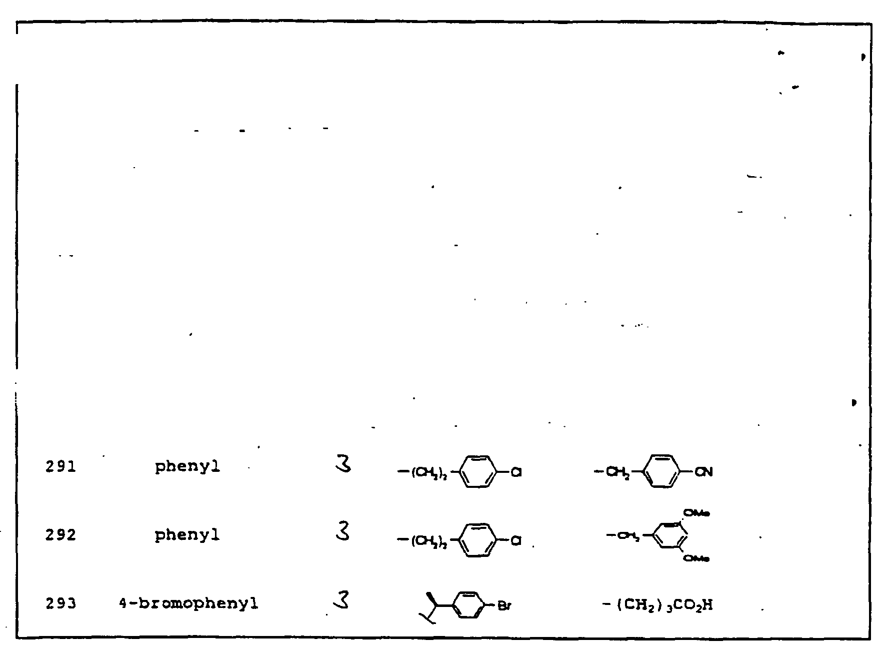

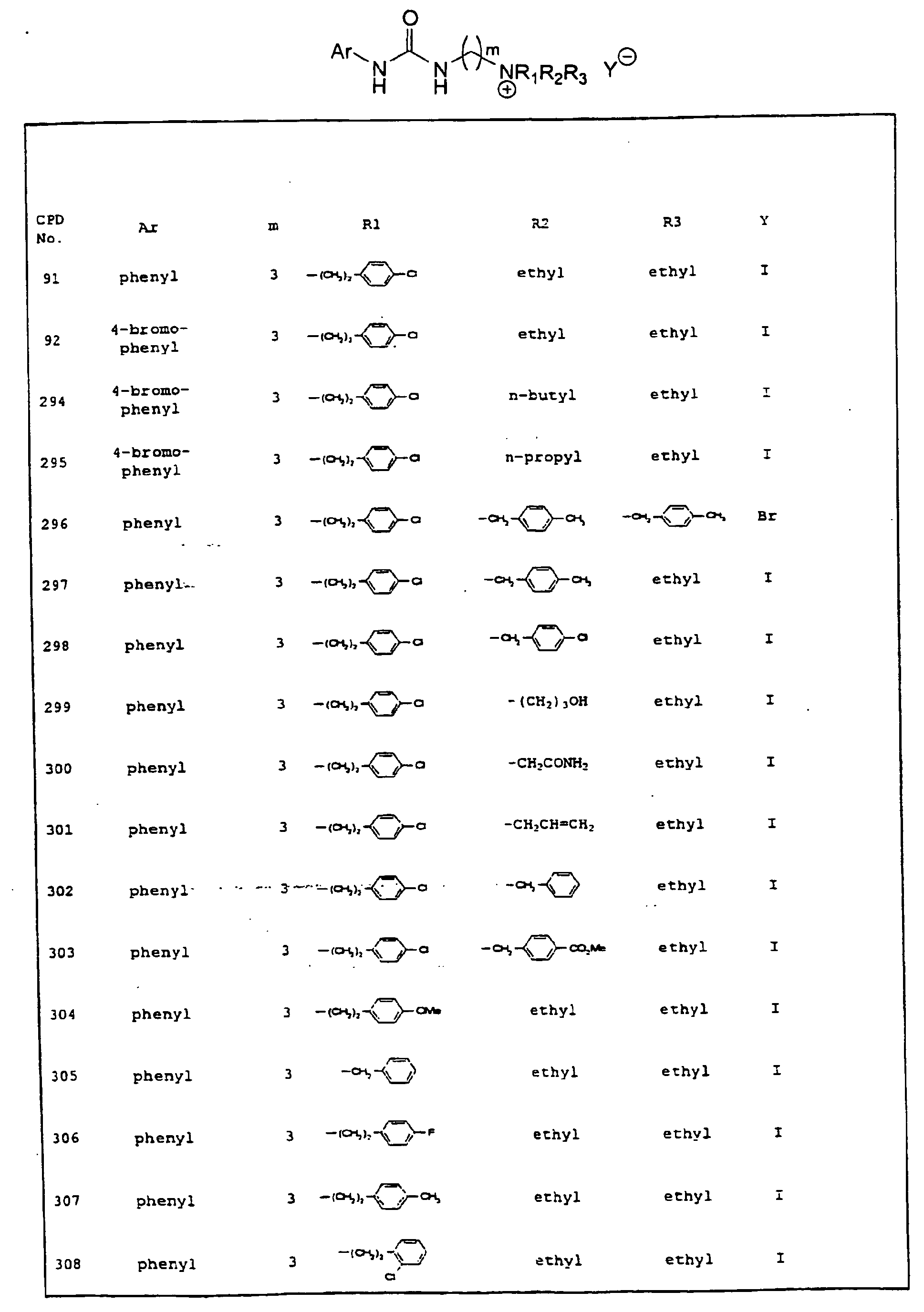

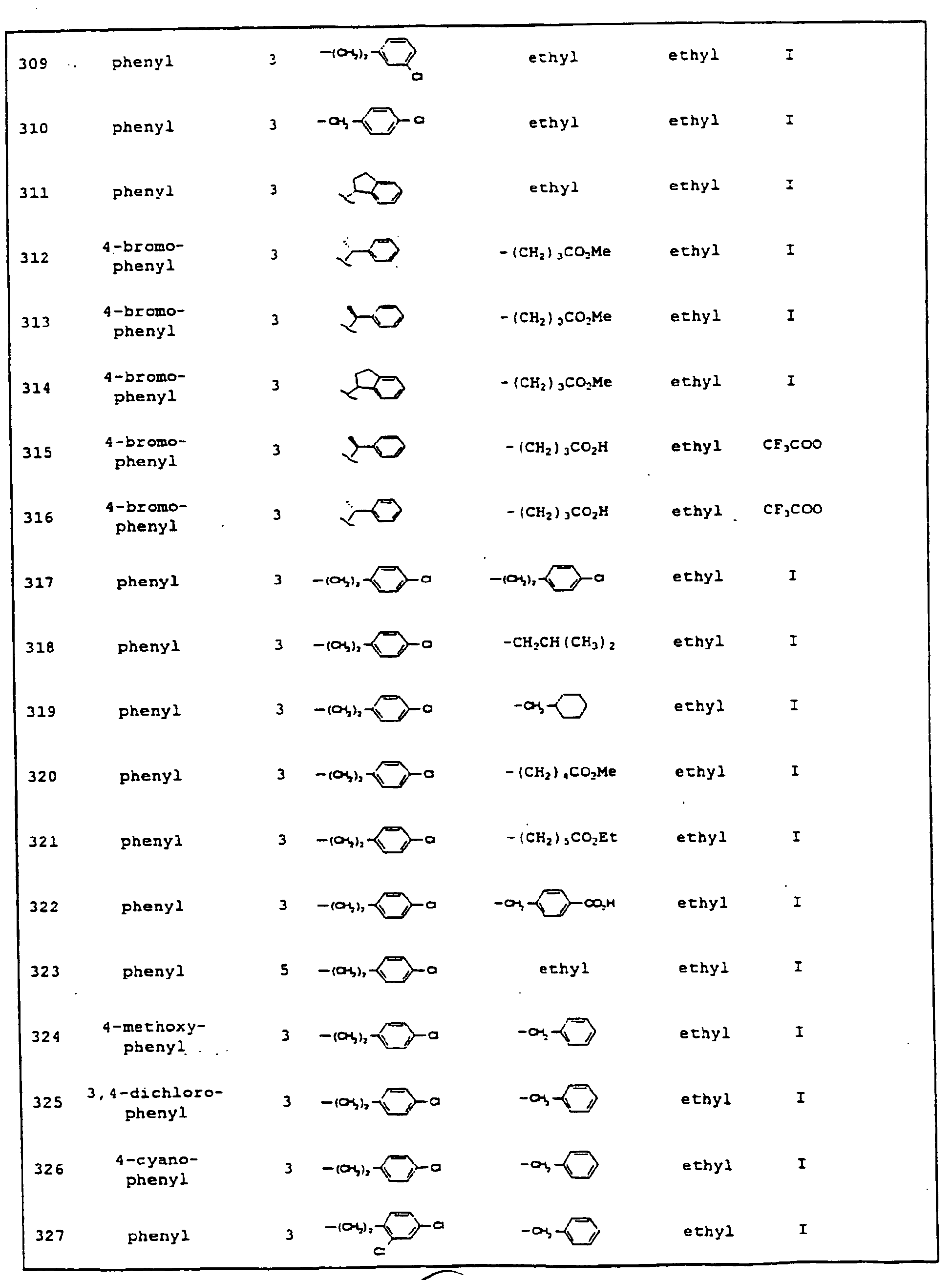

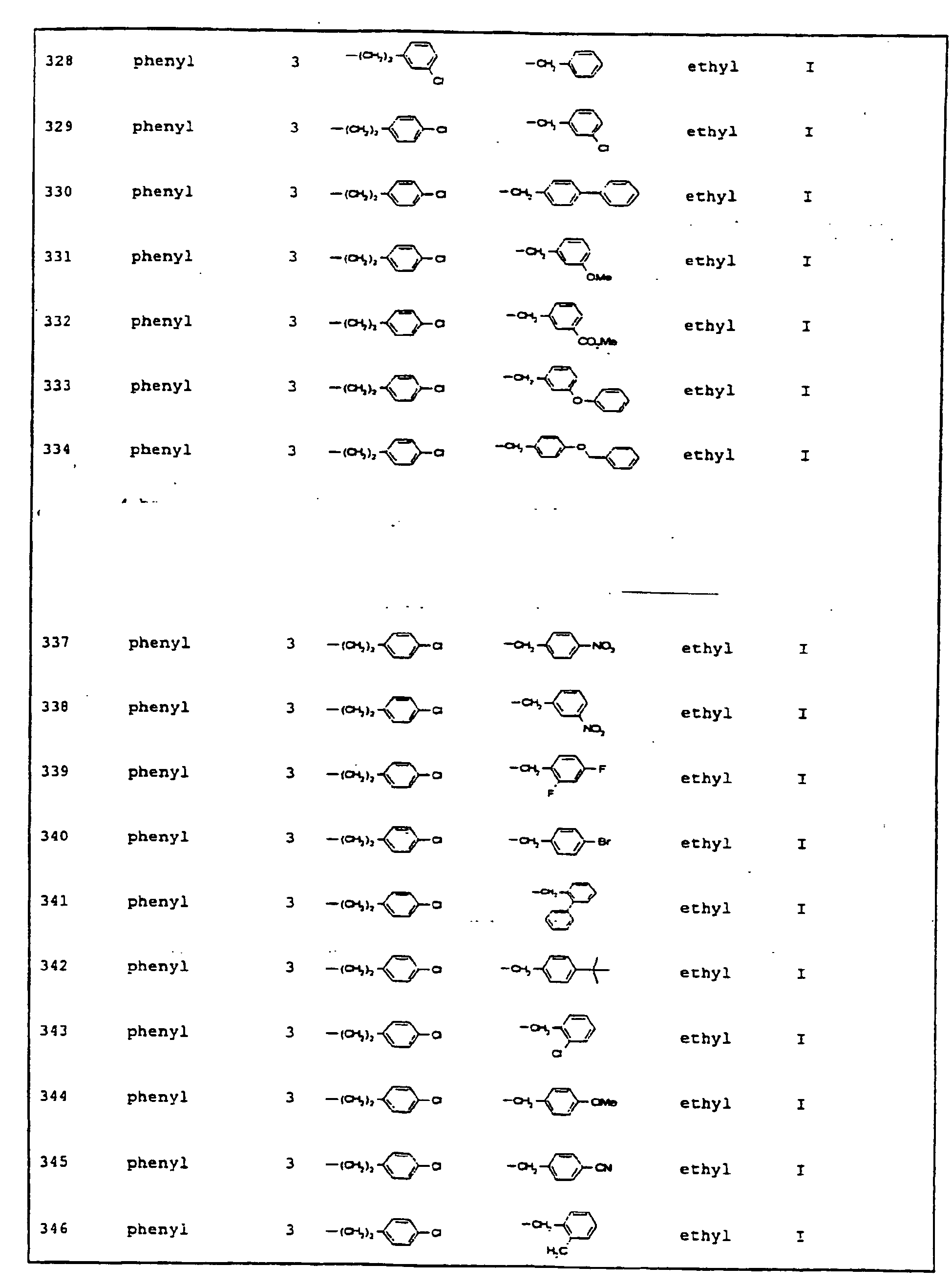

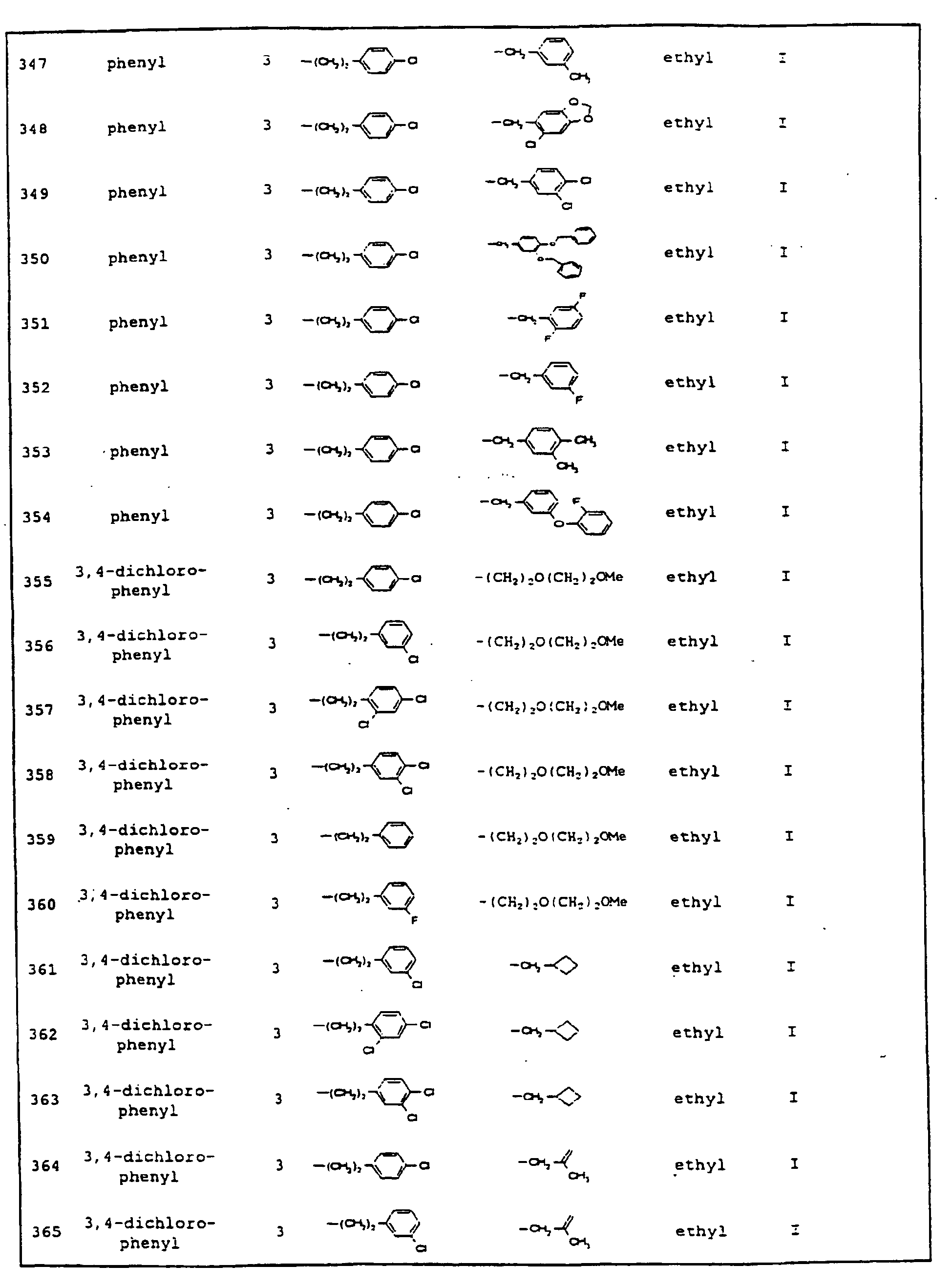

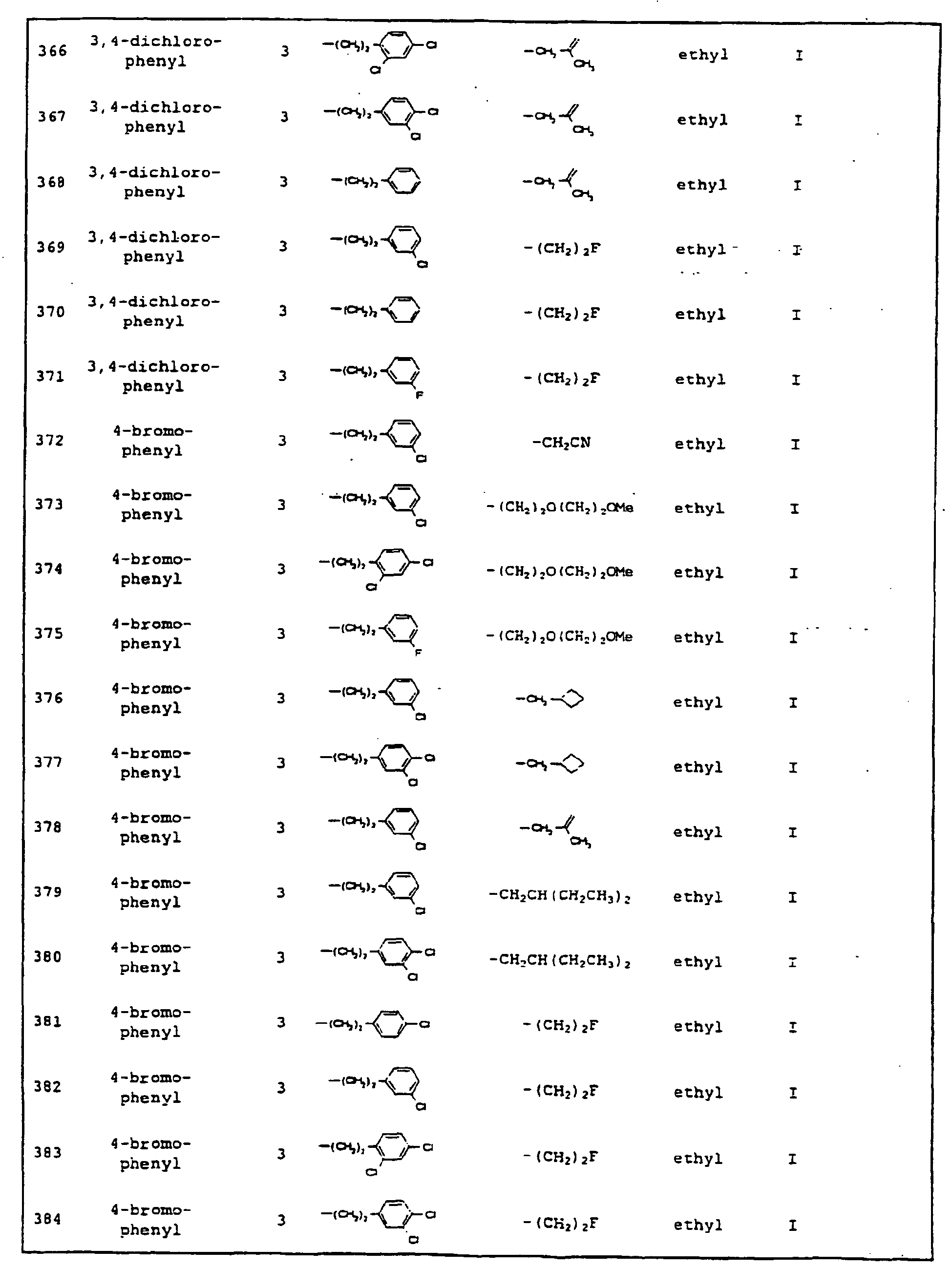

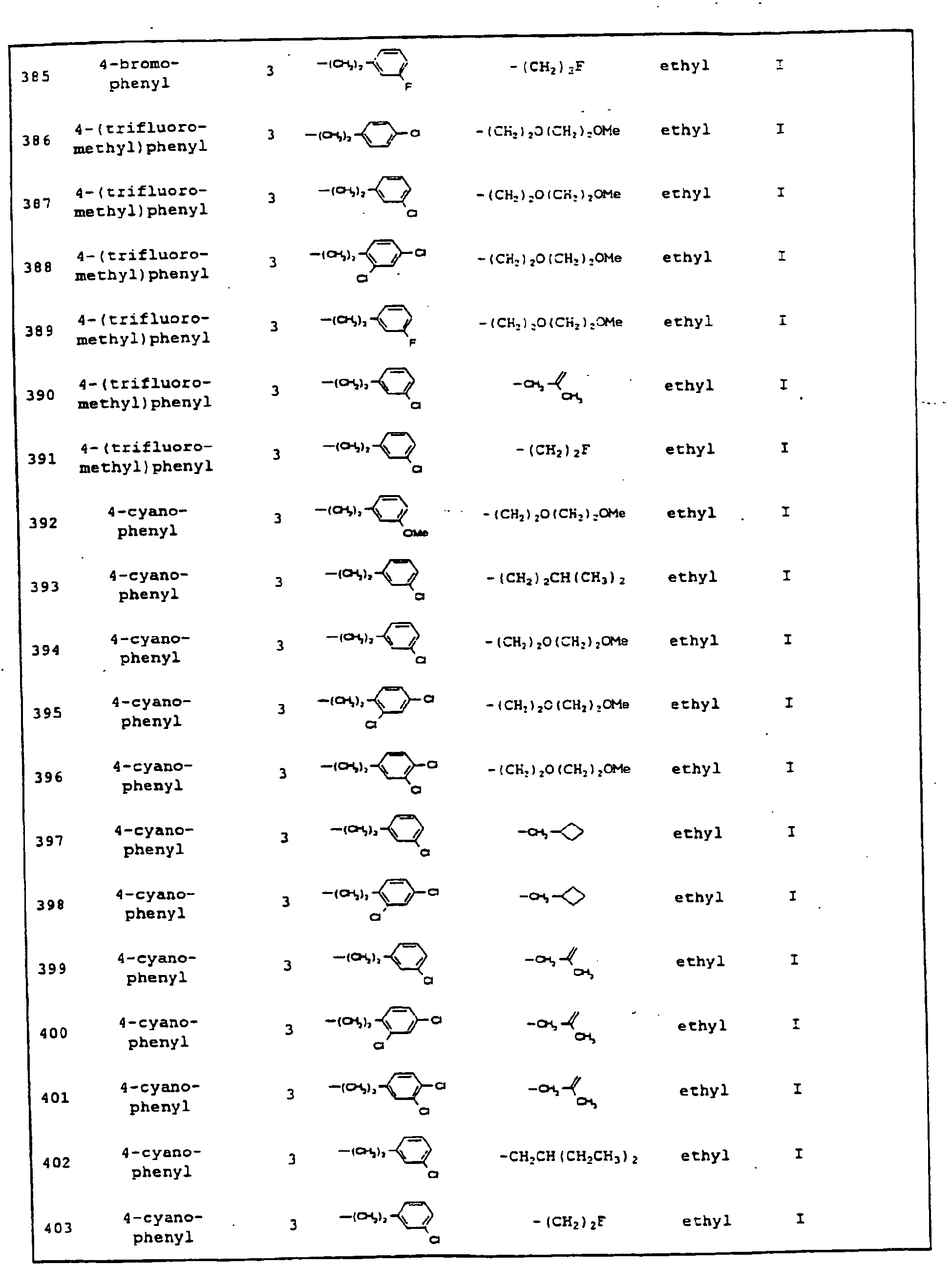

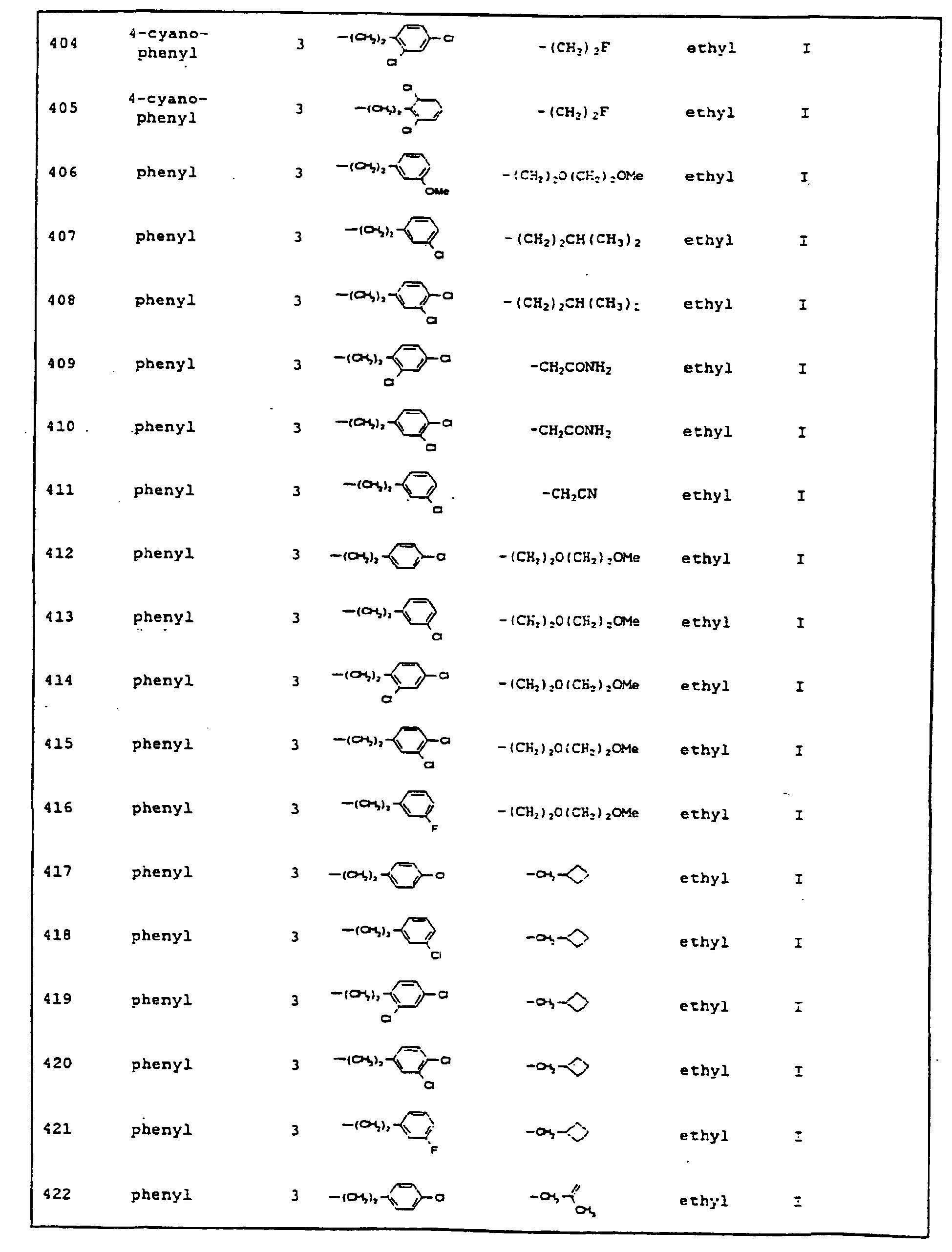

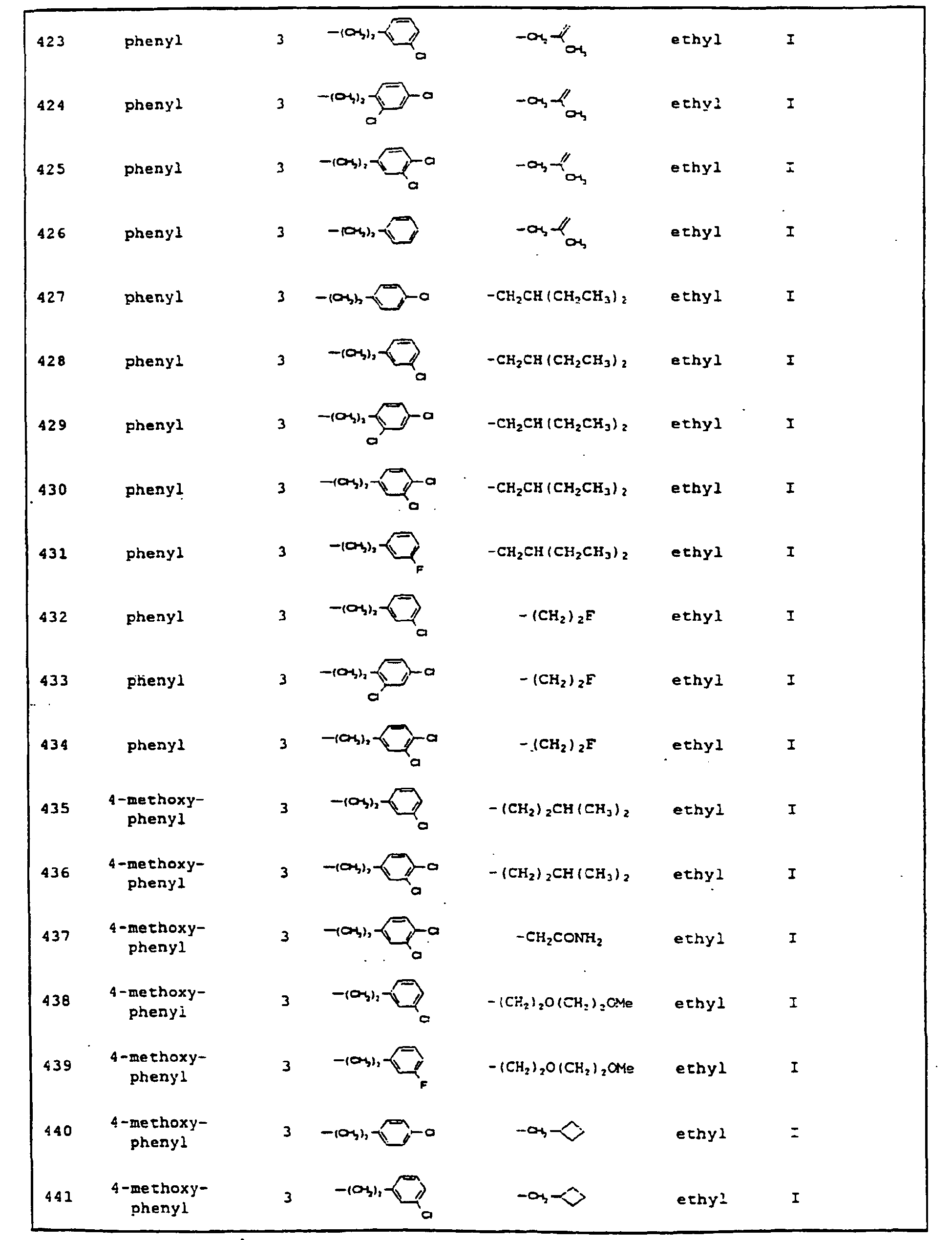

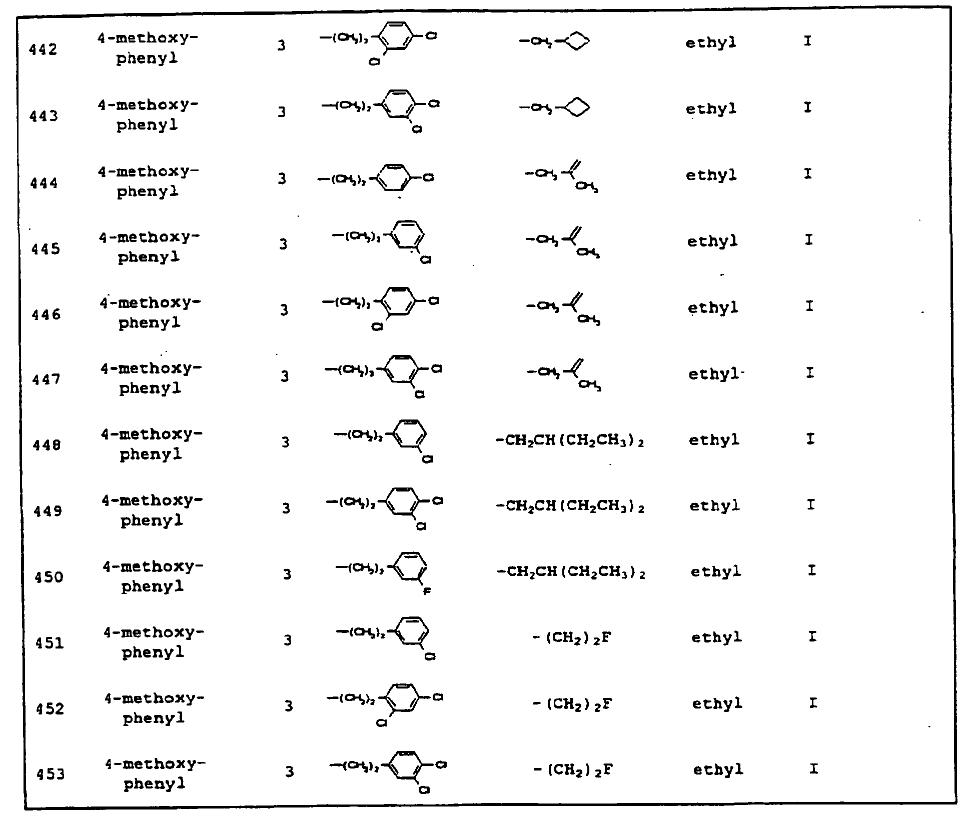

15. Pharmazeutische Zusammensetzung, die eine Verbindung gemäss mindestens einem der Ansprüche 1 bis 14 umfasst.

16. Verwendung einer Verbindung gemäss mindestens einem der Ansprüche 1 bis 14 zur Herstellung eines Medikaments zur Behandlung einer durch CCR-3 übertragenen Erkrankung.

17. Verwendung gemäss Anspruch 16, worin das Medikament zur Behandlung einer durch CCR-3 übertragenen Erkrankung bei einem Säuger dient.

18. Verwendung gemäss Anspruch 16 oder 17, worin die durch CCR-3 übertragene Erkrankung eine durch Eosinophil übertragene Erkrankung ist.

19. Verwendung gemäss Anspruch 18, worin die Erkrankung ausgewählt ist aus Asthma, Rhinitis, Ekzem, entzündlichen Darmerkrankungen und Parasiteninfektionen.

20. Verwendung gemäss Anspruch 16 oder 17, worin die durch CCR-3 übertragene Erkrankung eine durch T-Zellen oder Dendritenzellen übertragene Erkrankung ist.

21. Verwendung gemäss Anspruch 20, worin die Erkrankung ausgewählt ist aus Autoimmunerkrankungen und HIV.

## Revendications

1. Composé ayant la formule (I) suivante ; 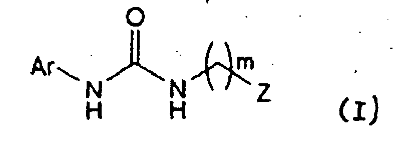 ou un sel, hydrate ou complexe de celui-ci, dans lequel :
m est 3, 4, 5 ou 6;
Ar est un aryle ou hétéroaryle
éventuellement substitué avec un ou plusieurs groupes indépendamment choisis dans le groupe constitué par un hydroxy, un halogène, un trihalométhyle, un trihalométhoxy, un alkyle en C₁₋₅, un alcoxy en C₁₋₅, un cyano, un nitro, un amino, un carboxy, un alkyloxycarbonyle, un arylméthyloxycarbonyle, un carbamoyle, un alkylcarbamoyle, un arylcarbamoyle, un acyle, un acyloxy, un sulfonyle, un alkylsulfonyle, un arylsulfonyle, un sulfamoyle, un alkylsulfamoyle, un arylsulfamoyle, un alkylthio, un alkylsulfonamide, un arylsulfonamide, un hydrazino, un acylamino, un alkylamino, un hydroxyamino, un amidino, un guanidino, un cyanoguanidino, un aryle
éventuellement substitué avec un ou plusieurs groupes indépendamment choisis parmi un hydroxy, un halogène, un trihalométhyle, un alkyle en C₁₋₅, un alcoxy en C₁₋₅, un cyano, un nitro, un amino et un carboxy,
et un aryloxy
éventuellement substitué avec un ou plusieurs groupes indépendamment choisis parmi un hydroxy, un halogène, un trihalométhyle, un alkyle en C₁₋₅, un alcoxy en C₁₋₅, un cyano, un nitro, un amino et un carboxy,
Z est : 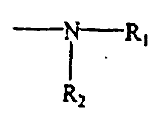 ou 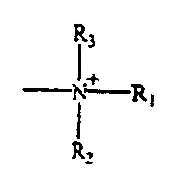
où R₁ est : 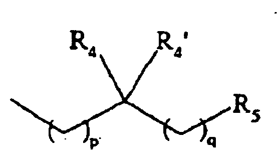 ou 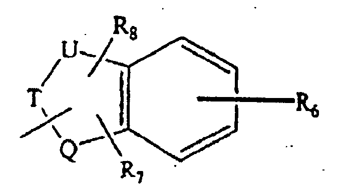 ou 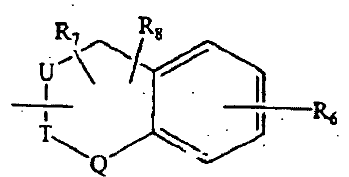 ou 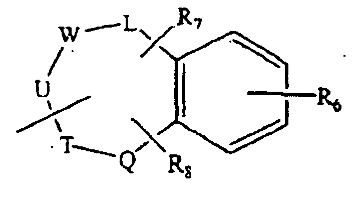
p est 0, 1 ou 2;
q est 0 , 1 ou 2;
R₄ et R_{4'} sont indépendamment choisis parmi un hydrogène, un halogène, un alkyle en C₁₋₅, un aryle, un hétéroaryle
où l'aryle ou hétéroaryle est éventuellement substitué avec un ou plusieurs groupes indépendamment choisis parmi un hydrogène, un hydroxy, un halogène, un trihalométhyle, un alkyle en C₁₋₅, un alcoxy en C₁₋₅, un cyano, un nitro, un amino, un carboxy, un carbamoyle, un alkylcarbamoyle, un arylcarbamoyle, un acyle, un acyloxy, un sulfonyle, un alkylsulfonyle, un arylsulfonyle, un sulfamoyle, un alkylsulfamoyle, un arylsulfamoyle, un alkylthio, un alkylsulfonamide, un arylsulfonamide, un hydrazino, un acylamino, un alkylamino, un hydroxyamino, un amidino, un guanidino, et un cyanoguanidino;
et COR₉, où R₉ est un hydroxy, un alkyle en C₁₋₅, un alcoxy en C₁₋₅, un amino, un alkylamino ou un arylamino;
R₅ est un aryle ou hétéroaryle
éventuellement substitué avec un ou plusieurs groupes indépendamment choisis parmi un hydroxy, un halogène, un trihalométhyle, un alkyle en C₁₋₅, un alcoxy en C₁₋₅, un cyano, un nitro, un amino, un carboxy, un carbamoyle, un alkylcarbamoyle, un arylcarbamoyle, un acyle, un acyloxy, un sulfonyle, un alkylsulfonyle, un arylsulfonyle, un sulfamoyle, un alkylsulfamoyle, un arylsulfamoyle, un alkylthio, un alkylsulfonamide, un arylsulfonamide, un hydrazino, un acylamino, un alkylamino, un hydroxyamino, un amidino, un guanidino, un cyanoguanidino, un aryle
éventuellement substitué avec un ou plusieurs groupes indépendamment choisis parmi un hydroxy, un halogène, un trihalométhyle, un alkyle en C₁₋₅, un alcoxy en C₁₋₅, un cyano, un nitro, un amino et un carboxy,
et un aryloxy
éventuellement substitué avec un ou plusieurs groupes indépendamment choisis parmi un hydroxy, un halogène, un trihalométhyle, un alkyle en C₁₋₅, un alcoxy en C₁₋₅, un cyano, un nitro, un amino et un
carboxy,
R₆ est choisi parmi un hydrogène, hydroxy, un halogène, un trihalométhyle, un alkyle en C₁₋₅, un alcoxy en C₁₋₅, un cyano, un nitro, un amino et un carboxy;
R₇ et R₈ sont indépendamment choisis parmi un hydrogène, un hydroxy, un halogène, un trihalométhyle, un alkyle en C₁₋₅, un alcoxy en C₁₋₅, un cyano, un nitro, un amino et un carboxy;
Q, T, U, W et L sont indépendamment choisis dans le groupe d'atomes constitué par C, N, O et S; où des atomes adjacents U-T, T-Q, U-W, W-L peuvent former une ou plusieurs doublés liaisons;
R₂ est choisi parmi un alkyle en C₁₋₈, alcényle en C₁₋₈ et alcynyle en C₁₋₈
substitué avec un ou plusieurs groupes indépendamment choisis parmi un carboxy, un carbamoyle, un alkylcarbamoyle, un arylcarbamoyle, un alkylsulfonylcarbamoyle, un arylsulfonylcarbamoyle, un alkyloxycarbonyle, un tétrazolyle, un isoxazolyle, un isothiazolyle; un alkylsulfonamido, un arylsulfonamido, un sulfonyle, un alkylsulfonyle, un arylsulfonyle, un sulfamoyle, un alkylsulfamoyle, un arylsulfamoyle, un alkylsulfonamide, un arylsulfonamide, un acyloxy, un acylamino, un aryle
substitué avec un ou plusieurs groupes indépendamment choisis parmi un alkyle en C₁₋₅ ou un alcoxy en C₁₋₅, où l'alkyle ou l'alcoxy est substitué avec un carboxy ou alkyloxycarbonyle, un carboxy, un carbamoyle, un alkylcarbamoyle, un arylcarbamoyle, un alkylsulfonylcarbamoyle, un arylsulfonylcarbamoyle, un alkyloxycarbonyle, un tétrazolyle, un isoxazolyle, un isothiazolyle, un alkylsulfonamido, un arylsulfonamido, un sulfonyle, un alkylsulfonyle, un arylsulfonyle, un sulfamoyle, un alkylsulfamoyle, un arylsulfamoyle, un alkylsulfonamide, un arylsulfonamide, un aryloxy, un arylméthyloxy, un acylamino, un hydroxy, et un halogène,
un hétéroaryle
substitué avec un ou plusieurs groupes indépendamment choisis parmi un alkyle en C₁₋₅ ou alcoxy en C₁₋₅ qui est substitué avec un carboxy ou alkyloxycarbonyle, un acylamino, un carboxy, un carbamoyle, un alkylcarbamoyle, un arylcarbamoyle, un alkylsulfonylcarbamoyle, un arylsulfonylcarbamoyle, un alkyloxycarbonyle, un tétrazolyle, un isoxazolyle, un isothiazolyle, un alkylsulfonamido, un arylsulfonamido, un sulfonyle, un alkylsulfonyle, un arylsulfonyle, un sulfamoyle, un alkylsulfamoyle, un arylsulfamoyle, un alkylsulfonamide, un arylsulfonamide, un hydroxy, et un halogène,
un alcoxy en C₁₋₅
éventuellement substitué avec un ou plusieurs groupes indépendamment choisis parmi un alkyle en C₁₋₅ ou alcoxy en C₁₋₅ qui peut être éventuellement substitué avec un carboxy ou alkyloxycarbonyle, un cyano, un nitro, un amino, un acylamino, un alkylamino, un carboxy, un carbamoyle, un alkylcarbamoyle, un arylcarbamoyle, un alkylsulfonylcarbamoyle, un arylsulfonylcarbamoyle, un alkyloxycarbonyle, un tétrazolyle, un isoxazolyle, un isothiazolyle, un alkylsulfonamido, un arylsulfonamido, un sulfonyle, un alkylsulfonyle, un arylsulfonyle, un sulfamoyle, un alkylsulfamoyle, un arylsulfamoyle, un alkylsulfonamide, un arylsulfonamide, un alkylthio, un acyle, un acyloxy, un hydrazino, un hydroxyamino, un amidino, un guanidino, un cyanoguanidino, un hydroxy, et un halogène,
un arylméthyloxy
substitué avec un ou plusieurs groupes indépendamment choisis parmi un alkyle en C₁₋₅ ou alcoxy en C₁₋₅ qui est substitué avec un carboxy ou alkyloxycarbonyle, un acylamino, un carboxy, un carbamoyle, un alkylcarbamoyle, un arylcarbamoyle, un alkylsulfonylcarbamoyle, un arylsulfonylcarbamoyle, un alkyloxycarbonyle, un tétrazolyle, un isoxazolyle, un isothiazolyle, un alkylsulfonamido, un arylsulfonamido, un sulfonyle, un alkylsulfonyle, un arylsulfonyle, un sulfamoyle, un alkylsulfamoyle, un arylsulfamoyle, un alkylsulfonamide, un arylsulfonamide, un hydroxy, et un halogène,
et un cycloalkyle en C₃₋₇
substitué avec un ou plusieurs groupes indépendamment choisis parmi un alkyle en C₁₋₅ ou alcoxy en C₁₋₅ qui est substitué avec un carboxy ou alkyloxycarbonyle, un acylamino, un carboxy, un carbamoyle, un alkylcarbamoyle, un arylcarbamoyle, un alkylsulfonylcarbamoyle, un arylsulfonylcarbamoyle, un alkyloxycarbonyle, un tétrazolyle, un isoxazolyle, un isothiazolyle, un alkylsulfonamido, un arylsulfonamido, un sulfonyle, un alkylsulfonyle, un arylsulfonyle, un sulfamoyle, un alkylsulfamoyle, un arylsulfamoyle, un
alkylsulfonamide et un arylsulfonamide;
R₃ est choisi parmi un alkyle en C₁₋₈, alcényle en C₁₋₈ et alcynyle en C₁₋₈
éventuellement substitué avec un ou plusieurs groupes indépendamment choisis dans le groupe constitué par un carboxy, un carbamoyle, un alkylcarbamoyle, un arylcarbamoyle, un alkylsulfonylcarbamoyle, un arylsulfonylcarbamoyle, un alkyloxycarbonyle, un tétrazolyle, un isoxazolyle, un isothiazolyle, un alkylsulfonamido, un arylsulfonamido, un sulfonyle, un alkylsulfonyle, un arylsulfonyle, un sulfamoyle, un alkylsulfamoyle, un arylsulfamoyle, un
alkylsulfonamide, un arylsulfonamide, un alkylthio, un halogène, un acyloxy, un hydroxy, un nitro, un amino, un acylamino, un alkylamino, un cyano, un aryle
éventuellement substitué avec un ou plusieurs groupes indépendamment choisis dans le groupe constitué par un alkyle en C₁₋₅ ou un alcoxy en C₁₋₅, où l'alkyle ou alcoxy peut être éventuellement substitué avec un carboxy ou alkyloxycarbonyle, un cyano, un nitro, un amino, un acylamino, un alkylamino, un carboxy, un carbamoyle, un alkylcarbamoyle, un arylcarbamoyle, un alkylsulfonylcarbamoyle, un arylsulfonylcarbamoyle, un alkyloxycarbonyle, un tétrazolyle, un isoxazolyle, un isothiazolyle, un alkylsulfonamido, un arylsulfonamido, un sulfonyle, un alkylsulfonyle, un arylsulfonyle, un sulfamoyle, un alkylsulfamoyle, un arylsulfamoyle, un alkylsulfonamide, un arylsulfonamide, un alkylthio, un acyle, un acyloxy, un aryloxy, un arylméthyloxy, un hydrazino, un hydroxyamino, un amidino, un guanidino, un cyanoguanidino, un hydroxy, et un halogène,
un hétéroaryle
éventuellement substitué avec un ou plusieurs groupes indépendamment choisis dans le groupe constitué par un alkyle en C₁₋₅ ou alcoxy en C₁₋₅ qui peut être éventuellement substitué avec un carboxy ou alkyloxycarbonyle, un cyano, un nitro, un amino, un acylamino, un alkylamino, un carboxy, un carbamoyle, un alkylcarbamoyle, un arylcarbamoyle, un alkylsulfonylcarbamoyle, un arylsulfonylcarbamoyle, un alkyloxycarbonyle, un tétrazolyle, un isoxazolyle, un isothiazolyle, un alkylsulfonamido, un arylsulfonamido, un sulfonyle, un alkylsulfonyle, un arylsulfonyle, un sulfamoyle, un alkylsulfamoyle, un arylsulfamoyle, un alkylsulfonamide, un arylsulfonamide, un alkylthio, un acyle, un acyloxy, un hydrazino, un hydroxyamino, un amidino, un guanidino, un cyanoguanidino, un hydroxy, et un halogène,
un alcoxy en C₁₋₅
éventuellement substitué avec un ou plusieurs groupes indépendamment choisis dans le groupe constitué par un alkyle en C₁₋₅ ou alcoxy en C₁₋₅ qui peut être éventuellement substitué avec un carboxy ou alkyloxycarbonyle, un cyano, un nitro, un amino, un acylamino, un alkylamino, un carboxy, un carbamoyle, un alkylcarbamoyle, un arylcarbamoyle, un alkylsulfonylcarbamoyle, un arylsulfonylcarbamoyle, un alkyloxycarbonyle, un tétrazolyle, un isoxazolyle, un isothiazolyle, un alkylsulfonamido, un arylsulfonamido, un sulfonyle, un alkylsulfonyle, un arylsulfonyle, un sulfamoyle, un alkylsulfamoyle, un arylsulfamoyle, un alkylsulfonamide, un arylsulfonamide, un alkylthio, un acyle, un acyloxy, un hydrazino, un hydroxyamino, un amidino, un guanidino, un cyanoguanidino, un hydroxy, et un halogène,
un arylméthoxy
éventuellement substitué avec un ou plusieurs groupes indépendamment choisis dans le groupe constitué par un alkyle en C₁₋₅ ou alcoxy en C₁₋₅ qui est éventuellement substitué avec un carboxy ou alkyloxycarbonyle, un cyano, un nitro, un amino, un acylamino, un alkylamino, un carboxy, un carbamoyle, un alkylcarbamoylé, un arylcarbamoyle, un alkylsulfonylcarbamoyle, un arylsulfonylcarbamoyle, un alkyloxycarbonyle, un tétrazolyle, un isoxazolyle, un isothiazolyle, un alkylsulfonamido, un arylsulfonamido, un sulfonyle, un alkylsulfonyle, un arylsulfonyle, un sulfamoyle, un alkylsulfamoyle, un arylsulfamoyle, un alkylsulfonamide, un arylsulfonamide, un alkylthio, un acyle, un acyloxy, un hydrazino, un hydroxyamino, un amidino, un guanidino, un cyanoguanidino, un hydroxy, et un halogène,
et un cycloalkyle en C₃₋₇
éventuellement substitué avec un ou plusieurs groupes indépendamment choisis dans le groupe constitué par un alkyle en C₁₋₅ ou alcoxy en C₁₋₅ qui est éventuellement substitué avec un carboxy ou alkyloxycarbonyle, un cyano, un nitro, un amino, un acylamino, un alkylamino, un carboxy, un carbamoyle, un alkylcarbamoyle, un arylcarbamoyle, un alkylsulfonylcarbamoyle, un arylsulfonylcarbamoyle, un alkyloxycarbonyle, un tétrazolyle, un isoxazolyle, un isothiazolyle, un alkylsulfonamido, un arylsulfonamido, un sulfonyle, un alkylsulfonyle, un arylsulfonyle, un sulfamoyle, un alkylsulfamoyle, un arylsulfamoyle, un alkylsulfonamide, un arylsulfonamide, un alkylthio, un acyle, un acyloxy, un hydrazino, un hydroxyamino, un amidino, un guanidino, un cyanoguanidino, un hydroxy, et un halogène, et un hétérocyle;
avec la condition que aucun de R₁, R₂ et R₃ ne se lient entre eux;
avec la condition supplémentaire que Ar ne soit pas un 2-hydroxy-5-méthoxyphényle, un 2-hydroxy-5-alcoxy(inférieur)phényle, et lorsque Ar est un pyrène, un chrysène ou un phénanthrène, alors m ≠ 3 et 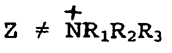 NR₁R₂R₃ avec R₁ = R₂ = Me et R₃ = Bz, et de plus avec la condition que lorsque Ar est un phényle,
Z est 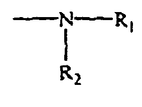 et R₂ est un méthyle, alors R₁ n'est pas

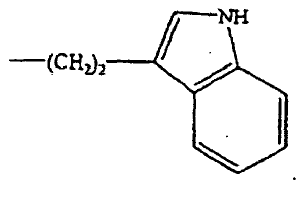

2. Composé selon la revendication 1, dans lequel Z est

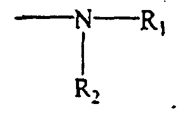

3. Composé selon la revendication 2, dans lequel m est 3, 4 ou 5.

4. Composé selon la revendication 3, dans lequel m est 3 ou 4.

5. Composé de la revendication 1, dans lequel R₂ est indépendamment choisi parmi un alkyle en C₁₋₈, alcényle en C₁₋₈ et alcynyle en C₁₋₈
substitué avec un ou plusieurs groupes indépendamment choisi parmi un carboxy, un carbamoyle, un alkylcarbamoyle, un arylcarbamoyle, un alkylsulfonylcarbamoyle, un arylsulfonylcarbamoyle, un alkyloxycarbonyle, un tétrazolyle, un isoxazolyle, un isothiazolyle, un alkylsulfonamido, un arylsulfonamido, un sulfonyle, un alkylsulfonyle, un arylsulfonyle, un sulfamoyle, un alkylsulfamoyle, un arylsulfamoyle, un alkylsulfonamide, un arylsulfonamide et un acylamino.

6. Composé de la revendication 5, dans lequel R₂ est indépendamment choisi parmi un alkyle en C₁₋₈, alcényle en C₁₋₈ et alcynyle en C₁₋₈, substitué avec un ou plusieurs groupes indépendamment choisis parmi un carboxy et un alkyloxycarbonyle.

7. Composé de la revendication 1, dans lequel Z est

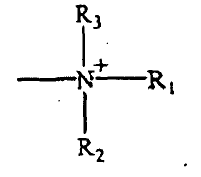

8. Composé selon la revendication 7, dans lequel m est 3, 4 ou 5.

9. Composé selon la revendication 8, dans lequel m est 3 ou 4.

10. Composé selon la revendication 9, dans lequel R₃ est un alkyle en C₁₋₈ éventuellement substitué avec un ou plusieurs groupes indépendamment choisis dans le groupe constitué par un hydroxy, un halogène, un trihalométhyle, un alkyle en C₁₋₅, un alcoxy en C₁₋₅, un cyano, un nitro, un amino et un carboxy.

11. Composé selon la revendication 1, dans lequel
Ar est tel que défini dans la revendication 1 excepté pour un aryle substitué avec un trihalométhoxy; '
R₂ est choisi parmi un alkyle en C₁₋₈, alcényle en C₁₋₈ et alcynyle en C₁₋₈,substitué avec un ou plusieurs groupes tels que définis dans la revendication 1, excepté pour
(i) un acyloxy substitué,
(ii) un carboxy, un carbamoyle, un alkylcarbamoyle, un arylcarbamoyle, un alkylsulfonylcarbamoyle, un arylsulfonylcarbamoyle, un alcoxycarbonyle, un tétrazolyle, un isoxazolyle, un isothiazolyle, un alkylsulfonamido, un arylsulfonamido, un sulfonyle, un alkylsulfonyle, un arylsulfonyle, un acylamino, un aryle, chacun substitué avec un acylamino, un aryloxy, un arylméthyloxy et
(iii) un alcoxy en C₁₋₅ éventuellement substitué;
R₃ est choisi parmi un alkyle en C₁₋₈, alcényle en C₁₋₈ et alcynyle en C₁₋₈, éventuellement substitué avec un ou plusieurs groupes tels que définis dans la revendication 1, excepté pour
(i) un acyloxy éventuellement substitué, et
(ii) un carboxy, un carbamoyle, un alkylcarbamoyle, un arylcarbamoyle, un alkylsulfonylcarbamoyle, un arylsulfonylcarbamoyle, un alcoxycarbonyle, un tétrazolyle, un isoxazolyle, un isothiazolyle, un alkylsulfonylamido, un arylsulfonylamido, un sulfonyle, un alkylsulfonyle, un arylsulfonyle, un acylamino, un aryle, chacun substitué avec un aryloxy, un arylméthyloxy et
(iii) un alcoxy en C₁₋₅ éventuellement substitué; et
R₄, est un hydrogène.

12. Composé selon la revendication 11, dans lequel Ar est un aryle
éventuellement substitué avec un ou plusieurs groupes indépendamment choisis parmi un hydroxy, un halogène, un trihalométhyle, un alkyle en C₁₋₅, un alcoxy en C₁₋₅, un cyano, un nitro, un amino, un carboxy, un carbamoyle, un alkylcarbamoyle, un arylcarbamoyle, un acyle, un acyloxy, un sulfonyle, un alkylsulfonyle, un arylsulfonyle, un sulfamoyle, un alkylsulfamoyle, un arylsulfamoyle, un alkylthio, un alkylsulfonamide, un arylsulfonamide, un hydrazino, un acylamino, un alkylamino, un hydroxyamino, un amidino, un guanidino, un cyanoguanidino, un aryle
éventuellement substitué avec un ou plusieurs groupes indépendamment choisis dans le groupe constitué par un hydroxy, un halogène, un trihalométhyle, un alkyle en C₁₋₅, un alcoxy en C₁₋₅, un cyano, nitro, amino et un carboxy,
et un aryloxy
éventuellement substitué avec un ou plusieurs groupes indépendamment choisis parmi un hydroxy, un halogène, un trihalométhyle, un alkyle en C₁₋₅, un alcoxy en C₁₋₅, un cyano, nitro, amino et un carboxy.

13. Composé selon la revendication 1 choisi parmi
le N-phénylcarbamoyl-N'-[2-(4-chlorophényl)éthyl]-N'-éthyl-1,3-diaminopropane;
le N-(4-nitrophénylcarbamoyl-N'-[2-(4-chlorophényl)éthyl]-N'-éthyl-1,3 diaminopropane;
le N-(4-bromophénylcarbamoyl-N'-[2-(4-chlorophényl)éthyl]-N'-éthyl-1,3-diaminopropane;
le N-phénylcarbamoyl-N'-[2-(4-chlorophényl)éthyl]-N'-propyl-1,3-diaminopropane;
le 4-[[3-(4-bromophényluréido)propyl](1,2,3,4-tétrahydro-1-naphtyl)amino]butylate de méthyle;
le 4-[[3-(4-bromophényluréido)propyl][(1R)-1-phényléthyl]amino]butylate de méthyle;
le 4-[[3-(4-bromophényluréido)propyl][2-(4-chlorophényl)éthyl]amino]butylate de méthyle
le 4-[[4-(4-bromophényluréido)butyl)(1,2,3,4-tétrahydro-1-naphtyl)amino]butylate de méthyle;
le 4-[[5-(4-bromophényluréido)pentyl](1,2,3,4-tétrahydro-1-naphtyl)amino]butylate de méthyle;
le 4-[[3-(4-méthylphényluréido)propyl](1,2,3,4-tétrahydro-1-naphtyl)amino]butylate de méthyle;
le 4-[[3-(3,4-dichlorophényluréido)propyl](1,2,3,4-tétrahydro-1-naphtyl)amino]butylate de méthyle;
l'acide 4-[[3-(4-bromophényluréido)propyl](1,2,3,4-tétrahydro-1-naphtyl)amino]butanoïque;
l'acide 4-[[3-(4-bromophényluréido)propyl][(1R)-1-phényléthyl]amino]butanoïque;
l'acide 4-[[4-(4-bromophényluréido)butyl](1,2,3,4-tétrahydro-1-naphtyl)amino]butanoique;
l'acide 4-[[5-(4-bromophényluréido)pentyl](1.,2,3,4-tétrahydro-1-naphtyl)amino)butanoïque;
l'acide 4-[[3-(4-méthylphényluréido)propyl](1,2,3,4-tétrahydro-1-naphtyl)amino]butanoique;
l'acide 4-[[3-(3,4-dichlorophényluréido)propyl](1,2,3,4-tétrahydro-1-naphtyl)amino]butanoique;
le iodure de [3-(phényluréido)propyl] [2-(4-chlorophényl)éthyl]diéthylammonium;
le iodure de [3-(4-bromophényluréido)propyl][2-(4-chlorophényl)éthyl]diéthylammonium;
le N-phénylcarbamoyl-N'-[2-(4-chlorophényl)éthyl]-N'-éthyl-2-hydroxy-1,3-diaminopropane;
l'acide 4-[[(3S)-3-(4-bromophényluréido)-3-(tert-butoxycarbonyl)propyl](1,2,3,4-tétrahydro-1-naphtyl)amino]butanoique;
l'acide 4-[[3-(4-bromophényluréido)-2-hydroxypropyl] (1,2,3,4-tétrahydro-1-naphtyl)amino]butanoique;
l'acide 4-[[3-(4-chlorophényluréido)propyl](1,2,3,4-tétrabydro-1-naphtyl) amino] butanoique;
le 4- [[3- (4-bromophényluréido)propyl] (1-indanyl)amino]butylate de méthyle;
l'acide 4-[[3-(4-bromophényluréido)propyl](1-indanyl)amino]butanoique;
le 4-[[3-(4-bromophényluréido)propyl] [(1R)-1-indanyl]amino]butylate de méthyle;
l'acide 4-[[3-(4-bromophényluréido)propyl] [(1R)-1-indanyl]amino]butanoïque;
le 4- [[3- (4-bromophényluréido)propyl] [(1R)-1, 2, 3, 4-tétrahydro-1-naphtyl]amino]butylate de méthyle;
l'acide 4-[[3-(4-bromophényluréido)propyl][(1R)-1,2,3,4-tétrahydro-1-naphtyl]amino]butanoique;
le 4-[[3-(4-bromophényluréido)propyl] (1,2,3,4-tétrahydro-1-naphtyl)amino]butylate d'éthyle;
le 4-[[3-(4-bromophényluréido)propyl] (1,2,3,4-tétrahydro-1-naphtyl) amino] butanamide;
le 3- [[3- (4-bromophényluréido)propyl] (1, 2, 3, 4-tétrahydro-1-naphtyl)amino]-1-[(phénylsulfonyl)carbamoyl]-propane;
le 4- [[3- (4-bromophényluréido) propyl] (1,2,3,4-tétrahydro-1-naphtyl) amino]-1-butanol;
le 3- [[3- (4-bromophényluréido) propyl] (1,2,3,4-tétrahydro-1-naphtyl)amino]-1-[1-(triphénylméthyl)tétrazol-5-yl]propane ;
le 3- [[3- (4-bromophényluréido) propyl] (1,2,3,4-tétrahydro-1-naphtyl)amino]-1-(1H-tétrazol-5-yl)propane;
le 4-[[3-[4-(carboxy)phényluréido)propyl] (1,2,3,4-tétrahydro-1-naphtyl)amino]butylate de méthyle;
l'acide 4-[[3-(4-bromophényluréido)propyl] [(1R)-1-(4-methoxyphényl)éthyl]amino]butanoique;
l'acide 4-[[3-[4-(éthoxycarbonyl)phényluréido]propyl]-(1,2,3,4-tétrahydro-1-naphtyl)amino]butanoique
l'acide 4-[[3-(4-iodophényluréido)propyl] (1,2,3,4-tétrahydro-1-naphtyl)amino]butanoique;
l'acide 4-[[3-[4-(butoxycarbonyl)phényluréido]propyl]-(1,2,3,4-tétrahydro-1-naphtyl)amino]butanoique;
la [3-(phényluréido)propyl]bis[2-(4-chlorophényl)éthyl]amine;
l'acide 4-[[3-(4-bromophényluréido)propyl][(1R)-1-(4-bromophényl)éthyl]amino]butanoïque;
l'acide 4-[[3-(4-bromophényluréido)propyl][1-(4-fluorophényl)éthyl]amino]butanoïque;
l'acide 4-[[3-(4-bromophényluréido)propyl][1-(4-chlorophényl)éthyl]-amino]butanoïque;
le 4-[[(3S)-3-(4-bromophényluréido)-3-(tert-butoxycarbonyl)propyl](1,2,3,4-tétrahydro-1-naphtyl)amino]butylate de méthyle;
le 4-[[(3S)-3-(4-bromophényluréido)-3-(isopropylcarbamoyl)propyl](1,2,3,4-tétrahydro-1-naphtyl)amino]butylate de méthyle;
le 4-[[(3S)-3-(4-bromophényluréido)-3-(benzylcarbamoyl)propyl](1,2,3,4-tétrahydro-1-naphtyl)amino]butylate de méthyle;
l'acide 4-[[(3S)-3-(4-bromophényluréido)-3-(isopropylcarbamoyl)propyl](1,2,3,4-tétrahydro-1-naphtyl)amino]butanoïque;
l'acide 4-[[(3S)-3-(4-bromophényluréido)-3-(benzylcarbamoyl)propyl](1,2,3,4-tétrahydro-1-naphtyl)amino]butanoïque;
l'acide 4- [[3- (4-bromophényluréido) propyl] [(1S) -1- (4-bromophényl) éthyl]amino]butanoïque;
le iodure de [3-(phényluréido)propyl] [2- (4-chlorophényl) éthyl] bis (4-méthylbenzyl) ammonium;
le iodure de [3-(phényluréido)propyl] [2-(4-chlorophényl) éthyl] (4-chlorobenzyl) éthylammonium;
le iodure de [3- (phényluréido)propyl] [2-(4-chlorophényl) éthyl] (benzyl) éthylammonium;
le iodure de [3-(phényluréido)propyl] [2- (3-chlorophényl) éthyl] diéthylammonium;
le trifluoroacétate de [3-(4-bromophényluréido)propyl]-[(1S)-1-phényléthyl] [3- (carboxy)propyl] éthylammonium;
le trifluoroacétate de [3-(4-bromophényluréido)propyl)-[(1R)-1-phényléthyl] [3- (carboxy)propyl] éthylammonium;
le iodure de (3-(phényluréido)propyl] [2- (4-chlorophényl)éthyl] [4- (méthoxycarbonyl) butyl] éthylammonium;
le iodure de [3- (phényluréido)propyl] [2- (4-chlorophényl) éthyl] [4-carboxy) benzyl] éthylammonium;
le iodure de [5-(phényluréido)pentyl] [2- (4-chlorophényl) éthyl] diéthylammonium;
le iodure de [3-(phényluréido)propyl] [2-(4-chlorophényl)éthyl] (2-chlorobenzyl)éthylammonium;
le iodure de [3-(phényluréido)propyl] [2- (4-chlorophényl)éthyl](2,5-difluorobenzyl)éthylammonium;
le iodure de [3-(phényluréido)propyl] [2-(4-chlorophényl)éthyl] (3-fluorobenzyl)éthylammonium;
le iodure de [3-(4-cyanophényluréido)propyl] [2- (3-chlorophényl)éthyl][2-(2-méthoxyéthoxy)éthyl]éthylammonium; et
le iodure de [3- (4-méthoxyphényluréido)propyl] [2- (3-chlorophényl)éthyl][2-(2-méthoxyéthoxy)éthyl]éthylammonium.

14. Composé selon la revendication 1, dans lequel le composé est choisi parmi les formules suivantes : 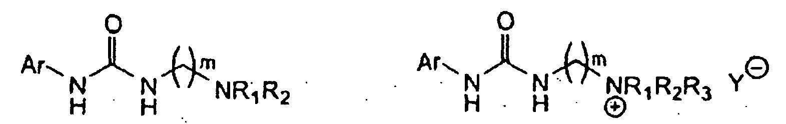 dans lesquelles Ar, R₁, R₂, R₃, Y et m sont définis comme montré dans les Tableaux suivants :

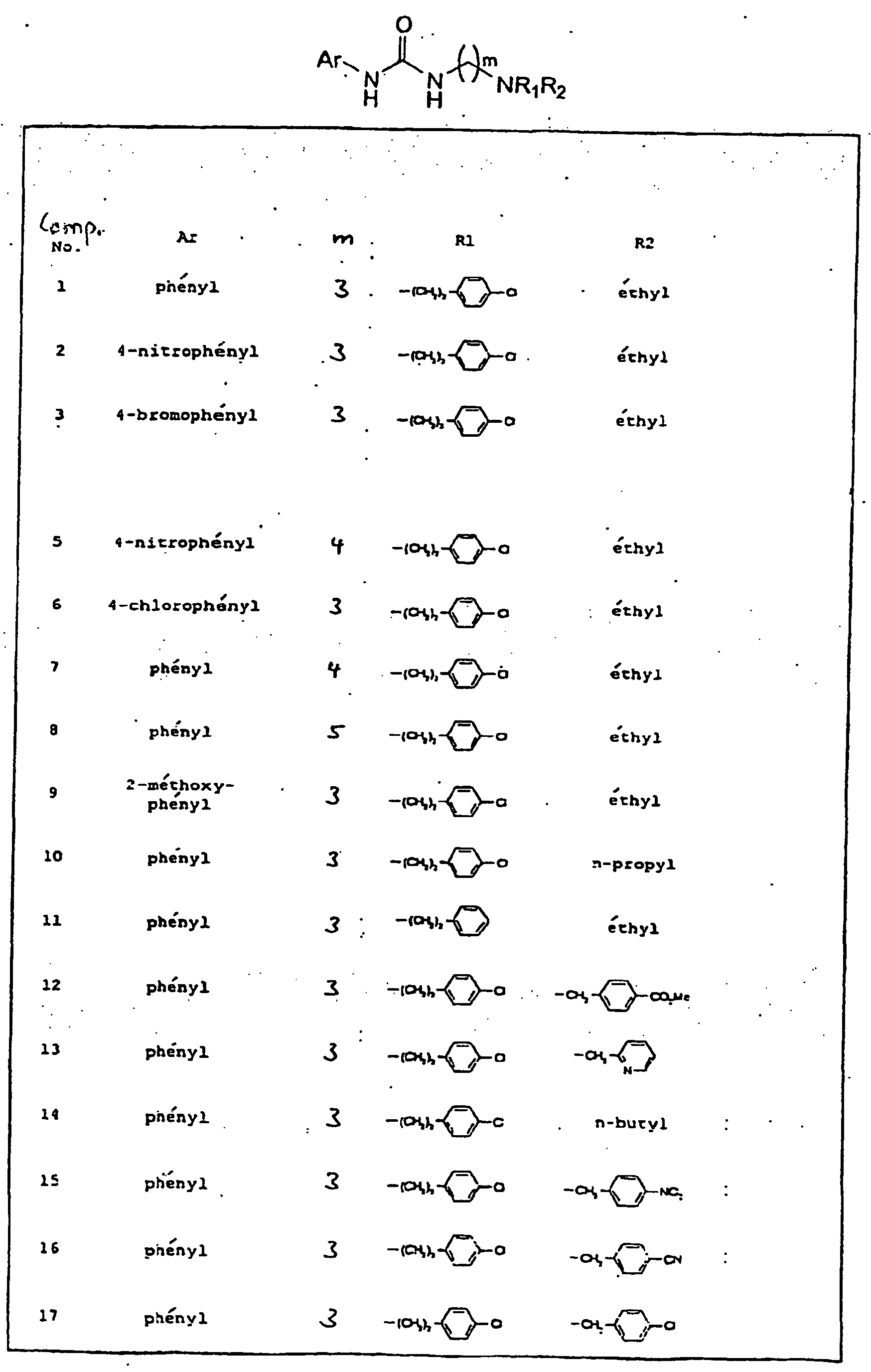

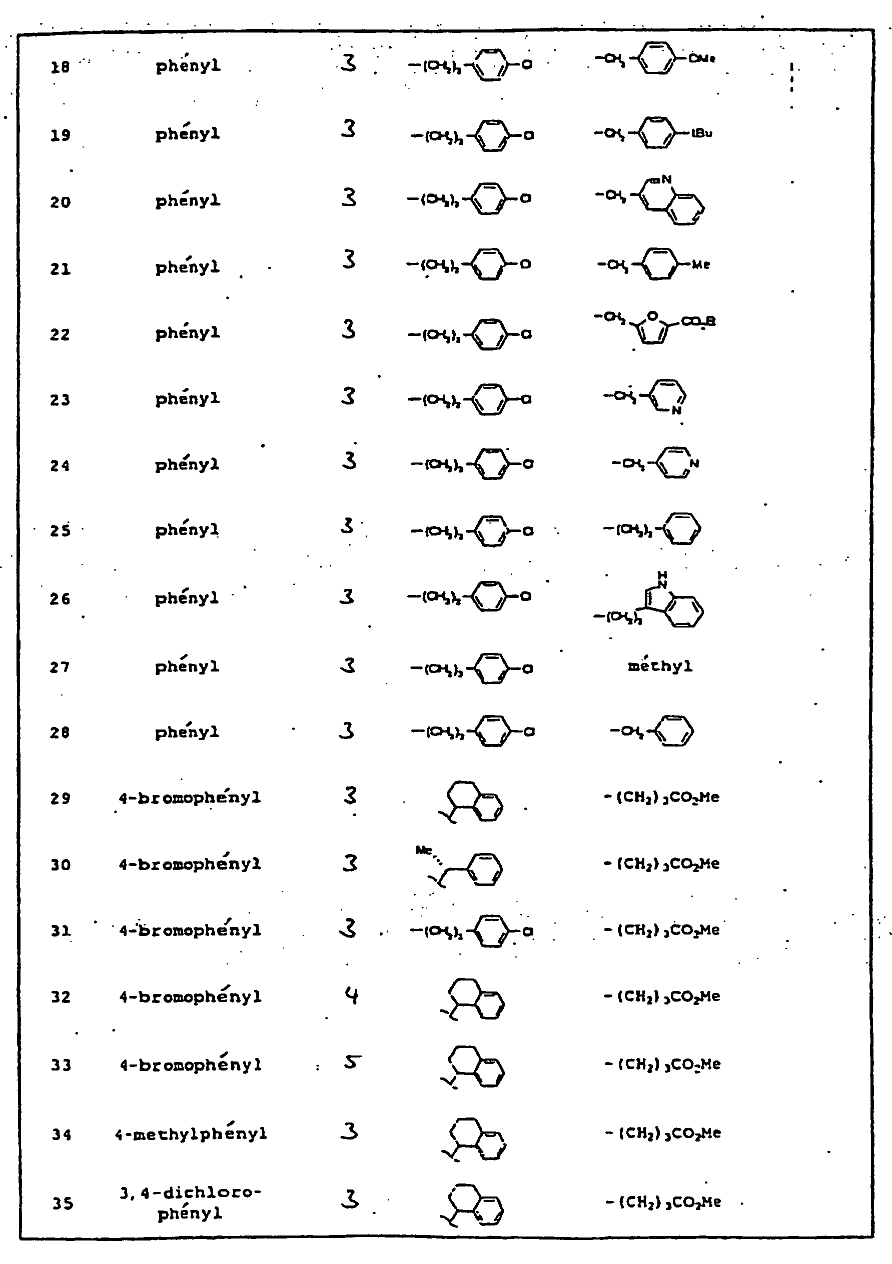

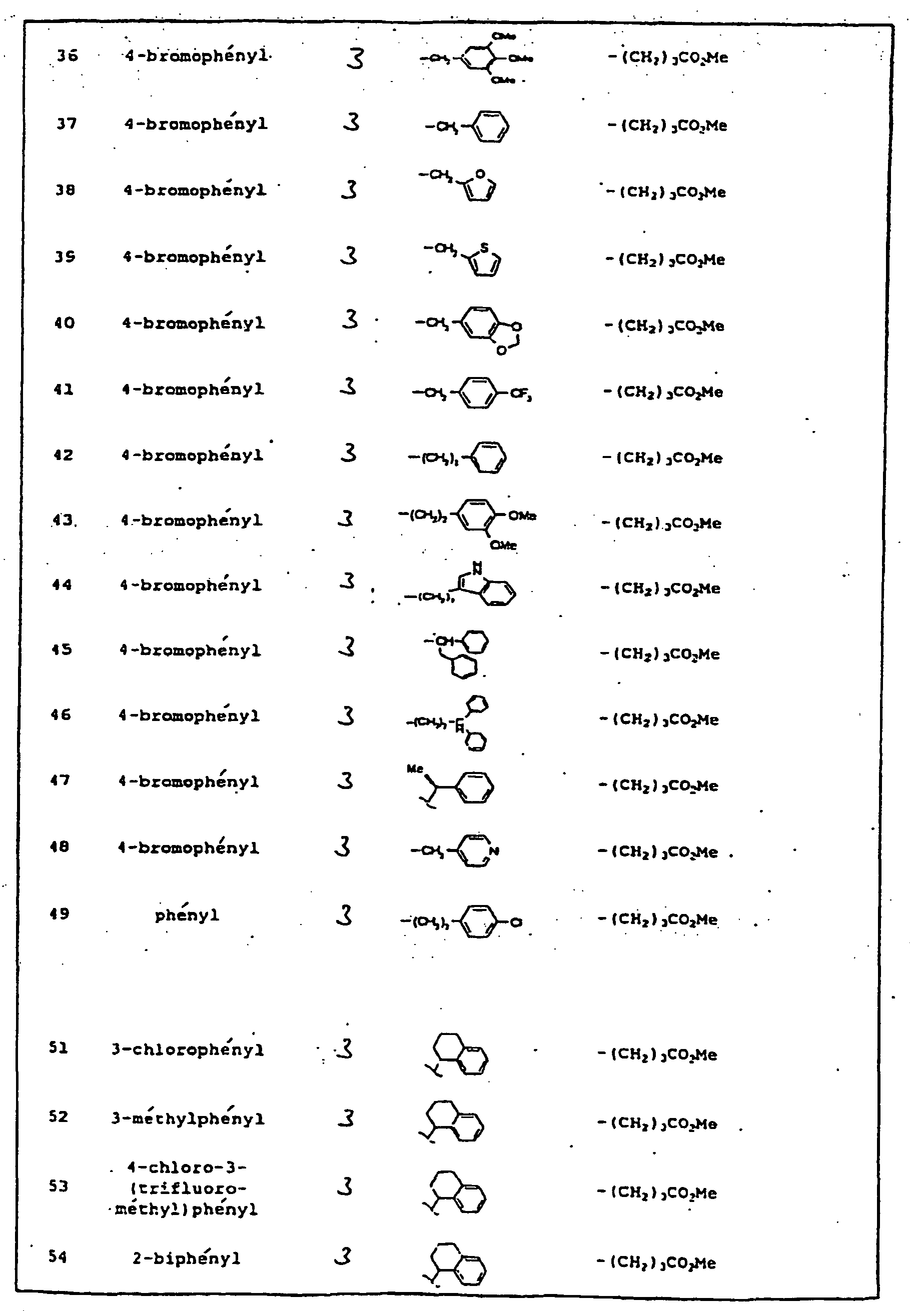

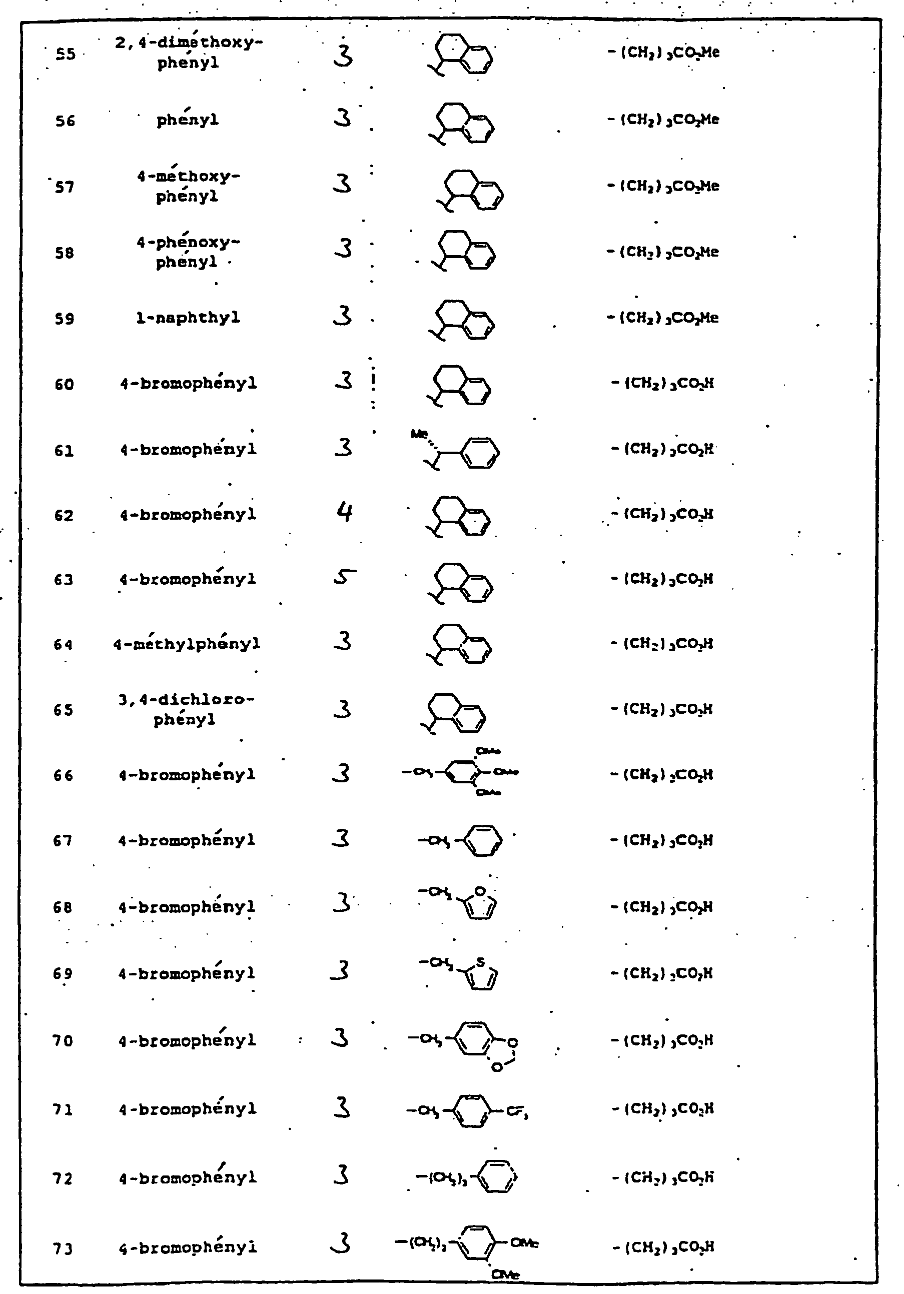

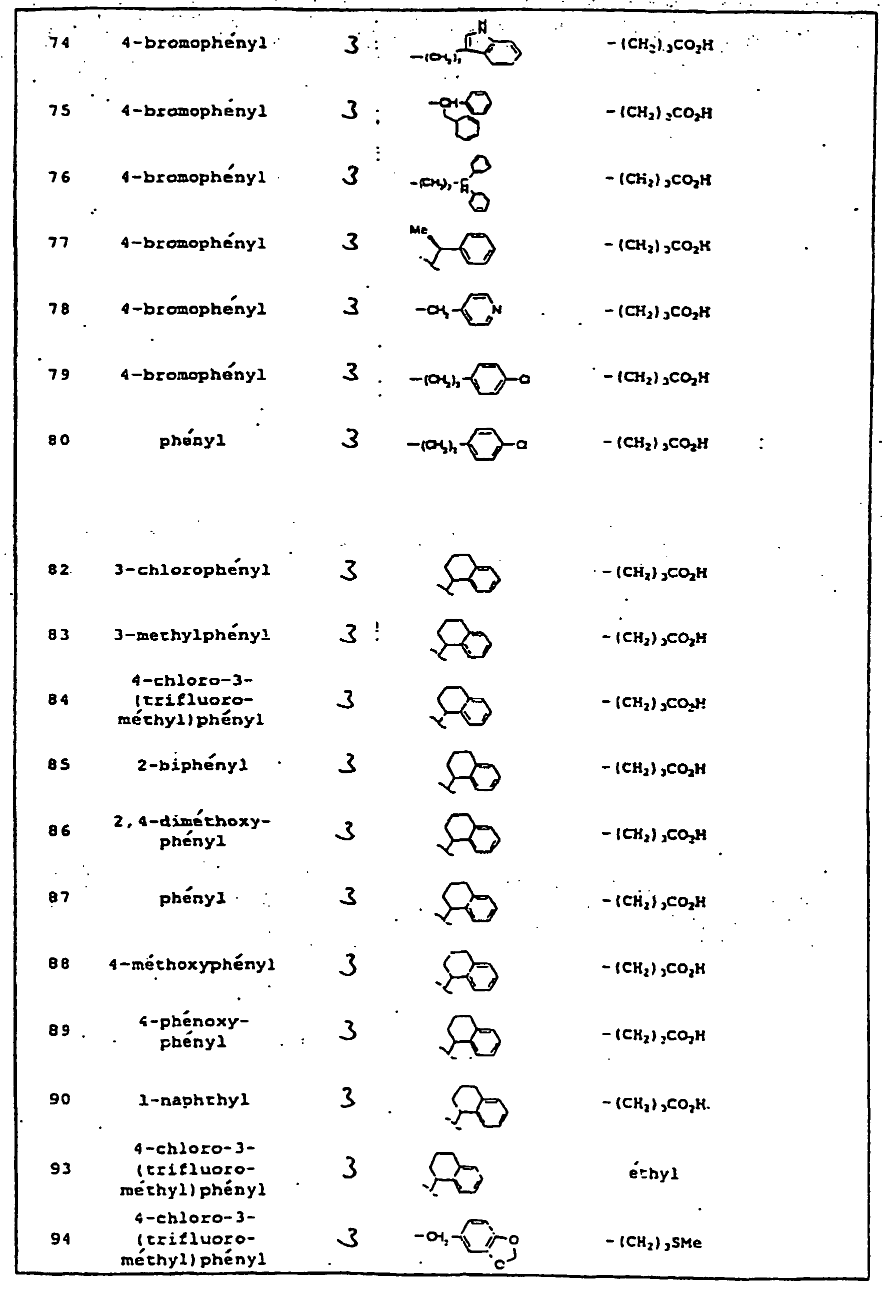

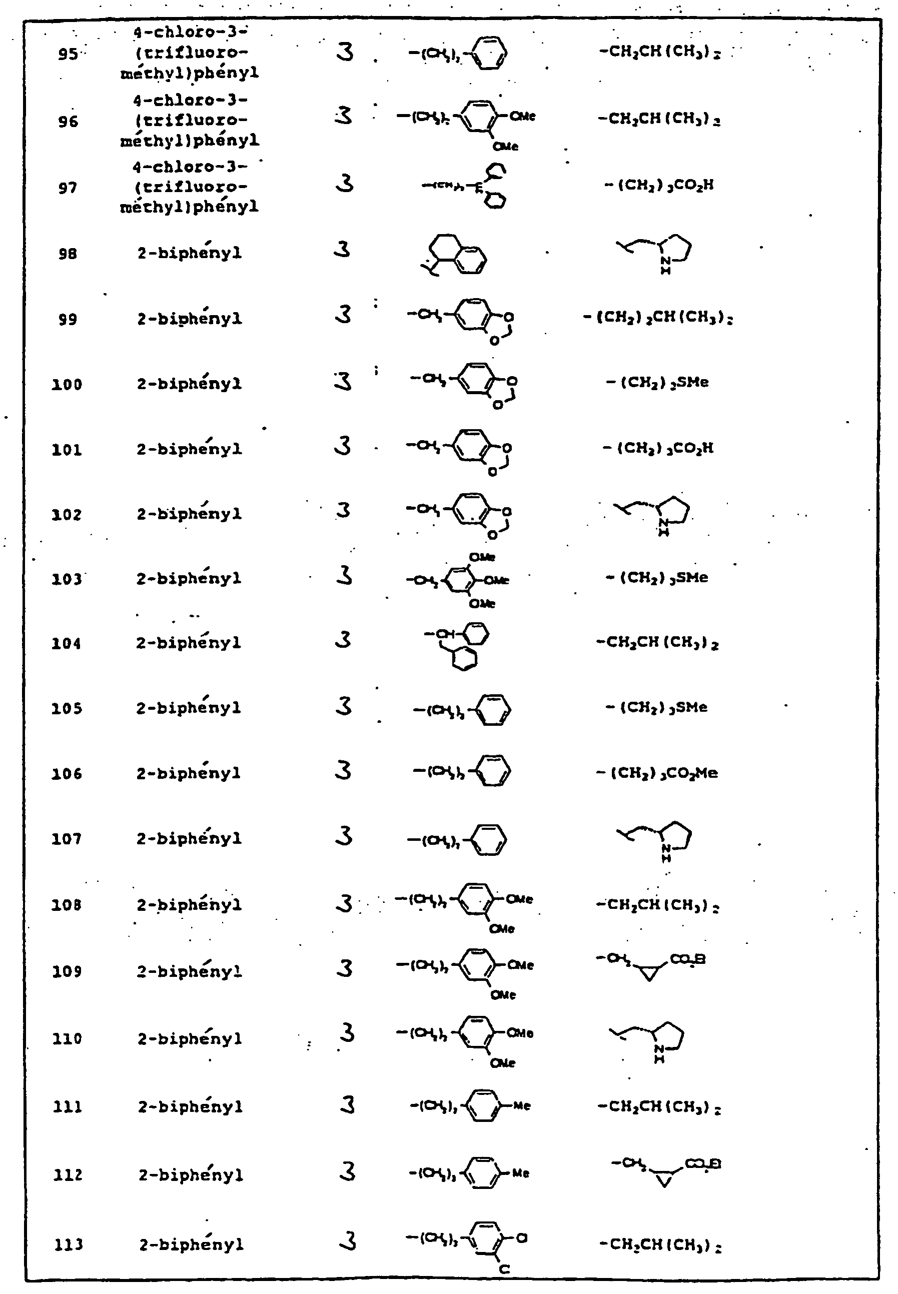

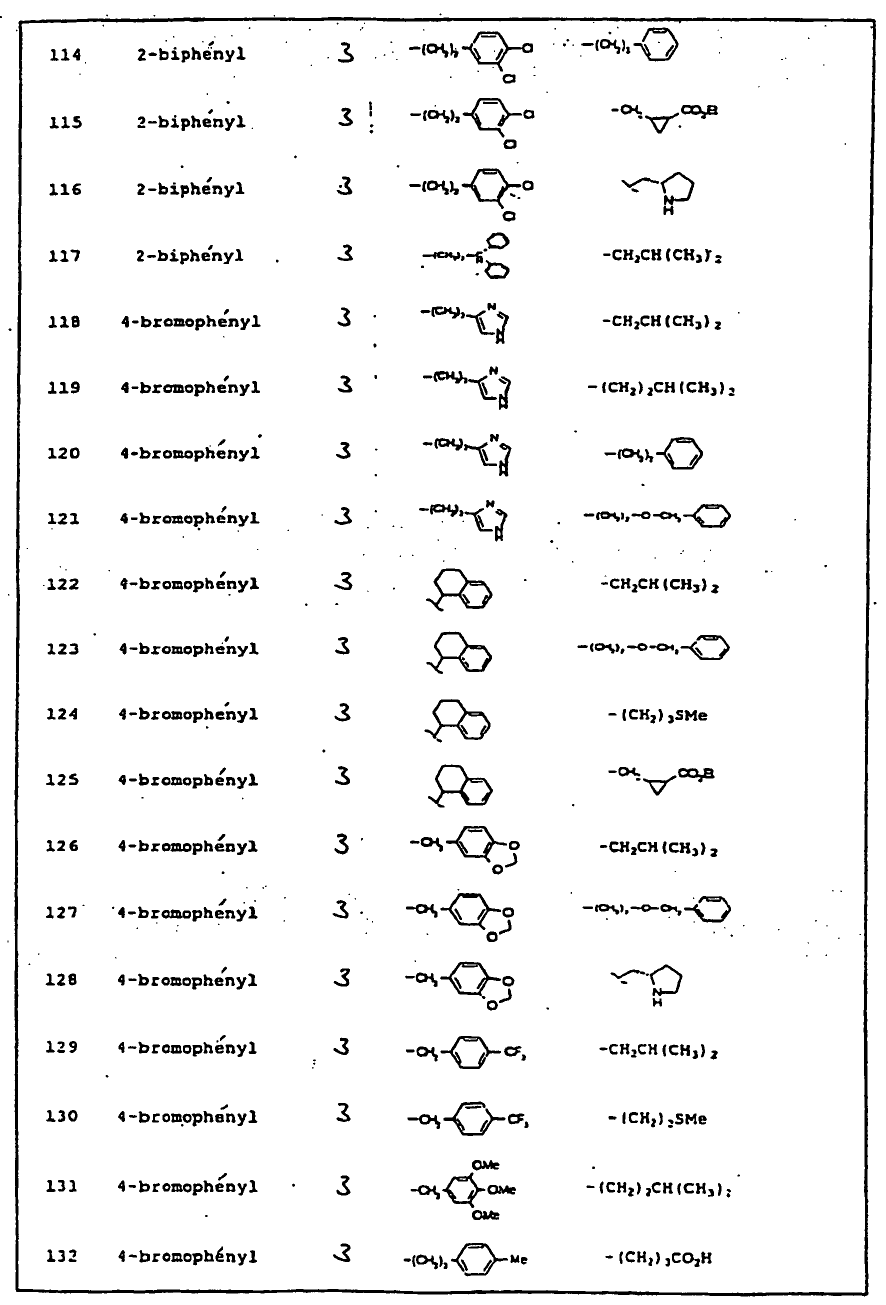

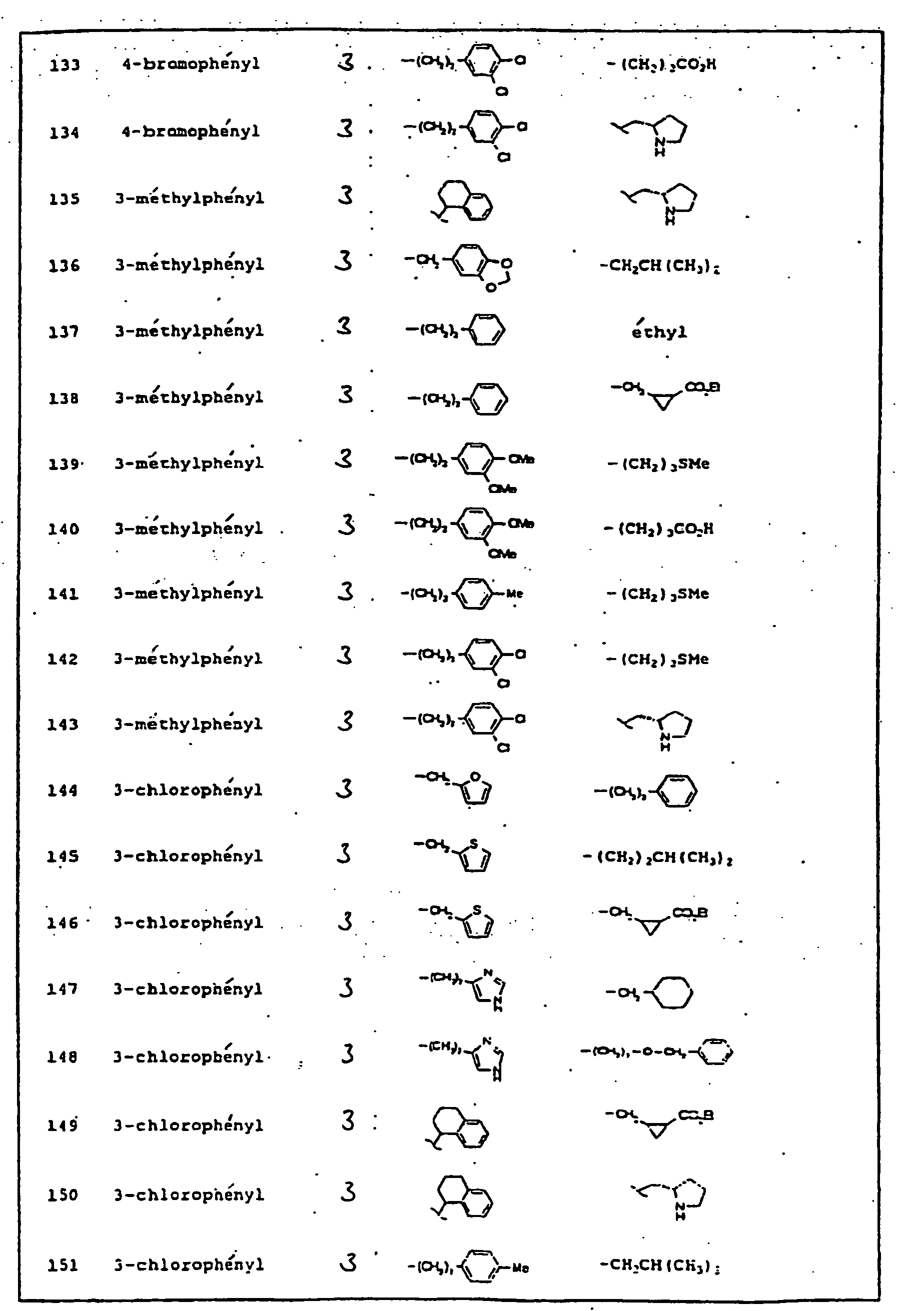

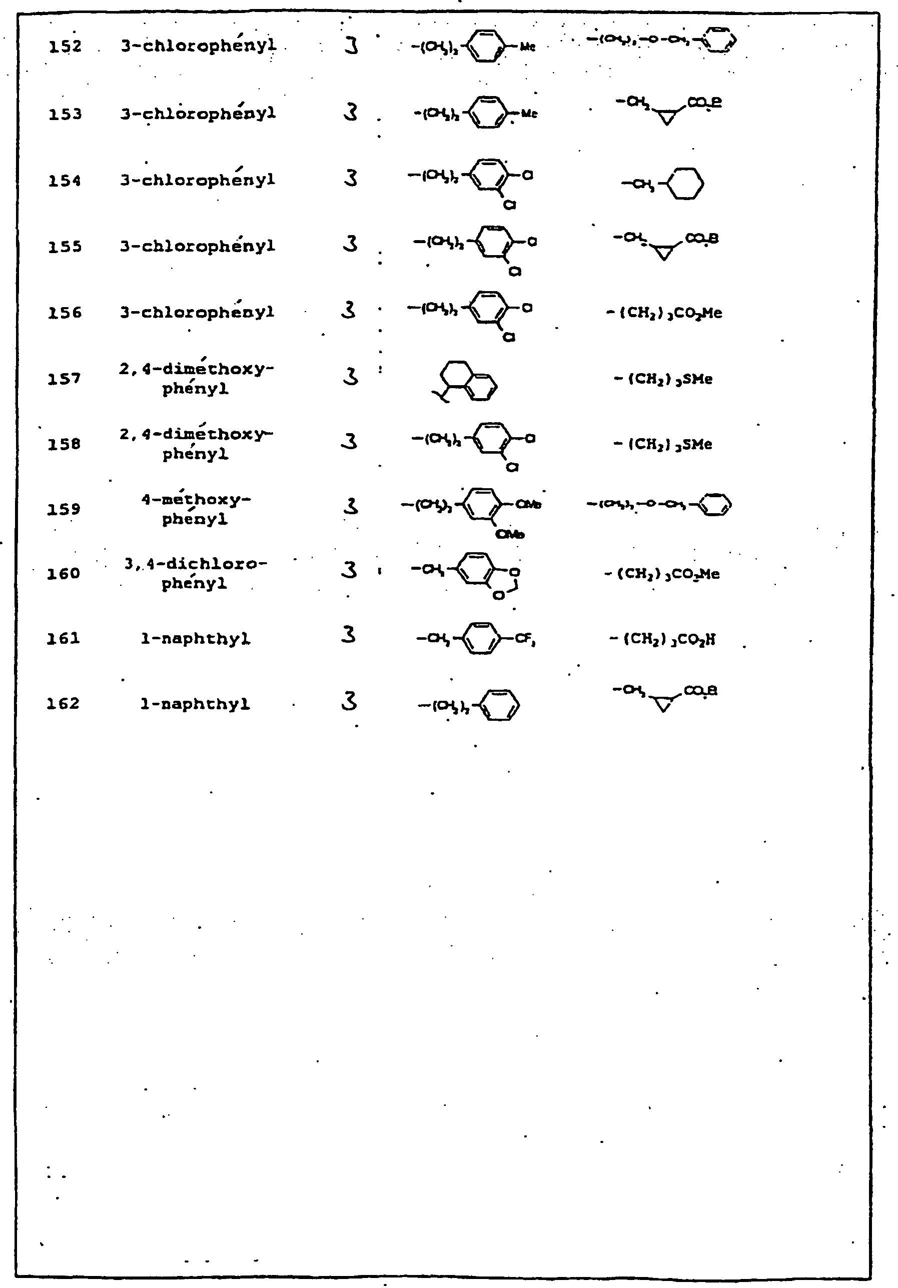

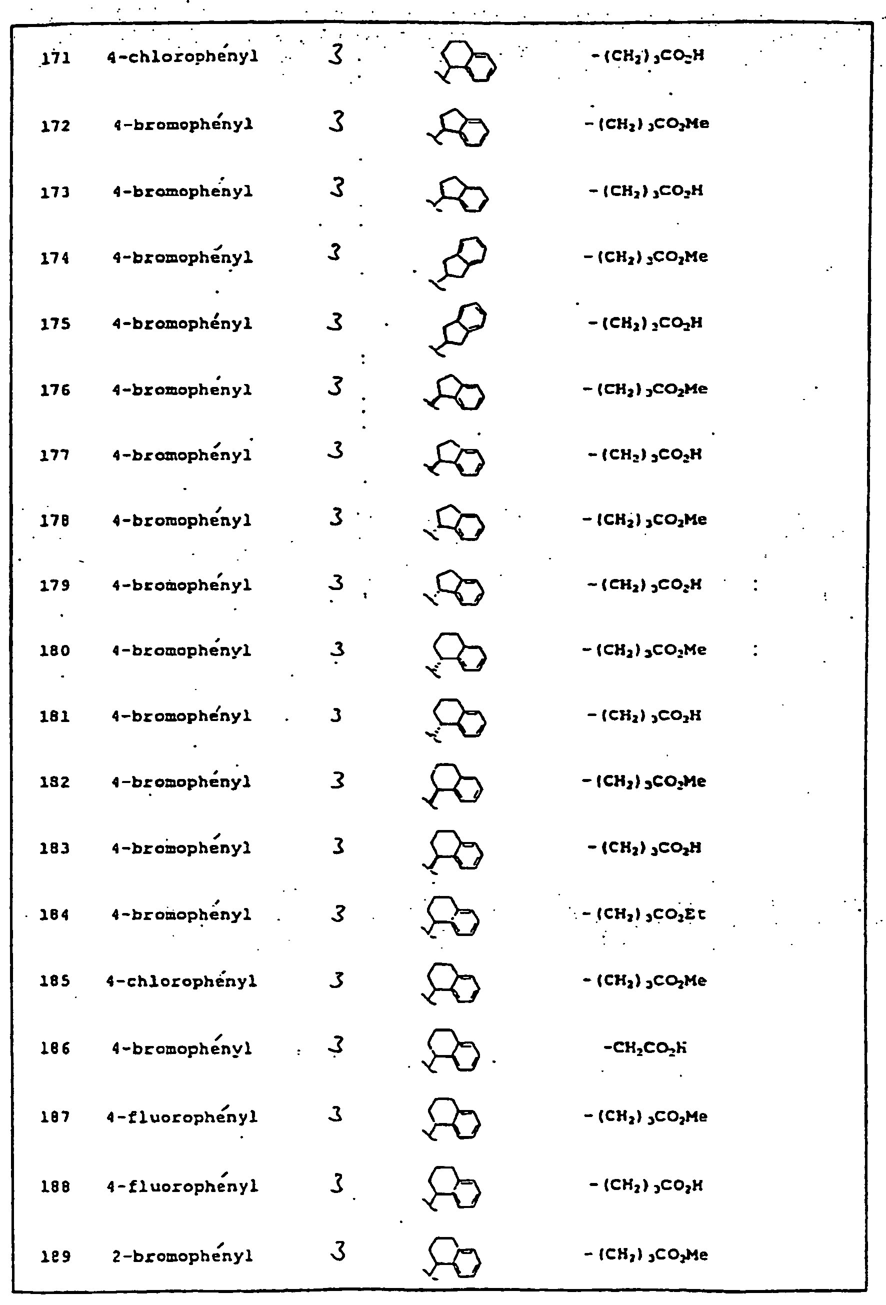

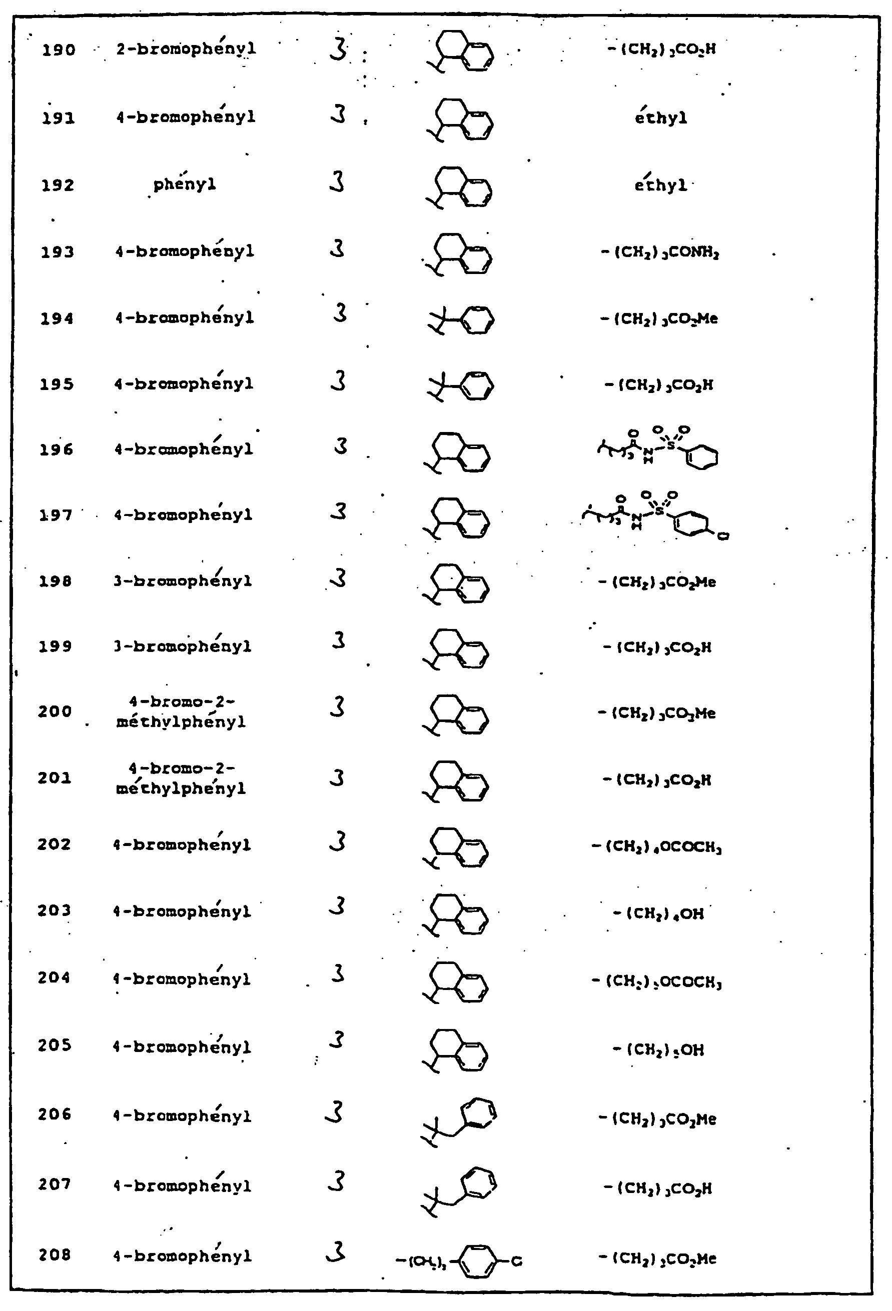

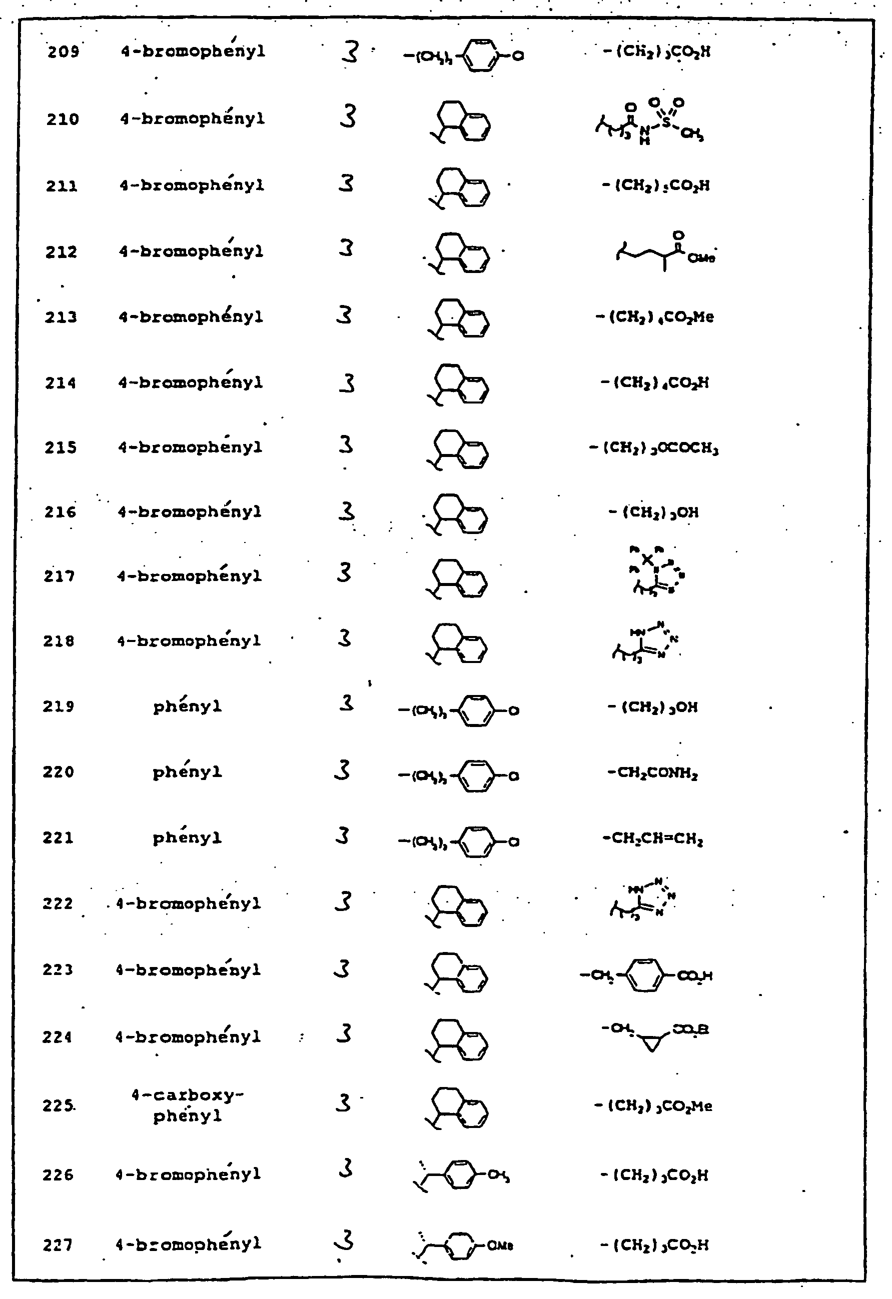

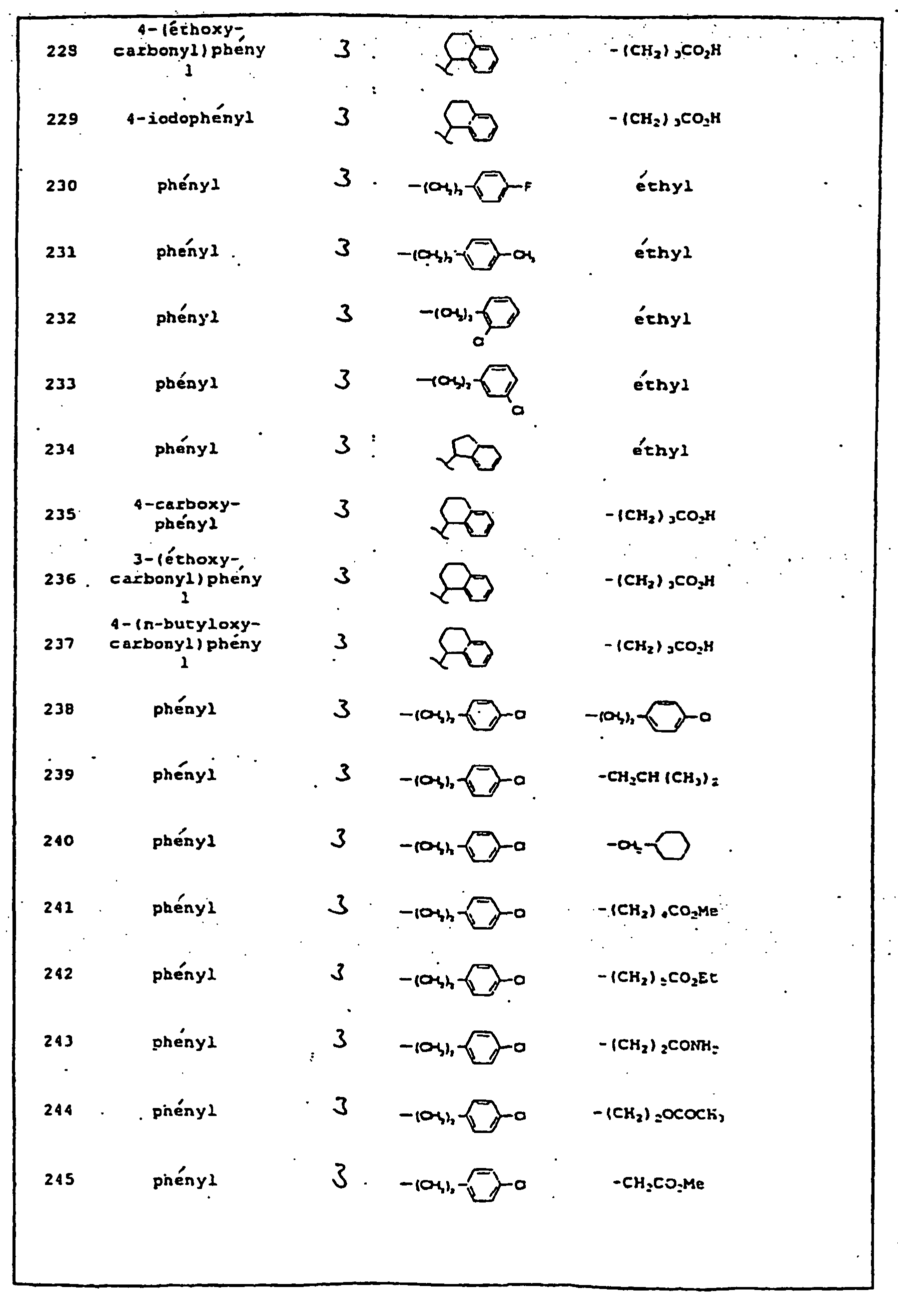

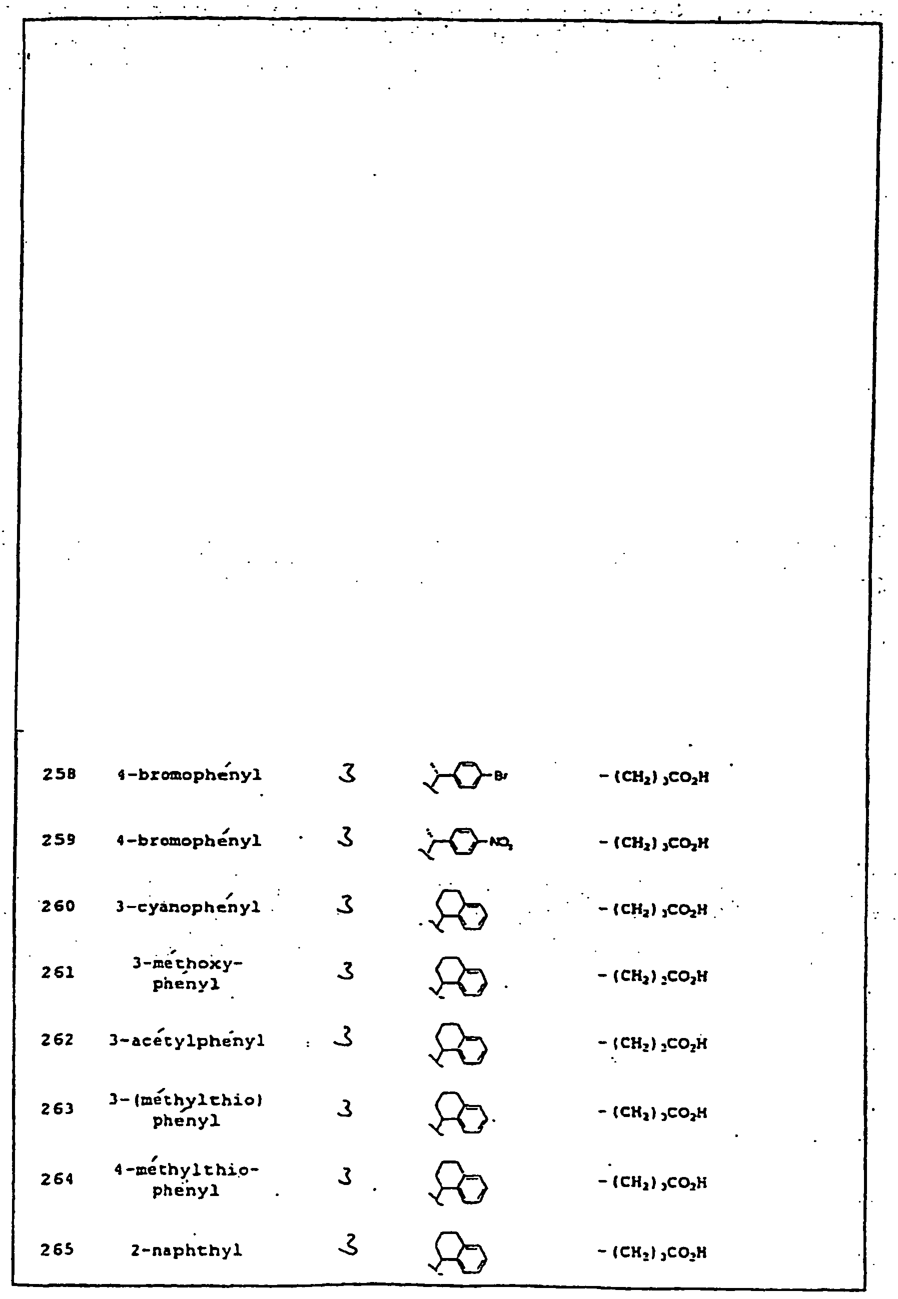

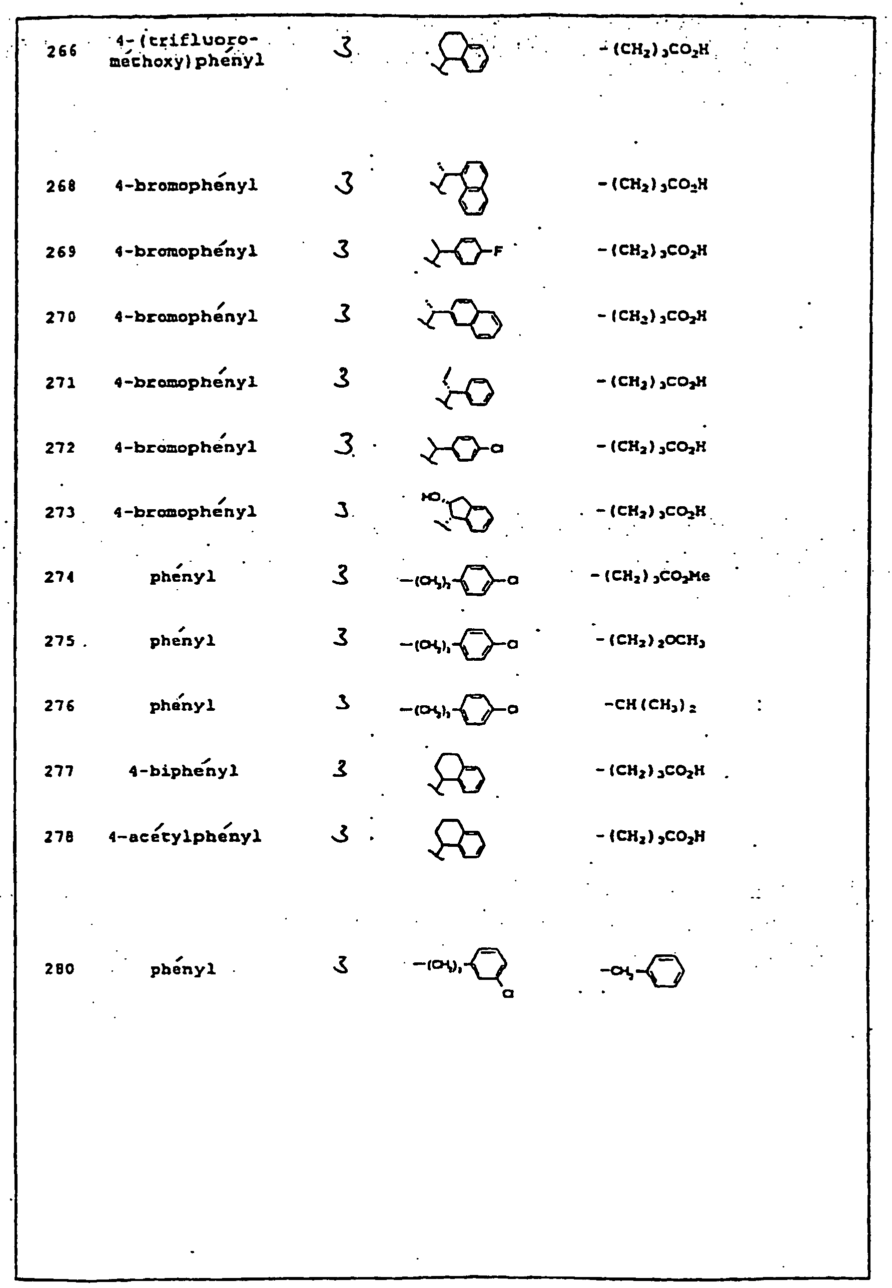

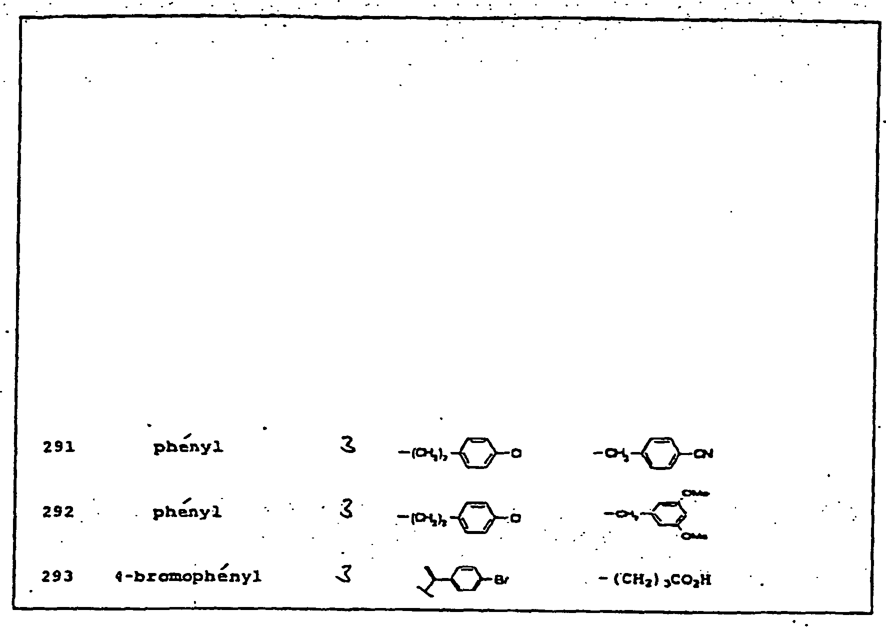

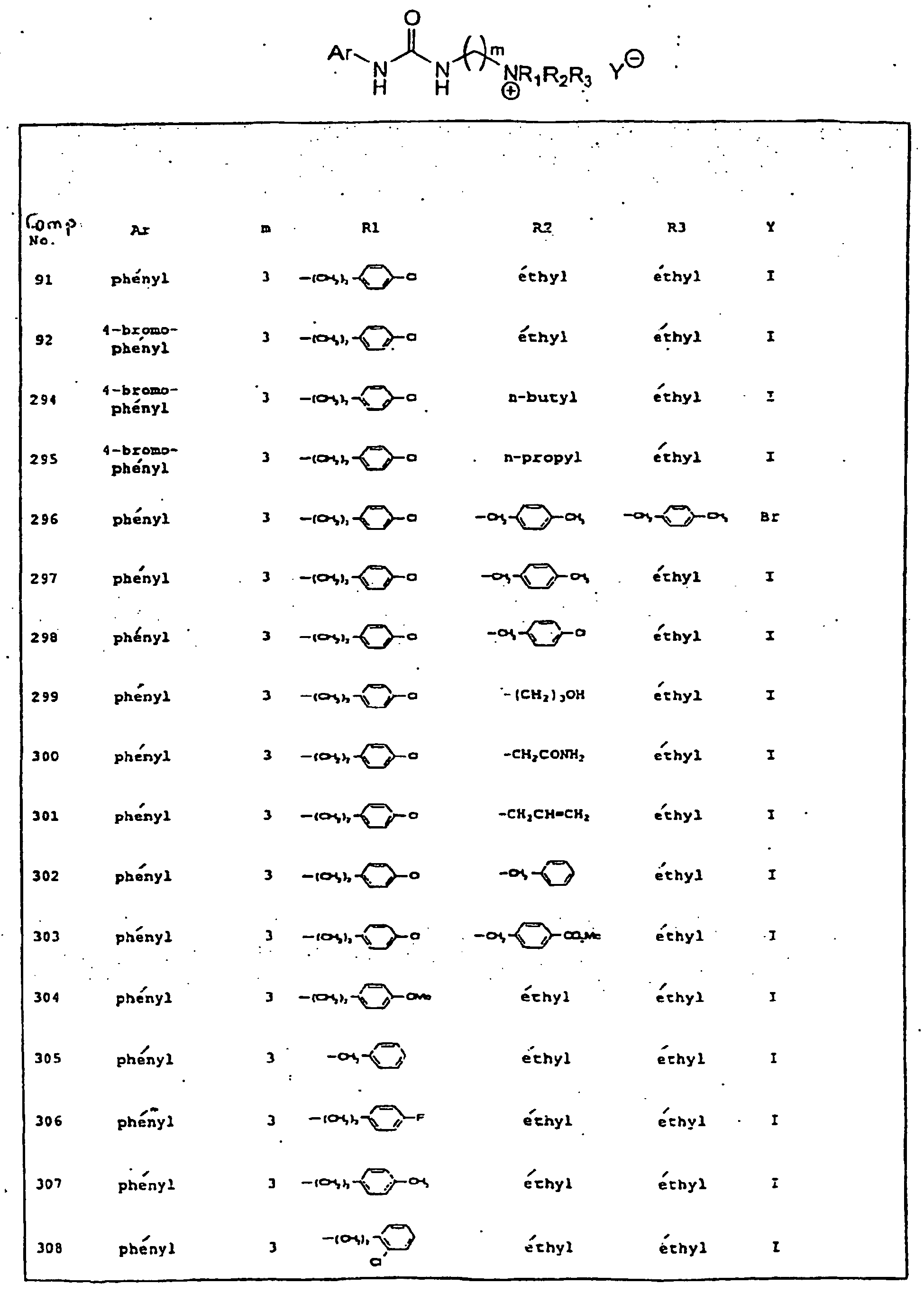

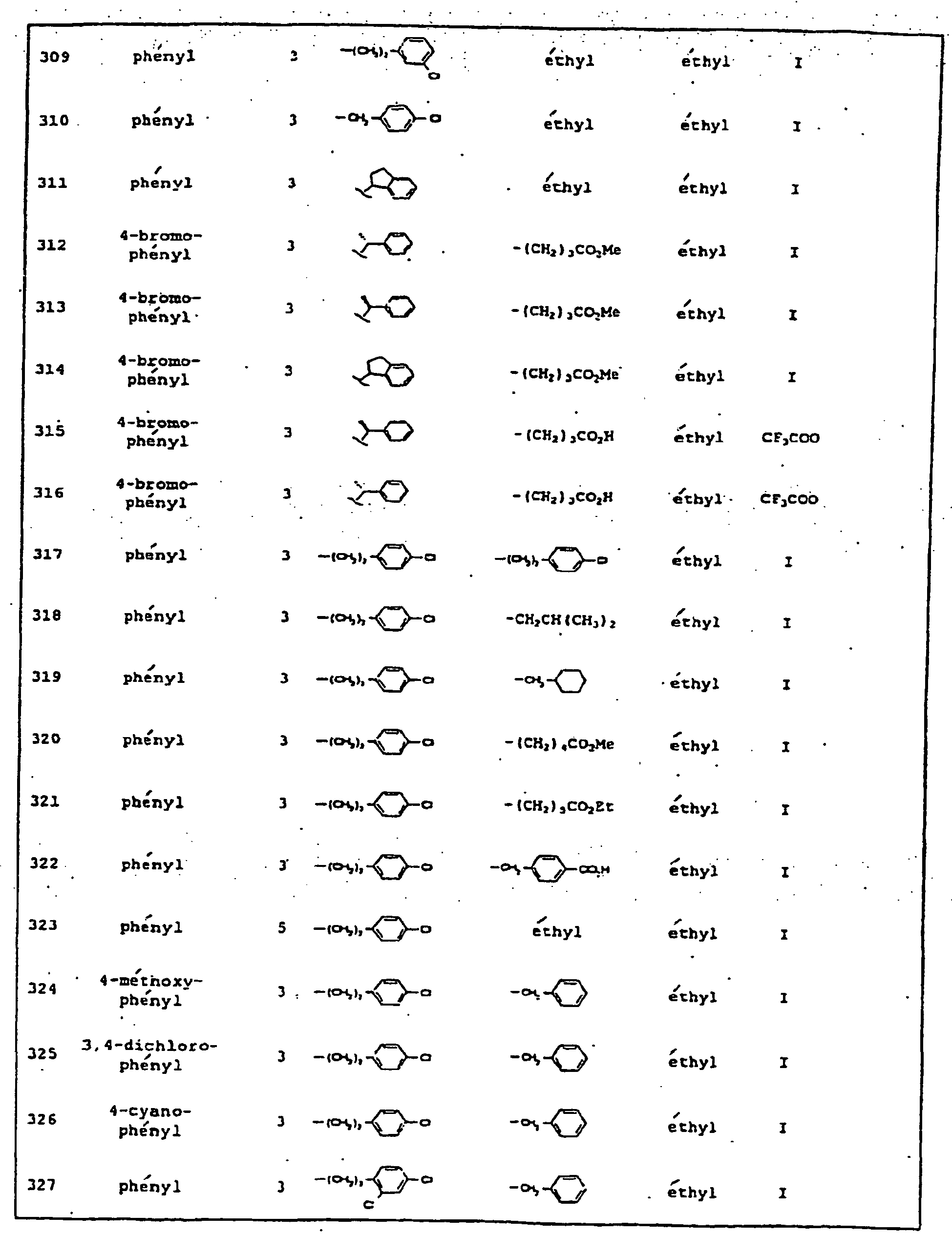

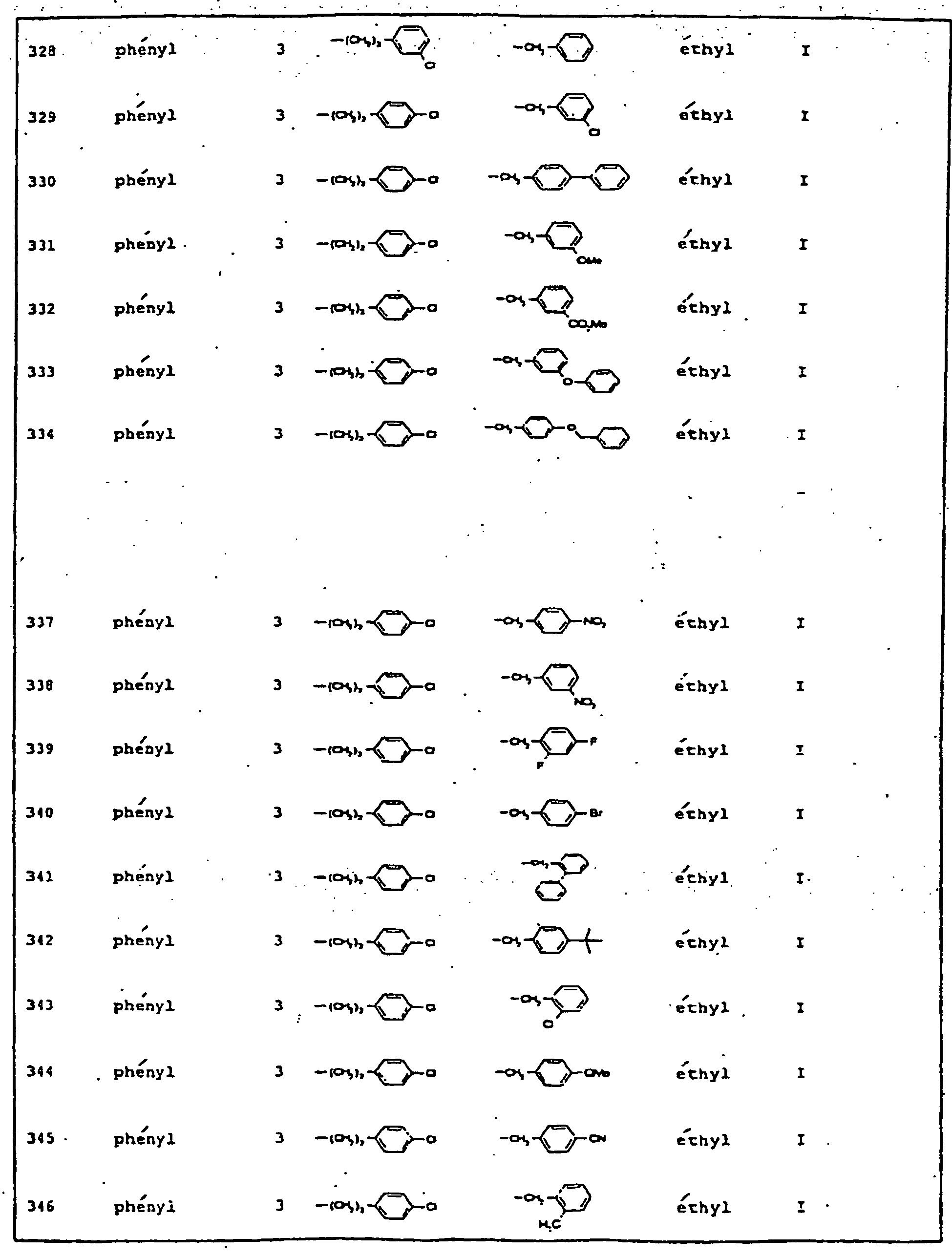

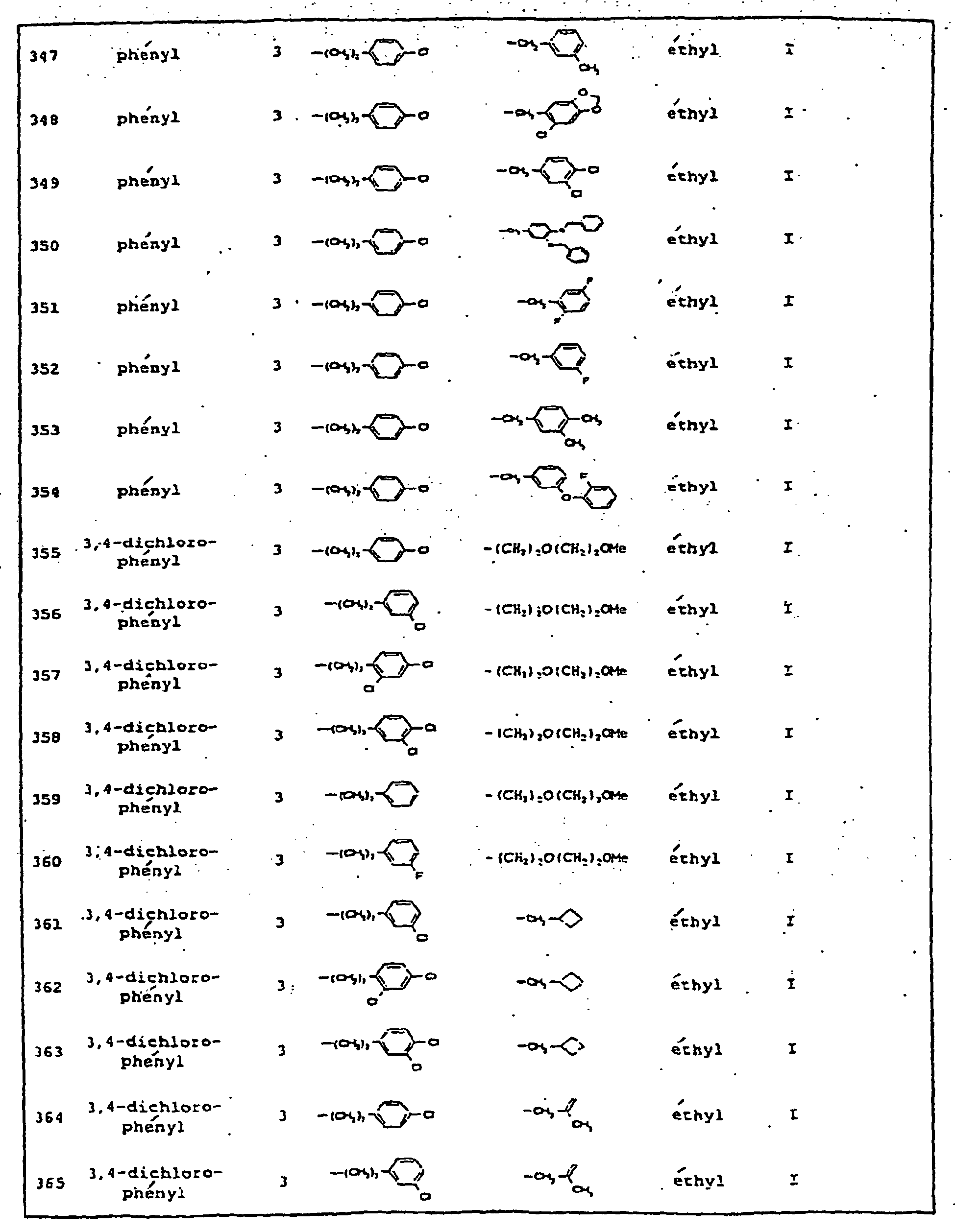

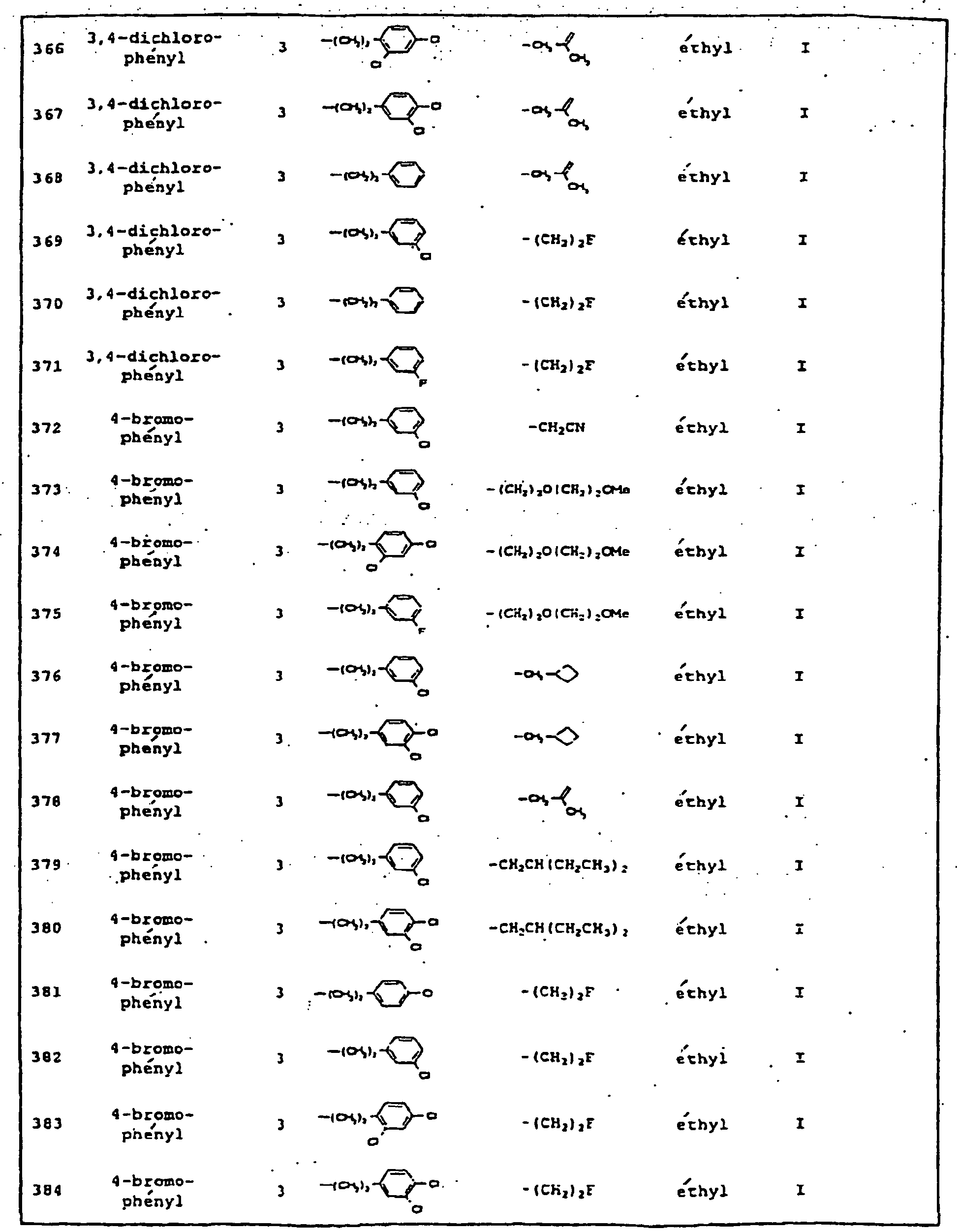

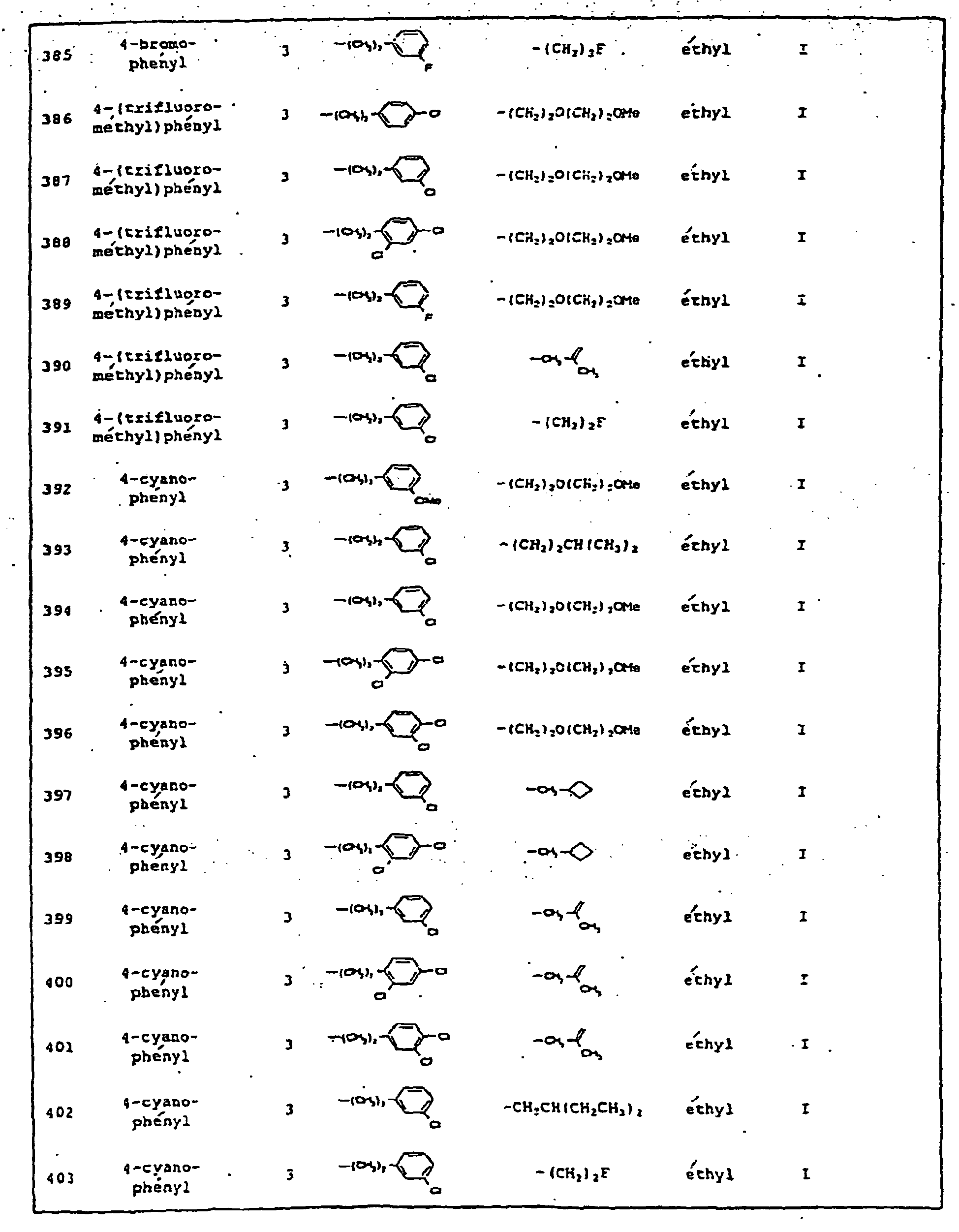

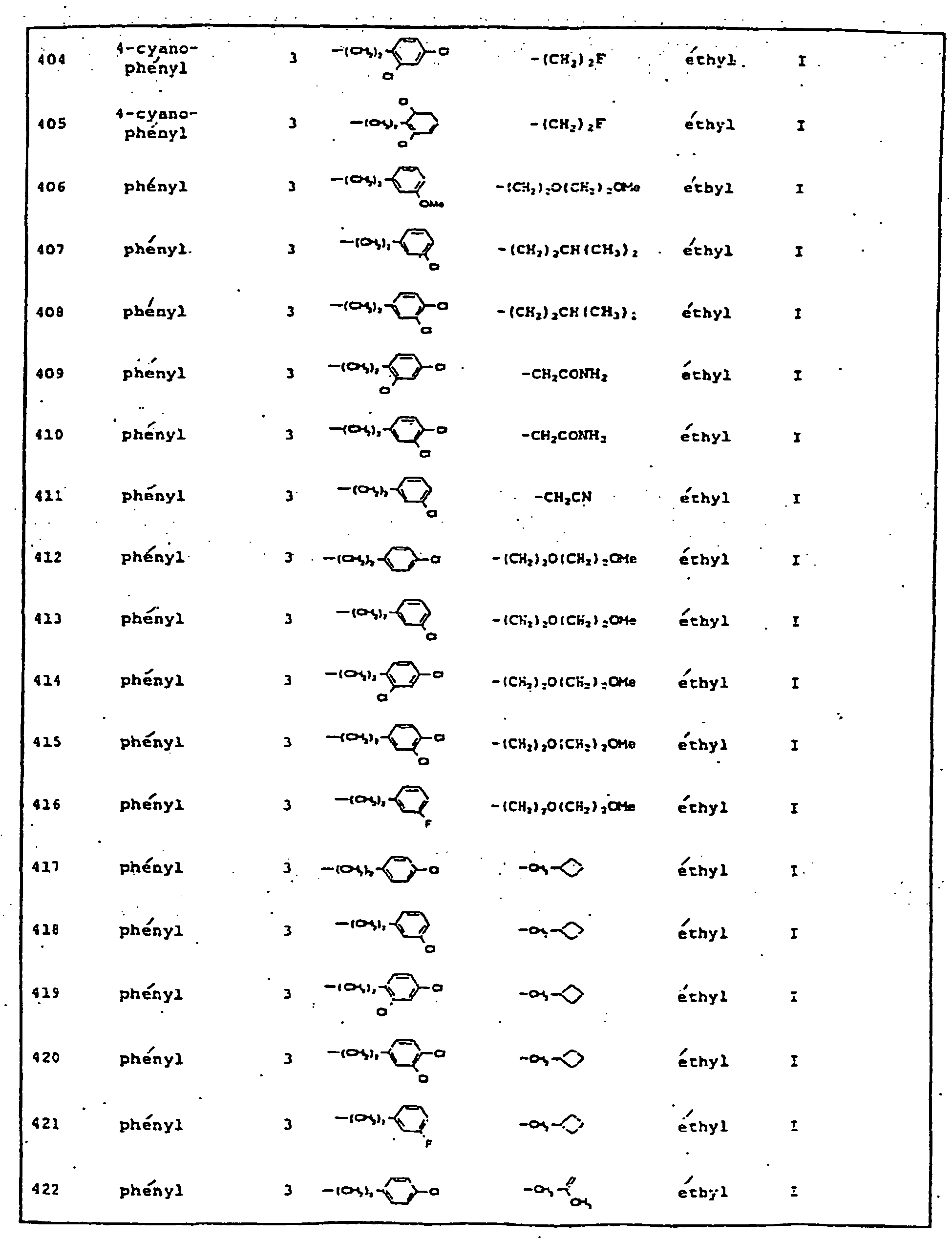

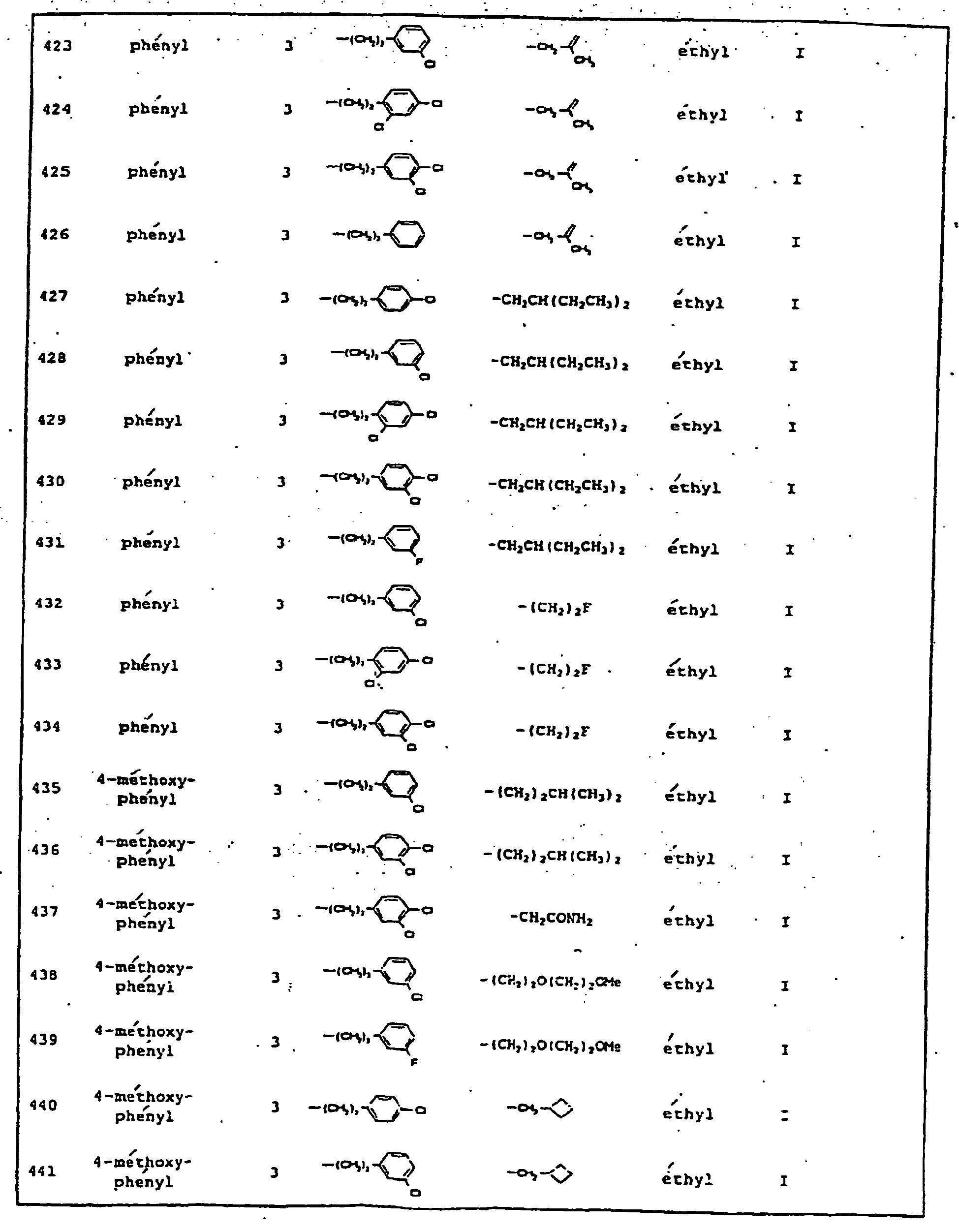

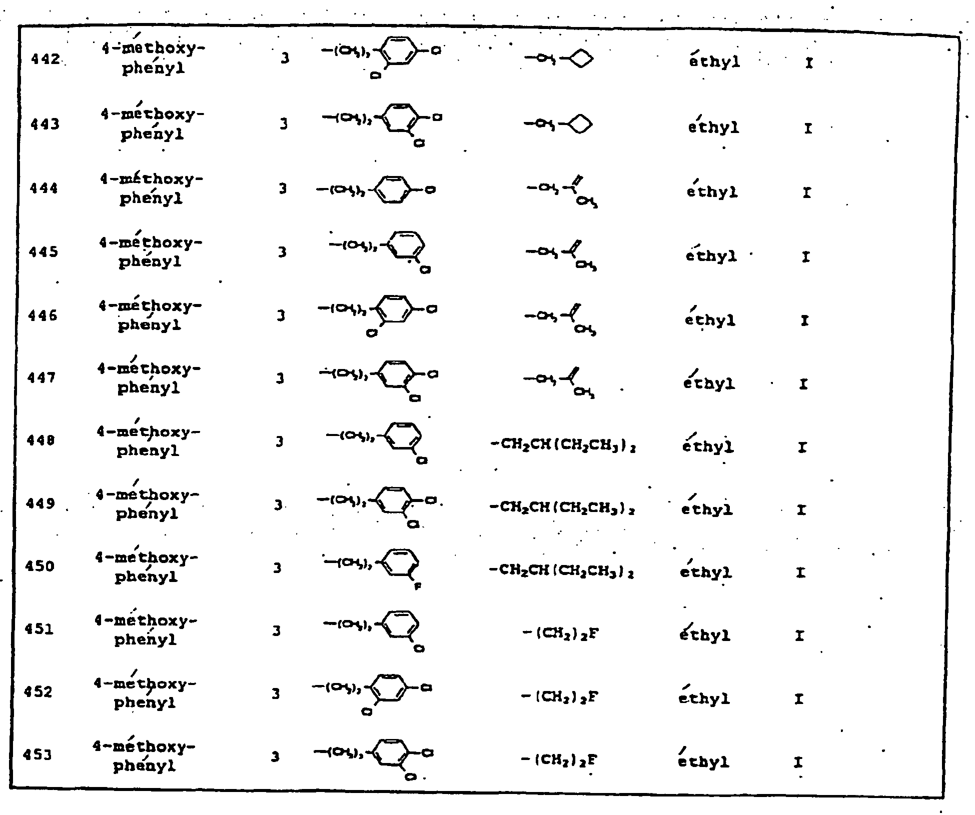

15. Composition pharmaceutique comprenant un composé selon l'une quelconque des revendications 1 à 14.

16. Utilisation d'un composé selon l'une quelconque des revendications 1 à 14 dans la fabrication d'un médicament pour traiter une maladie médiée par CCR-3.

17. Utilisation d'un composé selon la revendication 16, dans laquelle le médicament est pour traiter une maladie médiée par CCR-3 chez un mammifère.

18. Utilisation de la revendication 16 ou 17, dans laquelle la maladie médiée par CCR-3 est une maladie allegique médiée par des éosinophiles.

19. Utilisation de la revendication 18, dans laquelle la maladie est choisie parmi l'asthme, la rhinite, l'eczéma, les maladies des intestins inflammatoires et les infections parasitaires.

20. Utilisation de la revendication 16 ou 17, dans laquelle la maladie médiée par CCR-3 est une maladie médiée par des cellules T ou des cellules dendritiques.

21. Utilisation de la revendication 20, dans laquelle la maladie est choisie parmi les maladies auto-immunes et le VIH.
